# EUROPEAN PATENT APPLICATION

(11) **EP 4 782 437 A1**
(43) Date of publication of application: **29.07.2026**
(21) Application number: 24867553.0
(22) Date of filing: 20.09.2024
(51) Int. Cl.: C07D 239/47, C07D 307/78, C07D 307/87, C07D 307/94, C07D 311/20, C07D 317/50, C07D 319/18, C07D 333/78, A61K 31/506, A61P 35/00, A61P 9/00, A61P 11/00, A61P 3/00, A61P 31/00, A61P 37/00

(54) **HETEROARYL COMPOUND, AND PREPARATION METHOD THEREFOR AND USE THEREOF IN MEDICINE**

(30) Priority: 22.09.2023 CN 202311232055; 24.10.2023 CN 202311380203; 09.11.2023 CN 202311487086; 18.12.2023 CN 202311739484; 29.12.2023 CN 202311854680; 05.02.2024 CN 202410164328
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHANG, Zhigao, Shanghai 200245 (CN); LI, Xin, Shanghai 200245 (CN); CHEN, Lingxiang, Shanghai 200245 (CN); XIE, Zhichao, Shanghai 200245 (CN); DONG, Wenming, Shanghai 200245 (CN); CHEN, Lu, Shanghai 200245 (CN); HE, Feng, Shanghai 200245 (CN)
(74) Representative: Regimbeau
(86) International application number: PCT/CN2024/120037
(87) International publication number: WO 2025/061148

(57) **Abstract**

Provided are a heteroaryl compound, and a preparation method therefor and a use thereof in medicine. Specifically, provided are a heteroaryl compound as represented by general formula (I), a preparation method therefor and a pharmaceutical composition containing the compound, and a use thereof as a therapeutic agent, particularly a use thereof as a Bruton's tyrosine kinase (BTK) inhibitor and a use thereof for treating and/or preventing various diseases associated with excessive BTK activity, including cancer, autoimmune diseases, inflammatory diseases, allergic diseases, allergic reactions, respiratory diseases, cardiovascular diseases, viral infections, transplant rejection reactions, metabolic/endocrine disorders, and neurological disorders.

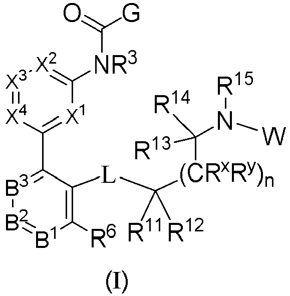

## Description

### Technical Field

The present disclosure belongs to the pharmaceutical field and relates to Bruton's tyrosine kinase (BTK) (including wild-type and mutant BTK) inhibitors for use in the treatment and/or prevention of BTK-related diseases or conditions, such as cancer, autoimmune diseases, inflammatory diseases, allergic diseases, allergic reactions, respiratory diseases, cardiovascular diseases, viral infections, transplant rejection reactions, metabolic/endocrine disorders, and neurological disorders.

### Background Art

Bruton's tyrosine kinase (BTK) is a cytoplasmic non-receptor tyrosine kinase belonging to the TEC family. The protein structure of BTK consists of an N-terminal Pleckstrin homology (PH) domain, a TEC homology (TH) domain, SRC homology (SH) domains SH2 and SH3, and a kinase domain with enzymatic activity (Hendriks RW et al., Nat Rev Cancer. 2014, 14: 219-232). The PH domain recruits BTK to the cell membrane by its interaction with phosphatidylinositol-3,4,5-triphosphate (PIP3), which is generated from phosphatidylinositol-3 kinase (PI3K). Transmembrane proteins (such as the B cell receptor (BCR) complex) promote phosphorylation of BTK at Y551 via SYK or SRC family kinases, leading to BTK kinase activation and subsequent autophosphorylation of Y223 in the SH3 domain (Rawlings DJ et al., Science. 1996, 271: 822-825). BTK is expressed in B lymphocytes and is essential at all stages of B lymphocyte development (Burger JA et al., Nat Rev Cancer. 2018, 18: 148-167). BTK was first identified to be mutated in X-linked agammaglobulinemia (XLA), a primary immunodeficiency in humans. XLA patients are characterised by a reduced number of B cells and an almost complete absence of circulating antibodies (Vetrie D, et al., Nature. 1993, 361: 226-233*;* Tsukada S et al., Cell, 1993, 72: 279-290*).* During the early stage of B-cell development in the bone marrow, BTK promotes the differentiation of pro-B cells into mature B cells by regulating IL-7-induced proliferation of cycling pro-B cells and transformation of quiescent pro-B cells, and the expression of immunoglobulin light chains (Rip, J. et al. Crit. Rev. Immunol. 2018, 38: 17-62). BTK also regulates the negative selection of autoreactive immature B cells in the bone marrow. In the peripheral lymphoid system, BTK regulates B-cell migration to follicles, B-cell maturation, and the activation and differentiation of memory B cells and plasma cells (Torke, S. et al., Exp. Opin. Investig. Drugs 2020, 29, 1143-1150).

Although the function of BTK has been extensively studied in the context of BCR signalling, BTK has also been reported to play important roles in signalling mediated by toll-like receptors (TLRs), Fc receptors (FCRs), and chemokine receptors (Crofford et al., Expert Rev Clin Immunol, 2016, 12: 763-773). The IgE receptor FcεR on the surface of mast cells and basophils binds to IgE. Crosslinking of IgE by the corresponding antigen activates intracellular ITAM-binding domain of FcεR to recruit downstream LYN and SYK kinases, thereby activating BTK and other kinases, mediating the generation of intracellular second messengers IP3 and DAG, inducing cell activation and degranulation, and simultaneously activating downstream transcription factors including NFAT and AP-1 to promote cytokine expression (Saitoh, S. et al. Immunity. 2000, 12, 525-535). Furthermore, BTK plays an important role in IgG-specific Fc receptor (FcγR) signalling in macrophages and microglia, promoting the expression of inflammatory cytokines such as IL-1β, IL-6 and TNF (Di Paolo, J. A. et al., Nat. Chem. Biol. 2011,7, 41-50).

BTK inhibitors have been developed for the treatment of cancers such as chronic lymphocytic leukaemia (CLL). Several covalent BTK inhibitors have been used clinically for B-cell malignancies. Beyond B-cell lymphomas, the function of BTK in the immune system has also prompted the development of BTK inhibitors for autoimmune diseases and allergic diseases, such as rheumatoid arthritis (RA), Sjogren's syndrome, systemic lupus erythematosus, and multiple sclerosis. To date, multiple compounds such as Evobrutinib, Tolebrutinib and Fenebrutinib have undergone clinical trials to evaluate their efficacy in multiple sclerosis, with promising results achieved. However, hepatotoxicity concerns have led to the suspension of several clinical trials (Montalban X et al., 2019, N Engl J Med, 380(25):2406-17). Therefore, an objective of the present disclosure is to provide covalent BTK inhibitors with an improved hepatotoxicity profile and enhanced safety for the treatment of autoimmune diseases.

### Summary of the Invention

The object of the present disclosure is to provide a compound as represented by general formula (I) or a pharmaceutically acceptable salt thereof: 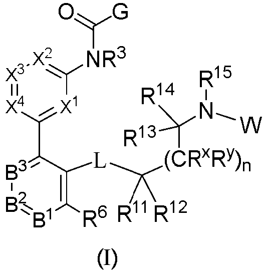 wherein:
G is selected from aryl, heteroaryl, cycloalkyl and heterocyclyl, wherein the aryl, heteroaryl, cycloalkyl and heterocyclyl are each independently and optionally substituted with one or more R^{G};
each R^{G} is the same or different, and is independently selected from R¹, R^{2a}, R² and R^{C}.
each R¹ and each R^{2a} are the same or different, and are each independently selected from halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, -(CR^{a}R^{b})ᵥ heteroaryl and oxo, wherein the alkyl, alkenyl, alkynyl, alkoxy, cycloalkyl, heterocyclyl, aryl and heteroaryl are each independently and optionally substituted with one or more R⁰;
or two R¹ together with the ring atoms to which they are attached form cycloalkyl or heterocyclyl, wherein the cycloalkyl or heterocyclyl is optionally substituted with one or more R⁰;
R² is -OR^{A} or -alkylene-R^{B}, wherein the alkylene is optionally substituted with one or more R⁰;
R^{A} is selected from alkyl, cycloalkyl, heterocyclyl, aryl and heteroaryl, wherein the alkyl, cycloalkyl, heterocyclyl, aryl and heteroaryl are each independently and optionally substituted with one or more R⁰;
R^{B} is selected from cycloalkyl, heterocyclyl, aryl and heteroaryl, wherein the cycloalkyl, heterocyclyl, aryl and heteroaryl are each independently and optionally substituted with one or more R⁰;
R^{C} is selected from cycloalkyl, heterocyclyl, aryl and heteroaryl, wherein the cycloalkyl, heterocyclyl, aryl and heteroaryl are each independently and optionally substituted with one or more R^{d};
each R^{d} is the same or different, and is independently selected from halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, - C(O)R¹⁷, -C(O)OR¹⁷, -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, - (CR^{a}R^{b})ᵥ heteroaryl and oxo, wherein the alkyl, alkenyl, alkynyl, alkoxy, cycloalkyl, heterocyclyl, aryl and heteroaryl are each independently and optionally substituted with one or more R⁰;
R³ is selected from a hydrogen atom, alkyl, alkenyl, alkynyl, haloalkyl, hydroxyalkyl, cycloalkyl, heterocyclyl, cycloalkylalkyl and heterocyclylalkyl;
X¹ is CR^{4a} or N;
X² is CR^{4b} or N;
X³ is CR^{4c} or N;
X⁴ is CR^{4d} or N;
B¹ is CR^{5a} or N;
B² is CR^{5b} or N;
B³ is CR^{5c} or N;
R^{4a}, R^{4b}, R^{4c}, R^{4d}, R^{5a}, R^{5b} and R^{5c} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, - C(O)NR¹⁸R¹⁹, -OC(O)NR¹⁸R¹⁹, -OC(O)R¹⁷, -OC(O)OR¹⁷, -NR²⁰C(O)R¹⁷, - NR²⁰C(O)OR¹⁷, -SR¹⁷, -S(O)R¹⁷, -S(O)₂R¹⁷, -S(O)₂NR¹⁸R¹⁹, -NR²⁰S(O)₂R¹⁷, - (CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or R^{4b} and R^{4c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{4c} and R^{4d} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5a} and R^{5b} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5b} and R^{5c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
R⁶ is selected from a hydrogen atom, -NHR⁷ and -NHC(O)R⁷;
R⁷ is selected from a hydrogen atom, alkyl, haloalkyl, hydroxyalkyl, alkoxyalkyl, hydroxyl, alkoxy, haloalkoxy, cycloalkyl, heterocyclyl, aryl, heteroaryl, cycloalkylalkyl and heterocyclylalkyl;
L is selected from CR⁸R⁹, C (O), O, NR¹⁰, S, S(O) and S(O)₂;
R⁸ and R⁹ are the same or different, and are each independently selected from a hydrogen atom, halogen, alkyl, haloalkyl, hydroxyalkyl, alkoxy, haloalkoxy, alkoxyalkyl, aminoalkyl, hydroxyl, cyano, amino, cycloalkyl and cycloalkylalkyl;
or R⁸ and R⁹ together with the carbon atoms to which they are attached form cycloalkyl or heterocyclyl, wherein the cycloalkyl or heterocyclyl is optionally substituted with one or more R⁰;
R¹⁰ is selected from a hydrogen atom, alkyl, alkenyl, alkynyl, haloalkyl, hydroxyalkyl, alkoxyalkyl, hydroxyl, alkoxy, haloalkoxy, cycloalkyl, heterocyclyl, cycloalkylalkyl and heterocyclylalkyl;
R¹¹, R¹², R¹³, R¹⁴, R^{x} and R^{y} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, hydroxyl, cyano, amino, nitro, -(CR^{a}R^{b})ᵥOR¹⁷, -(CR^{a}R^{b})ᵥNR¹⁸R¹⁹, -(CR^{a}R^{b})ᵥ cycloalkyl, - (CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or any two of R¹¹, R¹², R¹³, R¹⁴, R^{x} and R^{y}, together with the carbon atoms to which they are attached, form cycloalkyl or heterocyclyl, wherein the cycloalkyl or heterocyclyl is optionally substituted with one or more R⁰;
or R¹¹ and R¹² together form =O;
or R¹³ and R¹⁴ together form =O;
or R^{x} and R^{y} on the same carbon atom together form =O;
R¹⁵ is selected from a hydrogen atom, alkyl, alkoxy, alkenyl, alkynyl, haloalkyl, hydroxyalkyl, cycloalkyl, heterocyclyl, aryl, heteroaryl, cycloalkylalkyl, heterocyclylalkyl, arylalkyl and heteroarylalkyl, wherein the alkyl, alkoxy, alkenyl, alkynyl, cycloalkyl, heterocyclyl, aryl, heteroaryl, cycloalkylalkyl, heterocyclylalkyl, arylalkyl and heteroarylalkyl are each independently and optionally substituted with one or more R⁰;
or any one of R¹¹, R¹², R¹³, R¹⁴, R^{x} and R^{y} together with R¹⁵ and the atoms to which they are respectively attached forms nitrogen-containing heterocyclyl, wherein the nitrogen-containing heterocyclyl is optionally substituted with one or more R⁰;
W is selected from -C(O)R¹⁶, -S(O)₂R¹⁶ and -CN;
R¹⁶ is selected from alkenyl, alkynyl and alkenyl oxide, wherein the alkenyl, alkynyl and alkenyl oxide are each independently and optionally substituted with one or more substituents selected from oxo, halogen, alkyl, haloalkyl, hydroxyalkyl, alkoxy, haloalkoxy, alkoxyalkyl, aminoalkyl, hydroxyl, cyano, amino, nitro, - (CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R⁰ at each occurrence is the same or different, and is independently selected from oxo, halogen, alkenyl, alkynyl, cyano, nitro, hydroxyl, amino, alkyl, haloalkyl, hydroxyalkyl, alkoxy, haloalkoxy, alkoxyalkyl, aminoalkyl, -(CR^{a}R^{b})ᵥOR¹⁷, - (CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R¹⁷, R¹⁸, R¹⁹ and R²⁰ at each occurrence are the same or different, and are each independently selected from a hydrogen atom, alkyl, alkenyl, alkynyl, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, cycloalkyl, heterocyclyl, aryl and heteroaryl are each independently and optionally substituted with one or more R⁰¹;
or R¹⁸ and R¹⁹ together with the nitrogen atom to which they are attached form nitrogen-containing heterocyclyl, wherein the nitrogen-containing heterocyclyl is optionally substituted with one or more R⁰¹;
R⁰¹ at each occurrence is the same or different, and is independently selected from oxo, halogen, hydroxyl, alkenyl, alkynyl, cyano, nitro, amino, -NH alkyl, -N (alkyl)₂, -C(O)O alkyl, -C(O)OH, -C(O)NH₂, -C(O)halogen, -C(O)alkyl, alkyl, haloalkyl, hydroxyalkyl, alkoxy, haloalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
R^{a} and R^{b} at each occurrence are the same or different, and are each independently selected from a hydrogen atom, halogen, alkyl, haloalkyl, hydroxyalkyl, alkoxy, haloalkoxy, hydroxyl, cyano, amino, cycloalkyl and cycloalkylalkyl;
n is 0, 1, 2 or 3;
v is 0, 1, 2 or 3.

The object of the present disclosure is to provide a compound as represented by general formula (I) or a pharmaceutically acceptable salt thereof: 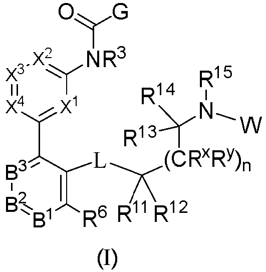 wherein:
G is selected from 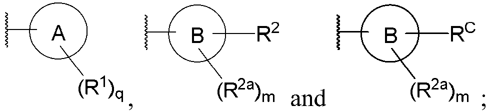
ring A is selected from polycyclic aryl, heteroaryl, cycloalkyl and heterocyclyl;
ring B is monocyclic aryl or monocyclic heteroaryl;
each R¹ and each R^{2a} are the same or different, and are each independently selected from a deuterium atom, halogen, alkyl, alkenyl, alkynyl, haloalkyl, deuterioalkyl, hydroxyalkyl, alkoxy, haloalkoxy, alkoxyalkyl, deuterioalkoxy, aminoalkyl, cyano, amino, hydroxyl, nitro, -(CR^{a}R^{b})ᵥOR¹⁷, -(CR^{a}R^{b})ᵥNR¹⁸R¹⁹, - C(O)R¹⁷, -C(O)OR¹⁷, -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, - (CR^{a}R^{b})ᵥ heteroaryl, oxo, =S and =CR^{p}R^{q}, wherein the alkyl, alkenyl, alkynyl, alkoxy, cycloalkyl, heterocyclyl, aryl and heteroaryl are each independently and optionally substituted with one or more R⁰;
or two R¹ together with the ring atoms to which they are attached form cycloalkyl or heterocyclyl, wherein the cycloalkyl or heterocyclyl is optionally substituted with one or more R⁰;
R² is -OR^{A} or -alkylene-R^{B}, wherein the alkylene is optionally substituted with one or more R⁰;
R^{A} is selected from alkyl, cycloalkyl, heterocyclyl, aryl and heteroaryl, wherein the alkyl, cycloalkyl, heterocyclyl, aryl and heteroaryl are each independently and optionally substituted with one or more R⁰;
R^{B} is selected from cycloalkyl, heterocyclyl, aryl and heteroaryl, wherein the cycloalkyl, heterocyclyl, aryl and heteroaryl are each independently and optionally substituted with one or more R⁰;
R^{C} is selected from cycloalkyl, heterocyclyl, aryl and heteroaryl, wherein the cycloalkyl, heterocyclyl, aryl and heteroaryl are each independently substituted with one or more R^{d};
each R^{d} is the same or different, and is independently selected from a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, - C(O)NR¹⁸R¹⁹, -OC(O)NR¹⁸R¹⁹, -OC(O)R¹⁷, -OC(O)OR¹⁷, -NR²⁰C(O)R¹⁷, - NR²⁰C(O)OR¹⁷, -SR¹⁷, -S(O)R¹⁷, -S(O)₂R¹⁷, -S(O)₂NR¹⁸R¹⁹, -NR²⁰S(O)₂R¹⁷, - (CR^{a}R^{b})ᵥ cycloalkyl, -(CR^{a}R^{b})ᵥ heterocyclyl, -(CR^{a}R^{b})ᵥ aryl, -(CR^{a}R^{b})ᵥ heteroaryl, oxo, =S and =CR^{p}R^{q}, wherein the alkyl, alkenyl, alkynyl, alkoxy, cycloalkyl, heterocyclyl, aryl and heteroaryl are each independently and optionally substituted with one or more R⁰;
R³ is selected from a hydrogen atom, alkyl, deuterioalkyl, alkenyl, alkynyl, haloalkyl, hydroxyalkyl, cycloalkyl, heterocyclyl, cycloalkylalkyl and heterocyclylalkyl;
X¹ is CR^{4a} or N;
X² is CR^{4b} or N;
X³ is CR^{4c} or N;
X⁴ is CR^{4d} or N;
B¹ is CR^{5a} or N;
B² is CR^{5b} or N;
B³ is CR^{5c} or N;
R^{4a}, R^{4b}, R^{4c} , R^{4d}, R^{5a}, R^{5b} and R^{5c} are the same or different, and are each independently selected from a hydrogen atom, a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, cyano, amino, hydroxyl, nitro, - (CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, - OC(O)NR¹⁸R¹⁹, -OC(O)R¹⁷, -OC(O)OR¹⁷, -NR²⁰C(O)R¹⁷, -NR²⁰C(O)OR¹⁷, -SR¹⁷, - S(O)R¹⁷, -S(O)₂R¹⁷, -S(O)₂NR¹⁸R¹⁹, -NR²⁰S(O)₂R¹⁷, -(CR^{a}R^{b})ᵥ cycloalkyl, - (CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or R^{4b} and R^{4c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{4c} and R^{4d} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5a} and R^{5b} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5b} and R^{5c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
R⁶ is selected from a hydrogen atom, -NHR⁷ and -NHC(O)R⁷;
R⁷ is selected from a hydrogen atom, alkyl, haloalkyl, deuterioalkyl, hydroxyalkyl, alkoxyalkyl, hydroxyl, alkoxy, haloalkoxy, deuterioalkoxy, cycloalkyl, heterocyclyl, aryl, heteroaryl, cycloalkylalkyl and heterocyclylalkyl;
L is selected from CR⁸R⁹, C (O), O, NR¹⁰, S, S(O) and S(O)₂;
R⁸ and R⁹ are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, alkoxyalkyl, aminoalkyl, hydroxyl, cyano, amino, cycloalkyl and cycloalkylalkyl;
or R⁸ and R⁹ together with the carbon atoms to which they are attached form cycloalkyl or heterocyclyl, wherein the cycloalkyl or heterocyclyl is optionally substituted with one or more R⁰;
R¹⁰ is selected from a hydrogen atom, alkyl, alkenyl, alkynyl, haloalkyl, deuterioalkyl, hydroxyalkyl, alkoxyalkyl, hydroxyl, alkoxy, haloalkoxy, deuterioalkoxy, cycloalkyl, heterocyclyl, cycloalkylalkyl and heterocyclylalkyl;
R¹¹, R¹², R¹³, R¹⁴, R^{x} and R^{y} are the same or different, and are each independently selected from a hydrogen atom, a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, hydroxyl, cyano, amino, nitro, - (CR^{a}R^{b})ᵥOR¹⁷, -(CR^{a}R^{b})ᵥNR¹⁸R¹⁹, -(CR^{a}R^{b})ᵥ cycloalkyl, -(CR^{a}R^{b})ᵥ heterocyclyl, - (CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or any two of R¹¹, R¹², R¹³, R¹⁴, R^{x} and R^{y}, together with the carbon atoms to which they are attached, form cycloalkyl or heterocyclyl, wherein the cycloalkyl or heterocyclyl is optionally substituted with one or more R⁰;
or R¹¹ and R¹² together form =O;
or R¹³ and R¹⁴ together form =O;
or R^{x} and R^{y} on the same carbon atom together form =O;
R¹⁵ is selected from a hydrogen atom, alkyl, alkoxy, alkenyl, alkynyl, haloalkyl, deuterioalkyl, hydroxyalkyl, cycloalkyl, heterocyclyl, aryl, heteroaryl, cycloalkylalkyl, heterocyclylalkyl, arylalkyl and heteroarylalkyl, wherein the alkyl, alkoxy, alkenyl, alkynyl, cycloalkyl, heterocyclyl, aryl, heteroaryl, cycloalkylalkyl, heterocyclylalkyl, arylalkyl and heteroarylalkyl are each independently and optionally substituted with one or more R⁰;
or any one of R¹¹, R¹², R¹³, R¹⁴, R^{x} and R^{y} together with R¹⁵ and the atoms to which they are respectively attached forms nitrogen-containing heterocyclyl, wherein the nitrogen-containing heterocyclyl is optionally substituted with one or more R⁰;
W is selected from -C(O)R¹⁶, -S(O)₂R¹⁶ and -CN;
R¹⁶ is selected from alkenyl, alkynyl and alkenyl oxide, wherein the alkenyl, alkynyl and alkenyl oxide are each independently and optionally substituted with one or more substituents selected from oxo, =S, =CR^{p}R^{q}, a deuterium atom, halogen, alkyl, haloalkyl, deuterioalkyl, hydroxyalkyl, alkoxy, haloalkoxy, deuterioalkoxy, alkoxyalkyl, aminoalkyl, hydroxyl, cyano, amino, nitro, -(CR^{a}R^{b})ᵥOR¹⁷, - (CR^{a}R^{b})ᵥNR¹⁸R¹⁹, -C(O)R¹⁷, -C(O)OR¹⁷, -C(O)NR¹⁸R¹⁹, -OC(O)R¹⁷, -OC(O)OR¹⁷, -NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and - (CR^{a}R^{b})ᵥ heteroaryl;
R⁰ at each occurrence is the same or different, and is independently selected from oxo, =S, =CR^{p}R^{q}, a deuterium atom, halogen, alkenyl, alkynyl, cyano, nitro, hydroxyl, amino, alkyl, haloalkyl, deuterioalkyl, hydroxyalkyl, alkoxy, haloalkoxy, deuterioalkoxy, alkoxyalkyl, aminoalkyl, -(CR^{a}R^{b})ᵥOR¹⁷, -(CR^{a}R^{b})ᵥNR¹⁸R¹⁹, - C(O)R¹⁷, -C(O)OR¹⁷, -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and - (CR^{a}R^{b})ᵥ heteroaryl;
R¹⁷, R¹⁸, R¹⁹ and R²⁰ at each occurrence are the same or different, and are each independently selected from a hydrogen atom, alkyl, alkenyl, alkynyl, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, cycloalkyl, heterocyclyl, aryl and heteroaryl are each independently and optionally substituted with one or more R⁰¹;
or R¹⁸ and R¹⁹ together with the nitrogen atom to which they are attached form nitrogen-containing heterocyclyl, wherein the nitrogen-containing heterocyclyl is optionally substituted with one or more R⁰¹;
R⁰¹ at each occurrence is the same or different, and is independently selected from oxo, =S, =CR^{p}R^{q}, a deuterium atom, halogen, hydroxyl, alkenyl, alkynyl, cyano, nitro, amino, -NH alkyl, -N (alkyl)₂, -C(O)O alkyl, -C(O)OH, -C(O)NH₂, - C(O)halogen, -C(O)alkyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
R^{a} and R^{b} at each occurrence are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, hydroxyl, cyano, amino, cycloalkyl and cycloalkylalkyl;
R^{p} and R^{q} at each occurrence are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, hydroxyl, cyano, amino, cycloalkyl and cycloalkylalkyl;
q is an integer from 0 to 25;
m is 0, 1, 2, 3 or 4;
n is 0, 1, 2 or 3;
v is 0, 1, 2 or 3.

The object of the present disclosure is to provide a compound as represented by general formula (I) or a pharmaceutically acceptable salt thereof: 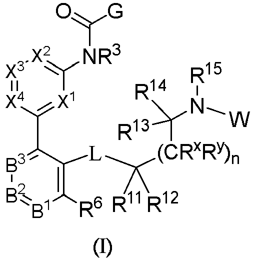 wherein:
G is selected from 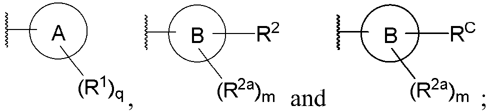
ring A is selected from polycyclic aryl, polycyclic heteroaryl, polycyclic cycloalkyl and polycyclic heterocyclyl;
ring B is monocyclic aryl or monocyclic heteroaryl;
each R¹ and each R^{2a} are the same or different, and are each independently selected from halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, -(CR^{a}R^{b})ᵥ heteroaryl and oxo, wherein the alkyl, alkenyl, alkynyl, alkoxy, cycloalkyl, heterocyclyl, aryl and heteroaryl are each independently and optionally substituted with one or more R⁰;
or two R¹ together with the ring atoms to which they are attached form cycloalkyl or heterocyclyl, wherein the cycloalkyl or heterocyclyl is optionally substituted with one or more R⁰;
R² is -OR^{A} or -alkylene-R^{B}, wherein the alkylene is optionally substituted with one or more R⁰;
R^{A} is selected from alkyl, cycloalkyl, heterocyclyl, aryl and heteroaryl, wherein the alkyl, cycloalkyl, heterocyclyl, aryl and heteroaryl are each independently and optionally substituted with one or more R⁰;
R^{B} is selected from cycloalkyl, heterocyclyl, aryl and heteroaryl, wherein the cycloalkyl, heterocyclyl, aryl and heteroaryl are each independently and optionally substituted with one or more R⁰;
R^{C} is selected from cycloalkyl, heterocyclyl, aryl and heteroaryl, wherein the cycloalkyl, heterocyclyl, aryl and heteroaryl are each independently substituted with one or more R^{d};
each R^{d} is the same or different, and is independently selected from halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, - C(O)R¹⁷, -C(O)OR¹⁷, -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, - (CR^{a}R^{b})ᵥ heteroaryl and oxo, wherein the alkyl, alkenyl, alkynyl, alkoxy, cycloalkyl, heterocyclyl, aryl and heteroaryl are each independently and optionally substituted with one or more R⁰;
R³ is selected from a hydrogen atom, alkyl, alkenyl, alkynyl, haloalkyl, hydroxyalkyl, cycloalkyl, heterocyclyl, cycloalkylalkyl and heterocyclylalkyl;
X¹ is CR^{4a} or N;
X² is CR^{4b} or N;
X³ is CR^{4c} or N;
X⁴ is CR^{4d} or N;
B¹ is CR^{5a} or N;
B² is CR^{5b} or N;
B³ is CR^{5c} or N;
R^{4a}, R^{4b}, R^{4c}, R^{4d}, R^{5a}, R^{5b} and R^{5c} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, - C(O)NR¹⁸R¹⁹, -OC(O)NR¹⁸R¹⁹, -OC(O)R¹⁷, -OC(O)OR¹⁷, -NR²⁰C(O)R¹⁷, - NR²⁰C(O)OR¹⁷, -SR¹⁷, -S(O)R¹⁷, -S(O)₂R¹⁷, -S(O)₂NR¹⁸R¹⁹, -NR²⁰S(O)₂R¹⁷, - (CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or R^{4b} and R^{4c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{4c} and R^{4d} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5a} and R^{5b} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5b} and R^{5c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
R⁶ is selected from a hydrogen atom, -NHR⁷ and -NHC(O)R⁷;
R⁷ is selected from a hydrogen atom, alkyl, haloalkyl, hydroxyalkyl, alkoxyalkyl, hydroxyl, alkoxy, haloalkoxy, cycloalkyl, heterocyclyl, aryl, heteroaryl, cycloalkylalkyl and heterocyclylalkyl;
L is selected from CR⁸R⁹, C (O), O, NR¹⁰, S, S(O) and S(O)₂;
R⁸ and R⁹ are the same or different, and are each independently selected from a hydrogen atom, halogen, alkyl, haloalkyl, hydroxyalkyl, alkoxy, haloalkoxy, alkoxyalkyl, aminoalkyl, hydroxyl, cyano, amino, cycloalkyl and cycloalkylalkyl;
or R⁸ and R⁹ together with the carbon atoms to which they are attached form cycloalkyl or heterocyclyl, wherein the cycloalkyl or heterocyclyl is optionally substituted with one or more R⁰;
R¹⁰ is selected from a hydrogen atom, alkyl, alkenyl, alkynyl, haloalkyl, hydroxyalkyl, alkoxyalkyl, hydroxyl, alkoxy, haloalkoxy, cycloalkyl, heterocyclyl, cycloalkylalkyl and heterocyclylalkyl;
R¹¹, R¹², R¹³, R¹⁴, R^{x} and R^{y} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, hydroxyl, cyano, amino, nitro, -(CR^{a}R^{b})ᵥOR¹⁷, -(CR^{a}R^{b})ᵥNR¹⁸R¹⁹, -(CR^{a}R^{b})ᵥ cycloalkyl, - (CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or any two of R¹¹, R¹², R¹³, R¹⁴, R^{x} and R^{y}, together with the carbon atoms to which they are attached, form cycloalkyl or heterocyclyl, wherein the cycloalkyl or heterocyclyl is optionally substituted with one or more R⁰;
or R¹¹ and R¹² together form =O;
or R¹³ and R¹⁴ together form =O;
or R^{x} and R^{y} on the same carbon atom together form =O;
R¹⁵ is selected from a hydrogen atom, alkyl, alkoxy, alkenyl, alkynyl, haloalkyl, hydroxyalkyl, cycloalkyl, heterocyclyl, aryl, heteroaryl, cycloalkylalkyl, heterocyclylalkyl, arylalkyl and heteroarylalkyl, wherein the alkyl, alkoxy, alkenyl, alkynyl, cycloalkyl, heterocyclyl, aryl, heteroaryl, cycloalkylalkyl, heterocyclylalkyl, arylalkyl and heteroarylalkyl are each independently and optionally substituted with one or more R⁰;
or any one of R¹¹, R¹², R¹³, R¹⁴, R^{x} and R^{y} together with R¹⁵ and the atoms to which they are respectively attached forms nitrogen-containing heterocyclyl, wherein the nitrogen-containing heterocyclyl is optionally substituted with one or more R⁰;
W is selected from -C(O)R¹⁶, -S(O)₂R¹⁶ and -CN;
R¹⁶ is selected from alkenyl, alkynyl and alkenyl oxide, wherein the alkenyl, alkynyl and alkenyl oxide are each independently and optionally substituted with one or more substituents selected from oxo, halogen, alkyl, haloalkyl, hydroxyalkyl, alkoxy, haloalkoxy, alkoxyalkyl, aminoalkyl, hydroxyl, cyano, amino, nitro, - (CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R⁰ at each occurrence is the same or different, and is independently selected from oxo, halogen, alkenyl, alkynyl, cyano, nitro, hydroxyl, amino, alkyl, haloalkyl, hydroxyalkyl, alkoxy, haloalkoxy, alkoxyalkyl, aminoalkyl, -(CR^{a}R^{b})ᵥOR¹⁷, - (CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R¹⁷, R¹⁸, R¹⁹ and R²⁰ at each occurrence are the same or different, and are each independently selected from a hydrogen atom, alkyl, alkenyl, alkynyl, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, cycloalkyl, heterocyclyl, aryl and heteroaryl are each independently and optionally substituted with one or more R⁰¹;
or R¹⁸ and R¹⁹ together with the nitrogen atom to which they are attached form nitrogen-containing heterocyclyl, wherein the nitrogen-containing heterocyclyl is optionally substituted with one or more R⁰¹;
R⁰¹ at each occurrence is the same or different, and is independently selected from oxo, halogen, hydroxyl, alkenyl, alkynyl, cyano, nitro, amino, -NH alkyl, -N (alkyl)₂, -C(O)O alkyl, -C(O)OH, -C(O)NH₂, -C(O)halogen, -C(O)alkyl, alkyl, haloalkyl, hydroxyalkyl, alkoxy, haloalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
R^{a} and R^{b} at each occurrence are the same or different, and are each independently selected from a hydrogen atom, halogen, alkyl, haloalkyl, hydroxyalkyl, alkoxy, haloalkoxy, hydroxyl, cyano, amino, cycloalkyl and cycloalkylalkyl;
q is an integer from 0 to 25;
m is 0, 1, 2, 3 or 4;
n is 0, 1, 2 or 3;
v is 0, 1, 2 or 3.

The object of the present disclosure is to provide a compound as represented by general formula (I) or a pharmaceutically acceptable salt thereof: 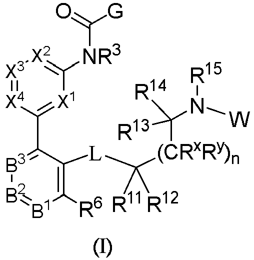 wherein:
G is 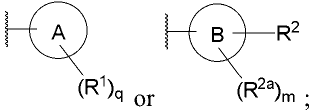
ring A is selected from polycyclic aryl, polycyclic heteroaryl, polycyclic cycloalkyl and polycyclic heterocyclyl;
ring B is monocyclic aryl or monocyclic heteroaryl;
each R¹ and each R^{2a} are the same or different, and are each independently selected from halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, -(CR^{a}R^{b})ᵥ heteroaryl and oxo, wherein the alkyl, alkenyl, alkynyl, alkoxy, cycloalkyl, heterocyclyl, aryl and heteroaryl are each independently and optionally substituted with one or more R⁰;
or two R¹ together with the ring atoms to which they are attached form cycloalkyl or heterocyclyl, wherein the cycloalkyl or heterocyclyl is optionally substituted with one or more R⁰;
R² is -OR^{A} or -alkylene-R^{B}, wherein the alkylene is optionally substituted with one or more R⁰;
R^{A} is selected from alkyl, cycloalkyl, heterocyclyl, aryl and heteroaryl, wherein the alkyl, cycloalkyl, heterocyclyl, aryl and heteroaryl are each independently and optionally substituted with one or more R⁰;
R^{B} is selected from cycloalkyl, heterocyclyl, aryl and heteroaryl, wherein the cycloalkyl, heterocyclyl, aryl and heteroaryl are each independently and optionally substituted with one or more R⁰;
R³ is selected from a hydrogen atom, alkyl, alkenyl, alkynyl, haloalkyl, hydroxyalkyl, cycloalkyl, heterocyclyl, cycloalkylalkyl and heterocyclylalkyl;
X¹ is CR^{4a} or N;
X² is CR^{4b} or N;
X³ is CR^{4c} or N;
X⁴ is CR^{4d} or N;
B¹ is CR^{5a} or N;
B² is CR^{5b} or N;
B³ is CR^{5c} or N;
R^{4a}, R^{4b}, R^{4c}, R^{4d}, R^{5a}, R^{5b} and R^{5c} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, - C(O)NR¹⁸R¹⁹, -OC(O)NR¹⁸R¹⁹, -OC(O)R¹⁷, -OC(O)OR¹⁷, -NR²⁰C(O)R¹⁷, - NR²⁰C(O)OR¹⁷, -SR¹⁷, -S(O)R¹⁷, -S(O)₂R¹⁷, -S(O)₂NR¹⁸R¹⁹, -NR²⁰S(O)₂R¹⁷, - (CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or R^{4b} and R^{4c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{4c} and R^{4d} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5a} and R^{5b} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5b} and R^{5c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
R⁶ is selected from a hydrogen atom, -NHR⁷ and -NHC(O)R⁷;
R⁷ is selected from a hydrogen atom, alkyl, haloalkyl, hydroxyalkyl, alkoxyalkyl, hydroxyl, alkoxy, haloalkoxy, cycloalkyl, heterocyclyl, aryl, heteroaryl, cycloalkylalkyl and heterocyclylalkyl;
L is selected from CR⁸R⁹, C (O), O, NR¹⁰, S, S(O) and S(O)₂;
R⁸ and R⁹ are the same or different, and are each independently selected from a hydrogen atom, halogen, alkyl, haloalkyl, hydroxyalkyl, alkoxy, haloalkoxy, alkoxyalkyl, aminoalkyl, hydroxyl, cyano, amino, cycloalkyl and cycloalkylalkyl;
or R⁸ and R⁹ together with the carbon atoms to which they are attached form cycloalkyl or heterocyclyl, wherein the cycloalkyl or heterocyclyl is optionally substituted with one or more R⁰;
R¹⁰ is selected from a hydrogen atom, alkyl, alkenyl, alkynyl, haloalkyl, hydroxyalkyl, alkoxyalkyl, hydroxyl, alkoxy, haloalkoxy, cycloalkyl, heterocyclyl, cycloalkylalkyl and heterocyclylalkyl;
R¹¹, R¹², R¹³, R¹⁴, R^{x} and R^{y} are the same or different, and are each independently selected from a hydrogen atom, halogen, alkyl, alkenyl, alkynyl, haloalkyl, hydroxyalkyl, alkoxy, haloalkoxy, alkoxyalkyl, aminoalkyl, hydroxyl, cyano, amino, nitro, -(CR^{a}R^{b})ᵥOR¹⁷, -(CR^{a}R^{b})ᵥNR¹⁸R¹⁹, -(CR^{a}R^{b})ᵥ cycloalkyl, - (CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or any two of R¹¹, R¹², R¹³, R¹⁴, R^{x} and R^{y}, together with the carbon atoms to which they are attached, form cycloalkyl or heterocyclyl, wherein the cycloalkyl or heterocyclyl is optionally substituted with one or more R⁰;
or R¹¹ and R¹² together form =O;
or R¹³ and R¹⁴ together form =O;
or R^{x} and R^{y} on the same carbon atom together form =O;
R¹⁵ is selected from a hydrogen atom, alkyl, alkoxy, alkenyl, alkynyl, haloalkyl, hydroxyalkyl, cycloalkyl, heterocyclyl, aryl, heteroaryl, cycloalkylalkyl, heterocyclylalkyl, arylalkyl and heteroarylalkyl, wherein the alkyl, alkoxy, alkenyl, alkynyl, cycloalkyl, heterocyclyl, aryl, heteroaryl, cycloalkylalkyl, heterocyclylalkyl, arylalkyl and heteroarylalkyl are each independently and optionally substituted with one or more R⁰;
or any one of R¹¹, R¹², R¹³, R¹⁴, R^{x} and R^{y} together with R¹⁵ and the atoms to which they are respectively attached forms nitrogen-containing heterocyclyl, wherein the nitrogen-containing heterocyclyl is optionally substituted with one or more R⁰;
W is selected from -C(O)R¹⁶, -S(O)₂R¹⁶ and -CN;
R¹⁶ is selected from alkenyl, alkynyl and alkenyl oxide, wherein the alkenyl, alkynyl and alkenyl oxide are each independently and optionally substituted with one or more substituents selected from oxo, halogen, alkyl, haloalkyl, hydroxyalkyl, alkoxy, haloalkoxy, alkoxyalkyl, aminoalkyl, hydroxyl, cyano, amino, nitro, - (CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R⁰ at each occurrence is the same or different, and is independently selected from oxo, halogen, alkenyl, alkynyl, cyano, nitro, hydroxyl, amino, alkyl, haloalkyl, hydroxyalkyl, alkoxy, haloalkoxy, alkoxyalkyl, aminoalkyl, -(CR^{a}R^{b})ᵥOR¹⁷, - (CR^{a}R^{b})ᵥNR¹⁸R¹⁹, -C(O)R¹⁷, -C(O)OR¹⁷, -C(O)NR¹⁸R¹⁹, -OC(O)NR¹⁸R¹⁹, - OC(O)R¹⁷, -OC(O)OR¹⁷, -NR²⁰C(O)R¹⁷, -NR²⁰C(O)OR¹⁷, -SR¹⁷, -S(O)R¹⁷, - S(O)₂R¹⁷, -S(O)₂NR¹⁸R¹⁹, -NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl;
R¹⁷, R¹⁸, R¹⁹ and R²⁰ at each occurrence are the same or different, and are each independently selected from a hydrogen atom, alkyl, alkenyl, alkynyl, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, cycloalkyl, heterocyclyl, aryl and heteroaryl are each independently and optionally substituted with one or more R⁰¹;
or R¹⁸ and R¹⁹ together with the nitrogen atom to which they are attached form nitrogen-containing heterocyclyl, wherein the nitrogen-containing heterocyclyl is optionally substituted with one or more R⁰¹;
R⁰¹ at each occurrence is the same or different, and is independently selected from oxo, halogen, hydroxyl, alkenyl, alkynyl, cyano, nitro, amino, -NH alkyl, -N (alkyl)₂, -C(O)O alkyl, -C(O)OH, -C(O)NH₂, -C(O)halogen, -C(O)alkyl, alkyl, haloalkyl, hydroxyalkyl, alkoxy, haloalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
R^{a} and R^{b} at each occurrence are the same or different, and are each independently selected from a hydrogen atom, halogen, alkyl, haloalkyl, hydroxyalkyl, alkoxy, haloalkoxy, hydroxyl, cyano, amino, cycloalkyl and cycloalkylalkyl;
q is an integer from 0 to 25;
m is 0, 1, 2, 3 or 4;
n is 0, 1, 2 or 3;
v is 0, 1, 2 or 3.

In some embodiments of the present disclosure, provided is the compound as represented by general formula (I) or the pharmaceutically acceptable salt thereof, which is a compound as represented by general formula (I-1) or a pharmaceutically acceptable salt thereof: 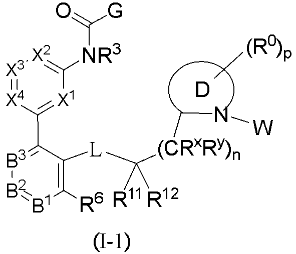 wherein:
ring D is nitrogen-containing heterocyclyl;
p is 0, 1, 2 or 3;
G, R³, X¹, X², X³, X⁴, B¹, B², B³, L, R⁶, R¹¹, R¹², R^{x}, R^{y}, n, W and R⁰ are as defined in general formula (I).

In some embodiments of the present disclosure, provided is the compound as represented by general formula (I) or the pharmaceutically acceptable salt thereof, which is a compound as represented by general formula (VI) or a pharmaceutically acceptable salt thereof: 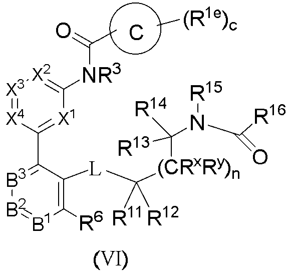 wherein:
ring C is selected from aryl, heteroaryl, cycloalkyl and heterocyclyl;
R^{1e} is R¹;
c is 0, 1, 2, 3, 4 or 5;
R¹, R³, X¹, X², X³, X⁴, B¹, B², B³, R⁶, L, R¹¹, R¹², R¹³, R¹⁴, R¹⁵, R¹⁶, R^{x}, R^{y} and n are as defined in general formula (I); provided that at least one of X¹, X², X³ and X⁴ is N.

In some embodiments of the present disclosure, provided is the compound as represented by general formula (I) or general formula (I-1) or the pharmaceutically acceptable salt thereof, wherein W is -C(O)R¹⁶; R¹⁶ is as defined in general formula (I); in some embodiments, W is -C (O)-CH=CH₂.

In some embodiments of the present disclosure, provided is the compound as represented by general formula (I), general formula (I-1) or general formula (VI) or the pharmaceutically acceptable salt thereof, wherein L is O.

In some embodiments of the present disclosure, provided is the compound as represented by general formula (I), general formula (I-1) or general formula (VI) or the pharmaceutically acceptable salt thereof, wherein B¹ is N.

In some embodiments of the present disclosure, provided is the compound as represented by general formula (I), general formula (I-1) or general formula (VI) or the pharmaceutically acceptable salt thereof, wherein B² is CR^{5b}; R^{5b} is as defined in general formula (I); in some embodiments, B² is CH.

In some embodiments of the present disclosure, provided is the compound as represented by general formula (I), general formula (I-1) or general formula (VI) or the pharmaceutically acceptable salt thereof, wherein R^{5a} is selected from a hydrogen atom, halogen and C₁₋₆ alkyl; in some embodiments, R^{5a} is a hydrogen atom.

In some embodiments of the present disclosure, provided is the compound as represented by general formula (I), general formula (I-1) or general formula (VI) or the pharmaceutically acceptable salt thereof, wherein R^{5b} is selected from a hydrogen atom, halogen and C₁₋₆ alkyl; in some embodiments, R^{5b} is a hydrogen atom.

In some embodiments of the present disclosure, provided is the compound as represented by general formula (I), general formula (I-1) or general formula (VI) or the pharmaceutically acceptable salt thereof, wherein B¹ is N; B² is CR^{5b}; B³ is CR^{5c} or N; R^{5b} and R^{5c} are as defined in general formula (I); in some embodiments, B¹ is N; B² is CH; B³ is CH or N; in some embodiments, B¹ is N; B² is CH; B³ is N.

In some embodiments of the present disclosure, provided is the compound as represented by general formula (I) or general formula (I-1) or the pharmaceutically acceptable salt thereof, wherein X¹ is CR^{4a}; X² is CR^{4b}; X³ is CR^{4c}; X⁴ is CR^{4d}; R^{4a}, R^{4b}, R^{4c} and R^{4d} are as defined in general formula (I); in some embodiments, X¹ is CR^{4a}; X² is N or CR^{4b}; X³ is CR^{4c}; X⁴ is CR^{4d}; R^{4a}, R^{4b}, R^{4c} and R^{4d} are as defined in general formula (I); in some embodiments, X¹ is CR^{4a}; X² is N or CH; X³ is CR^{4c}; X⁴ is CH; R^{4a} and R^{4c} are as defined in general formula (I); in some embodiments, X¹ is CR^{4a}; X² is CH; X³ is CR^{4c}; X⁴ is CH; R^{4a} and R^{4c} are as defined in general formula (I); in some embodiments, X¹ is CR^{4a}; R^{4a} is C₁₋₆ alkyl or C₁₋₆ hydroxyalkyl; X² is N or CH; X³ is CR^{4c}; R^{4c} is a hydrogen atom or halogen; X⁴ is CH; in some embodiments, X¹ is CR^{4a}; R^{4a} is C₁₋₆ alkyl; X² is CH; X³ is CR^{4c}; R^{4c} is halogen; X⁴ is CH; in some embodiments, X¹ is CR^{4a}; R^{4a} is methyl or hydroxymethyl; X² is CH; X³ is CR^{4c}; R^{4c} is a hydrogen atom or F; X⁴ is CH; in some embodiments, X¹ is CR^{4a}; R^{4a} is methyl; X² is CH; X³ is CR^{4c}; R^{4c} is F; X⁴ is CH.

In some embodiments of the present disclosure, provided is the compound as represented by general formula (VI) or the pharmaceutically acceptable salt thereof, wherein ring C is phenyl or 6-membered heteroaryl; in some embodiments, ring C is phenyl or pyridyl; in some embodiments, ring C is phenyl.

In some embodiments of the present disclosure, provided is the compound as represented by general formula (I) or general formula (VI) or the pharmaceutically acceptable salt thereof, which is a compound as represented by general formula (VI-1) or a pharmaceutically acceptable salt thereof: 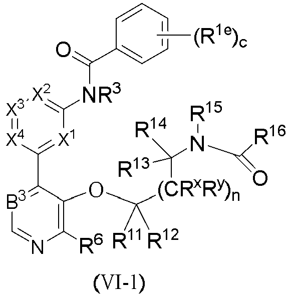 wherein:
R^{1e}, c, R³ , X¹, X², X³, X⁴ , B³ , R⁶ , R¹¹, R¹² , R¹³, R¹⁴, R¹⁵, R¹⁶, R^{x}, R^{y} and n are as defined in general formula (VI); provided that at least one of X¹, X², X³ and X⁴ is N.

In some embodiments of the present disclosure, provided is the compound as represented by general formula (I), general formula (I-1), general formula (VI) or general formula (VI-1) or the pharmaceutically acceptable salt thereof, wherein X¹ is N; X² is CR^{4b}; X³ is CR^{4c}; X⁴ is CR^{4d}; R^{4b}, R^{4c} and R^{4d} are as defined in general formula (I); in some embodiments, X¹ is CR^{4a}; X² is N; X³ is CR^{4c}; X⁴ is CR^{4d}; R^{4a}, R^{4c} and R^{4d} are as defined in general formula (I); in some embodiments, X¹ is CR^{4a}; X² is N; X³ is CR^{4c}; X⁴ is CR^{4d}; R^{4a}, R^{4c} and R^{4d} are the same or different, and are independently selected from a hydrogen atom, C₁₋₆ alkyl, halogen and C₁₋₆ hydroxyalkyl; in some embodiments, X¹ is CR^{4a}; X² is CR^{4b}; X³ is N; X⁴ is CR^{4a}; R^{4a}, R^{4b} and R^{4d} are as defined in general formula (I); in some embodiments, X¹ is CR^{4a}; X² is CR^{4b}; X³ is CR^{4c}; X⁴ is N; R^{4a}, R^{4b} and R^{4c} are as defined in general formula (I); in some embodiments, X¹ is CR^{4a}; X² is N; X³ is CH; X⁴ is CH; R^{4a} is as defined in general formula (I) (in some embodiments, R^{4a} is C₁₋₆ hydroxyalkyl; and in some embodiments, R^{4a} is hydroxymethyl).

In some embodiments of the present disclosure, provided is the compound as represented by general formula (I) or the pharmaceutically acceptable salt thereof, which is a compound as represented by general formula (II) or a pharmaceutically acceptable salt thereof: 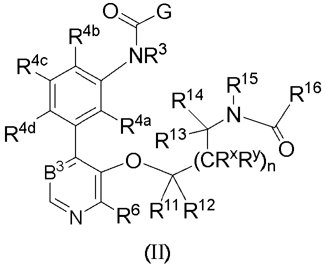 wherein:
G , R³ , R^{4a}, R^{4b}, R^{4c}, R^{4d}, B³ , R⁶ , R¹¹, R¹² , R¹³, R¹⁴, R¹⁵, R¹⁶, R^{x} , R^{y} and n are as defined in general formula (I).

In some embodiments of the present disclosure, provided is the compound as represented by general formula (I), general formula (I-1) or general formula (II) or the pharmaceutically acceptable salt thereof, which is a compound as represented by general formula (II-1) or a pharmaceutically acceptable salt thereof: 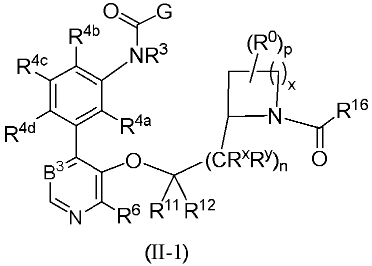 wherein:
p is 0, 1, 2 or 3;
x is 1, 2, 3 or 4;
G, R³, R^{4a}, R^{4b}, R^{4c}, R^{4d}, B³ , R⁶ , R¹¹, R¹², R¹⁶, R⁰, R^{x} , R^{y} and n are as defined in general formula (I).

In some embodiments of the present disclosure, provided is the compound as represented by general formula (I), general formula (I-1), general formula (II) or general formula (II-1) or the pharmaceutically acceptable salt thereof, wherein G is polycyclic aryl optionally substituted with one or more R^{G} or heteroaryl optionally substituted with one or more R^{G}; in some embodiments, G is 8- to 14-membered polycyclic aryl optionally substituted with one or more R^{G} or 5- to 14-membered heteroaryl optionally substituted with one or more R^{G}; in some embodiments, G is 8- to 14-membered polycyclic aryl optionally substituted with one or more R^{G} or 8-to 14-membered polycyclic heteroaryl optionally substituted with one or more R^{G}; in some embodiments, G is 8- to 14-membered polycyclic aryl optionally substituted with one or more R^{G}; in some embodiments, G is 5- to 14-membered heteroaryl optionally substituted with one or more R^{G}; in some embodiments, G is 8- to 14-membered polycyclic heteroaryl optionally substituted with one or more R^{G}; in some embodiments, G is phenyl optionally substituted with one or more R^{G}; the above R^{G} is as defined in general formula (I).

In some embodiments of the present disclosure, provided is the compound as represented by general formula (I), general formula (I-1), general formula (II) or general formula (II-1) or the pharmaceutically acceptable salt thereof, wherein each R^{G} is the same or different, and is independently selected from halogen, C₁₋₆ alkyl, C₁₋₆ haloalkyl, C₁₋₆ alkoxy, C₁₋₆ hydroxyalkyl, C₁₋₆ haloalkoxy, C₁₋₆ alkoxy C₁₋₆ alkyl, 3- to 6-membered cycloalkyl, 3- to 6-membered cycloalkyloxy and cyano; in some embodiments, each R^{G} is the same or different, and is independently selected from halogen, C₁₋₆ alkyl, C₁₋₆ haloalkyl, C₁₋₆ alkoxy and C₁₋₆ hydroxyalkyl; in some embodiments, each R^{G} is the same or different, and is independently halogen or C₁₋₆ alkyl; in some embodiments, each R^{G} is the same or different, and is independently halogen; in some embodiments, each R^{G} is the same or different, and is independently methyl or F; in some embodiments, R^{G} is F; in some embodiments, each R^{G} is the same or different, and is independently halogen or 3- to 6-membered cycloalkyl; in some embodiments, each R^{G} is the same or different, and is independently F or cyclopropyl; in some embodiments, each R^{G} is the same or different, and is independently halogen, or 3- to 6-membered cycloalkyl optionally substituted with one C₁₋₆ alkyl; in some embodiments, each R^{G} is the same or different, and is independently F or

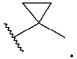

In some embodiments of the present disclosure, provided is the compound as represented by general formula (I), general formula (I-1), general formula (II) or general formula (II-1) or the pharmaceutically acceptable salt thereof, wherein G is 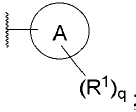 ; ring A, R¹ and q are as defined in general formula (I); in some embodiments, G is 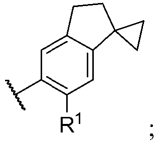 R¹ is as defined in general formula (I); in some embodiments, G is

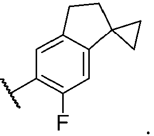

In some embodiments of the present disclosure, provided is the compound as represented by general formula (I), general formula (I-1), general formula (II) or general formula (II-1) or the pharmaceutically acceptable salt thereof, wherein G is 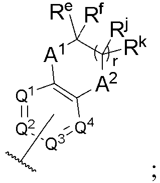 in some embodiments, G is 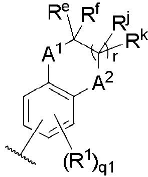 ; in some embodiments, G is 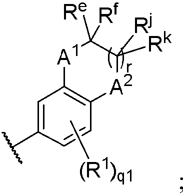 in some embodiments, G is 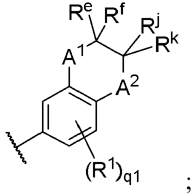 in some embodiments, G is 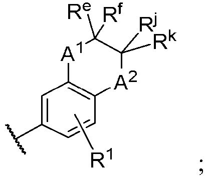 in some embodiments, G is 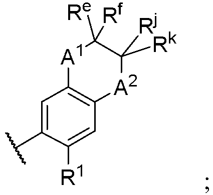 in some embodiments, G is 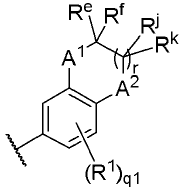 or 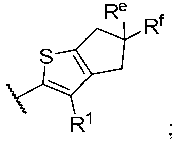 ; in some embodiments, G is 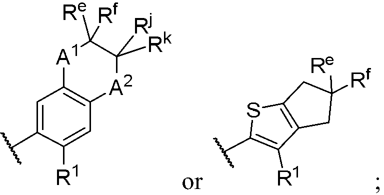 in some embodiments, G is 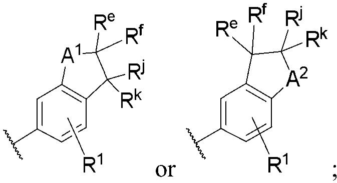 in some embodiments, G is

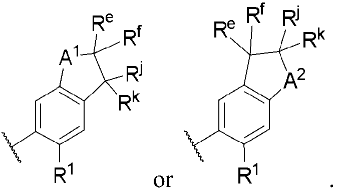

In each of the above embodiments, one of Q¹, Q², Q³ and Q⁴ is C, which is attached to C(O), and the remaining three are the same or different, and are each independently N or CR^{1a}; A¹ and A² are the same or different, and are each independently selected from a bond, C(O), O, NR, S, S (O), S(O)₂ and (CR^{1b}R^{1c})ⱼ; R^{1a}, R^{1b}, R^{1c}, R^{e}, R^{f}, R^{j} and R^{k} are the same or different, and are each independently a hydrogen atom or R¹; or R^{e} and R^{f} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R^{l}; or R^{j} and R^{k} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; or R^{f} and R^{j} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; or R^{1b} and R^{1c} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; j is 0, 1, 2 or 3; r is 0 or 1; q1 is 0, 1, 2 or 3; R is selected from a hydrogen atom, alkyl, alkenyl, alkynyl, haloalkyl, hydroxyalkyl, alkoxyalkyl, aminoalkyl, cycloalkyl, heterocyclyl, aryl and heteroaryl; R¹ is as defined in general formula (I).

In some embodiments of the present disclosure, provided is the compound as represented by general formula (I), general formula (I-1), general formula (II) or general formula (II-1) or the pharmaceutically acceptable salt thereof, wherein G is 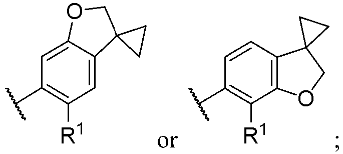 R¹ is as defined in general formula (I); in some embodiments, G is 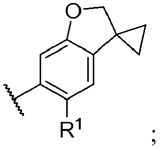 R¹ is as defined in general formula (I); in some embodiments, G is 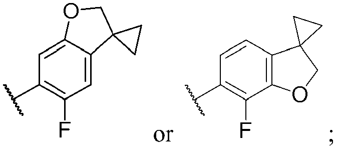 in some embodiments, G is

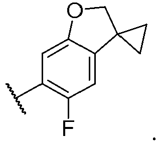

In some embodiments of the present disclosure, provided is the compound as represented by general formula (I), general formula (I-1), general formula (II) or general formula (II-1) or the pharmaceutically acceptable salt thereof, wherein G is selected from 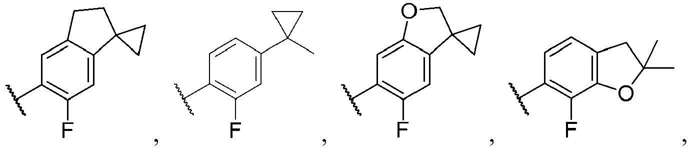 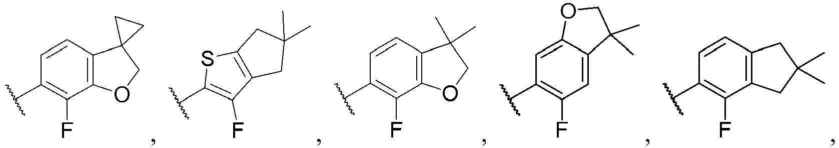 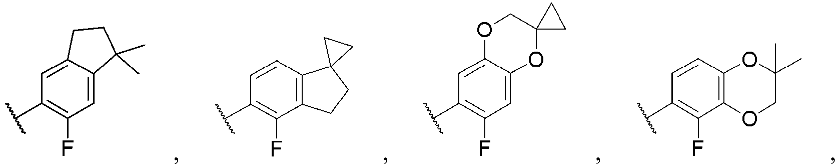 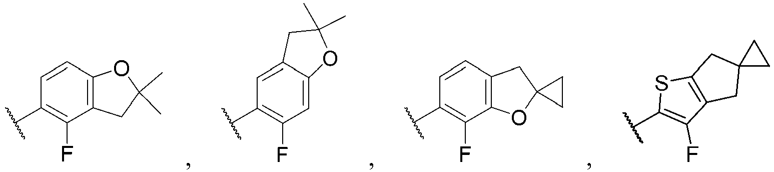 and 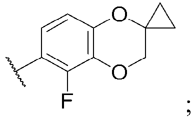 in some embodiments, G is selected from 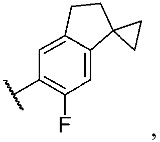 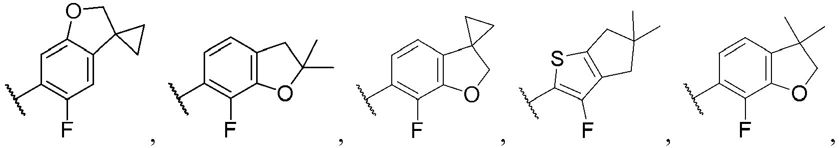 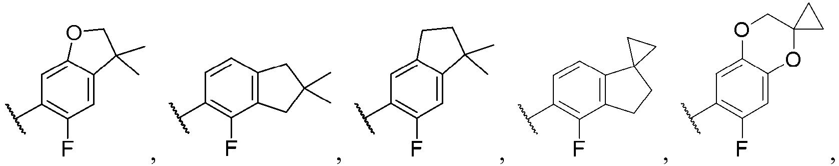 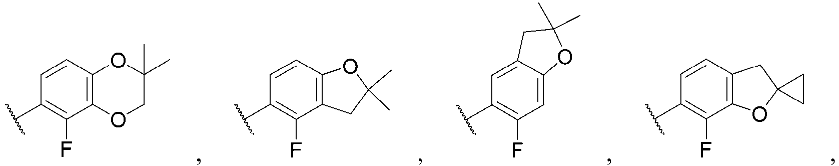 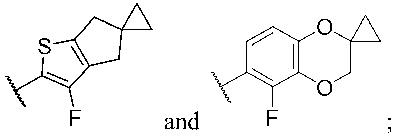 in some embodiments, G is selected from 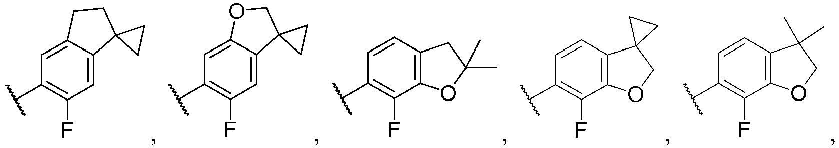 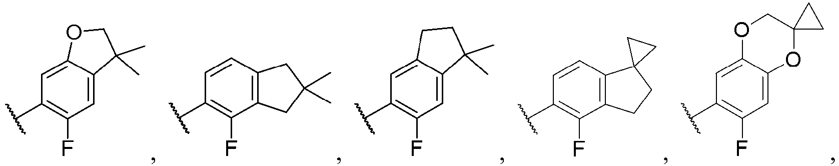 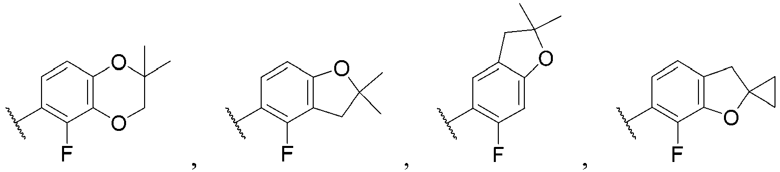 and

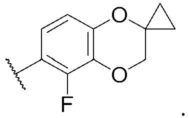

In some embodiments of the present disclosure, provided is the compound as represented by general formula (I), general formula (I-1), general formula (II) or general formula (II-1) or the pharmaceutically acceptable salt thereof, wherein G is 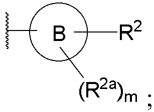 ring B, R², R^{2a} and m are as defined in general formula (I).

In some embodiments of the present disclosure, provided is the compound as represented by general formula (I), general formula (I-1), general formula (II) or general formula (II-1) or the pharmaceutically acceptable salt thereof, wherein G is 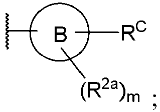 ring B, R^{C}, R^{2a} and m are as defined in general formula (I); in some embodiments, G is 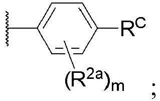 R^{C}, R^{2a} and m are as defined in general formula (I); in some embodiments, G is 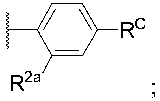 R^{C} and R^{2a} are as defined in general formula (I); in some embodiments, G is 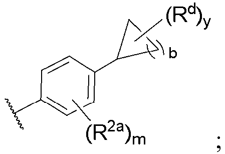 b is 1, 2, 3, 4 or 5 (in some embodiments, 1); y is 1, 2 or 3 (in some embodiments, 1); R^{d}, R^{2a} and m are as defined in general formula (I); in some embodiments, G is 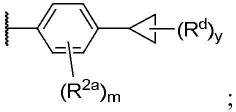 y is 1, 2 or 3 (in some embodiments, 1); R^{d}, R^{2a} and m are as defined in general formula (I); in some embodiments, G is 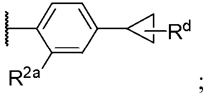 R^{2a} and R^{d} are as defined in general formula (I); in some embodiments, G is 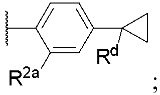 R^{2a} and R^{d} are as defined in general formula (I); in some embodiments, G is

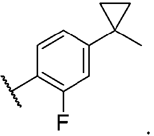

In some embodiments of the present disclosure, provided is the compound as represented by general formula (I), general formula (I-1), general formula (II) or general formula (II-1) or the pharmaceutically acceptable salt thereof, wherein ring A is 7- to 10-membered fused heterocyclyl; in some embodiments, ring A is 8- or 9-membered fused heterocyclyl.

In some embodiments of the present disclosure, provided is the compound as represented by general formula (I), general formula (I-1), general formula (II) or general formula (II-1) or the pharmaceutically acceptable salt thereof, wherein ring A is 11- to 14-membered tricyclic aryl or 12- to 19-membered tetracyclic aryl; in some embodiments, ring A is 11- or 12-membered tricyclic aryl or 13-membered tetracyclic aryl; in some embodiments, ring A is 11- to 14-membered tricyclic aryl; in some embodiments, ring A is 11- or 12-membered tricyclic aryl; in some embodiments, ring A is 11-membered tricyclic aryl; in some embodiments, ring A is 12- to 19-membered tetracyclic aryl; in some embodiments, ring A is 13-membered tetracyclic aryl; in some embodiments, ring A is 8- to 14-membered polycyclic aryl or 8- to 14-membered polycyclic heteroaryl; in some embodiments, ring A is 8- to 14-membered polycyclic aryl or 5- to 14-membered heteroaryl; in some embodiments, ring A is 8- to 14-membered polycyclic aryl; in some embodiments, ring A is 5- to 14-membered heteroaryl; in some embodiments, ring A is 5- or 6-membered heteroaryl; in some embodiments, ring A is 5-membered heteroaryl.

In some embodiments of the present disclosure, provided is the compound as represented by general formula (I), general formula (I-1), general formula (II) or general formula (II-1) or the pharmaceutically acceptable salt thereof, wherein ring A is selected from 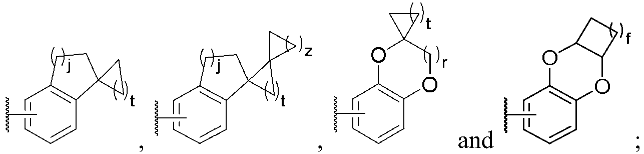 j is 0, 1, 2 or 3; r is 0 or 1; t is 1, 2, 3 or 4; z is 1, 2, 3 or 4; f is 1, 2, 3 or 4; in some embodiments, ring A is selected from 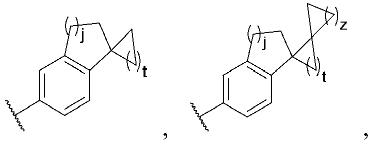 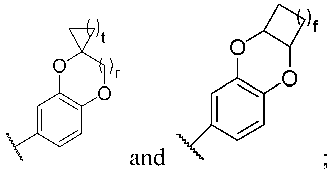 j is 0, 1, 2 or 3; r is 0 or 1; t is 1, 2, 3 or 4; z is 1, 2, 3 or 4; f is 1, 2, 3 or 4; in some embodiments, ring A is 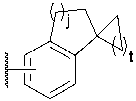 or 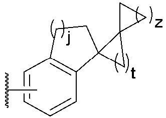 ;j is 0, 1, 2 or 3; t is 1, 2, 3 or 4; z is 1, 2, 3 or 4; in some embodiments, ring A is 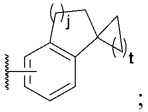 j is 0, 1, 2 or 3; t is 1, 2, 3 or 4; in some embodiments, ring 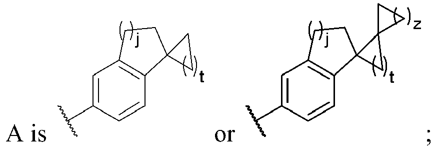 j is 0, 1, 2 or 3; t is 1, 2, 3 or 4; z is 1, 2, 3 or 4; in some embodiments, ring A is 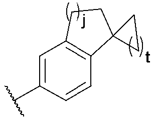 ;j is 0, 1, 2 or 3; t is 1, 2, 3 or 4; in some embodiments, ring A is selected from 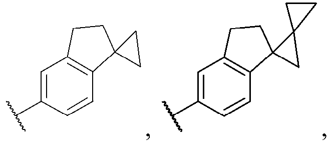 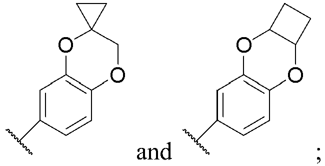 in some embodiments, ring A is 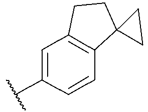 or 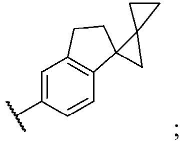 in some embodiments, ring A is 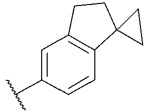 ;in some embodiments, ring A is selected from 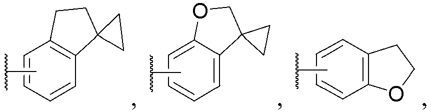 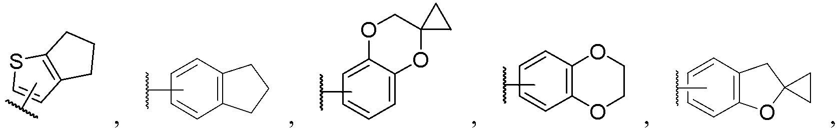 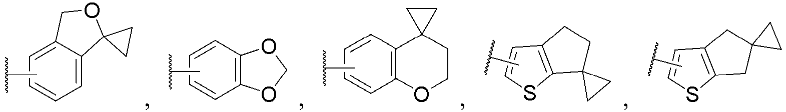 and 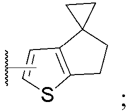 in some embodiments, ring A is selected from 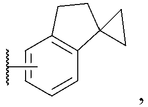 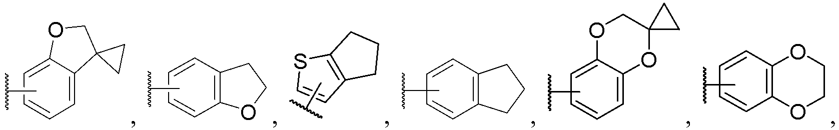 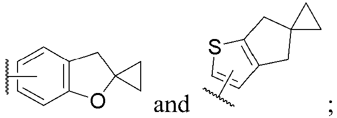 in some embodiments, ring A is selected from 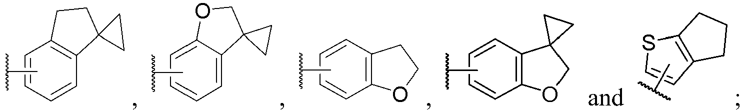 in some embodiments, ring A is selected from 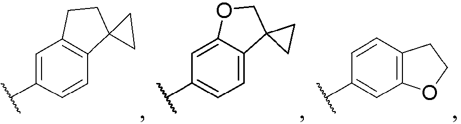 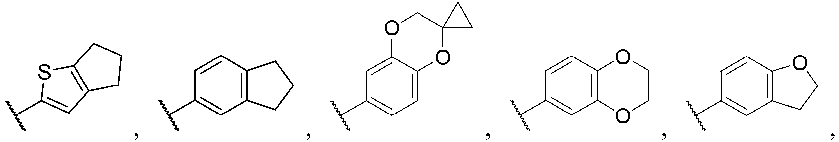 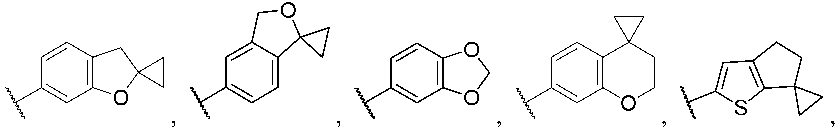 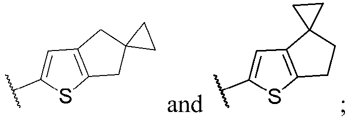 in some embodiments, ring A is selected from 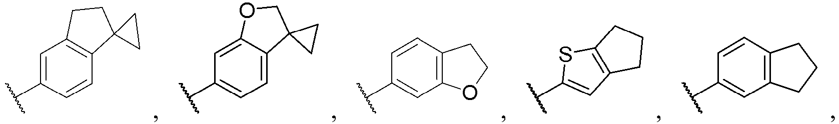 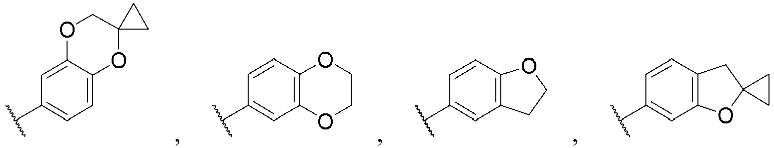 and 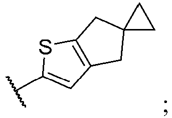 in some embodiments, ring A is selected from 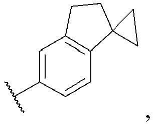 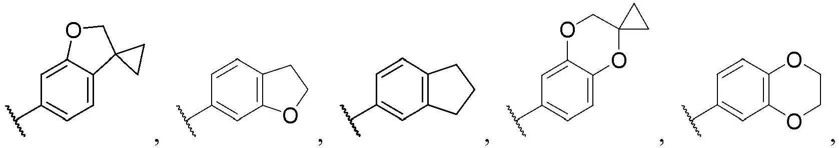 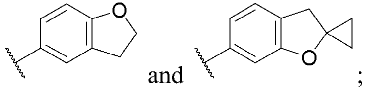 in some embodiments, ring A is selected from 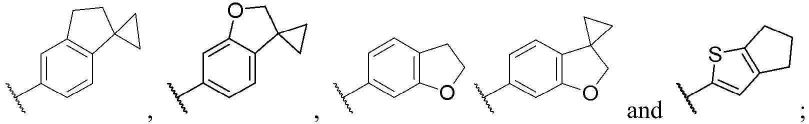 in some embodiments, ring A is

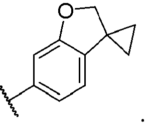

In some embodiments of the present disclosure, provided is the compound as represented by general formula (I), general formula (I-1), general formula (II) or general formula (II-1) or the pharmaceutically acceptable salt thereof, wherein 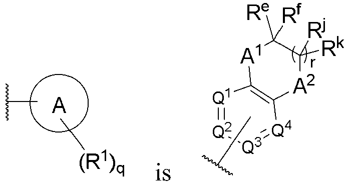 ;in some embodiments, 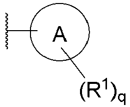 is 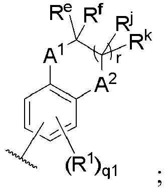 in some embodiments, 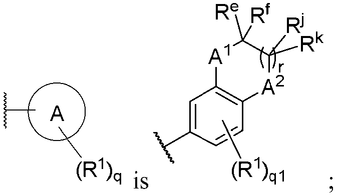 in some embodiments, 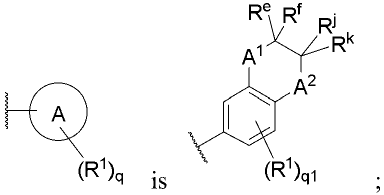 in some embodiments, 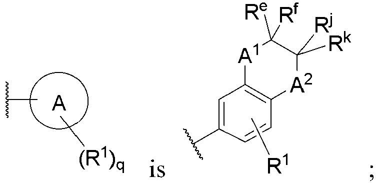 in some embodiments, 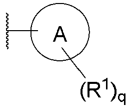 is 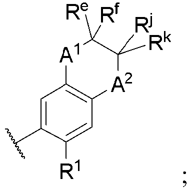 in some embodiments, 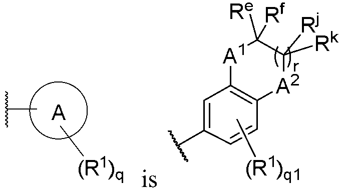 or 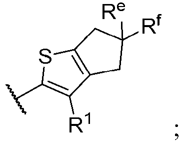 in some embodiments, 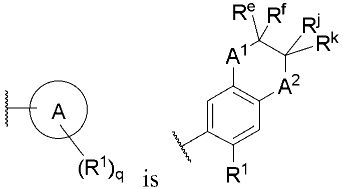 or 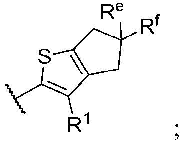 in some embodiments, 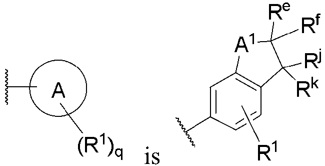 or 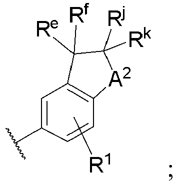 in some embodiments, 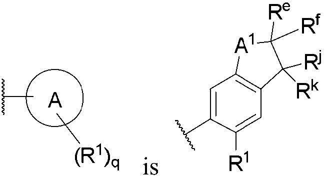 or 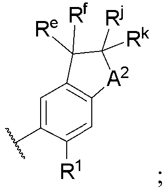 in some embodiments, 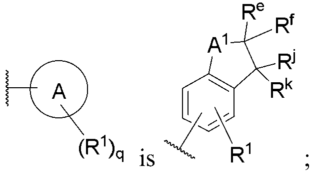 in some embodiments, 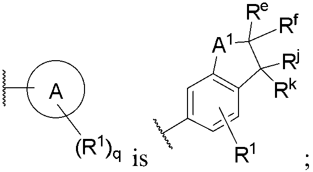 in some embodiments, 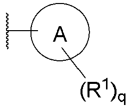 is 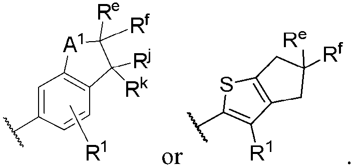 In each of the above embodiments, one of Q¹, Q², Q³ and Q⁴ is C, which is attached to C(O), and the remaining three are the same or different, and are each independently N or CR^{1a}; A¹ and A² are the same or different, and are each independently selected from a bond, C (O), O, NR, S, S (O), S(O)₂ and (CR^{1b}R^{1c})ⱼ; R^{1a}, R^{1b}, R^{1c}, R^{e}, R^{f}, R^{j} and R^{k} are the same or different, and are each independently a hydrogen atom or R¹; or R^{e} and R^{f} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; or R^{j} and R^{k} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; or R^{f} and R^{j} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; or R^{1b} and R^{1c} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹; j is 0, 1, 2 or 3; r is 0 or 1; q1 is 0, 1, 2 or 3; R is selected from a hydrogen atom, alkyl, alkenyl, alkynyl, haloalkyl, hydroxyalkyl, alkoxyalkyl, aminoalkyl, cycloalkyl, heterocyclyl, aryl and heteroaryl; R¹ is as defined in general formula (I).

In some embodiments of the present disclosure, provided is the compound as represented by general formula (I), general formula (I-1), general formula (II) or general formula (II-1) or the pharmaceutically acceptable salt thereof, wherein 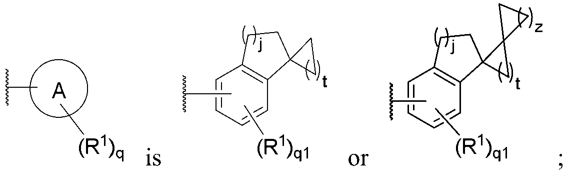 in some embodiments, 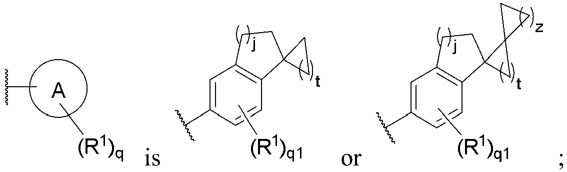 in some embodiments, 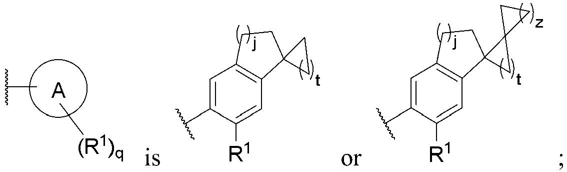 in some embodiments, 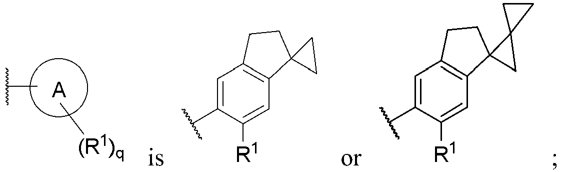 in some embodiments, 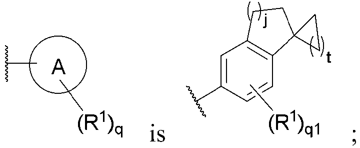 in some embodiments, 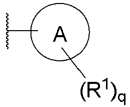 is 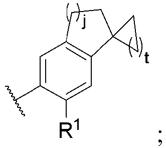 in some embodiments, 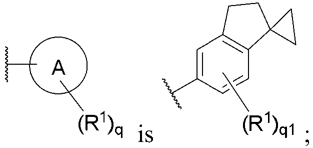 in some embodiments, 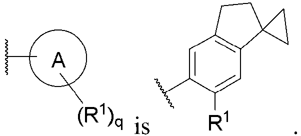 In each of the above embodiments, j is 0, 1, 2 or 3; t is 1, 2, 3 or 4; z is 1, 2, 3 or 4; q1 is 0, 1, 2 or 3; R¹ is as defined in general formula (I).

In some embodiments of the present disclosure, provided is the compound as represented by general formula (I), general formula (I-1), general formula (II) or general formula (II-1) or the pharmaceutically acceptable salt thereof, wherein 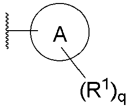 is selected from 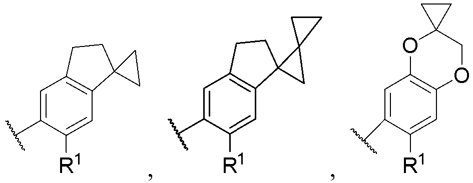 and 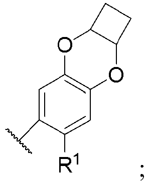 R¹ is as defined in general formula (I); in some embodiments, 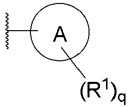 is selected from 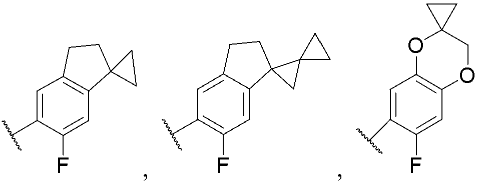 and 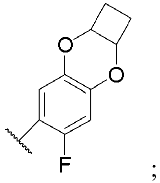 in some embodiments, 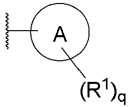 is 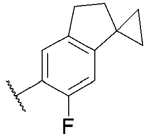 or 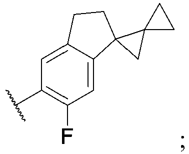 in some embodiments, 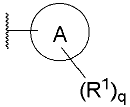 is

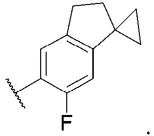

In some embodiments of the present disclosure, provided is the compound as represented by general formula (I), general formula (I-1), general formula (II) or general formula (II-1) or the pharmaceutically acceptable salt thereof, wherein 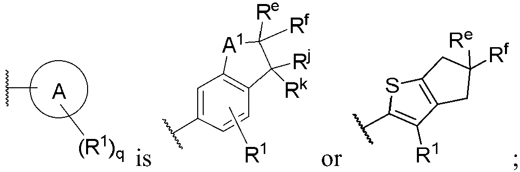 R^{e}, R^{f}, R^{j} and R^{k} are the same or different, and are each independently a hydrogen atom or R¹; or R^{e} and R^{f} together with the carbon atoms to which they are attached form cycloalkyl or heterocyclyl; or R^{j} and R^{k} together with the carbon atoms to which they are attached form cycloalkyl or heterocyclyl; A¹ is selected from a bond, CH₂, O and NR; R is selected from a hydrogen atom, alkyl and cycloalkyl; R¹ is as defined in general formula (I); in some embodiments, 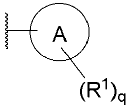 is selected from 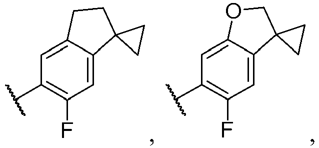 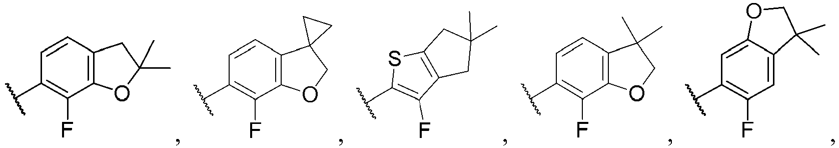 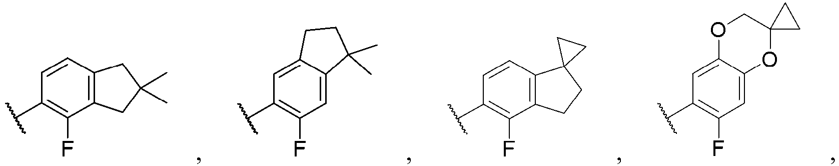 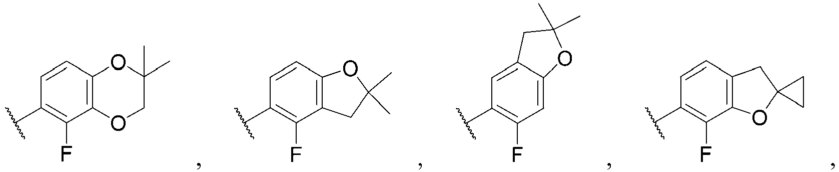 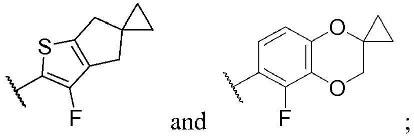 in some embodiments, 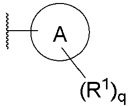 is selected from 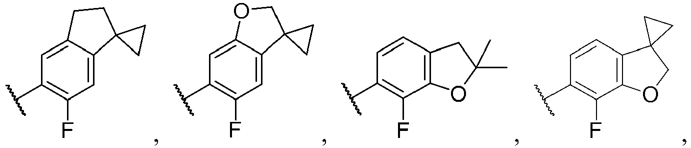 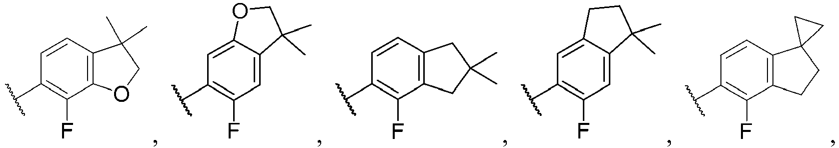 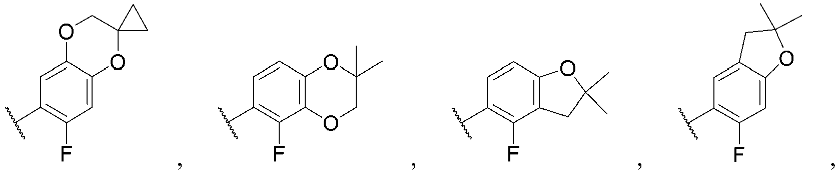 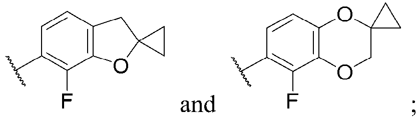 in some embodiments, 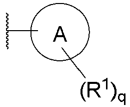 is selected from 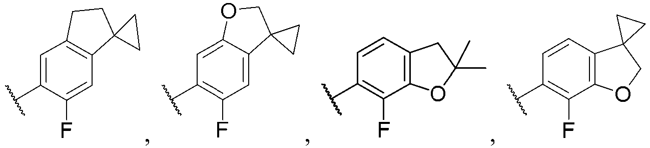 and 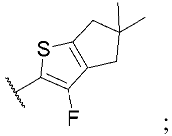 in some embodiments, 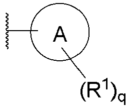 is 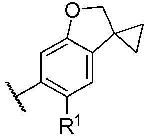 or 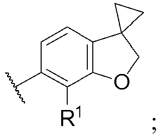 R¹ is as defined in general formula (I); in some embodiments, 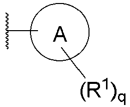 is 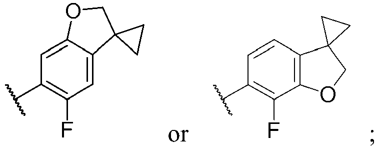 in some embodiments,

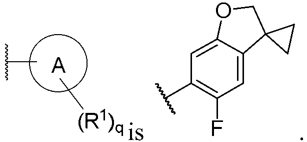

In some embodiments of the present disclosure, provided is the compound as represented by general formula (I), general formula (I-1), general formula (II) or general formula (II-1) or the pharmaceutically acceptable salt thereof, wherein G or 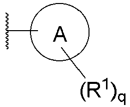 is selected from 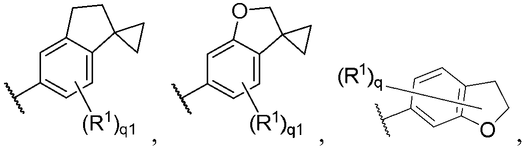 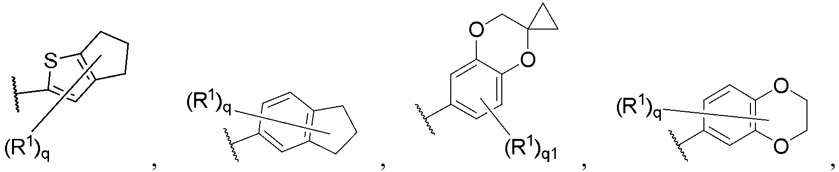 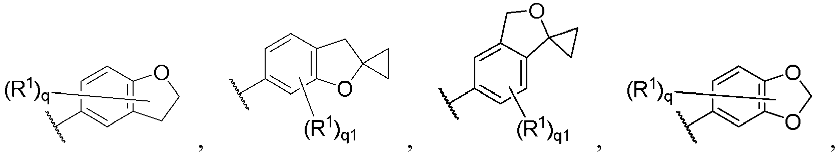 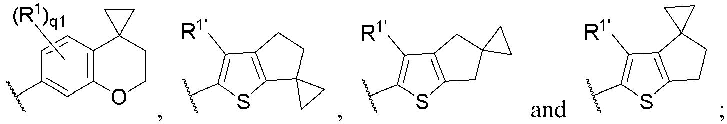 R¹' is a hydrogen atom or R¹; q1 is 0, 1, 2 or 3; q is 0, 1, 2, 3, 4, 5 or 6; R¹ is as defined in general formula (I); in some embodiments, G or 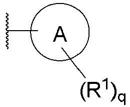 is selected from 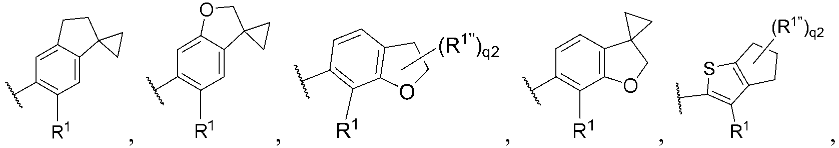 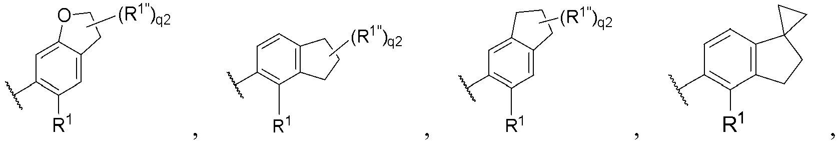 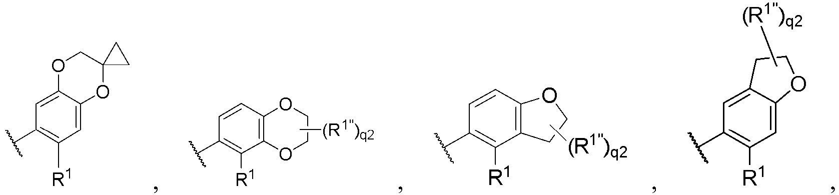 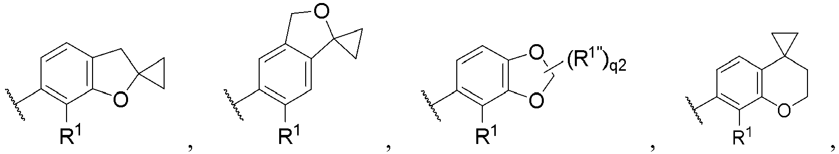 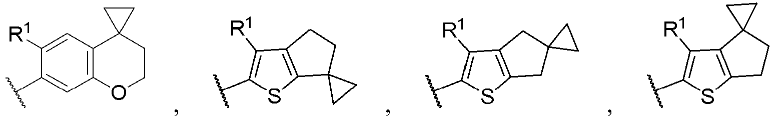 and 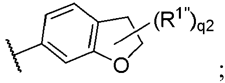 q2 is 0, 1, 2, 3 or 4; R¹" is R¹; R¹ is as defined in general formula (I); in some embodiments, G or 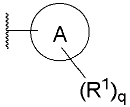 is selected from 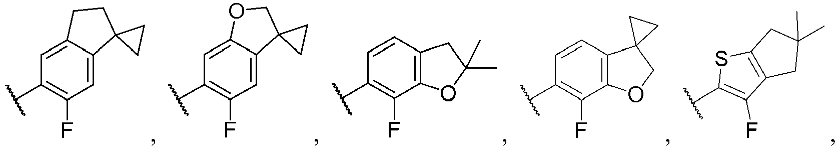 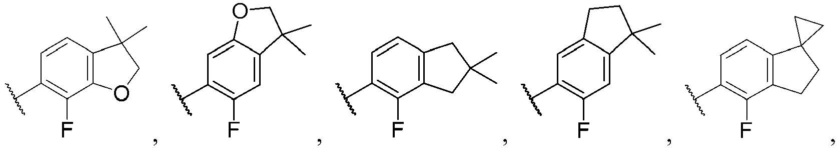 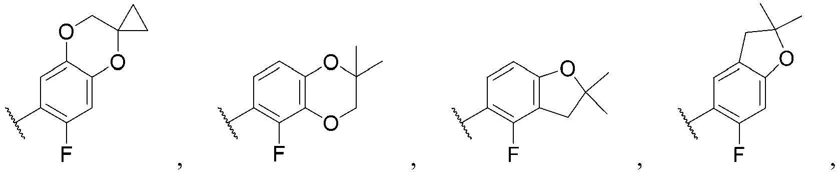 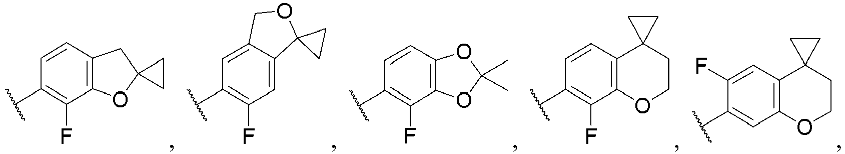 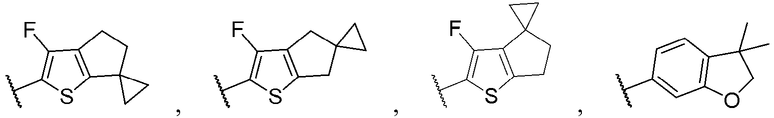 and

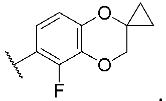

In some embodiments of the present disclosure, provided is the compound as represented by general formula (I), general formula (I-1), general formula (II) or general formula (II-1) or the pharmaceutically acceptable salt thereof, wherein ring B is phenyl or 5- or 6-membered heteroaryl; in some embodiments, ring B is phenyl or 6-membered heteroaryl; in some embodiments, ring B is phenyl.

In some embodiments of the present disclosure, provided is the compound as represented by general formula (I), general formula (I-1), general formula (II) or general formula (II-1) or the pharmaceutically acceptable salt thereof, wherein 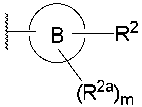 is 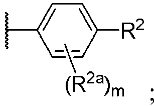 R², R^{2a} and m are as defined in general formula (I); in some embodiments, 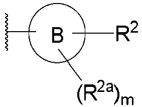 is 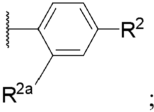 R² and R^{2a} are as defined in general formula (I).

In some embodiments of the present disclosure, provided is the compound as represented by general formula (I), general formula (I-1), general formula (II) or general formula (II-1) or the pharmaceutically acceptable salt thereof, wherein 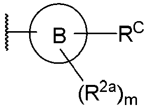 is 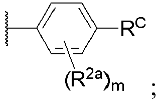 R^{C}, R^{2a} and m are as defined in general formula (I); in some embodiments, 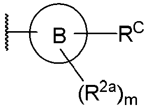 is 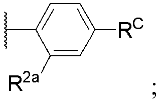 R^{C} and R^{2a} are as defined in general formula (I); in some embodiments, 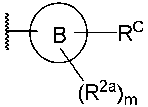 is 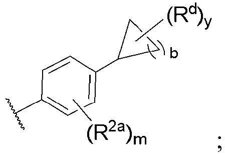 b is 1, 2, 3, 4 or 5 (in some embodiments, 1); y is 1, 2 or 3 (in some embodiments, 1); R^{d}, R^{2a} and m are as defined in general formula (I); in some embodiments, 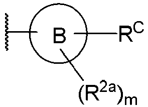 is 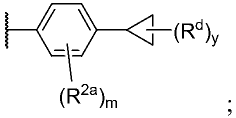 y is 1, 2 or 3 (in some embodiments, 1); R^{d}, R^{2a} and m are as defined in general formula (I); in some embodiments, 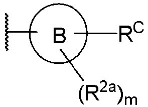 is 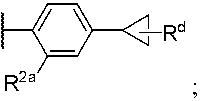 R^{d} and R^{2a} are as defined in general formula (I); in some embodiments, 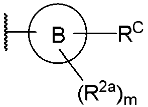 is 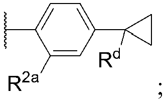 R^{2a} and R^{d} are as defined in general formula (I); in some embodiments, 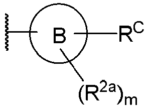 is

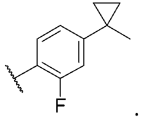

In some embodiments of the present disclosure, provided is the compound as represented by general formula (I), general formula (I-1), general formula (II) or general formula (II-1) or the pharmaceutically acceptable salt thereof, wherein 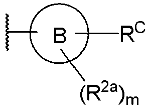 is 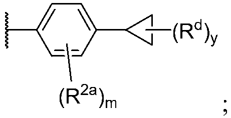 R^{2a} is halogen; m is 1; y is 1, 2 or 3 (in some embodiments, 1); each R^{d} is the same or different, and is independently selected from halogen, C₁₋₆ alkyl, C₁₋₆ haloalkyl and C₁₋₆ alkoxy; in some embodiments, 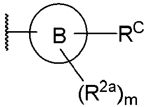 is 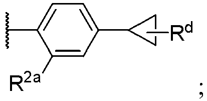 R^{2a} is halogen; R^{d} is C₁₋₆ alkyl; in some embodiments, 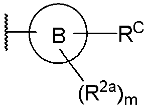 is 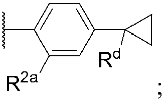 R^{2a} is halogen; R^{d} is C₁₋₆ alkyl; in some embodiments, 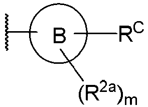 is

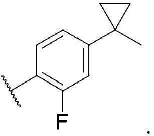

In some embodiments of the present disclosure, provided is the compound as represented by general formula (I) or general formula (II) or the pharmaceutically acceptable salt thereof, which is a compound as represented by general formula (III) or a pharmaceutically acceptable salt thereof: 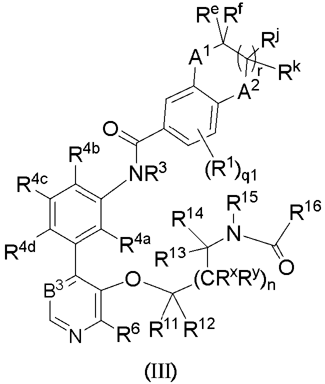 wherein:
A¹ and A² are the same or different, and are each independently selected from a bond, C (O), O, NR, S, S (O), S(O)₂ and (CR^{1b}R^{1c})ⱼ;
R^{1b}, R^{1c}, R^{e}, R^{f}, R^{j} and R^{k} are the same or different, and are each independently a hydrogen atom or R¹;
or R^{e} and R^{f} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹;
or R^{j} and R^{k} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹;
or R^{f} and R^{j} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹;
or R^{1b} and R^{1c} together with the carbon atoms to which they are attached form cycloalkyl or heterocyclyl, wherein the cycloalkyl or heterocyclyl is optionally substituted with 1, 2, 3 or 4 (in some embodiments, 1 or 2; and in some embodiments, 1) R¹;
R is selected from a hydrogen atom, alkyl, alkenyl, alkynyl, haloalkyl, hydroxyalkyl, alkoxyalkyl, aminoalkyl, cycloalkyl, heterocyclyl, aryl and heteroaryl;
j is 0, 1, 2 or 3;
q1 is 0, 1, 2 or 3;
r is 0 or 1;
R¹, R³, R^{4a}, R^{4b}, R^{4c}, R^{4d}, B³ , R⁶ , R¹¹, R¹² , R¹³, R¹⁴, R¹⁵, R¹⁶, R^{x} , R^{y} and n are as defined in general formula (I).

In some embodiments of the present disclosure, provided is the compound as represented by general formula (I) or general formula (II) or the pharmaceutically acceptable salt thereof, which is a compound as represented by general formula (IV) or a pharmaceutically acceptable salt thereof: 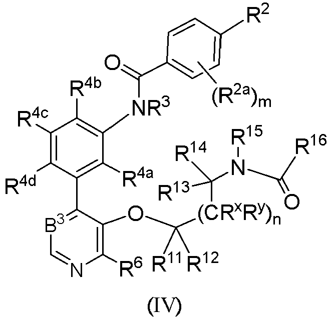 wherein:
R^{2a}, R², R³ , R^{4a}, R^{4b}, R^{4c}, R^{4d}, B³, R⁶, R¹¹, R¹² , R¹³, R¹⁴, R¹⁵, R¹⁶, R^{x}, R^{y}, m and n are as defined in general formula (I).

In some embodiments of the present disclosure, provided is the compound as represented by general formula (I) or general formula (II) or the pharmaceutically acceptable salt thereof, which is a compound as represented by general formula (V) or a pharmaceutically acceptable salt thereof: 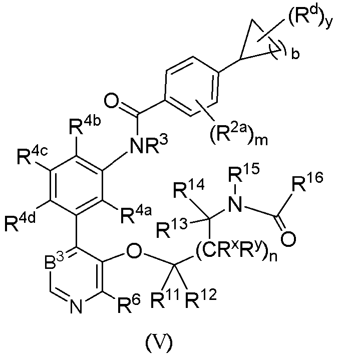 wherein:
y is 1, 2 or 3 (in some embodiments, 1);
b is 1, 2, 3, 4 or 5 (in some embodiments, 1);
R^{2a}, R^{d}, R³ , R^{4a}, R^{4b}, R^{4c}, R^{4d}, B³, R⁶ , R¹¹, R¹² , R¹³, R¹⁴, R¹⁵, R¹⁶, R^{x} , R^{y}, m and n are as defined in general formula (I).

In some embodiments of the present disclosure, provided is the compound as represented by general formula (I), general formula (I-1), general formula (II), general formula (II-1) or general formula (III) or the pharmaceutically acceptable salt thereof, wherein 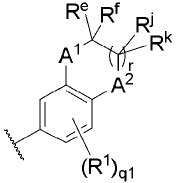 is 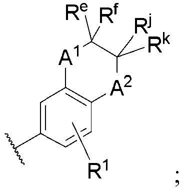 A¹, A², R¹, R^{e}, R^{f}, R^{j} and R^{k} are as defined in general formula (III); 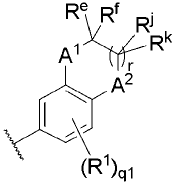 is 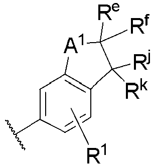 or 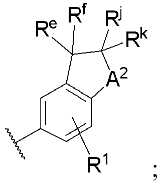 A¹, A², R¹, R^{e}, R^{f}, R^{j} and R^{k} are as defined in general formula (III); in some embodiments, 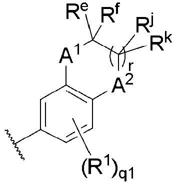 is 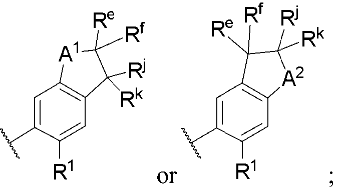 A¹, A², R¹, R^{e}, R^{f}, R^{j} and R^{k} are as defined in general formula (III); in some embodiments, 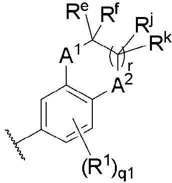 is 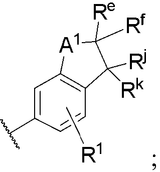 A¹, R¹, R^{e}, R^{f}, R^{j} and R^{k} are as defined in general formula (III); in some embodiments, 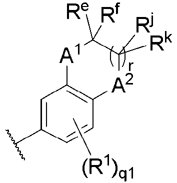 is 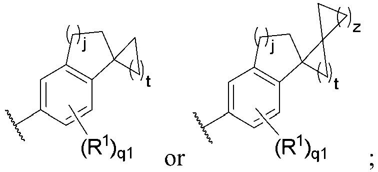 in some embodiments, 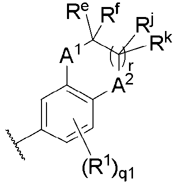 is 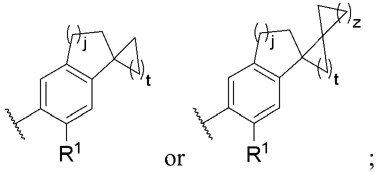 in some embodiments, 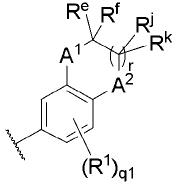 is 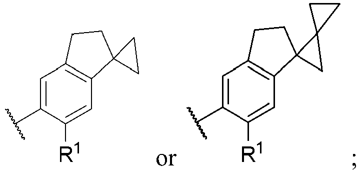 in some embodiments, 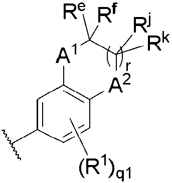 is 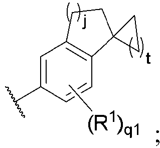 in some embodiments, 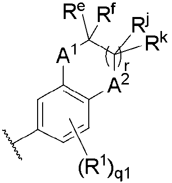 is 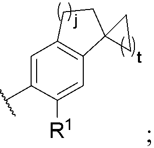 in some embodiments, 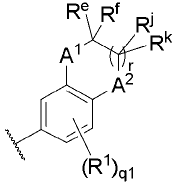 is 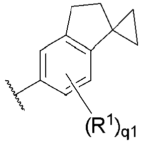 ; in some embodiments, 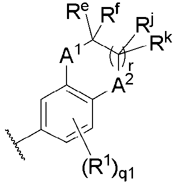 is 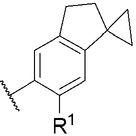 . In each of the above embodiments, j is 0, 1, 2 or 3; t is 1, 2, 3 or 4; z is 1, 2, 3 or 4; q1 is 0, 1, 2 or 3; R¹ is as defined in general formula (I).

In some embodiments of the present disclosure, provided is the compound as represented by general formula (I), general formula (I-1), general formula (II), general formula (II-1) or general formula (III) or the pharmaceutically acceptable salt thereof, wherein 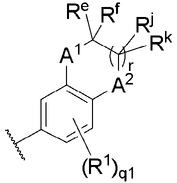 is selected from 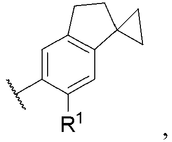 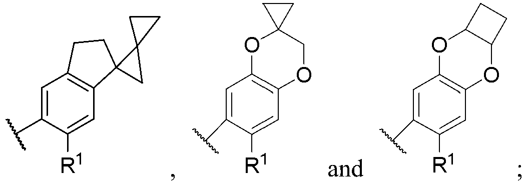 R¹ is as defined in general formula (I); in some embodiments, 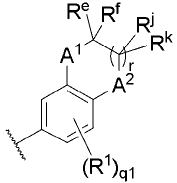 is selected from 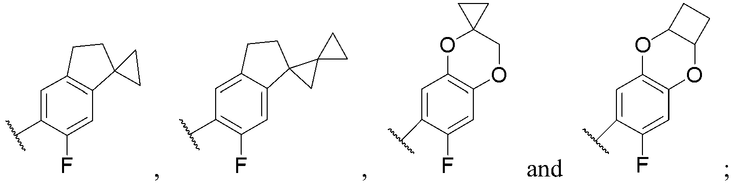 in some embodiments, 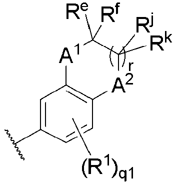 is 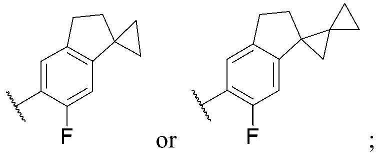 in some embodiments, 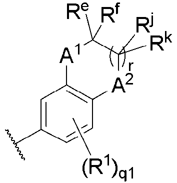 is 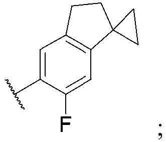 in some embodiments, 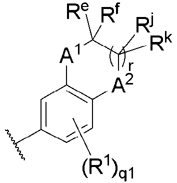 is selected from 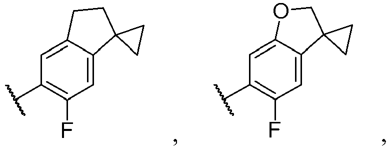 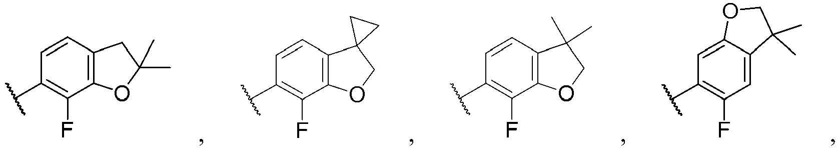 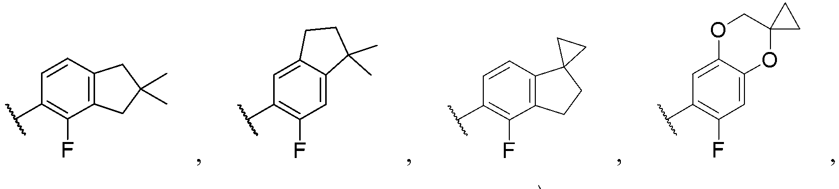 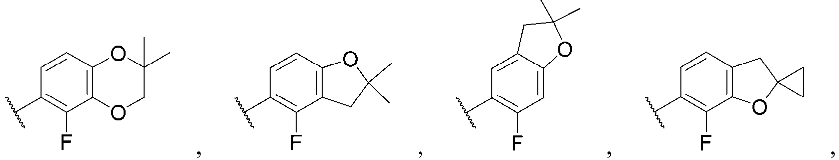 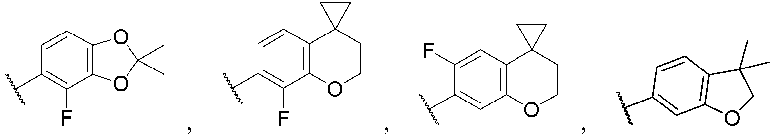 and 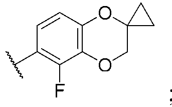 ; in some embodiments, 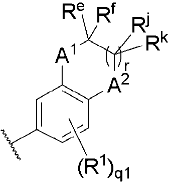 is selected from 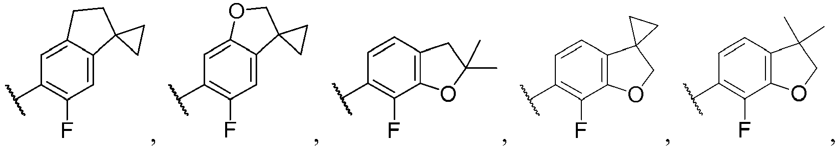 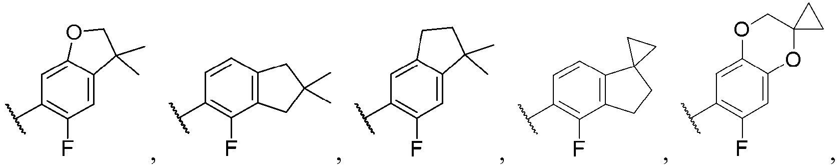 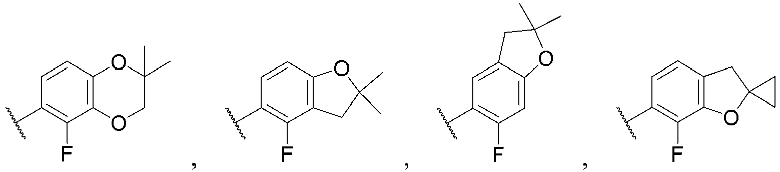 and 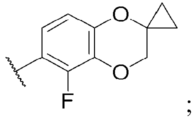 in some embodiments, 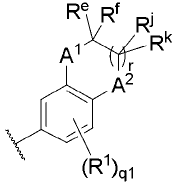 is 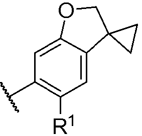 or 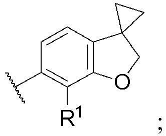 in some embodiments, 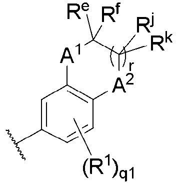 is 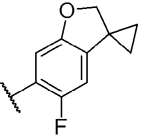 or 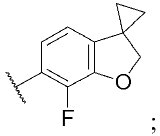 in some embodiments, 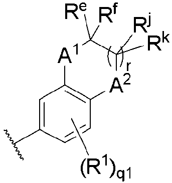 is 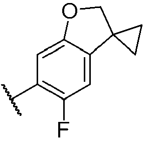 ; in some embodiments, 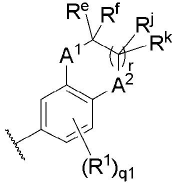 is selected from

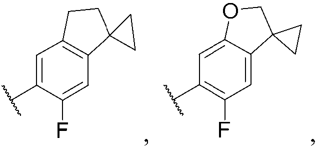

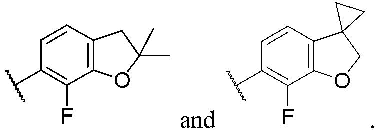

In some embodiments of the present disclosure, provided is the compound as represented by general formula (I), general formula (I-1), general formula (II), general formula (II-1) or general formula (III) or the pharmaceutically acceptable salt thereof, wherein each R¹ is the same or different, and is independently selected from halogen, C₁₋₆ alkyl, C₁₋₆ haloalkyl, C₁₋₆ alkoxy, C₁₋₆ hydroxyalkyl, C₁₋₆ haloalkoxy, C₁₋₆ alkoxy C₁₋₆ alkyl, 3- to 6-membered cycloalkyl, 3- to 6-membered cycloalkyloxy and cyano; in some embodiments, each R¹ is the same or different, and is independently selected from halogen, C₁₋₆ alkyl, C₁₋₆ haloalkyl, C₁₋₆ alkoxy and C₁₋₆ hydroxyalkyl; in some embodiments, each R¹ is the same or different, and is independently halogen or 3- to 6-membered cycloalkyl; in some embodiments, each R¹ is the same or different, and is independently F or cyclopropyl; in some embodiments, each R¹ is the same or different, and is independently halogen or C₁₋₆ alkyl; in some embodiments, each R¹ is the same or different, and is independently halogen; each R¹ is the same or different, and is independently C₁₋₆ alkyl; in some embodiments, each R¹ is the same or different, and is independently methyl or F; in some embodiments, R¹ is methyl; in some embodiments, R¹ is F.

In some embodiments of the present disclosure, provided is the compound as represented by general formula (I), general formula (I-1), general formula (II) or general formula (II-1) or the pharmaceutically acceptable salt thereof, wherein R¹' is selected from a hydrogen atom, halogen, C₁₋₆ alkyl, C₁₋₆ haloalkyl, C₁₋₆ alkoxy and C₁₋₆ hydroxyalkyl; in some embodiments, R¹' is selected from a hydrogen atom, halogen and C₁₋₆ alkyl; in some embodiments, R¹' is a hydrogen atom or halogen; in some embodiments, R¹' is halogen; in some embodiments, R¹' is F.

In some embodiments of the present disclosure, provided is the compound as represented by general formula (I), general formula (I-1), general formula (II) or general formula (II-1) or the pharmaceutically acceptable salt thereof, wherein each R¹" is the same or different, and is independently selected from halogen, C₁₋₆ alkyl, C₁₋₆ haloalkyl, C₁₋₆ alkoxy and C₁₋₆ hydroxyalkyl; in some embodiments, R¹" is halogen or C₁₋₆ alkyl; in some embodiments, R¹" is C₁₋₆ alkyl; in some embodiments, R¹" is methyl.

In some embodiments of the present disclosure, provided is the compound as represented by general formula (I), general formula (I-1), general formula (II) or general formula (II-1) or the pharmaceutically acceptable salt thereof, wherein q2 is 0, 1, 2 or 3; in some embodiments, q2 is 0, 1 or 2; in some embodiments, q2 is 2.

In some embodiments of the present disclosure, provided is the compound as represented by general formula (I), general formula (I-1), general formula (II) or general formula (II-1) or the pharmaceutically acceptable salt thereof, wherein R¹" is C₁₋₆ alkyl; q2 is 2; in some embodiments, R¹" is methyl; q2 is 2.

In some embodiments of the present disclosure, provided is the compound as represented by general formula (I), general formula (VI) or general formula (VI-1) or the pharmaceutically acceptable salt thereof, wherein each R^{1e} is the same or different, and is independently selected from halogen, C₁₋₆ alkyl, C₁₋₆ haloalkyl, C₁₋₆ alkoxy, C₁₋₆ hydroxyalkyl and 3- to 6-membered cycloalkyl, wherein the 3- to 6-membered cycloalkyl is optionally substituted with one or more substituents selected from halogen, C₁₋₆ alkyl, C₁₋₆ haloalkyl, hydroxyl and C₁₋₆ hydroxyalkyl; in some embodiments, each R^{1e} is the same or different, and is independently selected from halogen, C₁₋₆ alkyl, C₁₋₆ haloalkyl, C₁₋₆ alkoxy, C₁₋₆ hydroxyalkyl and 3- to 6-membered cycloalkyl; in some embodiments, each R^{1e} is the same or different, and is independently halogen or 3- to 6-membered cycloalkyl; in some embodiments, each R^{1e} is the same or different, and is independently halogen or cyclopropyl; in some embodiments, each R^{1e} is the same or different, and is independently F or cyclopropyl.

In some embodiments of the present disclosure, provided is the compound as represented by general formula (VI) or general formula (VI-1) or the pharmaceutically acceptable salt thereof, wherein c is 1, 2 or 3; in some embodiments, c is 2.

In some embodiments of the present disclosure, provided is the compound as represented by general formula (VI) or the pharmaceutically acceptable salt thereof, wherein 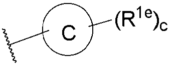 is 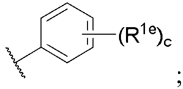 R^{1e} and c are as defined in general formula (VI); in some embodiments, 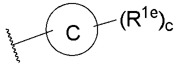 is 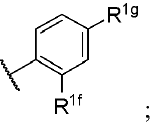 R^{1f} is a hydrogen atom or R^{1e}; R^{1g} is a hydrogen atom or R^{1e}; R^{1e} is as defined in general formula (VI); in some embodiments 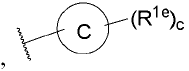 is

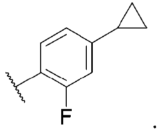

In some embodiments of the present disclosure, provided is the compound as represented by general formula (VI) or general formula (VI-1) or the pharmaceutically acceptable salt thereof, wherein 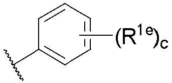 is 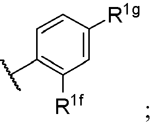 R^{1f} is a hydrogen atom or R^{1e}; R^{1g} is a hydrogen atom or R^{1e}; R1^{e} is as defined in general formula (VI-1); in some embodiments, 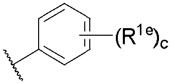 is

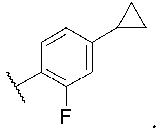

In some embodiments of the present disclosure, R^{1f} is selected from a hydrogen atom, halogen, C₁₋₆ alkyl, C₁₋₆ haloalkyl, C₁₋₆ alkoxy and C₁₋₆ hydroxyalkyl; in some embodiments, R^{1f} is a hydrogen atom or halogen; in some embodiments, R^{1f} is halogen; in some embodiments, R^{1f} is F.

In some embodiments of the present disclosure, R^{1g} is selected from a hydrogen atom, halogen, C₁₋₆ alkyl, C₁₋₆ haloalkyl, C₁₋₆ alkoxy, C₁₋₆ hydroxyalkyl and 3- to 6-membered cycloalkyl, wherein the 3- to 6-membered cycloalkyl is optionally substituted with one or more substituents selected from halogen, C₁₋₆ alkyl, C₁₋₆ haloalkyl, hydroxyl and C₁₋₆ hydroxyalkyl; in some embodiments, R^{1g} is 3- to 6-membered cycloalkyl, wherein the 3- to 6-membered cycloalkyl is optionally substituted with one or more substituents selected from halogen, C₁₋₆ alkyl, C₁₋₆ haloalkyl, hydroxyl and C₁₋₆ hydroxyalkyl; in some embodiments, R^{1g} is 3- to 6-membered cycloalkyl; in some embodiments, R^{1g} is cyclopropyl.

In some embodiments of the present disclosure, provided is the compound as represented by general formula (I), general formula (I-1), general formula (II) or general formula (II-1) or the pharmaceutically acceptable salt thereof, wherein q is an integer from 0 to 20 (i.e., q is 0, 1, 2, 3, 4, 5, 6, 7, 8, 9, 10, 11, 12, 13, 14, 15, 16, 17, 18, 19 or 20); in some embodiments, q is an integer from 0 to 10 (i.e., q is 0, 1, 2, 3, 4, 5, 6, 7, 8, 9 or 10); in some embodiments, q is 0, 1, 2, 3, 4, 5 or 6; in some embodiments, q is 0, 1, 2, 3 or 4; in some embodiments, q is 1, 2 or 3; in some embodiments, q is 0, 1, 2 or 3; in some embodiments, q is 1 or 3; in some embodiments, q is 0 or 1; in some embodiments, q is 1; in some embodiments, q is 3.

In some embodiments of the present disclosure, provided is the compound as represented by general formula (I), general formula (I-1), general formula (II), general formula (II-1) or general formula (III) or the pharmaceutically acceptable salt thereof, wherein q1 is 0, 1 or 2; in some embodiments, q1 is 0 or 1; in some embodiments, q1 is 1; in some embodiments, q1 is 0.

In some embodiments of the present disclosure, provided is the compound as represented by general formula (I), general formula (I-1), general formula (II), general formula (II-1) or general formula (IV) or the pharmaceutically acceptable salt thereof, wherein R^{A} is 3- to 6-membered cycloalkyl or C₁₋₆ alkyl; in some embodiments, R^{A} is cyclopropyl or isopropyl; in some embodiments, R^{A} is 3- to 6-membered cycloalkyl; in some embodiments, R^{A} is cyclopropyl; in some embodiments, R^{A} is C₁₋₆ alkyl; in some embodiments, R^{A} is isopropyl.

In some embodiments of the present disclosure, provided is the compound as represented by general formula (I), general formula (I-1), general formula (II), general formula (II-1) or general formula (IV) or the pharmaceutically acceptable salt thereof, wherein R^{B} is 3- to 6-membered cycloalkyl; in some embodiments, R^{B} is cyclopropyl.

In some embodiments of the present disclosure, provided is the compound as represented by general formula (I), general formula (I-1), general formula (II), general formula (II-1) or general formula (IV) or the pharmaceutically acceptable salt thereof, wherein R² is -OR^{A} or 3- to 6-membered cycloalkyl C₁₋₆ alkyl; R^{A} is as defined in general formula (I); in some embodiments, R² is selected from isopropyloxy, cyclopropyloxy and cyclopropylmethyl.

In some embodiments of the present disclosure, provided is the compound as represented by general formula (I), general formula (I-1), general formula (II), general formula (II-1) or general formula (V) or the pharmaceutically acceptable salt thereof, wherein 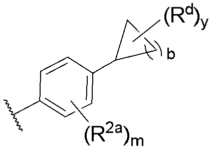 is 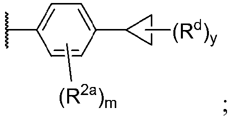 y is 1, 2 or 3 (in some embodiments, 1); R^{d}, R^{2a} and m are as defined in general formula (I); in some embodiments, 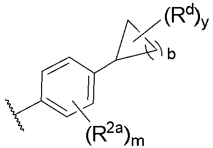 is 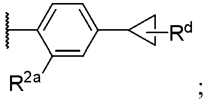 R^{2a} and R^{d} are as defined in general formula (I); in some embodiments, 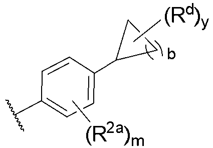 is 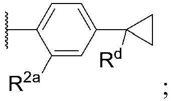 R^{2a} and R^{d} are as defined in general formula (I); in some embodiments, 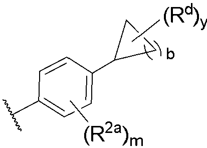 is

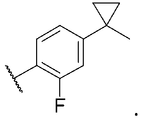

In some embodiments of the present disclosure, provided is the compound as represented by general formula (I), general formula (I-1), general formula (II), general formula (II-1) or general formula (V) or the pharmaceutically acceptable salt thereof, wherein R^{C} is 3- to 6-membered cycloalkyl, wherein the 3- to 6-membered cycloalkyl is substituted with one or more R^{d}; R^{d} is as defined in general formula (I); in some embodiments, R^{C} is cyclopropyl, wherein the cyclopropyl is substituted with one or more R^{d}; R^{d} is as defined in general formula (I); in some embodiments, R^{C} is cyclopropyl, wherein the cyclopropyl is substituted with one C₁₋₆ alkyl; in some embodiments, R^{C} is 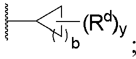 b is 1, 2, 3, 4 or 5 (in some embodiments, 1); y is 1, 2 or 3 (in some embodiments, 1); R^{d} is as defined in general formula (I); in some embodiments, R^{C} is 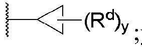 y is 1, 2 or 3 (in some embodiments, 1); R^{d} is as defined in general formula (I); in some embodiments, R^{C} is 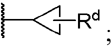 R^{d} is as defined in general formula (I); in some embodiments, R^{C} is 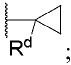 R^{d} is as defined in general formula (I); in some embodiments, R^{C} is

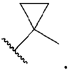

In some embodiments of the present disclosure, provided is the compound as represented by general formula (I), general formula (I-1), general formula (II), general formula (II-1) or general formula (V) or the pharmaceutically acceptable salt thereof, wherein each R^{d} is the same or different, and is independently selected from halogen, C₁₋₆ alkyl, C₁₋₆ haloalkyl, C₁₋₆ hydroxyalkyl, C₁₋₆ alkoxy and C₁₋₆ haloalkoxy; in some embodiments, each R^{d} is the same or different, and is independently selected from C₁₋₆ alkyl, C₁₋₆ haloalkyl, C₁₋₆ hydroxyalkyl, C₁₋₆ alkoxy and C₁₋₆ haloalkoxy; in some embodiments, each R^{d} is the same or different, and is independently selected from halogen, C₁₋₆ alkyl, C₁₋₆ haloalkyl and C₁₋₆ alkoxy; in some embodiments, each R^{d} is the same or different, and is independently selected from halogen, C₁₋₆ alkyl and C₁₋₆ haloalkyl; in some embodiments, each R^{d} is the same or different, and is independently selected from C₁₋₆ alkyl, C₁₋₆ haloalkyl and C₁₋₆ alkoxy; in some embodiments, each R^{d} is the same or different, and is independently C₁₋₆ alkyl or halogen; in some embodiments, each R^{d} is the same or different, and is independently C₁₋₆ alkyl; in some embodiments, R^{d} is methyl.

In some embodiments of the present disclosure, provided is the compound as represented by general formula (I), general formula (I-1), general formula (II), general formula (II-1), general formula (IV) or general formula (V) or the pharmaceutically acceptable salt thereof, wherein each R^{2a} is the same or different, and is independently selected from halogen, C₁₋₆ alkyl, C₁₋₆ haloalkyl, C₁₋₆ alkoxy and C₁₋₆ hydroxyalkyl; in some embodiments, each R^{2a} is the same or different, and is independently halogen; in some embodiments, R^{2a} is F.

In some embodiments of the present disclosure, provided is the compound as represented by general formula (I), general formula (I-1), general formula (II), general formula (II-1), general formula (IV) or general formula (V) or the pharmaceutically acceptable salt thereof, wherein m is 0, 1 or 2; in some embodiments, m is 0 or 1; in some embodiments, m is 1.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{4a} is selected from a hydrogen atom, halogen, C₁₋₆ alkyl, C₁₋₆ haloalkyl, C₁₋₆ alkoxy and C₁₋₆ hydroxyalkyl; in some embodiments, R^{4a} is C₁₋₆ alkyl or C₁₋₆ hydroxyalkyl; in some embodiments, R^{4a} is C₁₋₆ alkyl; in some embodiments, R^{4a} is methyl; in some embodiments, R^{4a} is C₁₋₆ hydroxyalkyl; in some embodiments, R^{4a} is methyl or hydroxymethyl; in some embodiments, R^{4a} is hydroxymethyl.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{4b} is selected from a hydrogen atom, halogen, C₁₋₆ alkyl, C₁₋₆ haloalkyl, C₁₋₆ alkoxy and C₁₋₆ hydroxyalkyl; in some embodiments, R^{4b} is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{4c} is selected from a hydrogen atom, halogen, C₁₋₆ alkyl, C₁₋₆ haloalkyl, C₁₋₆ alkoxy and C₁₋₆ hydroxyalkyl; in some embodiments, R^{4c} is a hydrogen atom or halogen; in some embodiments, R^{4c} is halogen; in some embodiments, R^{4c} is a hydrogen atom or F; in some embodiments, R^{4c} is a hydrogen atom; in some embodiments, R^{4c} is F.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{4d} is selected from a hydrogen atom, halogen, C₁₋₆ alkyl, C₁₋₆ haloalkyl, C₁₋₆ alkoxy and C₁₋₆ hydroxyalkyl; in some embodiments, R^{4d} is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{4a}, R^{4b}, R^{4c} and R^{4d} are the same or different, and are each independently selected from a hydrogen atom, halogen, C₁₋₆ alkyl, C₁₋₆ haloalkyl, C₁₋₆ alkoxy and C₁₋₆ hydroxyalkyl; in some embodiments, R^{4a} is C₁₋₆ alkyl or C₁₋₆ hydroxyalkyl; R^{4b} is a hydrogen atom; R^{4c} is a hydrogen atom or halogen; R^{4d} is a hydrogen atom; in some embodiments, R^{4a} is C₁₋₆ alkyl; R^{4b} is a hydrogen atom; R^{4c} is halogen; R^{4d} is a hydrogen atom; in some embodiments, R^{4a} is methyl or hydroxymethyl; R^{4b} is a hydrogen atom; R^{4c} is a hydrogen atom or F; R^{4d} is a hydrogen atom; in some embodiments, R^{4a} is methyl; R^{4b} is a hydrogen atom; R^{4c} is F; R^{4d} is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R³ is selected from a hydrogen atom, C₁₋₆ alkyl and cyclopropyl; in some embodiments, R³ is a hydrogen atom or C₁₋₆ alkyl; in some embodiments, R³ is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{5c} is selected from a hydrogen atom, halogen and C₁₋₆ alkyl; in some embodiments, R^{5c} is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein B³ is CH or N; in some embodiments, B³ is N.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R⁶ is -NHR⁷; R⁷ is as defined in general formula (I); in some embodiments, R⁶ is amino. In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R⁷ is a hydrogen atom or C₁₋₆ alkyl; in some embodiments, R⁷ is a hydrogen atom.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein n is 0 or 1; in some embodiments, n is 0.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹¹, R¹², R¹³ and R¹⁴ are the same or different, and are each independently selected from a hydrogen atom, halogen, C₁₋₆ alkyl and C₁₋₆ haloalkyl; in some embodiments, R¹¹, R¹², R¹³ and R¹⁴ are the same or different, and are each independently a hydrogen atom or C₁₋₆ alkyl; in some embodiments, R¹¹, R¹², R¹³ and R¹⁴ are the same or different, and are each independently a hydrogen atom or a deuterium atom; in some embodiments, R¹¹, R¹², R¹³ and R¹⁴ are hydrogen atoms.

In some embodiments of the present disclosure, provided is the compound as represented by general formula (I-1) or general formula (II-1) or the pharmaceutically acceptable salt thereof, wherein R¹¹ and R¹² are the same or different, and are each independently selected from a hydrogen atom, halogen, C₁₋₆ alkyl and C₁₋₆ haloalkyl; in some embodiments, R¹¹ and R¹² are the same or different, and are each independently a hydrogen atom or a deuterium atom; in some embodiments, R¹¹ and R¹² are the same or different, and are each independently a hydrogen atom or C₁₋₆ alkyl; in some embodiments, R¹¹ and R¹² are deuterium atoms; in some embodiments, R¹¹ and R¹² are hydrogen atoms.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹¹, R¹², R¹³ and R¹⁴ are hydrogen atoms; and/or n is 0.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{x} and R^{y} are the same or different, and are each independently selected from a hydrogen atom, halogen, C₁₋₆ alkyl and C₁₋₆ haloalkyl; in some embodiments, R^{x} and R^{y} are the same or different, and are each independently a hydrogen atom or C₁₋₆ alkyl; in some embodiments, R^{x} and R^{y} are hydrogen atoms.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹⁵ is selected from a hydrogen atom, C₁₋₆ alkyl, C₁₋₆ deuterioalkyl, C₁₋₆ haloalkyl and 3- to 6-membered cycloalkyl; in some embodiments, R¹⁵ is selected from C₁₋₆ alkyl, C₁₋₆ deuterioalkyl and C₁₋₆ haloalkyl; in some embodiments, R¹⁵ is selected from a hydrogen atom, C₁₋₆ alkyl, C₁₋₆ deuterioalkyl and 3- to 6-membered cycloalkyl; in some embodiments, R¹⁵ is selected from a hydrogen atom, C₁₋₆ alkyl and 3- to 6-membered cycloalkyl; in some embodiments, R¹⁵ is C₁₋₆ alkyl or 3- to 6-membered cycloalkyl; in some embodiments, R¹⁵ is C₁₋₆ alkyl or C₁₋₆ deuterioalkyl; in some embodiments, R¹⁵ is C₁₋₆ alkyl; in some embodiments, R¹⁵ is methyl or -CD₃; in some embodiments, R¹⁵ is methyl or cyclopropyl; in some embodiments, R¹⁵ is methyl; in some embodiments, R¹⁵ is -CD₃; in some embodiments, R¹⁵ is cyclopropyl; in some embodiments, R¹⁵ is selected from methyl, -CD₃, ethyl and -CH₂CH₂F.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹⁴ and R¹⁵ together with the atoms to which they are respectively attached form 4- to 7-membered nitrogen-containing heterocyclyl, wherein the 4- to 7-membered nitrogen-containing heterocyclyl is optionally substituted with one or more R⁰; R⁰ is as defined in general formula (I); in some embodiments, R¹⁴ and R¹⁵ together with the atoms to which they are respectively attached form 5-membered nitrogen-containing heterocyclyl, wherein the 5-membered nitrogen-containing heterocyclyl is optionally substituted with one or more R⁰; R⁰ is the same or different, and is independently halogen or C₁₋₆ alkoxy.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹³ is a hydrogen atom; R¹⁴ and R¹⁵ together with the atoms to which they are respectively attached form 4- to 7-membered nitrogen-containing heterocyclyl, wherein the 4- to 7-membered nitrogen-containing heterocyclyl is optionally substituted with one or more R⁰; R⁰ is as defined in general formula (I); in some embodiments, R¹³ is a hydrogen atom; R¹⁴ and R¹⁵ together with the atoms to which they are respectively attached form 5-membered nitrogen-containing heterocyclyl, wherein the 5-membered nitrogen-containing heterocyclyl is optionally substituted with one or more R⁰; R⁰ is as defined in general formula (I); in some embodiments, R¹³ is a hydrogen atom; R¹⁴ and R¹⁵ together with the atoms to which they are respectively attached form pyrrolidyl, wherein the pyrrolidyl is optionally substituted with one or more R⁰; R⁰ is as defined in general formula (I); in some embodiments, R¹³ is a hydrogen atom; R¹⁴ and R¹⁵ together with the atoms to which they are respectively attached form pyrrolidyl.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹¹, R¹², R¹³, R¹⁴, R^{x} and R^{y} are the same or different, and are each independently selected from a hydrogen atom, halogen, C₁₋₆ alkyl and C₁₋₆ haloalkyl; R¹⁵ is selected from a hydrogen atom, C₁₋₆ alkyl and 3- to 6-membered cycloalkyl; or R¹¹, R¹², R^{x} and R^{y} are the same or different, and are each independently selected from a hydrogen atom, halogen, C₁₋₆ alkyl and C₁₋₆ haloalkyl; R¹³ is a hydrogen atom; R¹⁴ and R¹⁵ together with the atoms to which they are respectively attached form 4- to 7-membered nitrogen-containing heterocyclyl, wherein the 4- to 7-membered nitrogen-containing heterocyclyl is optionally substituted with one or more R⁰; R⁰ is as defined in general formula (I);
in some embodiments, R¹¹, R¹², R¹³, R¹⁴, R^{x} and R^{y} are the same or different, and are each independently a hydrogen atom or C₁₋₆ alkyl; R¹⁵ is C₁₋₆ alkyl or 3- to 6-membered cycloalkyl; or R¹¹, R¹², R^{x} and R^{y} are the same or different, and are each independently a hydrogen atom or C₁₋₆ alkyl; R¹³ is a hydrogen atom; R¹⁴ and R¹⁵ together with the atoms to which they are respectively attached form 5-membered nitrogen-containing heterocyclyl, wherein the 5-membered nitrogen-containing heterocyclyl is optionally substituted with one or more R⁰; R⁰ is as defined in general formula (I);
in some embodiments, R¹¹, R¹², R¹³, R¹⁴, R^{x} and R^{y} are hydrogen atoms; R¹⁵ is C₁₋₆ alkyl or 3- to 6-membered cycloalkyl; or R¹¹, R¹², R^{x} and R^{y} are hydrogen atoms; R¹³ is a hydrogen atom; R¹⁴ and R¹⁵ together with the atoms to which they are respectively attached form 5-membered nitrogen-containing heterocyclyl;
in some embodiments, R¹¹, R¹², R¹³, R¹⁴, R^{x} and R^{y} are hydrogen atoms; R¹⁵ is C₁₋₆ alkyl; or R¹¹, R¹², R^{x} and R^{y} are hydrogen atoms; R¹³ is a hydrogen atom; R¹⁴ and R¹⁵ together with the atoms to which they are respectively attached form pyrrolidyl.

In some embodiments of the present disclosure, provided is the compound as represented by general formula (I), general formula (II), general formula (III), general formula (IV), general formula (V), general formula (VI) or general formula (VI-1) or the pharmaceutically acceptable salt thereof, wherein R¹¹, R¹², R¹³ and R¹⁴ are each independently a hydrogen atom or a deuterium atom; R¹⁵ is selected from a hydrogen atom, C₁₋₆ alkyl, C₁₋₆ deuterioalkyl and C₁₋₆ haloalkyl; or R¹⁴ and R¹⁵ together with the atoms to which they are respectively attached form 4- to 7-membered nitrogen-containing heterocyclyl, wherein the 4- to 7-membered nitrogen-containing heterocyclyl is optionally substituted with one or more R⁰; R⁰ is the same or different, and is independently halogen or C₁₋₆ alkoxy; in some embodiments, R¹¹, R¹², R¹³ and R¹⁴ are hydrogen atoms; R¹⁵ is C₁₋₆ alkyl or C₁₋₆ deuterioalkyl; in some embodiments, R¹¹, R¹², R¹³ and R¹⁴ are hydrogen atoms; R¹⁵ is C₁₋₆ alkyl; in some embodiments, R¹¹, R¹², R¹³ and R¹⁴ are hydrogen atoms; R¹⁵ is methyl.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R¹⁶ is C₂₋₆ alkenyl, wherein the C₂₋₆ alkenyl is optionally substituted with one or more substituents selected from halogen, C₁₋₆ alkyl, C₁₋₆ alkoxy and -NR¹⁸R¹⁹; R¹⁸ and R¹⁹ are each independently a hydrogen atom or C₁₋₆ alkyl; in some embodiments, R¹⁶ is C₂₋₆ alkenyl; in some embodiments, R¹⁶ is -CH=CH₂.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R⁰ at each occurrence is the same or different, and is independently selected from halogen, C₁₋₆ alkyl, C₁₋₆ haloalkyl, C₁₋₆ alkoxy, C₁₋₆ haloalkoxy and C₁₋₆ hydroxyalkyl; in some embodiments, R⁰ at each occurrence is the same or different, and is independently selected from halogen, C₁₋₆ alkyl and C₁₋₆ haloalkyl; in some embodiments, R⁰ at each occurrence is the same or different, and is independently halogen or C₁₋₆ alkoxy; in some embodiments, R⁰ at each occurrence is the same or different, and is independently F or methoxy.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R⁰¹ at each occurrence is the same or different, and is independently selected from halogen, C₁₋₆ alkyl, C₁₋₆ haloalkyl, C₁₋₆ alkoxy, C₁₋₆ haloalkoxy and C₁₋₆ hydroxyalkyl; in some embodiments, R⁰¹ at each occurrence is the same or different, and is independently selected from halogen, C₁₋₆ alkyl and C₁₋₆ haloalkyl.

In some embodiments of the present disclosure, provided is the compound as represented by general formula (I-1) or general formula (II-1) or the pharmaceutically acceptable salt thereof, wherein p is 0 or 1; in some embodiments, p is 0; in some embodiments, p is 1.

In some embodiments of the present disclosure, provided is the compound as represented by general formula (I-1) or the pharmaceutically acceptable salt thereof, wherein ring D is 4- to 7-membered nitrogen-containing heterocyclyl; in some embodiments, ring D is 5-membered nitrogen-containing heterocyclyl; in some embodiments, ring D is pyrrolidyl.

In some embodiments of the present disclosure, provided is the compound as represented by general formula (II-1) or the pharmaceutically acceptable salt thereof, wherein x is 2 or 3; in some embodiments, x is 2.

In some embodiments of the present disclosure, provided is the compound as represented by general formula (I), general formula (I-1), general formula (II), general formula (II-1) or general formula (III) or the pharmaceutically acceptable salt thereof, wherein r is 0; in some embodiments, r is 1.

In some embodiments of the present disclosure, provided is the compound as represented by general formula (I), general formula (I-1), general formula (II), general formula (II-1) or general formula (III) or the pharmaceutically acceptable salt thereof, wherein t is 1.

In some embodiments of the present disclosure, provided is the compound as represented by general formula (I), general formula (I-1), general formula (II), general formula (II-1) or general formula (III) or the pharmaceutically acceptable salt thereof, wherein z is 1.

In some embodiments of the present disclosure, provided is the compound as represented by general formula (I), general formula (I-1), general formula (II), general formula (II-1) or general formula (III) or the pharmaceutically acceptable salt thereof, wherein f is 1 or 2; in some embodiments, f is 1.

In some embodiments of the present disclosure, provided is the compound as represented by general formula (I), general formula (I-1), general formula (II), general formula (II-1) or general formula (III) or the pharmaceutically acceptable salt thereof, wherein j is 0, 1 or 2; in some embodiments, j is 0 or 1; in some embodiments, j is 1 or 2; in some embodiments, j is 1; in some embodiments, j is 0.

In some embodiments of the present disclosure, provided is the compound as represented by general formula (I), general formula (I-1), general formula (II), general formula (II-1) or general formula (III) or the pharmaceutically acceptable salt thereof, wherein R^{1b} and R^{1c} are the same or different, and are each independently selected from a hydrogen atom, halogen and C₁₋₆ alkyl; in some embodiments, R^{1b} and R^{1c} are hydrogen atoms.

In some embodiments of the present disclosure, provided is the compound as represented by general formula (I), general formula (I-1), general formula (II), general formula (II-1) or general formula (III) or the pharmaceutically acceptable salt thereof, wherein A¹ is selected from a bond, O and CH₂; A² is selected from a bond, O and CR^{1b}R^{1c}; R^{1b} and R^{1c} are hydrogen atoms; or R^{1b} and R^{1c} together with the carbon atoms to which they are attached form 3- to 6-membered cycloalkyl; in some embodiments, A¹ is selected from a bond, O and CH₂; A² is selected from a bond, O and CR^{1b}R^{1c}; R^{1b} and R^{1c} are hydrogen atoms; or R^{1b} and R^{1c} together with the carbon atoms to which they are attached form cyclopropyl; in some embodiments, A¹ and A² are the same or different, and are each independently selected from a bond, O and CH₂; in some embodiments, A¹ is CH₂ or O, and A² is a bond; in some embodiments, A¹ is CH₂, and A² is a bond; in some embodiments, A¹ is O, and A² is a bond; in some embodiments, A¹ is O, and A² is O; in some embodiments, A¹ is a bond, and A² is CH₂ or O; in some embodiments, A¹ is a bond, and A² is O; in some embodiments, A¹ is a bond, and A² is CH₂; in some embodiments, A¹ is O or CH₂; A² is a bond; or A² is O or CH₂; A¹ is a bond.

In some embodiments of the present disclosure, provided is the compound as represented by general formula (I), general formula (I-1), general formula (II), general formula (II-1) or general formula (III) or the pharmaceutically acceptable salt thereof, wherein A² is selected from a bond, O and CR^{1b}R^{1c}, R^{1b} and R^{1c} are hydrogen atoms; or R^{1b} and R^{1c} together with the carbon atoms to which they are attached form cyclopropyl; in some embodiments, A² is a bond or O; in some embodiments, A² is a bond; in some embodiments, A² is CH₂ or O;

In some embodiments of the present disclosure, provided is the compound as represented by general formula (I), general formula (I-1), general formula (II), general formula (II-1) or general formula (III) or the pharmaceutically acceptable salt thereof, wherein in some embodiments, A¹ is O; in some embodiments, A¹ is a bond; in some embodiments, A¹ is CH₂ or O.

In some embodiments of the present disclosure, provided is the compound as represented by general formula (I), general formula (I-1), general formula (II), general formula (II-1) or general formula (III) or the pharmaceutically acceptable salt thereof, wherein R^{e}, R^{f}, R^{j} and R^{k} are the same or different, and are each independently selected from a hydrogen atom, halogen and C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; or R^{f} and R^{j} together with the carbon atoms to which they are attached form 4- to 6-membered cycloalkyl; in some embodiments, R^{e}, R^{f}, R^{j} and R^{k} are hydrogen atoms; or R^{e} and R^{f} together with the carbon atoms to which they are attached form cyclopropyl; or R^{j} and R^{k} together with the carbon atoms to which they are attached form cyclopropyl; or R^{f} and R^{j} together with the carbon atoms to which they are attached form cyclobutyl; in some embodiments, R^{e}, R^{f}, R^{j} and R^{k} are hydrogen atoms or C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; in some embodiments, R^{e}, R^{f}, R^{j} and R^{k} are hydrogen atoms or methyl; or R^{e} and R^{f} together with the carbon atoms to which they are attached form cyclopropyl; or R^{j} and R^{k} together with the carbon atoms to which they are attached form cyclopropyl; in some embodiments, R^{e} and R^{f} are C₁₋₆ alkyl; or R^{e} and R^{f} together with the carbon atoms to which they are attached form 3- to 6-membered cycloalkyl; R^{j} and R^{k} are hydrogen atoms; in some embodiments, R^{j} and R^{k} are C₁₋₆ alkyl; or R^{j} and R^{k} together with the carbon atoms to which they are attached form 3- to 6-membered cycloalkyl; R^{e} and R^{f} are hydrogen atoms.

In some embodiments of the present disclosure, provided is the compound as represented by general formula (I), general formula (I-1), general formula (II), general formula (II-1) or general formula (III) or the pharmaceutically acceptable salt thereof, wherein r is 0 or 1; R^{e} and R^{f} together with the carbon atoms to which they are attached form 3- to 6-membered cycloalkyl, and R^{j} and R^{k} are hydrogen atoms; or r is 1; R^{j} and R^{k} together with the carbon atoms to which they are attached form 3- to 6-membered cycloalkyl, and R^{e} and R^{f} are hydrogen atoms; or r is 1, R^{f} and R^{j} together with the carbon atoms to which they are attached form 3- to 6-membered cycloalkyl, and R^{e} and R^{k} are hydrogen atoms; in some embodiments, r is 0 or 1, R^{e} and R^{f} together with the carbon atoms to which they are attached form cyclopropyl, and R^{j} and R^{k} are hydrogen atoms; or r is 1, R^{j} and R^{k} together with the carbon atoms to which they are attached form cyclopropyl, and R^{e} and R^{f} are hydrogen atoms; or r is 1, R^{f} and R^{j} together with the carbon atoms to which they are attached form cyclobutyl, and R^{e} and R^{k} are hydrogen atoms; in some embodiments, r is 1, R^{j} and R^{k} together with the carbon atoms to which they are attached form cyclopropyl, and R^{e} and R^{f} are hydrogen atoms.

In some embodiments of the present disclosure, provided is the compound as represented by general formula (I), general formula (I-1), general formula (II), general formula (II-1) or general formula (III) or the pharmaceutically acceptable salt thereof, wherein R^{e} and R^{f} are each independently a hydrogen atom or C₁₋₆ alkyl; or R^{e} and R^{f} together with the carbon atoms to which they are attached form 3- to 6-membered cycloalkyl; in some embodiments, R^{e} and R^{f} together with the carbon atoms to which they are attached form 3- to 6-membered cycloalkyl; in some embodiments, R^{e} and R^{f} together with the carbon atoms to which they are attached form cyclopropyl; in some embodiments, R^{e} and R^{f} together with the carbon atoms to which they are attached form 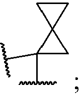 in some embodiments, R^{e} and R^{f} are each independently C₁₋₆ alkyl; in some embodiments, R^{e} and R^{f} are methyl; in some embodiments, R^{e} and R^{f} are hydrogen atoms.

In some embodiments of the present disclosure, provided is the compound as represented by general formula (I), general formula (I-1), general formula (II), general formula (II-1) or general formula (III) or the pharmaceutically acceptable salt thereof, wherein R^{j} and R^{k} are each independently a hydrogen atom or C₁₋₆ alkyl; or R^{j} and R^{k} together with the carbon atoms to which they are attached form 3- to 6-membered cycloalkyl; in some embodiments, R^{j} and R^{k} together with the carbon atoms to which they are attached form 3- to 6-membered cycloalkyl; in some embodiments, R^{j} and R^{k} together with the carbon atoms to which they are attached form cyclopropyl; in some embodiments, R^{j} and R^{k} together with the carbon atoms to which they are attached form 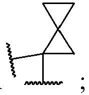 in some embodiments, R^{j} and R^{k} are each independently C₁₋₆ alkyl; in some embodiments, R^{j} and R^{k} are methyl; in some embodiments, R^{j} and R^{k} are hydrogen atoms.

In some embodiments of the present disclosure, provided is the compound as represented by general formula (I), general formula (I-1), general formula (II), general formula (II-1) or general formula (III) or the pharmaceutically acceptable salt thereof, wherein R^{e}, R^{f}, R^{j} and R^{k} are the same or different, and are each independently a hydrogen atom or C₁₋₆ alkyl; or R^{e} and R^{f} together with the carbon atoms to which they are attached form cyclopropyl; or R^{j} and R^{k} together with the carbon atoms to which they are attached form cyclopropyl; r is 0 or 1; A¹ is selected from a bond, O and CH₂; A² is selected from a bond, O and CR^{1b}R^{1c}; R^{1b} and R^{1c} are hydrogen atoms; or R^{1b} and R^{1c} together with the carbon atoms to which they are attached form cyclopropyl.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R¹⁷, R¹⁸, R¹⁹ and R²⁰ at each occurrence are the same or different, and are each independently a hydrogen atom or C₁₋₆ alkyl.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein v is 0 or 1; in some embodiments, v is 0.

In some embodiments of the present disclosure, provided is the compound as represented by general formula (I), general formula (I-1), general formula (II), general formula (II-1), general formula (III), general formula (IV), general formula (V), general formula (VI) or general formula (VI-1) or the pharmaceutically acceptable salt thereof, wherein R^{a} and R^{b} are the same or different, and are each independently selected from a hydrogen atom, halogen and C₁₋₆ alkyl; in some embodiments, R^{a} and R^{b} are hydrogen atoms.

In some embodiments of the present disclosure, G is 5- or 6-membered heteroaryl optionally substituted with one or more R^{G}; in some embodiments, G is 5-membered heteroaryl optionally substituted with one or more R^{G}; in some embodiments, G is thienyl optionally substituted with one or more R^{G}; in some embodiments, G is 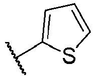 optionally substituted with one or more R^{G}; in some embodiments, G is

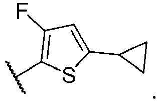

In some embodiments of the present disclosure, provided is the compound as represented by general formula (I) or the pharmaceutically acceptable salt thereof, wherein G is selected from 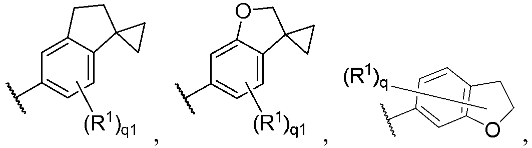 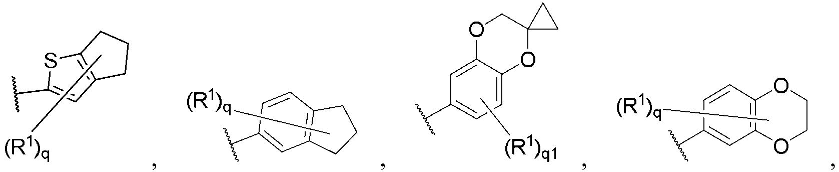 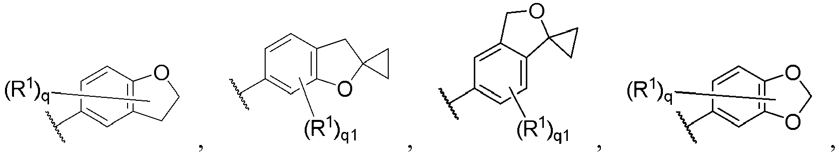 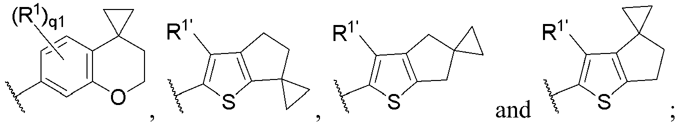 R¹' is selected from a hydrogen atom, halogen, C₁₋₆ alkyl, C₁₋₆ haloalkyl, C₁₋₆ alkoxy and C₁₋₆ hydroxyalkyl; each R¹ is the same or different, and is independently selected from halogen, C₁₋₆ alkyl, C₁₋₆ haloalkyl, C₁₋₆ alkoxy and C₁₋₆ hydroxyalkyl; q1 is 0 or 1; q is 0, 1, 2, 3 or 4; R³ is a hydrogen atom; X¹ is CR^{4a}; R^{4a} is C₁₋₆ alkyl or C₁₋₆ hydroxyalkyl; X² is N or CH; X³ is CR^{4c}; R^{4c} is a hydrogen atom or halogen; X⁴ is CH; B¹ is N; B² is CH; B³ is N; L is O; R⁶ is amino; n is 0; R¹¹, R¹², R¹³ and R¹⁴ are each independently a hydrogen atom or a deuterium atom; R¹⁵ is selected from a hydrogen atom, C₁₋₆ alkyl, C₁₋₆ deuterioalkyl and C₁₋₆ haloalkyl; or R¹⁴ and R¹⁵ together with the atoms to which they are respectively attached form 4- to 7-membered nitrogen-containing heterocyclyl, wherein the 4- to 7-membered nitrogen-containing heterocyclyl is optionally substituted with one or more R⁰; R⁰ is the same or different, and is independently halogen or C₁₋₆ alkoxy; W is -C(O)-CH=CH₂.

In some embodiments of the present disclosure, provided is the compound as represented by general formula (II) or the pharmaceutically acceptable salt thereof, wherein G is selected from 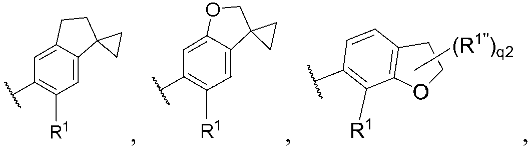 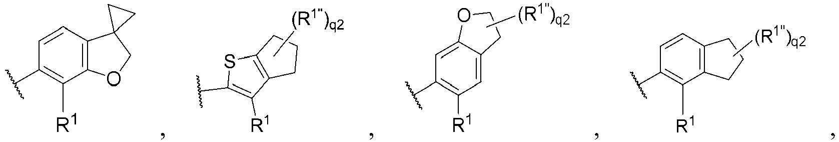 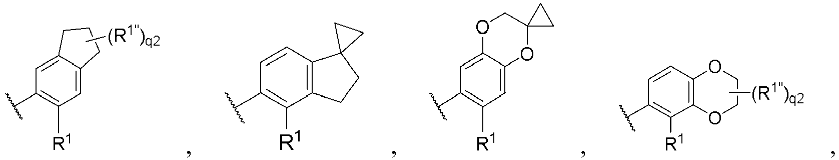 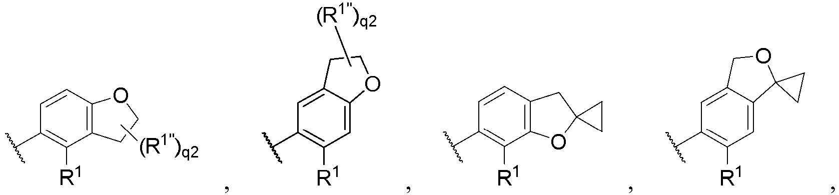 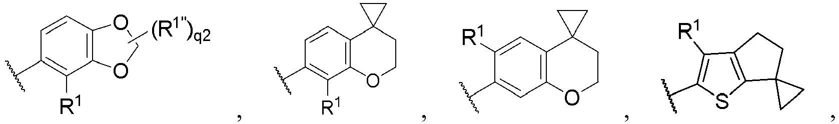 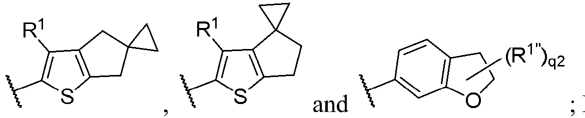 R¹ is halogen; R¹" is C₁₋₆ alkyl; q2 is 0, 1, 2 or 3; R³ is a hydrogen atom; R^{4a} is C₁₋₆ alkyl or C₁₋₆ hydroxyalkyl; R^{4b} is a hydrogen atom; R^{4c} is a hydrogen atom or halogen; R^{4d} is a hydrogen atom; B³ is N; R⁶ is amino; n is O; R¹¹, R¹², R¹³ and R¹⁴ are each independently a hydrogen atom or a deuterium atom; R¹⁵ is selected from C₁₋₆ alkyl, C₁₋₆ deuterioalkyl and C₁₋₆ haloalkyl; or R¹⁴ and R¹⁵ together with the atoms to which they are respectively attached form 5-membered nitrogen-containing heterocyclyl, wherein the 5-membered nitrogen-containing heterocyclyl is optionally substituted with one or more R⁰; R⁰ is the same or different, and is independently halogen or C₁₋₆ alkoxy; R¹⁶ is -CH=CH₂.

In some embodiments of the present disclosure, provided is the compound as represented by general formula (I) or the pharmaceutically acceptable salt thereof, wherein G is 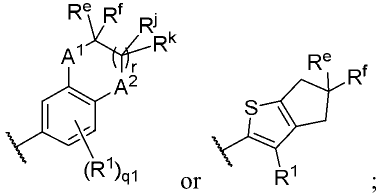 r is 1; q1 is 1; R¹ is halogen; A¹ and A² are the same or different, and are each independently selected from a bond, O and CH₂; R^{e}, R^{f}, R^{j} and R^{k} are the same or different, and are each independently a hydrogen atom or C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; R³ is a hydrogen atom; X¹ is CR^{4a}; R^{4a} is C₁₋₆ alkyl or C₁₋₆ hydroxyalkyl; X² is N or CH; X³ is CR^{4c}; R^{4c} is a hydrogen atom or halogen; X⁴ is CH; B¹ is N; B² is CH; B³ is N; R⁶ is amino; L is O; R¹¹, R¹², R¹³ and R¹⁴ are hydrogen atoms; R¹⁵ is C₁₋₆ alkyl or C₁₋₆ deuterioalkyl; n is 0; W is -C (O)-CH=CH₂.

In some embodiments of the present disclosure, provided is the compound as represented by general formula (I) or the pharmaceutically acceptable salt thereof, wherein G is 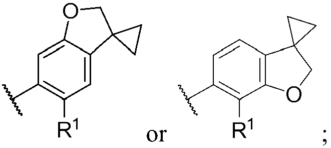 R¹ is halogen; R³ is a hydrogen atom; X¹ is CR^{4a}; R^{4a} is C₁₋₆ alkyl; X² is CH; X³ is CR^{4c}; R^{4c} is halogen; X⁴ is CH; B¹ is N; B² is CH; B³ is N; R⁶ is amino; L is O; R¹¹, R¹², R¹³ and R¹⁴ are hydrogen atoms; R¹⁵ is C₁₋₆ alkyl; n is 0; W is -C (O)-CH=CH₂.

In some embodiments of the present disclosure, provided is the compound as represented by general formula (II) or the pharmaceutically acceptable salt thereof, wherein G is 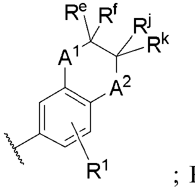 R¹ is halogen; R^{e}, R^{f}, R^{j} and R^{k} are the same or different, and are each independently a hydrogen atom or C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; A¹ and A² are the same or different, and are each independently selected from a bond, O and CH₂; R³ is a hydrogen atom; R^{4a} is C₁₋₆ alkyl or C₁₋₆ hydroxyalkyl; R^{4b} is a hydrogen atom; R^{4c} is a hydrogen atom or halogen; R^{4d} is a hydrogen atom; B³ is N; R⁶ is amino; R¹¹, R¹², R¹³ and R¹⁴ are hydrogen atoms; R¹⁵ is C₁₋₆ alkyl or C₁₋₆ deuterioalkyl; n is 0; R¹⁶ is -CH=CH₂.

In some embodiments of the present disclosure, provided is the compound as represented by general formula (II) or the pharmaceutically acceptable salt thereof, wherein G is 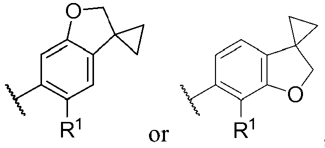 ; R¹ is halogen; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; R¹⁵ is methyl or -CD₃; n is 0; R¹⁶ is -CH=CH₂.

In some embodiments of the present disclosure, provided is the compound as represented by general formula (III) or the pharmaceutically acceptable salt thereof, wherein R¹ is halogen; q1 is 1; A¹ and A² are the same or different, and are each independently selected from a bond, O and CH₂; r is 1; R^{e}, R^{f}, R^{j} and R^{k} are the same or different, and are each independently a hydrogen atom or C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; R³ is a hydrogen atom; R^{4a} is C₁₋₆ alkyl or C₁₋₆ hydroxyalkyl; R^{4b} is a hydrogen atom; R^{4c} is a hydrogen atom or halogen; R^{4d} is a hydrogen atom; B³ is N; R⁶ is amino; R¹¹, R¹², R¹³ and R¹⁴ are hydrogen atoms; R¹⁵ is C₁₋₆ alkyl or C₁₋₆ deuterioalkyl; n is 0; R¹⁶ is -CH=CH₂.

In some embodiments of the present disclosure, provided is the compound as represented by general formula (III) or the pharmaceutically acceptable salt thereof, wherein R¹ is halogen; q1 is 1; A¹ is O or CH₂; A² is a bond; or A² is O or CH₂; A¹ is a bond; r is 1; R^{e}, R^{f}, R^{j} and R^{k} are the same or different, and are each independently a hydrogen atom or methyl; or R^{e} and R^{f} together with the carbon atoms to which they are attached form cyclopropyl; or R^{j} and R^{k} together with the carbon atoms to which they are attached form cyclopropyl; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; R¹⁵ is C₁₋₆ alkyl; n is 0; R¹⁶ is -CH=CH₂.

In some embodiments of the present disclosure, provided is the compound as represented by general formula (II) or the pharmaceutically acceptable salt thereof, wherein G is 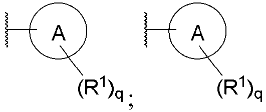 is 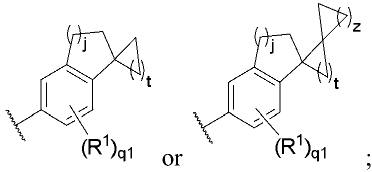 q1 is 1; R¹ is halogen; j is 1; t is 1; z is 1; R³ is a hydrogen atom; R^{4a} is C₁₋₆ alkyl; R^{4b} is a hydrogen atom; R^{4c} is halogen; R^{4d} is a hydrogen atom; B³ is CH or N; R⁶ is amino; R¹¹, R¹², R¹³ and R¹⁴ are hydrogen atoms; n is 0; R¹⁵ is selected from a hydrogen atom, C₁₋₆ alkyl and 3- to 6-membered cycloalkyl; R¹⁶ is C₂₋₆ alkenyl.

In some embodiments of the present disclosure, provided is the compound as represented by general formula (II) or the pharmaceutically acceptable salt thereof, wherein G is 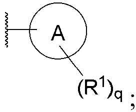 ring A is selected from 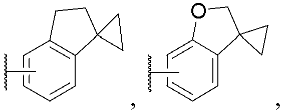 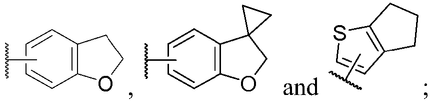 each R¹ is the same or different, and is independently selected from halogen, C₁₋₆ alkyl, C₁₋₆ haloalkyl, C₁₋₆ alkoxy, C₁₋₆ hydroxyalkyl, C₁₋₆ haloalkoxy, C₁₋₆ alkoxy C₁₋₆ alkyl, 3- to 6-membered cycloalkyl, 3- to 6-membered cycloalkyloxy and cyano; q is 0, 1, 2 or 3; R^{4a} is C₁₋₆ alkyl; R^{4b} is a hydrogen atom; R^{4c} is halogen; R^{4d} is a hydrogen atom; B³ is CH or N; R⁶ is amino; R¹¹, R¹², R¹³ and R¹⁴ are hydrogen atoms; n is 0; R¹⁵ is selected from a hydrogen atom, C₁₋₆ alkyl and 3- to 6-membered cycloalkyl; R¹⁶ is C₂₋₆ alkenyl.

In some embodiments of the present disclosure, provided is the compound as represented by general formula (II) or the pharmaceutically acceptable salt thereof, wherein G is 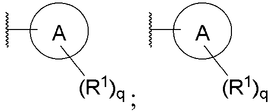 is 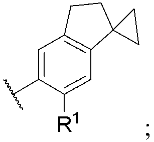 R¹ is halogen; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; n is 0; R¹⁵ is C₁₋₆ alkyl; R¹⁶ is -CH=CH₂.

In some embodiments of the present disclosure, provided is the compound as represented by general formula (III) or the pharmaceutically acceptable salt thereof, wherein r is 0 or 1; R^{e} and R^{f} together with the carbon atoms to which they are attached form cyclopropyl, and R^{j} and R^{k} are hydrogen atoms; or r is 1, R^{j} and R^{k} together with the carbon atoms to which they are attached form cyclopropyl, and R^{e} and R^{f} are hydrogen atoms; or r is 1, R^{f} and R^{j} together with the carbon atoms to which they are attached form cyclobutyl, and R^{e} and R^{k} are hydrogen atoms; A¹ and A² are the same or different, and are each independently selected from a bond, O and CH₂; q1 is 1; R¹ is halogen; R³ is a hydrogen atom; R^{4a} is C₁₋₆ alkyl; R^{4b} is a hydrogen atom; R^{4c} is halogen; R^{4d} is a hydrogen atom; B³ is CH or N; R⁶ is amino; R¹¹, R¹², R¹³ and R¹⁴ are hydrogen atoms; n is 0; R¹⁵ is selected from a hydrogen atom, C₁₋₆ alkyl and 3- to 6-membered cycloalkyl; R¹⁶ is C₂₋₆ alkenyl.

In some embodiments of the present disclosure, provided is the compound as represented by general formula (III) or the pharmaceutically acceptable salt thereof, wherein r is 1; R^{j} and R^{k} together with the carbon atoms to which they are attached form cyclopropyl; R^{e} and R^{f} are hydrogen atoms; A¹ is CH₂; A² is a bond; q1 is 1; R¹ is halogen; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; n is 0; R¹⁵ is C₁₋₆ alkyl; R¹⁶ is -CH=CH₂.

In some embodiments of the present disclosure, provided is the compound as represented by general formula (III) or the pharmaceutically acceptable salt thereof, wherein r is 0 or 1; A¹ and A² are the same or different, and are each independently selected from a bond, O and CH₂; R^{e}, R^{f}, R^{j} and R^{k} are the same or different, and are each independently a hydrogen atom or C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; q1 is 0 or 1; R¹ is halogen; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; n is 0; R¹⁵ is C₁₋₆ alkyl; R¹⁶ is-CH=CH₂.

In some embodiments of the present disclosure, provided is the compound as represented by general formula (III) or the pharmaceutically acceptable salt thereof, wherein r is 1; A¹ is CH₂ or O, and A² is a bond; R^{e}, R^{f}, R^{j} and R^{k} are the same or different, and are each independently a hydrogen atom or methyl; or R^{e} and R^{f} together with the carbon atoms to which they are attached form cyclopropyl; or R^{j} and R^{k} together with the carbon atoms to which they are attached form cyclopropyl; q1 is 1; R¹ is halogen; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; n is O; R¹⁵ is C₁₋₆ alkyl; R¹⁶ is -CH=CH₂.

In some embodiments of the present disclosure, provided is the compound as represented by general formula (V) or the pharmaceutically acceptable salt thereof, wherein R^{2a} is halogen; m is 1; b is 1; y is 1; R^{d} is selected from halogen, C₁₋₆ alkyl, C₁₋₆ haloalkyl and C₁₋₆ alkoxy; R³ is a hydrogen atom; R^{4a} is C₁₋₆ alkyl; R^{4b} is a hydrogen atom; R^{4c} is halogen; R^{4d} is a hydrogen atom; B³ is N; R⁶ is amino; R¹¹, R¹², R¹³ and R¹⁴ are hydrogen atoms; n is 0; R¹⁵ is C₁₋₆ alkyl; R¹⁶ is -CH=CH₂.

In some embodiments of the present disclosure, provided is the compound as represented by general formula (VI-1) or the pharmaceutically acceptable salt thereof, wherein 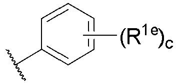 is 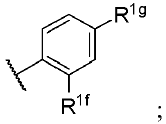 R^{1g} is 3- to 6-membered cycloalkyl; R^{1f} is halogen; X¹ is CR^{4a}; X² is N; X³ is CR^{4c}; X⁴ is CR^{4d}; R^{4a}, R^{4c} and R^{4d} are the same or different, and are independently selected from a hydrogen atom, C₁₋₆ alkyl, halogen and C₁₋₆ hydroxyalkyl; R³ is a hydrogen atom; B³ is N; R⁶ is amino; R¹¹, R¹², R¹³ and R¹⁴ are hydrogen atoms; n is 0; R¹⁵ is C₁₋₆ alkyl; R¹⁶ is-CH=CH₂.

**Table A. Typical compounds of the present disclosure, including but not limited to:**

| Compound No. | Compound structure | Name |
|---|---|---|
| 1 | 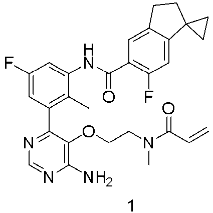 | *N*-(3-(6-amino-5-(2-(*N-*methacrylamido)ethoxy)pyrimidin-4-yl)-5-fluoro-2-methylphenyl)-6'-fluoro-2',3'-dihydrospiro[cyclopropane-1,1'-indene]-5'-carboxamide **1** |
| 2 | 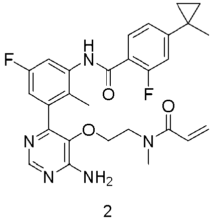 | *N*-(3-(6-amino-5-(2-(*N-*methacrylamido)ethoxy)pyrimidin-4-yl)-5-fluoro-2-methylphenyl)-2-fluoro-4-(1-methylcyclopropyl)benzamide **2** |
| | 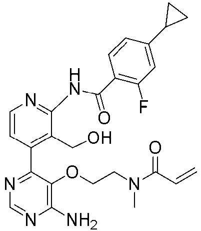 | |
| | 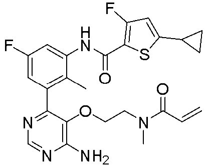 | |
| 3 | 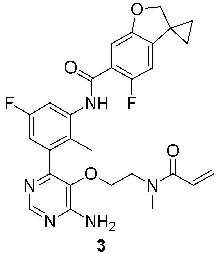 | *N*-(3-(6-amino-5-(2-(*N-*methacrylamido)ethoxy)pyrimidin-4-yl)-5-fluoro-2-methylphenyl)-5-fluoro-2*H-*spiro[benzofuran-3,1'-cyclopropane]-6-carboxamide **3** |
| 4 | 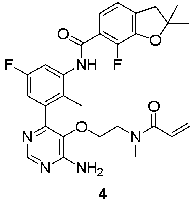 | *N*-(3-(6-amino-5-(2-(*N-*methacrylamido)ethoxy)pyrimidin-4-yl)-5-fluoro-2-methylphenyl)-7-fluoro-2,2-dimethyl-2,3-dihydrobenzofuran-6-carboxamide **4** |
| 5 | 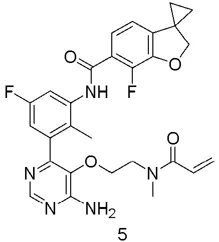 | *N*-(3-(6-amino-5-(2-(*N-*methacrylamido)ethoxy)pyrimidin-4-yl)-5-fluoro-2-methylphenyl)-7-fluoro-2*H-*spiro[benzofuran-3,1'-cyclopropane]-6-carboxamide **5** |
| 6 | 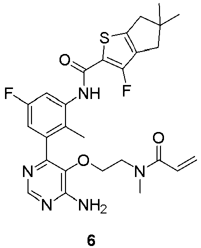 | *N*-(3-(6-amino-5-(2-(*N-*methacrylamido)ethoxy)pyrimidin-4-yl)-5-fluoro-2-methylphenyl)-3-fluoro-5,5-dimethyl-5,6-dihydro-4*H-*cyclopenta[b]thiophene-2-carboxamide 6 |
| 7 | 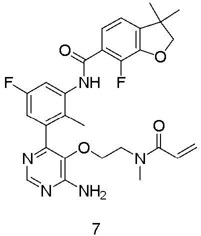 | *N*-(3-(6-amino-5-(2-(*N-*methacrylamido)ethoxy)pyrimidin-4-yl)-5-fluoro-2-methylphenyl)-7-fluoro-3,3-dimethyl-2,3-dihydrobenzofuran-6-carboxamide **7** |
| 8 | 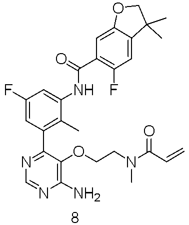 | *N*-(3-(6-amino-5-(2-(*N-*methacrylamido)ethoxy)pyrimidin-4-yl)-5-fluoro-2-methylphenyl)-5-fluoro-3,3-dimethyl-2,3-dihydrobenzofuran-6-carboxamide **8** |
| 9 | 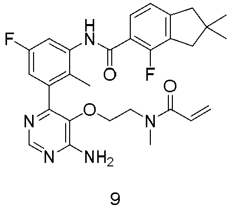 | *N*-(3-(6-amino-5-(2-(*N-*methacrylamido)ethoxy)pyrimidin-4-yl)-5-fluoro-2-methylphenyl)-4-fluoro-2,2-dimethyl-2,3-dihydro-1*H*-indene-5-carboxamide **9** |
| 10 | 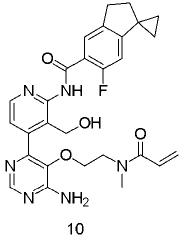 | *N*-(4-(6-amino-5-(2-(*N-*methacrylamido)ethoxy)pyrimidin-4-yl)-3-(hydroxymethyl)pyridin-2-yl)-6'-fluoro-2',3'-dihydrospiro[cyclopropane-1,1'-indene]-5'-carboxamide **10** |
| 11 | 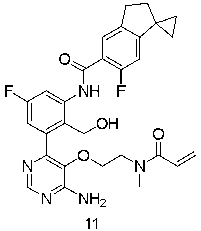 | *N*-(3-(6-amino-5-(2-(*N-*methacrylamido)ethoxy)pyrimidin-4-yl)-5-fluoro-2-(hydroxymethyl)phenyl)-6'-fluoro-2',3'-dihydrospiro[cyclopropane-1,1'-indene]-5'-carboxamide **11** |
| 12 | 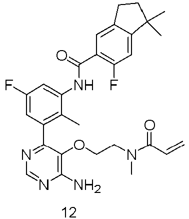 | *N*-(3-(6-amino-5-(2-(*N-*methacrylamido)ethoxy)pyrimidin-4-yl)-5-fluoro-2-methylphenyl)-6-fluoro-1,1-dimethyl-2,3-dihydro-1*H*-indene-5-carboxamide **12** |
| 13 | 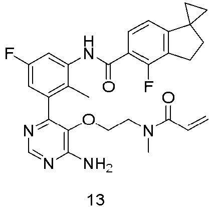 | *N*-(3-(6-amino-5-(2-(*N-*methacrylamido)ethoxy)pyrimidin-4-yl)-5-fluoro-2-methylphenyl)-4'-fluoro-2',3'-dihydrospiro[cyclopropane-1,1'-indene]-5'-carboxamide **13** |
| 14 | 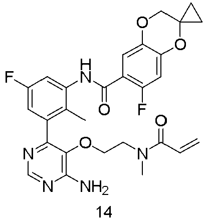 | *N*-(3-(6-amino-5-(2-(*N-*methacrylamido)ethoxy)pyrimidin-4-yl)-5-fluoro-2-methylphenyl)-7-fluoro-3*H-*spiro[benzo[*b*][1,4]dioxine-2,1'-cyclopropane]-6-carboxamide **14** |
| 15 | 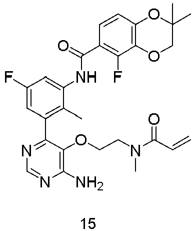 | *N*-(3-(6-amino-5-(2-(*N-*methacrylamido)ethoxy)pyrimidin-4-yl)-5-fluoro-2-methylphenyl)-5-fluoro-2,2-dimethyl-2,3-dihydrobenzo[*b*][1,4]dioxine-6-carboxamide **15** |
| 16-1 | 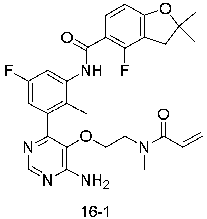 | *N*-(3-(6-amino-5-(2-(*N-*methacrylamido)ethoxy)pyrimidin-4-yl)-5-fluoro-2-methylphenyl)-4-fluoro-2,2-dimethyl-2,3-dihydrobenzofuran-5-carboxamide **16-1** |
| 16-2 | 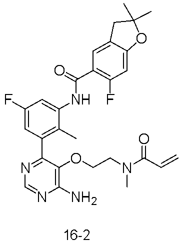 | *N*-(3-(6-amino-5-(2-(*N-*methacrylamido)ethoxy)pyrimidin-4-yl)-5-fluoro-2-methylphenyl)-6-fluoro-2,2-dimethyl-2,3-dihydrobenzofuran-5-carboxamide **16-2** |
| 17 | 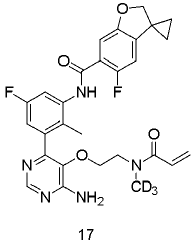 | *N*-(3-(6-amino-5-(2-(*N*-(methyl-d₃)acrylamido)ethoxy)pyrimidin-4-yl)-5-fluoro-2-methylphenyl)-5-fluoro-2*H-*spiro[benzofuran-3,1'-cyclopropane]-6-carboxamide **17** |
| 18 | 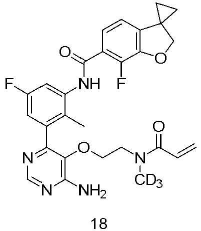 | *N*-(3-(6-amino-5-(2-(*N*-(methyl-*d*₃)acrylamido)ethoxy)pyrimidin-4-yl)-5-fluoro-2-methylphenyl)-7-fluoro-2*H-*spiro[benzofuran-3,1'-cyclopropane]-6-carboxamide **18** |
| 19 | 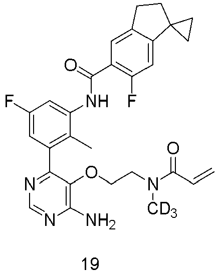 | *N*-(3-(6-amino-5-(2-(*N*-(methyl-*d*3)acrylamido)ethoxy)pyrimidin-4-yl)-5-fluoro-2-methylphenyl)-6'-fluoro-2',3'-dihydrospiro[cyclopropane-1,1'-indene]-5'-carboxamide **19** |
| 20 | 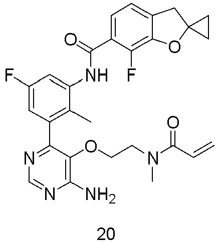 | *N*-(3-(6-amino-5-(2-(*N-*methacrylamido)ethoxy)pyrimidin-4-yl)-5-fluoro-2-methylphenyl)-7-fluoro-3H-spiro[benzofuran-2,1'-cyclopropane]-6-carboxamide **20** |
| 21 | 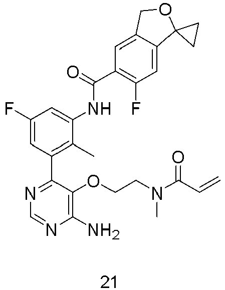 | *N*-(3-(6-amino-5-(2-(*N-*methacrylamido)ethoxy)pyrimidin-4-yl)-5-fluoro-2-methylphenyl)-6'-fluoro-3'*H-*spiro[cyclopropane-1,1'-isobenzofuran]-5'-carboxamide **21** |
| 22 | 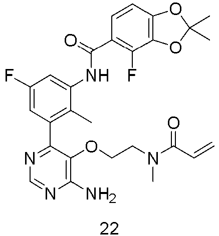 | *N*-(3-(6-amino-5-(2-(*N-*methacrylamido)ethoxy)pyrimidin-4-yl)-5-fluoro-2-methylphenyl)-4-fluoro-2,2-dimethylbenzo[*d*][1,3]dioxole-5-carboxamide **22** |
| 23 | 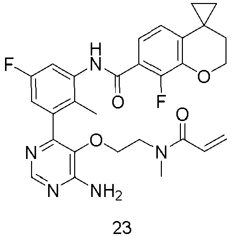 | *N*-(3-(6-amino-5-(2-(*N-*methacrylamido)ethoxy)pyrimidin-4-yl)-5-fluoro-2-methylphenyl)-8-fluorospiro[chromane-4,1'-cyclopropane]-7-carboxamide **23** |
| 24 | 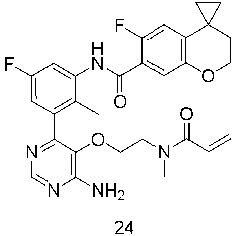 | *N*-(3-(6-amino-5-(2-(*N-*methacrylamido)ethoxy)pyrimidin-4-yl)-5-fluoro-2-methylphenyl)-6-fluorospiro[chromane-4,1'-cyclopropane]-7-carboxamide **24** |
| 25 | 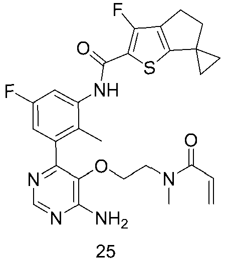 | *N*-(3-(6-amino-5-(2-(*N-*methacrylamido)ethoxy)pyrimidin-4-yl)-5-fluoro-2-methylphenyl)-3-fluoro-4,5-dihydrospiro[cyclopenta[*b*]thiophene-6,1'-cyclopropane]-2-carboxamide **25** |
| 26-1 | 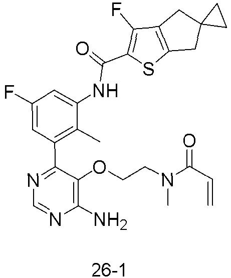 | *N*-(3-(6-amino-5-(2-(*N-*methacrylamido)ethoxy)pyrimidin-4-yl)-5-fluoro-2-methylphenyl)-3-fluoro-4,6-dihydrospiro[cyclopenta[*b*]thiophene-5,1'-cyclopropane]-2-carboxamide **26-1** |
| 26-2 | 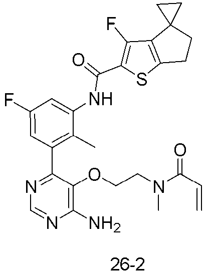 | *N*-(3-(6-amino-5-(2-(*N-*methacrylamido)ethoxy)pyrimidin-4-yl)-5-fluoro-2-methylphenyl)-3-fluoro-5,6-dihydrospiro[cyclopenta[b]thiophene-4,1'-cyclopropane]-2-carboxamide **26-2** |
| 27 | 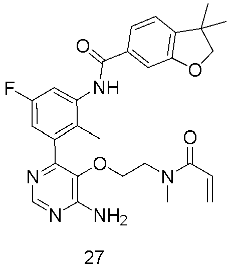 | *N*-(3-(6-amino-5-(2-(*N-*methacrylamido)ethoxy)pyrimidin-4-yl)-5-fluoro-2-methylphenyl)-3,3-dimethyl-2,3-dihydrobenzofuran-6-carboxamide **27** |
| 28 | 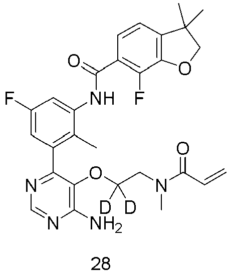 | *N*-(3-(6-amino-5-(2-(*N-*methacrylamido)ethoxy-1,1-*d*₂)pyrimidin-4-yl)-5-fluoro-2-methylphenyl)-7-fluoro-3,3-dimethyl-2,3-dihydrobenzofuran-6-carboxamide **28** |
| 29 | 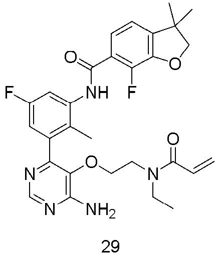 | *N*-(3-(6-amino-5-(2-(*N-*ethylacrylamido)ethoxy)pyrimidin-4-yl)-5-fluoro-2-methylphenyl)-7-fluoro-3,3-dimethyl-2,3-dihydrobenzofuran-6-carboxamide **29** |
| 30 | 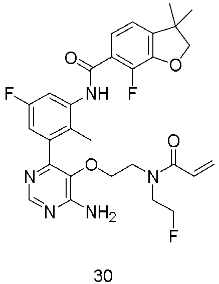 | *N*-(3-(6-amino-5-(2-(*N*-(2-fluoroethyl)acrylamido)ethoxy)pyrimidin-4-yl)-5-fluoro-2-methylphenyl)-7-fluoro-3,3-dimethyl-2,3-dihydrobenzofuran-6-carboxamide **30** |
| 31 | 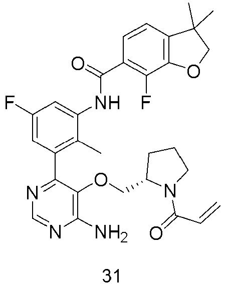 | (*S*)-*N*-(3-(5-((1-acryloylpyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide **31** |
| 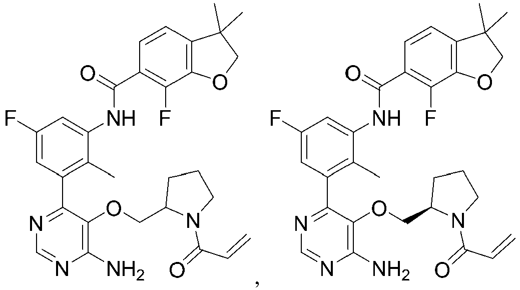 | | |
| 32 | 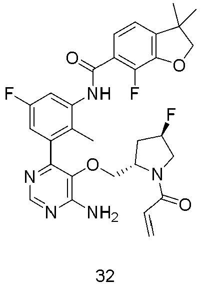 | *N*-(3-(5-(((2*S*,4*R*)-1-acryloyl-4-fluoropyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide **32** |
| 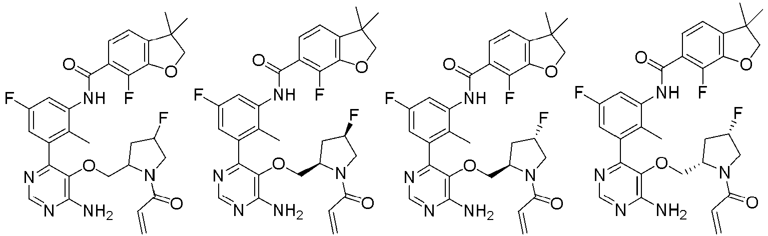 | | |
| 33 | 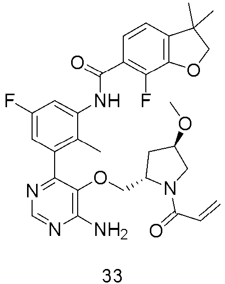 | *N*-(3-(5-(((2*S*,4*R*)-1-acryloyl-4-methoxypyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide **33** |
| 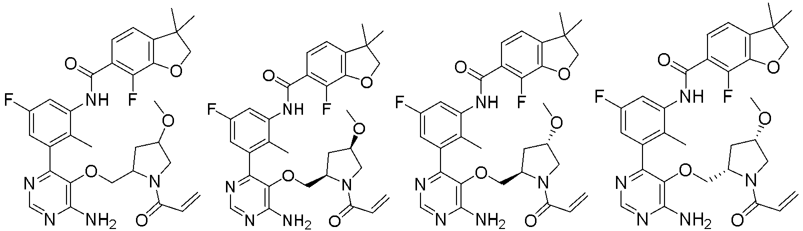 | | |
| | 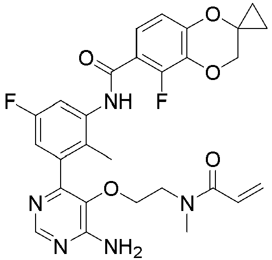 | |

Another aspect of the present disclosure relates to a compound as represented by general formula (IA) or a salt thereof: 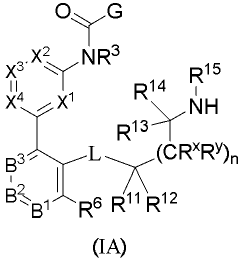 wherein:
G, R³ , X¹, X², X³, X⁴, B¹, B², B³, R⁶, L, R¹¹, R¹², R¹³, R¹⁴, R¹⁵, R^{x}, R^{y} and n are as defined in general formula (I).

Another aspect of the present disclosure relates to a compound as represented by general formula (I-1A) or a salt thereof: 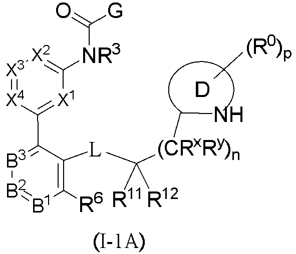 wherein:
G, R³, X¹, X², X³, X⁴, B¹, B², B³, R⁶, L, R¹¹, R¹², R^{x}, R^{y}, n, ring D, R⁰ and p are as defined in general formula (I-1).

Another aspect of the present disclosure relates to a compound as represented by general formula (IIA) or a salt thereof: 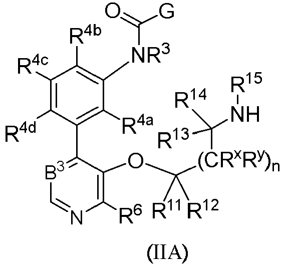 wherein:
G, R³, R^{4a}, R^{4b}, R^{4c}, R^{4d}, B³, R⁶, R¹¹, R¹², R¹³, R¹⁴, R¹⁵, R^{x}, R^{y} and n are as defined in general formula (II).

Another aspect of the present disclosure relates to a compound as represented by general formula (II-1A) or a salt thereof: 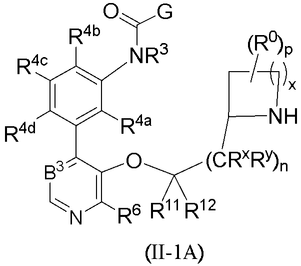 wherein:
G, R³, R^{4a}, R^{4b}, R^{4c}, R^{4d}, B³, R⁶, R¹¹, R¹², R^{x}, R^{y}, n, R⁰, p and x are as defined in general formula (II-1).

Another aspect of the present disclosure relates to a compound as represented by general formula (IIIA) or a salt thereof: 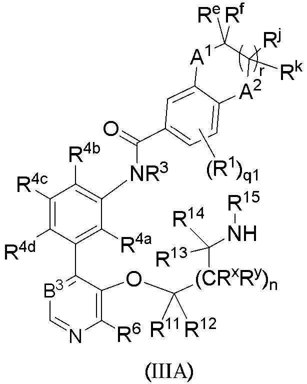 wherein:
A¹, A², R^{e}, R^{f}, R^{j}, R^{k}, r, R¹, q1, R³, R^{4a}, R^{4b}, R^{4c}, R^{4d}, B³, R⁶, R¹¹, R¹², R¹³, R¹⁴, R¹⁵, R^{x}, R^{y} and n are as defined in general formula (III).

Another aspect of the present disclosure relates to a compound as represented by general formula (IVA) or a salt thereof: 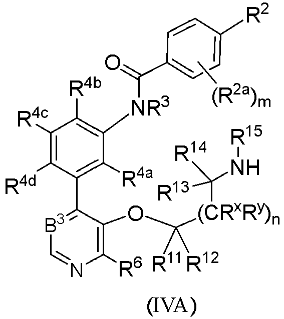 wherein:
R² , R^{2a} , m, R³, R^{4a}, R^{4b}, R^{4c}, R^{4d}, B³, R⁶, R¹¹, R¹², R¹³, R¹⁴, R¹⁵, R^{x}, R^{y} and n are as defined in general formula (IV).

Another aspect of the present disclosure relates to a compound as represented by general formula (VA) or a salt thereof: 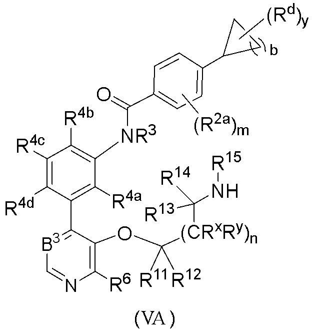 wherein:
R^{d}, y, b, R^{2a}, m, R³, R^{4a}, R^{4b}, R^{4c}, R^{4d}, B³, R⁶, R¹¹, R¹², R¹³, R¹⁴ , R¹⁵, R^{x} , R^{y} and n are as defined in general formula (V).

**Table B. Typical intermediate compounds of the present disclosure, including but not limited to:**

| Compound No. | Compound structure | Name |
|---|---|---|
| 1f | 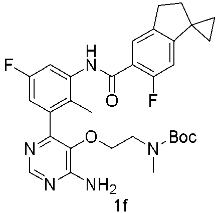 | Tert-butyl (2-((4-amino-6-(5-fluoro-3-(6'-fluoro-2',3'-dihydrospiro[cyclopropane-1,1'-indene]-5'-carboxamido)-2-methylphenyl)pyrimidin-5-yl)oxy)ethyl)(methyl)carbamate **1f** |
| 1g | 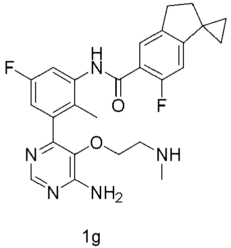 | *N*-(3-(6-amino-5-(2-(methylamino)ethoxy)pyrimidin-4-yl)-5-fluoro-2-methylphenyl)-6'-fluoro-2',3'-dihydrospiro[cyclopropane-1,1'-indene]-5'-carboxamide **1g** |
| 2f | 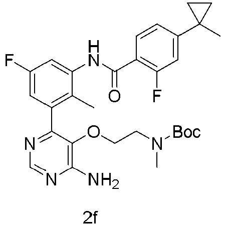 | Tert-butyl (2-((4-amino-6-(5-fluoro-3-(2-fluoro-4-(1-methylcyclopropyl)benzamido)-2-methylphenyl)pyrimidin-5-yl)oxy)ethyl)(methyl)carbamate **2f** |
| 2g | 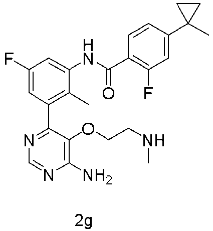 | *N*-(3-(6-amino-5-(2-(methylamino)ethoxy)pyrimidin-4-yl)-5-fluoro-2-methylphenyl)-2-fluoro-4-(1-methylcyclopropyl)benzamide **2g** |
| 3l | 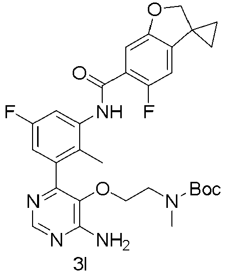 | Tert-butyl (2-((4-amino-6-(5-fluoro-3-(5-fluoro-2*H*-spiro[benzofuran-3,1'-cyclopropane]-6-carboxamido)-2-methylphenyl)pyrimidin-5-yl)oxy)ethyl)(methyl)carbamate **3l** |
| 3m | 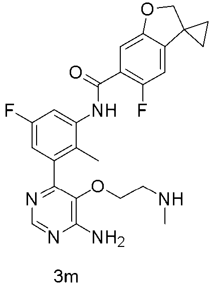 | *N*-(3-(6-amino-5-(2-(methylamino)ethoxy)pyrimidin-4-yl)-5-fluoro-2-methylphenyl)-5-fluoro-2*H-*spiro[benzofuran-3,1'-cyclopropane]-6-carboxamide **3m** |
| 4f | 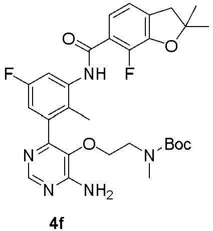 | Tert-butyl (2-((4-amino-6-(5-fluoro-3-(7-fluoro-2,2-dimethyl-2,3-dihydrobenzofuran-6-carboxamido)-2-methylphenyl)pyrimidin-5-yl)oxy)ethyl)(methyl)carbamate **4f** |
| 4g | 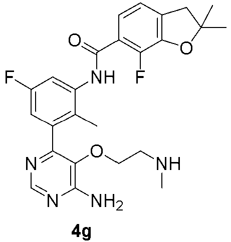 | *N*-(3-(6-amino-5-(2-(methylamino)ethoxy)pyrimidin-4-yl)-5-fluoro-2-methylphenyl)-7-fluoro-2,2-dimethyl-2,3-dihydrobenzofuran-6-carboxamide **4g** |
| 5j | 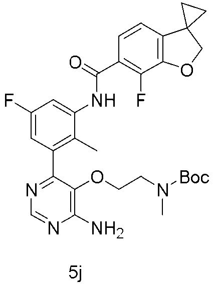 | Tert-butyl (2-((4-amino-6-(5-fluoro-3-(7-fluoro-2*H*-spiro[benzofuran-3,1'-cyclopropane]-6-carboxamido)-2-methylphenyl)pyrimidin-5-yl)oxy)ethyl)(methyl)carbamate **5j** |
| 5k | 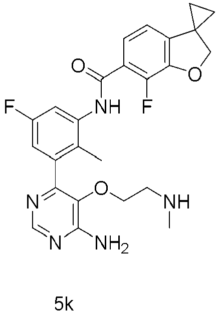 | *N*-(3-(6-amino-5-(2-(methylamino)ethoxy)pyrimidin-4-yl)-5-fluoro-2-methylphenyl)-7-fluoro-2*H-*spiro[benzofuran-3,1'-cyclopropane]-6-carboxamide **5k** |
| 6g | 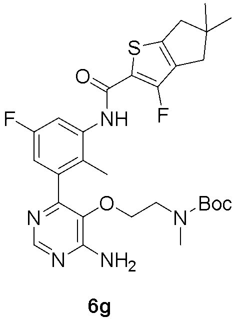 | Tert-butyl (2-((4-amino-6-(5-fluoro-3-(3-fluoro-5,5-dimethyl-5,6-dihydro-4*H-*cyclopenta[*b*]thiophene-2-carboxamido)-2-methylphenyl)pyrimidin-5-yl)oxy)ethyl)(methyl)carbamate **6g** |
| 6h | 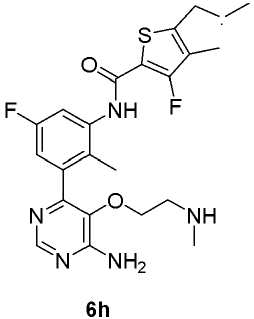 | *N*-(3-(6-amino-5-(2-(methylamino)ethoxy)pyrimidin-4-yl)-5-fluoro-2-methylphenyl)-3-fluoro-5,5-dimethyl-5,6-dihydro-4*H-*cyclopenta[b]thiophene-2-carboxamide **6h** |
| 7f | 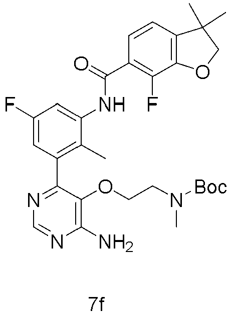 | Tert-butyl (2-((4-amino-6-(5-fluoro-3-(7-fluoro-3,3-dimethyl-2,3-dihydrobenzofuran-6-carboxamido)-2-methylphenyl)pyrimidin-5-yl)oxy)ethyl)(methyl)carbamate **7f** |
| 7g | 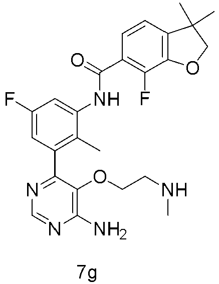 | *N*-(3-(6-amino-5-(2-(methylamino)ethoxy)pyrimidin-4-yl)-5-fluoro-2-methylphenyl)-7-fluoro-3,3-dimethyl-2,3-dihydrobenzofuran-6-carboxamide **7g** |
| 8f | 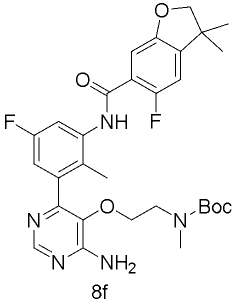 | Tert-butyl (2-((4-amino-6-(5-fluoro-3-(5-fluoro-3,3-dimethyl-2,3-dihydrobenzofuran-6-carboxamido)-2-methylphenyl)pyrimidin-5-yl)oxy)ethyl)(methyl)carbamate **8f** |
| 8g | 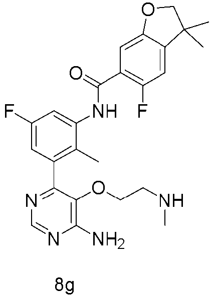 | *N*-(3-(6-amino-5-(2-(methylamino)ethoxy)pyrimidin-4-yl)-5-fluoro-2-methylphenyl)-5-fluoro-3,3-dimethyl-2,3-dihydrobenzofuran-6-carboxamide **8g** |
| 9f | 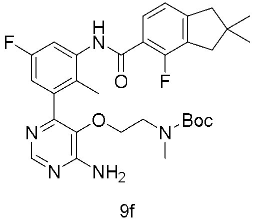 | Tert-butyl (2-((4-amino-6-(5-fluoro-3-(4-fluoro-2,2-dimethyl-2,3-dihydro-1*H-*indene-5-carboxamido)-2-methylphenyl)pyrimidin-5-yl)oxy)ethyl)(methyl)carbamate **9f** |
| 9g | 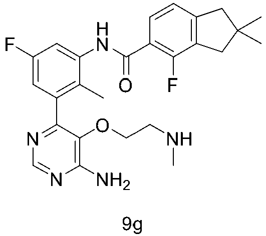 | *N*-(3-(6-amino-5-(2-(methylamino)ethoxy)pyrimidin-4-yl)-5-fluoro-2-methylphenyl)-4-fluoro-2,2-dimethyl-2,3-dihydro-1*H*-indene-5-carboxamide **9g** |
| 10g | 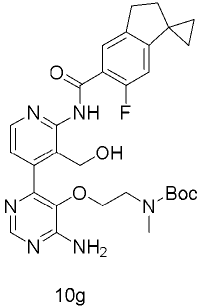 | Tert-butyl (2-((4-amino-6-(2-(6'-fluoro-2',3'-dihydrospiro[cyclopropane-1,1'-indene]-5'-carboxamido)-3-(hydroxymethyl)pyridin-4-yl)pyrimidin-5-yl)oxy)ethyl)(methyl)carbamate **10g** |
| 10h | 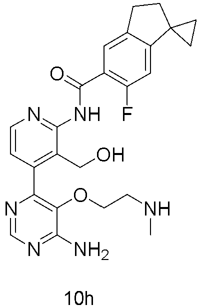 | *N*-(4-(6-amino-5-(2-(methylamino)ethoxy)pyrimidin-4-yl)-3-(hydroxymethyl)pyridin-2-yl)-6'-fluoro-2',3'-dihydrospiro[cyclopropane-1,1'-indene]-5'-carboxamide **10h** |
| 11f | 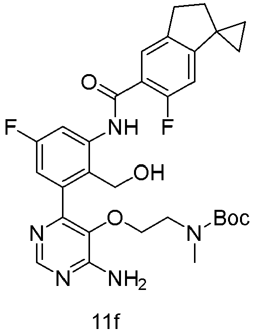 | Tert-butyl (2-((4-amino-6-(5-fluoro-3-(6'-fluoro-2',3'-dihydrospiro[cyclopropane-1,1'-indene]-5'-carboxamido)-2-(hydroxymethyl)phenyl)pyrimidin-5-yl)oxy)ethyl)(methyl)carbamate **11f** |
| 11g | 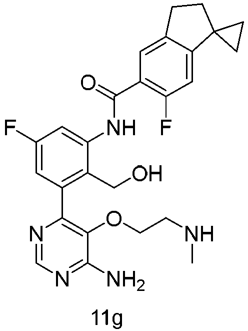 | *N*-(3-(6-amino-5-(2-(methylamino)ethoxy)pyrimidin-4-yl)-5-fluoro-2-(hydroxymethyl)phenyl)-6'-fluoro-2',3'-dihydrospiro[cyclopropane-1,1'-indene]-5'-carboxamide **11g** |
| 12e | 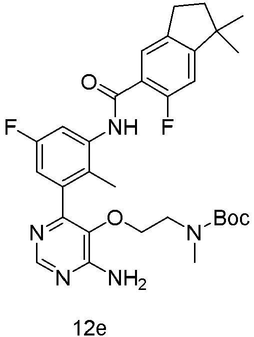 | Tert-butyl (2-((4-amino-6-(5-fluoro-3-(6-fluoro-1,1-dimethyl-2,3-dihydro-1*H-*indene-5-carboxamido)-2-methylphenyl)pyrimidin-5-yl)oxy)ethyl)(methyl)carbamate **12e** |
| 12f | 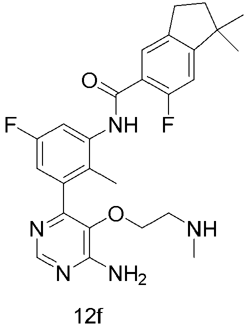 | *N*-(3-(6-amino-5-(2-(methylamino)ethoxy)pyrimidin-4-yl)-5-fluoro-2-methylphenyl)-6-fluoro-1,1-dimethyl-2,3-dihydro-1*H*-indene-5-carboxamide **12f** |
| 13f | 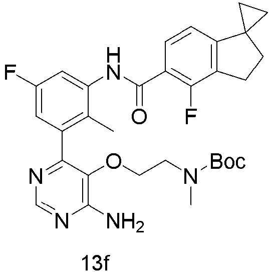 | Tert-butyl (2-((4-amino-6-(5-fluoro-3-(4'-fluoro-2',3'-dihydrospiro[cyclopropane-1,1'-indene]-5'-carboxamido)-2-methylphenyl)pyrimidin-5-yl)oxy)ethyl)(methyl)carbamate **13f** |
| 13g | 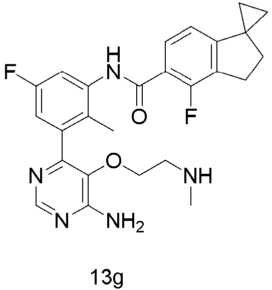 | *N*-(3-(6-amino-5-(2-(methylamino)ethoxy)pyrimidin-4-yl)-5-fluoro-2-methylphenyl)-4'-fluoro-2',3'-dihydrospiro[cyclopropane-1,1'-indene]-5'-carboxamide **13g** |
| 14h | 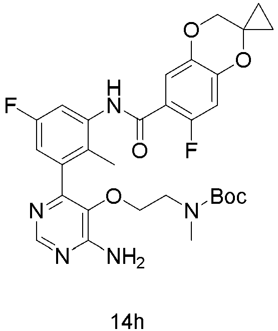 | Tert-butyl (2-((4-amino-6-(5-fluoro-3-(7-fluoro-3*H*-spiro[benzo[*b*][1,4]dioxine-2,1'-cyclopropane]-6-carboxamido)-2-methylphenyl)pyrimidin-5-yl)oxy)ethyl)(methyl)carbamate **14h** |
| 14i | 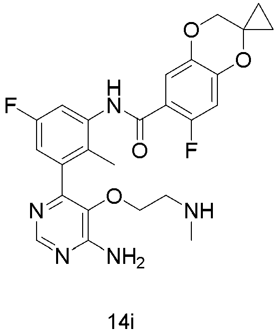 | *N*-(3-(6-amino-5-(2-(methylamino)ethoxy)pyrimidin-4-yl)-5-fluoro-2-methylphenyl)-7-fluoro-3*H-*spiro[benzo[*b*][1,4]dioxine-2,1'-cyclopropane]-6-carboxamide **14i** |
| 15f | 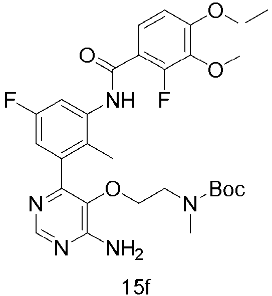 | Tert-butyl (2-((4-amino-6-(5-fluoro-3-(5-fluoro-2,2-dimethyl-2,3-dihydrobenzo[*b*][1,4]dioxine-6-carboxamido)-2-methylphenyl)pyrimidin-5-yl)oxy)ethyl)(methyl)carbamate **15f** |
| 15g | 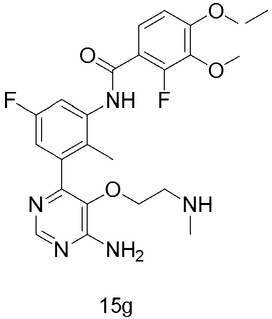 | *N*-(3-(6-amino-5-(2-(methylamino)ethoxy)pyrimidin-4-yl)-5-fluoro-2-methylphenyl)-5-fluoro-2,2-dimethyl-2,3-dihydrobenzo[*b*][1,4]dioxine-6-carboxamide **15g** |
| 16f-1 | 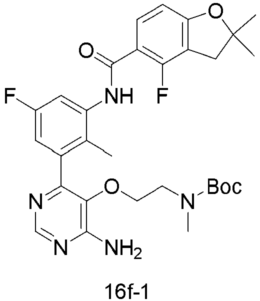 | Tert-butyl (2-((4-amino-6-(5-fluoro-3-(4-fluoro-2,2-dimethyl-2,3-dihydrobenzofuran-5-carboxamido)-2-methylphenyl)pyrimidin-5-yl)oxy)ethyl)(methyl)carbamate **16f-1** |
| 16g-1 | 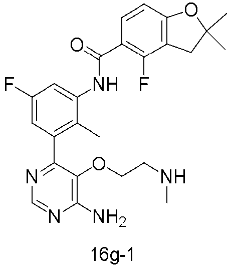 | *N*-(3-(6-amino-5-(2-(methylamino)ethoxy)pyrimidin-4-yl)-5-fluoro-2-methylphenyl)-4-fluoro-2,2-dimethyl-2,3-dihydrobenzofuran-5-carboxamide **16g-1** |
| 16f-2 | 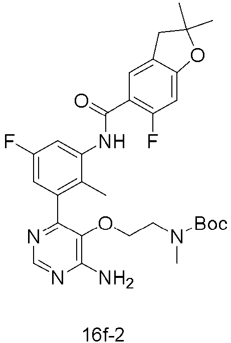 | Tert-butyl (2-((4-amino-6-(5-fluoro-3-(6-fluoro-2,2-dimethyl-2,3-dihydrobenzofuran-5-carboxamido)-2-methylphenyl)pyrimidin-5-yl)oxy)ethyl)(methyl)carbamate **16f-2** |
| 16g-2 | 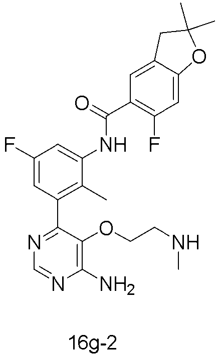 | *N*-(3-(6-amino-5-(2-(methylamino)ethoxy)pyrimidin-4-yl)-5-fluoro-2-methylphenyl)-6-fluoro-2,2-dimethyl-2,3-dihydrobenzofuran-5-carboxamide **16g-2** |
| 17g | 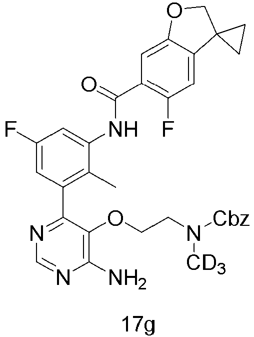 | Benzyl (2-((4-amino-6-(5-fluoro-3-(5-fluoro-2*H*-spiro[benzofuran-3,1'-cyclopropane]-6-carboxamido)-2-methylphenyl)pyrimidin-5-yl)oxy)ethyl)(methyl-*d*₃)carbamate **17g** |
| 17h | 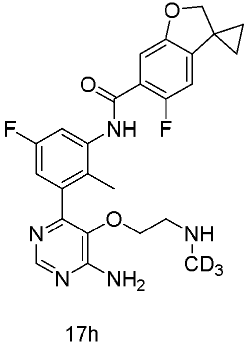 | *N*-(3-(6-amino-5-(2-((methyl-*d*₃)amino)ethoxy)pyrimidin-4-yl)-5-fluoro-2-methylphenyl)-5-fluoro-2*H-*spiro[benzofuran-3,1'-cyclopropane]-6-carboxamide **17h** |
| 18a | 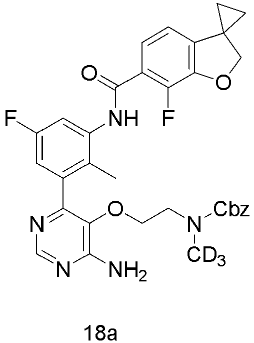 | Benzyl (2-((4-amino-6-(5-fluoro-3-(7-fluoro-2*H*-spiro[benzofuran-3,1'-cyclopropane]-6-carboxamido)-2-methylphenyl)pyrimidin-5-yl)oxy)ethyl)(methyl-*d*₃)carbamate **18a** |
| 18b | 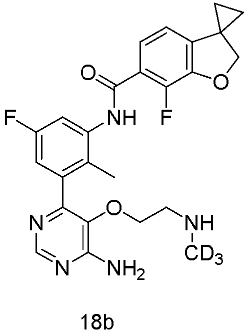 | *N*-(3-(6-amino-5-(2-((methyl-*d*₃)amino)ethoxy)pyrimidin-4-yl)-5-fluoro-2-methylphenyl)-7-fluoro-2*H-*spiro[benzofuran-3,1'-cyclopropane]-6-carboxamide **18b** |
| **19a** | 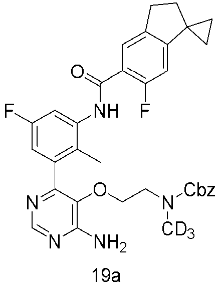 | Benzyl (2-((4-amino-6-(5-fluoro-3-(6'-fluoro-2',3'-dihydrospiro[cyclopropane-1,1'-indene]-5'-carboxamido)-2-methylphenyl)pyrimidin-5-yl)oxy)ethyl)(methyl-*d*3)carbamate **19a** |
| **19b** | 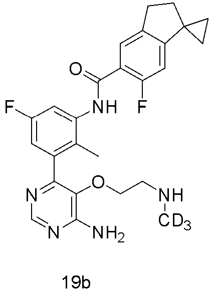 | *N*-(3-(6-amino-5-(2-((methyl-*d*3)amino)ethoxy)pyrimidin-4-yl)-5-fluoro-2-methylphenyl)-6'-fluoro-2',3'-dihydrospiro[cyclopropane-1,1'-indene]-5'-carboxamide **19b** |
| **20e** | 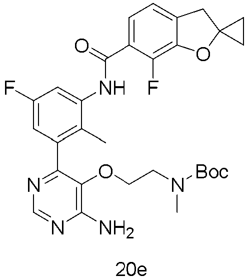 | Tert-butyl (2-((4-amino-6-(5-fluoro-3-(7-fluoro-3*H*-spiro[benzofuran-2,1'-cyclopropane]-6-carboxamido)-2-methylphenyl)pyrimidin-5-yl)oxy)ethyl)(methyl)carbamate **20e** |
| **20f** | 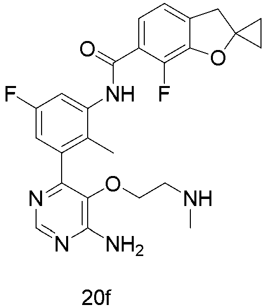 | *N*-(3-(6-amino-5-(2-(methylamino)ethoxy)pyrimidin-4-yl)-5-fluoro-2-methylphenyl)-7-fluoro-3*H-*spiro[benzofuran-2,1'-cyclopropane]-6-carboxamide 20f |
| 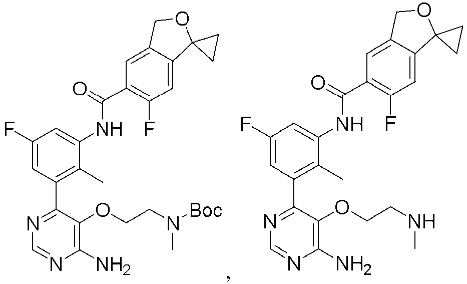 | | |
| 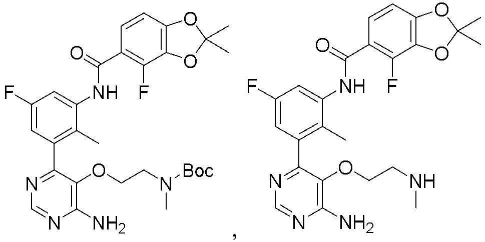 | | |
| 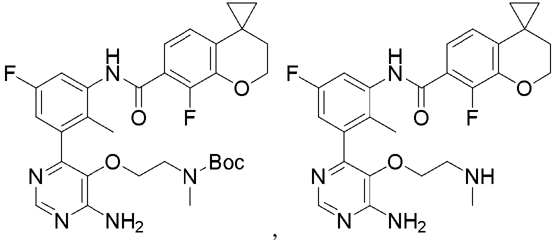 | | |
| 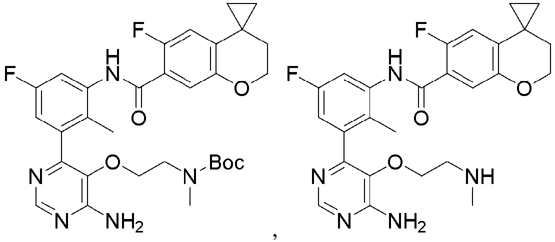 | | |
| 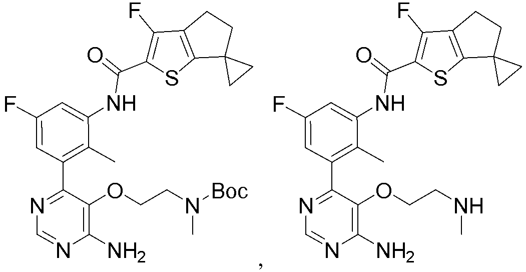 | | |
| 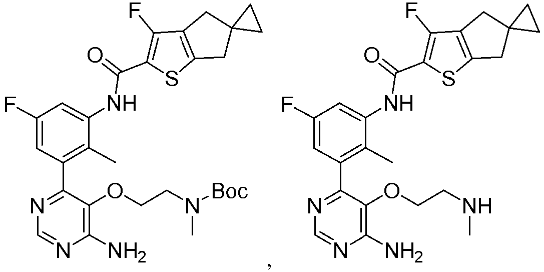 | | |
| 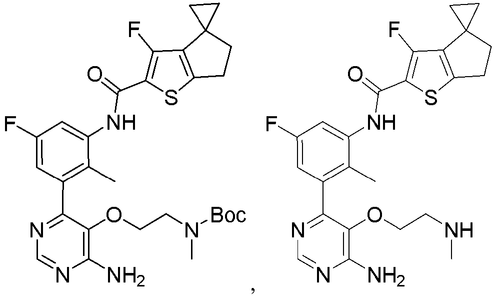 | | |
| 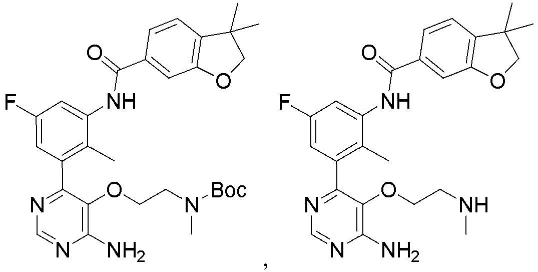 | | |
| 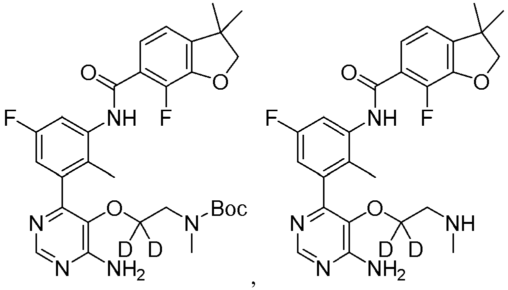 | | |
| 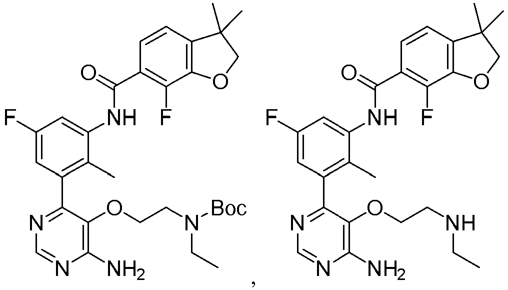 | | |
| 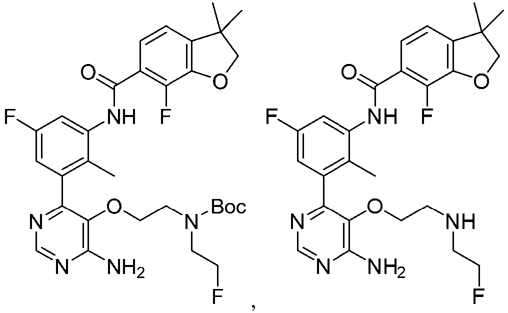 | | |
| 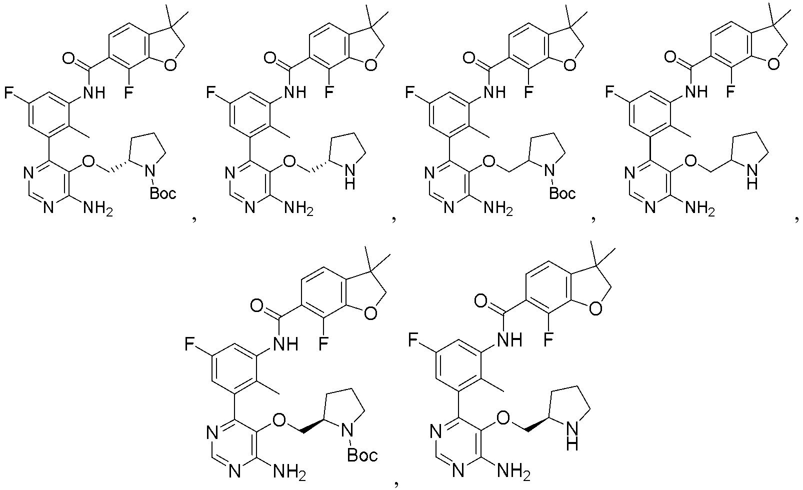 | | |
| 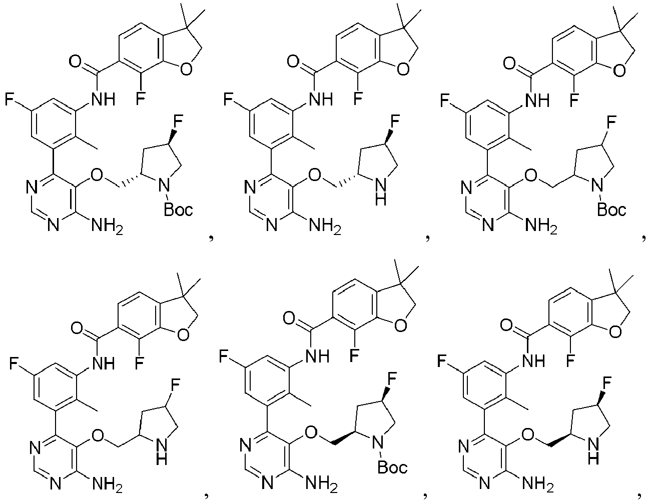 | | |
| 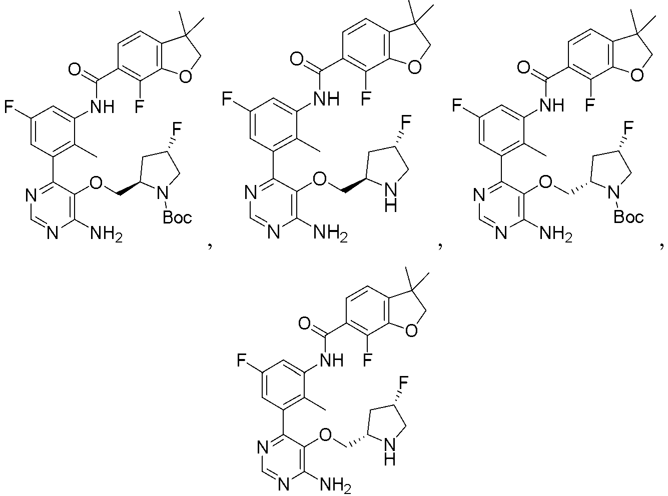 | | |
| 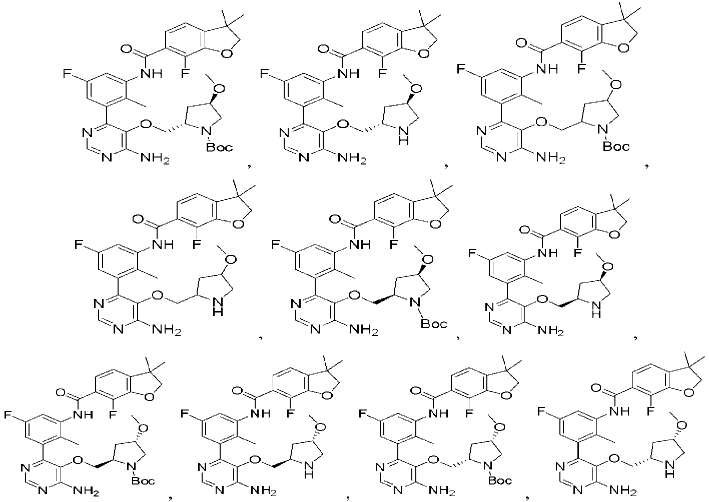 | | |

Another aspect of the present disclosure relates to a method for preparing the compound as represented by general formula (I) or the pharmaceutically acceptable salt thereof, wherein the method comprises: 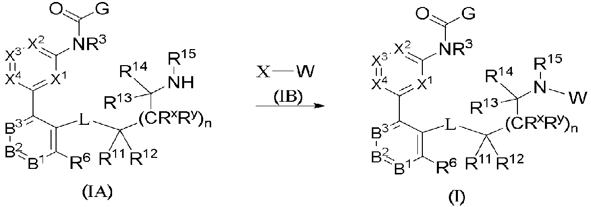
subjecting a compound as represented by general formula (IA) or a salt thereof, or a compound as represented by general formula (IB) or a salt thereof to a condensation reaction to obtain the compound as represented by general formula (I) or the pharmaceutically acceptable salt thereof,
wherein:
   X is halogen, in some embodiments, C1;
   W is -C(O)R¹⁶ or -S(O)₂R¹⁶;
   G, R³ , X¹, X², X³, X⁴, B¹, B², B³, R⁶, L, R¹¹, R¹², R¹³, R¹⁴, R¹⁵, R¹⁶, R^{x}, R^{y} and n are as defined in general formula (I).

Another aspect of the present disclosure relates to a method for preparing the compound as represented by general formula (I-1) or the pharmaceutically acceptable salt thereof, wherein the method comprises: 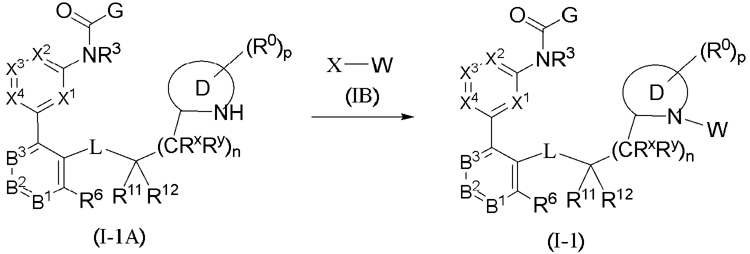
subjecting a compound as represented by general formula (I-1A) or a salt thereof, or a compound as represented by general formula (IB) or a salt thereof to a condensation reaction to obtain the compound as represented by general formula (I-1) or the pharmaceutically acceptable salt thereof,
wherein:
   X is halogen, in some embodiments, C1;
   W is -C(O)R¹⁶ or -S(O)₂R¹⁶;
   G, R³, X¹, X², X³, X⁴, B¹, B², B³, R⁶, L, R¹¹, R¹², R¹⁶, R^{x}, R^{y}, n, ring D, R⁰ and p are as defined in general formula (I-1).

Another aspect of the present disclosure relates to a method for preparing the compound as represented by general formula (II) or the pharmaceutically acceptable salt thereof, wherein the method comprises: 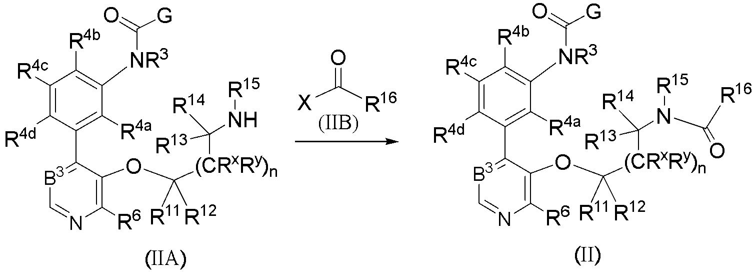
subjecting a compound as represented by general formula (IIA) or a salt thereof, or a compound as represented by general formula (IIB) or a salt thereof to a condensation reaction to obtain the compound as represented by general formula (II) or the pharmaceutically acceptable salt thereof,
wherein:
   X is halogen, in some embodiments, C1;
   G, R³, R^{4a}, R^{4b}, R^{4c}, R^{4d}, B³, R⁶, R¹¹, R¹², R¹³, R¹⁴, R¹⁵, R¹⁶, R^{x}, R^{y} and n are as defined in general formula (II).

Another aspect of the present disclosure relates to a method for preparing the compound as represented by general formula (II-1) or the pharmaceutically acceptable salt thereof, wherein the method comprises: 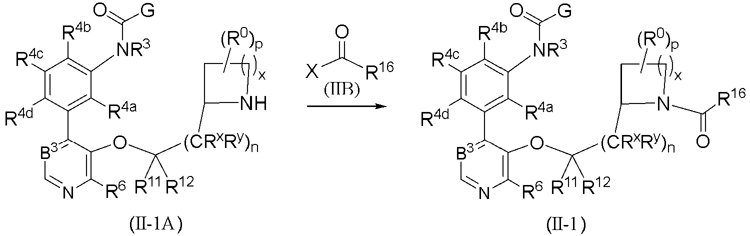
subjecting a compound as represented by general formula (II-1A) or a salt thereof, or a compound as represented by general formula (IIB) or a salt thereof to a condensation reaction to obtain the compound as represented by general formula (II-1) or the pharmaceutically acceptable salt thereof,
wherein:
   X is halogen, in some embodiments, C1;
   G, R³, R^{4a}, R^{4b}, R^{4c}, R^{4d}, B³, R⁶, R¹¹, R¹², R¹⁶, R^{x}, R^{y}, n, R⁰, p and x are as defined in general formula (II-1).

Another aspect of the present disclosure relates to a method for preparing the compound as represented by general formula (III) or the pharmaceutically acceptable salt thereof, wherein the method comprises: 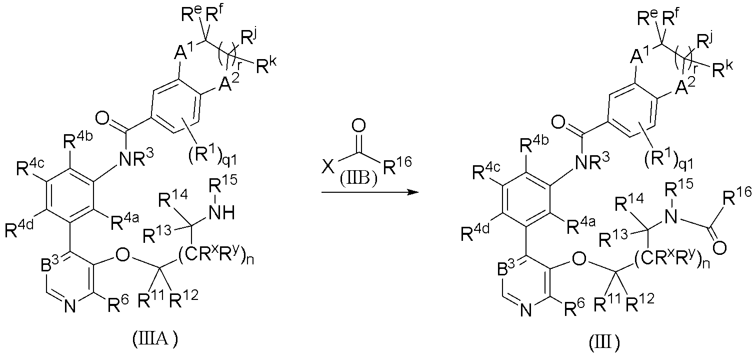
subjecting a compound as represented by general formula (IIIA) or a salt thereof, or a compound as represented by general formula (IIB) or a salt thereof to a condensation reaction to obtain the compound as represented by general formula (III) or the pharmaceutically acceptable salt thereof,
wherein:
   X is halogen, in some embodiments, C1;
   A¹, A², R^{e}, R^{f}, R^{j}, R^{k}, r, R^{l}, q1, R³, R^{4a}, R^{4b}, R^{4c}, R^{4d}, B³, R⁶, R¹¹, R¹², R¹³, R¹⁴, R¹⁵, R¹⁶, R^{x}, R^{y} and n are as defined in general formula (III).

Another aspect of the present disclosure relates to a method for preparing the compound as represented by general formula (IV) or the pharmaceutically acceptable salt thereof, wherein the method comprises: 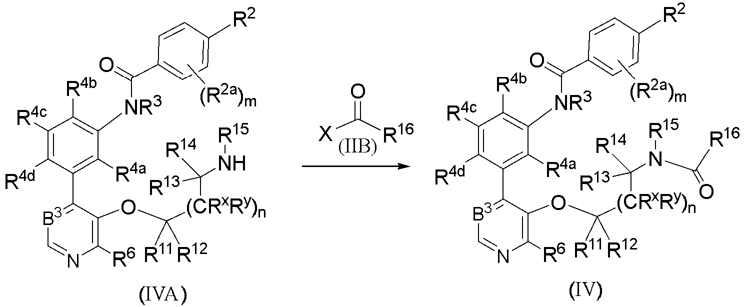
subjecting a compound as represented by general formula (IVA) or a salt thereof, or a compound as represented by general formula (IIB) or a salt thereof to a condensation reaction to obtain the compound as represented by general formula (IV) or the pharmaceutically acceptable salt thereof,
wherein:
   X is halogen, in some embodiments, C1;
   R², R^{2a}, m, R³, R^{4a}, R^{4b}, R^{4c}, R^{4d}, B³, R⁶, R¹¹, R¹², R¹³, R¹⁴, R¹⁵, R¹⁶, R^{x}, R^{y} and n are as defined in general formula (IV).

Another aspect of the present disclosure relates to a method for preparing the compound as represented by general formula (V) or the pharmaceutically acceptable salt thereof, wherein the method comprises: 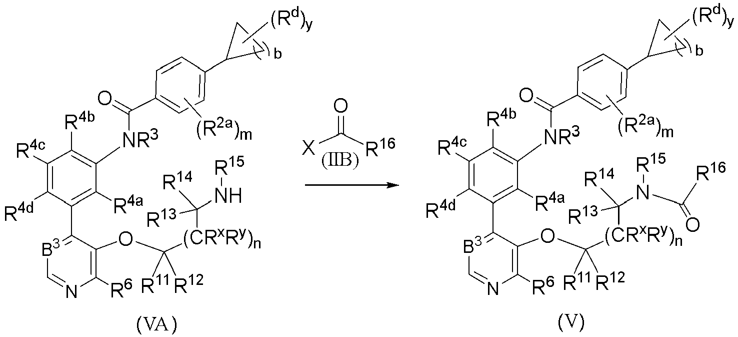
subjecting a compound as represented by general formula (VA) or a salt thereof, or a compound as represented by general formula (IIB) or a salt thereof to a condensation reaction to obtain the compound as represented by general formula (V) or the pharmaceutically acceptable salt thereof,
wherein:
   X is halogen, in some embodiments, C1;
   R^{d}, y, b, R^{2a}, m, R³, R^{4a}, R^{4b}, R^{4c}, R^{4a}, B³, R⁶, R¹¹, R¹², R¹³, R¹⁴, R¹⁵, R^{x}, R^{y}, n and R¹⁶ are as defined in general formula (V).

In some embodiments of the present disclosure, provided is a method for preparing the compound as represented by general formula (I), general formula (I-1), general formula (II), general formula (II-1), general formula (III), general formula (IV), general formula (V), general formula (VI) or general formula (VI-1), wherein the condensation reaction is performed under alkaline conditions; the reagents used to provide the alkaline conditions include organic bases and inorganic bases; the organic bases include, but are not limited to, triethylamine, *N,N-*diisopropylethylamine, n-butyllithium, lithium diisopropylamide, sodium acetate, potassium acetate, sodium ethoxide, sodium tert-butoxide, and potassium tert-butoxide; the inorganic bases include, but are not limited to, sodium hydride, potassium phosphate, sodium carbonate, potassium carbonate, caesium carbonate, sodium hydroxide, lithium hydroxide monohydrate, lithium hydroxide, and potassium hydroxide; in some embodiments, the reagents used to provide the alkaline conditions is *N,N*-diisopropylethylamine.

In some embodiments of the present disclosure, provided is a method for preparing the compound as represented by general formula (I), general formula (I-1), general formula (II), general formula (II-1), general formula (III), general formula (IV), general formula (V), general formula (VI) or general formula (VI-1), wherein the condensation reaction is performed in a solvent, which includes but is not limited to: N-methylpyrrolidone, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane and mixtures thereof.

Another aspect of the present disclosure relates to a pharmaceutical composition, wherein the pharmaceutical composition comprises the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof according to the present disclosure, and one or more pharmaceutically acceptable carriers, diluents or excipients.

The present disclosure further relates to the use of the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same in the preparation of a medicament for treating and/or preventing a BTK-mediated disease or condition.

The present disclosure further relates to the use of the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same in the preparation of a BTK inhibitor.

The present disclosure further relates to the use of the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same in the preparation of a medicament for treating and/or preventing a disease or condition, wherein the disease or condition is selected from cancer, autoimmune diseases, inflammatory diseases, allergic diseases, allergic reactions, respiratory diseases, cardiovascular diseases, viral infections, transplant rejection reactions, metabolic/endocrine disorders, and neurological disorders; in some embodiments, the disease or condition is selected from autoimmune diseases, inflammatory diseases and cancer; in some embodiments, the disease or condition is autoimmune disease or inflammatory disease; in some embodiments, the disease or condition is selected from B-cell malignancies, multiple myeloma, leukaemia (such as acute lymphoblastic leukaemia, chronic lymphocytic leukaemia, acute myeloid leukaemia, chronic myelogenous leukaemia, acute promyelocytic leukaemia, acute granulocytic leukaemia, chronic granulocytic leukaemia and hairy cell leukaemia), malignant lymphoma (such as small lymphocytic lymphoma, diffuse large B-cell lymphoma, intravascular large B-cell lymphoma, primary effusion lymphoma, follicular lymphoma, mantle cell lymphoma, non-Hodgkin lymphoma, B-cell non-Hodgkin lymphoma, B-cell lymphoma and follicular lymphoma), Waldenström macroglobulinemia, polycythaemia vera, essential thrombocythemia, myelofibrosis with myeloid metaplasia, Waldenstroem macroglobulinemia (Waldenstroem), bone cancer, bone metastasis, arthritis (such as rheumatoid arthritis, and systemic-onset juvenile idiopathic arthritis (SOJIA)), multiple sclerosis (such as relapsing multiple sclerosis), amyotrophic lateral sclerosis, osteoporosis, diabetes mellitus (such as type I diabetes mellitus and type II diabetes mellitus), arteriosclerosis, irritable bowel syndrome, inflammatory bowel disease, ulcerative colitis, Crohn's disease, Sjogren's syndrome, autoimmune thyroid diseases, Alzheimer's disease, lupus, systemic lupus erythematosus, psoriasis, urticaria (such as chronic spontaneous urticaria, chronic inducible urticaria, and chronic autoimmune urticaria), hepatic insufficiency, allergies (such as atopic eczema, atopic dermatitis, contact dermatitis, and allergic rhinitis), ankylosing spondylitis, dermatitis (such as atopic dermatitis), pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa, myasthenia gravis, autoimmune haemolytic anaemia, antineutrophil cytoplasmic antibody (ANCA)-associated vasculitis, cryoglobulinemia, thrombotic thrombocytopenic purpura, immune thrombocytopenia, complications caused by organ transplantation, xenotransplantation, antibody-mediated rejection (AMR), graft versus host disease, B cell-mediated hyperacute, and acute and chronic transplant rejection reactions.

The present disclosure further relates to a method for inhibiting BTK, wherein the method comprises administering to a patient in need thereof the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same.

The present disclosure further relates to a method for treating and/or preventing a BTK-mediated disease or condition, wherein the method comprises administering to a patient in need thereof the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same.

The present disclosure further relates to a method for treating and/or preventing a disease or condition, wherein the method comprises administering to a patient in need thereof the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same; the disease or condition is selected from cancer, autoimmune diseases, inflammatory diseases, allergic diseases, allergic reactions, respiratory diseases, cardiovascular diseases, viral infections, transplant rejection reactions, metabolic/endocrine disorders, and neurological disorders; in some embodiments, the disease or condition is selected from autoimmune diseases, inflammatory diseases and cancer; in some embodiments, the disease or condition is autoimmune disease or inflammatory disease; in some embodiments, the disease or condition is selected from B-cell malignancies, multiple myeloma, leukaemia (such as acute lymphoblastic leukaemia, chronic lymphocytic leukaemia, acute myeloid leukaemia, chronic myelogenous leukaemia, acute promyelocytic leukaemia, acute granulocytic leukaemia, chronic granulocytic leukaemia and hairy cell leukaemia), malignant lymphoma (such as small lymphocytic lymphoma, diffuse large B-cell lymphoma, intravascular large B-cell lymphoma, primary effusion lymphoma, follicular lymphoma, mantle cell lymphoma, non-Hodgkin lymphoma, B-cell non-Hodgkin lymphoma, B-cell lymphoma and follicular lymphoma), Waldenström macroglobulinemia, polycythaemia vera, essential thrombocythemia, myelofibrosis with myeloid metaplasia, Waldenstroem macroglobulinemia (Waldenstroem), bone cancer, bone metastasis, arthritis (such as rheumatoid arthritis, and systemic-onset juvenile idiopathic arthritis (SOJIA)), multiple sclerosis (such as relapsing multiple sclerosis), amyotrophic lateral sclerosis, osteoporosis, diabetes mellitus (such as type I diabetes mellitus and type II diabetes mellitus), arteriosclerosis, irritable bowel syndrome, inflammatory bowel disease, ulcerative colitis, Crohn's disease, Sjogren's syndrome, autoimmune thyroid diseases, Alzheimer's disease, lupus, systemic lupus erythematosus, psoriasis, urticaria (such as chronic spontaneous urticaria, chronic inducible urticaria, and chronic autoimmune urticaria), hepatic insufficiency, allergies (such as atopic eczema, atopic dermatitis, contact dermatitis, and allergic rhinitis), ankylosing spondylitis, dermatitis (such as, atopic dermatitis), pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa, myasthenia gravis, autoimmune haemolytic anaemia, antineutrophil cytoplasmic antibody (ANCA)-associated vasculitis, cryoglobulinemia, thrombotic thrombocytopenic purpura, immune thrombocytopenia, complications caused by organ transplantation, xenotransplantation, antibody-mediated rejection (AMR), graft versus host disease, B cell-mediated hyperacute, and acute and chronic transplant rejection reactions.

The present disclosure further relates to the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same, for use as a medicament.

The present disclosure further relates to the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same, for use as a BTK inhibitor.

The present disclosure further relates to the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same, for use in the treatment and/or prevention of a BTK-mediated disease or condition.

The present disclosure further relates to the compounds as represented by general formula (I), general formula (I-1), general formula (II), general formula (II-1), general formula (III), general formula (IV), general formula (V), general formula (VI) or general formula (VI-1) and the compounds shown in Table A or the pharmaceutically acceptable salts thereof, or the pharmaceutical composition comprising same, for use in the treatment and/or prevention of a disease or condition, wherein the disease or condition is selected from cancer, autoimmune diseases, inflammatory diseases, allergic diseases, allergic reactions, respiratory diseases, cardiovascular diseases, viral infections, transplant rejection reactions, metabolic/endocrine disorders, and neurological disorders; in some embodiments, the disease or condition is selected from autoimmune diseases, inflammatory diseases and cancer; in some embodiments, the disease or condition is autoimmune disease or inflammatory disease; in some embodiments, the disease or condition is selected from B-cell malignancies, multiple myeloma, leukaemia (such as acute lymphoblastic leukaemia, chronic lymphocytic leukaemia, acute myeloid leukaemia, chronic myelogenous leukaemia, acute promyelocytic leukaemia, acute granulocytic leukaemia, chronic granulocytic leukaemia and hairy cell leukaemia), malignant lymphoma (such as small lymphocytic lymphoma, diffuse large B-cell lymphoma, intravascular large B-cell lymphoma, primary effusion lymphoma, follicular lymphoma, mantle cell lymphoma, non-Hodgkin lymphoma, B-cell non-Hodgkin lymphoma, B-cell lymphoma and follicular lymphoma), Waldenström macroglobulinemia, polycythaemia vera, essential thrombocythemia, myelofibrosis with myeloid metaplasia, Waldenstroem macroglobulinemia (Waldenstroem), bone cancer, bone metastasis, arthritis (such as rheumatoid arthritis, and systemic-onset juvenile idiopathic arthritis (SOJIA)), multiple sclerosis (such as relapsing multiple sclerosis), amyotrophic lateral sclerosis, osteoporosis, diabetes mellitus (such as type I diabetes mellitus and type II diabetes mellitus), arteriosclerosis, irritable bowel syndrome, inflammatory bowel disease, ulcerative colitis, Crohn's disease, Sjogren's syndrome, autoimmune thyroid diseases, Alzheimer's disease, lupus, systemic lupus erythematosus, psoriasis, urticaria (such as chronic spontaneous urticaria, chronic inducible urticaria, and chronic autoimmune urticaria), hepatic insufficiency, allergies (such as atopic eczema, atopic dermatitis, contact dermatitis, and allergic rhinitis), ankylosing spondylitis, dermatitis (such as atopic dermatitis), pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa, myasthenia gravis, autoimmune haemolytic anaemia, antineutrophil cytoplasmic antibody (ANCA)-associated vasculitis, cryoglobulinemia, thrombotic thrombocytopenic purpura, immune thrombocytopenia, complications caused by organ transplantation, xenotransplantation, antibody-mediated rejection (AMR), graft versus host disease, B cell-mediated hyperacute, and acute and chronic transplant rejection reactions.

In some embodiments, the arthritis described in the present disclosure is rheumatoid arthritis.

In some embodiments, the diabetes mellitus described in the present disclosure is type I diabetes mellitus.

In some embodiments, the urticaria described in the present disclosure is chronic urticaria or acute urticaria. In some embodiments, the urticaria described in the present disclosure is chronic urticaria.

In some embodiments, the urticaria described in the present disclosure is chronic spontaneous urticaria (CSU) or chronic inducible urticaria. In some embodiments, the urticaria described in the present disclosure is chronic spontaneous urticaria.

In some embodiments, the dermatitis described in the present disclosure is atopic dermatitis.

In some embodiments, the multiple sclerosis described in the present disclosure is relapsing multiple sclerosis (RMS).

In some embodiments, the allergies described in the present disclosure are food allergies.

In some embodiments, the disease or condition or the BTK-mediated disease or condition described in the present disclosure is selected from cancer, autoimmune diseases, inflammatory diseases, allergic diseases, allergic reactions, respiratory diseases, cardiovascular diseases, viral infections, transplant rejection reactions, metabolic/endocrine disorders, and neurological disorders; in some embodiments, the disease or condition or the BTK-mediated disease or condition described in the present disclosure is selected from cancer, autoimmune diseases and inflammatory diseases; the disease or condition or the BTK-mediated disease or condition described in the present disclosure is autoimmune disease or inflammatory disease. In some embodiments, the disease or condition or the BTK-mediated disease or condition described in the present disclosure is selected from B-cell malignancies, B-cell lymphoma, diffuse large B-cell lymphoma, chronic lymphocytic leukaemia, non-Hodgkin lymphoma (such as ABC-DLBCL), mantle cell lymphoma, follicular lymphoma, hairy cell leukaemia, B-cell non-Hodgkin lymphoma, Waldenstroem macroglobulinemia, multiple myeloma, bone cancer, bone metastasis, follicular lymphoma, chronic lymphocytic lymphoma, B-cell prolymphocytic leukaemia, lymphoplasmacytic lymphoma, splenic marginal zone lymphoma, plasma cell myeloma, plasmacytoma, extranodal marginal zone B-cell lymphoma, nodal marginal zone B-cell lymphoma, mediastinal (thymic) large B-cell lymphoma, intravascular large B-cell lymphoma, primary effusion lymphoma, burkitt lymphoma/leukaemia, lymphomatoid granulomatosis, inflammatory bowel disease, arthritis, lupus, rheumatoid arthritis, psoriatic arthritis, osteoarthritis, Still's disease, juvenile arthritis, diabetes mellitus, myasthenia gravis, Hashimoto's thyroiditis, Ord's thyroiditis, Graves' disease, Sjogren's syndrome, multiple sclerosis, Guillain-Barre syndrome, acute disseminated encephalomyelitis, Addison's disease, opsoclonus-myoclonus syndrome, ankylosing spondylitis, antiphospholipid antibody syndrome, aplastic anaemia, autoimmune hepatitis, celiac disease, Goodpasture's syndrome, idiopathic thrombocytopenic purpura, optic neuritis, scleroderma, primary biliary cholangitis, Reiter's syndrome, Takayasu's arteritis, temporal arteritis, warm autoimmune haemolytic anaemia, Wegener's granulomatosis, psoriasis, alopecia Universalis, Behcet's disease, chronic fatigue, familial dysautonomia, endometriosis, interstitial cystitis, neuromyotonia, scleroderma, vulvodynia, graft versus host disease, transplantation reaction, transfusion reaction, anaphylaxis, allergy, type I hypersensitivity, allergic conjunctivitis, allergic rhinitis, atopic dermatitis, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis, myocarditis, myositis, nephritis, oophoritis, orchitis, osteitis, otitis, pancreatitis, parotitis, pericarditis, peritonitis, pharyngitis, pleurisy, phlebitis, focal pneumonia, pneumonia, proctitis, prostatitis, pyelonephritis, rhinitis, salpingitis, sinusitis, stomatitis, synovitis, tendinitis, tonsillitis, uveitis, vaginitis, vasculitis, vulvitis, pulmonary fibrosis, idiopathic pulmonary fibrosis (IPF), usual interstitial pneumonia (UIP), interstitial lung disease, cryptogenic fibrosing alveolitis (CFA), bronchiolitis obliterans, bronchiectasis, fatty liver disease, steatosis (such as non-alcoholic steatohepatitis (NASH)), cholestatic liver disease (such as primary biliary cholangitis (PBC)), cirrhosis, alcohol-induced liver fibrosis, bile duct injury, bile duct fibrosis, cholestasis or biliary tract disease, liver or hepatic fibrosis (including but not limited to liver fibrosis associated with alcoholism), viral infections (such as hepatitis, including hepatitis C, hepatitis B, or hepatitis D), autoimmune hepatitis, non-alcoholic fatty liver disease (NAFLD), progressive massive fibrosis, exposure to toxins or irritants (such as alcohol, drugs and environmental toxins), renal fibrosis (such as chronic renal fibrosis), kidney diseases associated with injury/fibrosis (such as chronic kidney diseases associated with diabetes mellitus (e.g., diabetic nephropathy)), lupus, scleroderma renal crisis, glomerulonephritis, focal segmental glomerulosclerosis, IgA nephropathy, renal fibrosis associated with human chronic kidney disease (CKD), chronic progressive nephropathy (CPN), tubulointerstitial fibrosis, ureteral obstruction, chronic uraemia, chronic interstitial nephritis, radiation nephropathy, glomerulosclerosis, progressive glomerulonephritis (PGN), endothelial/thrombotic microangiopathy injury, HIV-associated nephropathy, or fibrosis associated with exposure to toxins, irritants or chemotherapeutic agents, fibrosis associated with scleroderma; radiation-induced intestinal fibrosis; fibrosis associated with foregut inflammatory diseases (such as Barrett's oesophagus and chronic gastritis) and/or fibrosis associated with hindgut inflammatory diseases (such as inflammatory bowel disease (IBD), ulcerative colitis and Crohn's disease); age-related macular degeneration; diabetic retinopathy; retinopathy of prematurity, and neovascular glaucoma.

In some embodiments, the disease or condition or the BTK-mediated disease or condition described in the present disclosure is selected from B-cell malignancies, multiple myeloma, leukaemia (such as acute lymphoblastic leukaemia, chronic lymphocytic leukaemia, acute myeloid leukaemia, chronic myelogenous leukaemia, acute promyelocytic leukaemia, acute granulocytic leukaemia, chronic granulocytic leukaemia and hairy cell leukaemia), malignant lymphoma (such as small lymphocytic lymphoma, diffuse large B-cell lymphoma, intravascular large B-cell lymphoma, primary effusion lymphoma, follicular lymphoma, mantle cell lymphoma, non-Hodgkin lymphoma, B-cell non-Hodgkin lymphoma, B-cell lymphoma and follicular lymphoma), Waldenström macroglobulinemia, polycythaemia vera, essential thrombocythemia, myelofibrosis with myeloid metaplasia, Waldenstroem macroglobulinemia (Waldenstroem), bone cancer, bone metastasis, arthritis (such as rheumatoid arthritis, and systemic-onset juvenile idiopathic arthritis (SOJIA)), multiple sclerosis (such as relapsing multiple sclerosis), amyotrophic lateral sclerosis, osteoporosis, diabetes mellitus (such as type I diabetes mellitus and type II diabetes mellitus), arteriosclerosis, irritable bowel syndrome, inflammatory bowel disease, ulcerative colitis, Crohn's disease, Sjogren's syndrome, autoimmune thyroid diseases, Alzheimer's disease, lupus, systemic lupus erythematosus, psoriasis, urticaria (such as chronic spontaneous urticaria, chronic inducible urticaria, and chronic autoimmune urticaria), hepatic insufficiency, allergies (such as atopic eczema, atopic dermatitis, contact dermatitis, and allergic rhinitis), ankylosing spondylitis, dermatitis (such as atopic dermatitis), pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa, myasthenia gravis, autoimmune haemolytic anaemia, antineutrophil cytoplasmic antibody (ANCA)-associated vasculitis, cryoglobulinemia, thrombotic thrombocytopenic purpura, immune thrombocytopenia, complications caused by organ transplantation, xenotransplantation, antibody-mediated rejection (AMR), graft versus host disease, B cell-mediated hyperacute, and acute and chronic transplant rejection reactions;
in some embodiments, the disease or condition or the BTK-mediated disease or condition described in the present disclosure is selected from rheumatoid arthritis, multiple sclerosis, amyotrophic lateral sclerosis, osteoporosis, diabetes mellitus, arteriosclerosis, irritable bowel syndrome, inflammatory bowel disease, ulcerative colitis, Crohn's disease, Sjogren's syndrome, autoimmune thyroid diseases, lupus, systemic lupus erythematosus, psoriasis, urticaria, hepatic insufficiency, allergies, ankylosing spondylitis, dermatitis, pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa and myasthenia gravis.

In some embodiments, the cancer described in the present disclosure is hematologic malignancy; in some embodiments, the cancer is selected from leukaemia, multiple myeloma and malignant lymphoma; in some embodiments, the cancer is selected from acute lymphoblastic leukaemia, chronic lymphocytic leukaemia, acute myeloid leukaemia, chronic myelogenous leukaemia, acute promyelocytic leukaemia, acute granulocytic leukaemia, chronic granulocytic leukaemia, hairy cell leukaemia, small lymphocytic lymphoma, diffuse large B-cell lymphoma, intravascular large B-cell lymphoma, primary effusion lymphoma, follicular lymphoma, mantle cell lymphoma, non-Hodgkin lymphoma, B-cell non-Hodgkin lymphoma, B-cell lymphoma, follicular lymphoma, Waldenström macroglobulinemia and multiple myeloma.

In some embodiments, the cancer described in the present disclosure is selected from the group consisting of:
heart: sarcoma (angiosarcoma, fibrosarcoma, rhabdomyosarcoma, or liposarcoma), myxoma, rhabdomyoma, fibroma, lipoma and teratoma;
lung: lung bronchogenic carcinoma (squamous cell carcinoma, small cell undifferentiated carcinoma, large cell undifferentiated carcinoma, or adenocarcinoma), alveolar (bronchiolar) carcinoma, bronchial adenoma, sarcoma, lymphoma, chondromatous hamartoma, and mesothelioma;
gastrointestinal tract: oesophagus (squamous cell carcinoma, adenocarcinoma, leiomyosarcoma, or lymphoma), stomach (carcinoma, lymphoma, or leiomyosarcoma), pancreas (ductal adenocarcinoma, insulinoma, glucagonoma, gastrinoma, carcinoid tumour, or VIPoma), small intestine (adenocarcinoma, lymphoma, carcinoid tumour, Kaposi sarcoma, leiomyoma, haemangioma, lipoma, neurofibroma, or fibroma), large intestine (adenocarcinoma, tubular adenoma, villous adenoma, hamartoma, or leiomyoma);
urogenital tract: kidneys (adenocarcinoma, Wilms tumour (nephroblastoma), lymphoma, or leukaemia), bladder and urethra (squamous cell carcinoma, transitional cell carcinoma, or adenocarcinoma), prostate (adenocarcinoma, or sarcoma), testes (seminoma, teratoma, embryonal carcinoma, teratoma, choriocarcinoma, sarcoma, stromal cell carcinoma, fibroma, fibroadenoma, adenomatoid tumour, or lipoma);
liver: liver cancer (liver cell carcinoma), cholangiocarcinoma, hepatoblastoma, angiosarcoma, hepatocellular adenoma, or haemangioma;
biliary tract: gallbladder carcinoma, ampullary carcinoma, or cholangiocarcinoma;
bone: osteoblastic sarcoma (osteosarcoma), fibrosarcoma, malignant fibrous histiocytoma, chondrosarcoma, Ewing sarcoma, malignant lymphoma (reticular cell sarcoma), multiple myeloma, malignant giant cell tumour, chordoma, osteochondroma (osteocartilaginous exostosis), benign chondroma, chondroblastoma, chondromyxoid fibroma, osteoid osteoma and giant cell tumour;
nervous system: skull (osteoma, haemangioma, granuloma, xanthoma, or osteitis deformans), meninges (meningioma, meningosarcoma, or gliomatosis), brain (astrocytoma, medulloblastoma, glioma, ependymoma, germ cell tumour, glioblastoma multiforme, oligodendroglioma, schwannoma, retinoblastoma, or congenital tumours, spinal neurofibroma, meningioma, glioma, or sarcoma;
gynaecological: uterus (endometrial cancer), cervix (cervical cancer, or pre-tumour cervical dysplasia), ovary (ovarian cancer (serous cystadenocarcinoma, mucinous cystadenocarcinoma, or unclassified carcinoma), granulosa-thecal cell tumour, Sertoli-stromal cell tumour, dysgerminoma, or malignant teratoma), vulva (squamous cell carcinoma, intraepithelial carcinoma, adenocarcinoma, fibrosarcoma, or melanoma), vagina (clear cell carcinoma, squamous cell carcinoma, botryoid sarcoma (embryonal rhabdomyosarcoma), fallopian tube (carcinoma);
haematological: blood (myeloid leukaemia (acute and chronic), acute lymphoblastic leukaemia, chronic lymphocytic leukaemia, myeloproliferative diseases, multiple myeloma, or myelodysplastic syndrome), Hodgkin disease, or non-Hodgkin lymphoma (malignant lymphoma);
skin: malignant melanoma, basal cell carcinoma, squamous cell carcinoma, Kaposi sarcoma, hydatidiform mole, lipoma, haemangioma, dermatofibroma, keloid, or psoriasis;
and adrenal glands: neuroblastoma.

The active compound can be formulated into a form suitable for administration via any appropriate route, and the compositions of the present disclosure can be formulated using conventional methods with one or more pharmaceutically acceptable carriers. Therefore, the active compounds of the present disclosure can be formulated into various dosage forms for oral administration, injection (e.g., intravenous injection, intramuscular injection, or subcutaneous injection), inhalation, or insufflation. The compounds of the present disclosure can also be formulated into dosage forms such as tablets, hard or soft capsules, aqueous or oily suspensions, emulsions, injectable solutions, dispersible powders or granules, suppositories, lozenges or syrups.

As a general guide, in some embodiments, the active compound is presented in a unit dosage form, or in a manner that allows the patient to self-administer a single dose. The compounds or compositions of the present disclosure can be presented in unit dosage forms such as tablets, capsules, cachets, bottled oral solutions, powders, granules, lozenges, suppositories, reconstitutable powders, or liquid preparations. An appropriate unit dose can be 0.1-1000 mg.

In addition to the active compound, the pharmaceutical composition of the present disclosure can contain one or more auxiliary materials selected from: fillers (diluents), binders, wetting agents, disintegrants, excipients, etc. Depending on the administration method, the composition can contain 0.1% to 99% by weight of the active compound.

In some embodiments, the unit dose of the pharmaceutical composition is 0.001 mg-1000 mg.

In certain embodiments, the pharmaceutical composition comprises 0.01%-99.99% of the aforementioned compound or a pharmaceutically acceptable salt thereof, or an isotopic substitute thereof, on the basis of the total weight of the composition. In certain embodiments, the pharmaceutical composition comprises 0.1%-99.9% of the aforementioned compound or a pharmaceutically acceptable salt thereof, or an isotopic substitute thereof. In certain embodiments, the pharmaceutical composition comprises 0.5%-99.5% of the aforementioned compound or a pharmaceutically acceptable salt thereof, or an isotopic substitute thereof. In certain embodiments, the pharmaceutical composition comprises 1%-99% of the aforementioned compound or a pharmaceutically acceptable salt thereof, or an isotopic substitute thereof. In certain embodiments, the pharmaceutical composition comprises 2%-98% of the aforementioned compound or a pharmaceutically acceptable salt thereof, or an isotopic substitute thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The tablet contains an active ingredient, and a non-toxic, pharmaceutically acceptable excipient suitable for compounding into a tablet. These excipients can be inert excipients, granulators, disintegrants, binders, and lubricants. These tablets can be uncoated, or coated using known techniques to mask the taste of the drug or provide a sustained-release effect over an extended period by delaying disintegration and absorption in the gastrointestinal tract.

Oral preparations can also be provided as soft gelatine capsules in which the active ingredient is mixed with an inert solid diluent or in which the active ingredient is mixed with a water-soluble carrier or an oil-based vehicle.

The aqueous suspension contains an active substance, and an excipient suitable for compounding into an aqueous suspension. Such excipients are suspending agents, dispersants, or wetting agents. The aqueous suspension can also contain one or more preservatives, one or more colourants, one or more flavouring agents, and one or more sweeteners.

The oil suspension can be prepared by suspending active ingredients in vegetable oil or mineral oil. The oil suspension can contain thickeners. The above-described sweeteners and flavouring agents can be added to provide a palatable preparation. These compositions can be preserved by adding antioxidants.

The pharmaceutical composition of the present disclosure can also be in the form of an oil-in-water emulsion. The oil phase can be vegetable oil, mineral oil, or a mixture thereof. Suitable emulsifiers can be naturally occurring phospholipids, and emulsions can also contain sweeteners, flavouring agents, preservatives, and antioxidants. Such preparations can also contain demulcents, preservatives, colourants, and antioxidants.

The pharmaceutical composition of the present disclosure can be in the form of a sterile injectable aqueous solution. Acceptable vehicles or solvents that can be used include water, Ringer's solution, and isotonic sodium chloride solution. Sterile injectable preparations can be sterile injectable oil-in-water microemulsions in which the active ingredient is dissolved in the oil phase. The injectable solutions or microemulsions can be injected into the patient's bloodstream through local high-volume injection. Alternatively, the solutions and microemulsions are preferably administered in a manner that maintains a constant circulating concentration of the compounds of the present disclosure. To maintain such a constant concentration, a continuous intravenous delivery device can be used. An example of such a device is the Deltec CADD-PLUS. TM. 5400 intravenous injection pump.

The pharmaceutical composition of the present disclosure can be in the form of a sterile injectable water or an oil suspension for intramuscular and subcutaneous administration. The suspension can be prepared using the above-described suitable dispersants or wetting agents and suspending agents according to known techniques. Sterile injectable preparations can also be sterile injectable solutions or suspensions prepared in non-toxic diluents or solvents that are acceptable for parenteral administration. Furthermore, sterile fixed oils can conveniently be used as solvents or suspension media. For this purpose, any blended fixed oil can be used. In addition, fatty acids can also be used in the preparation of injections.

The compounds of the present disclosure can be administered in suppository form for rectal administration. These pharmaceutical compositions can be prepared by mixing the drug with a suitable, non-irritating excipient that is solid at normal temperatures but liquid in the rectum, and thus dissolves in the rectum to release the drug.

The compounds of the present disclosure can be prepared by adding water to form water-dispersible powders and granules. These pharmaceutical compositions can be prepared by mixing the active ingredient with a dispersant or a wetting agent, a suspending agent, or one or more preservatives.

As will be appreciated by a person skilled in the art, the administration dosage of the drug depends on a variety of factors, including, but not limited to: the activity of the specific compound used, the patient's age, weight, health status, behaviour and diet, administration time, administration method, excretion rate, combination of drugs, and severity of disease. Furthermore, the optimal treatment method, such as the mode of treatment, the daily dosage of the compound, or the type of pharmaceutically acceptable salts, can be validated by conventional therapeutic protocols.

### Description of Terminology

Unless stated to the contrary, the terms used in the description and claims have the following meanings.

The term "alkyl" refers to a saturated, straight or branched aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). In some embodiments, the alkyl has 1 to 12 carbon atoms (i.e., C₁₋₁₂ alkyl). In some embodiments, the alkyl has 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When substituted, the alkyl can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkylene" refers to a divalent alkyl group, wherein the alkyl, as defined above, has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). In some embodiments, the alkylene has 1 to 12 carbon atoms (i.e., C₁₋₁₂ alkylene). In some embodiments, the alkylene has 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene can be substituted or unsubstituted. When substituted, the alkylene can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkenyl" refers to an alkyl group containing at least one carbon-carbon double bond in the molecule, wherein the alkyl, as defined above, has 2 to 12 (e.g., 2, 3, 4, 5, 6, 7, 8, 9, 10, 11, or 12) carbon atoms (i.e., C₂₋₁₂ alkenyl). In some embodiments, the alkenyl has 2 to 6 carbon atoms (i.e., C₂₋₆ alkenyl). Non-limiting examples include: ethenyl, propenyl, isopropenyl, butenyl, etc. The alkenyl can be substituted or unsubstituted. When substituted, the alkenyl can be substituted at any available point of attachment, and in some embodiments, the substituents are selected from one or more of a D atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkenyl oxide" refers to a branched or unbranched hydrocarbon group containing an epoxy group and having 2-12 carbon atoms, and in some embodiments, it refers to C₂₋₆ alkenyl oxides. Representative examples of the "alkenyl oxides" include ethenyl oxides, propenyl 1, 2-oxides, butenyl 1, 2-oxides, etc.

The term "alkynyl" refers to an alkyl group containing at least one carbon-carbon triple bond in the molecule, wherein the alkyl, as defined above, has 2 to 12 (e.g., 2, 3, 4, 5, 6, 7, 8, 9, 10, 11, or 12) carbon atoms (i.e., C₂₋₁₂ alkynyl). In some embodiments, the alkynyl has 2 to 6 carbon atoms (i.e., C₂₋₆ alkynyl). Non-limiting examples include: ethynyl, propynyl, butynyl, pentynyl, hexynyl, etc. The alkynyl can be substituted or unsubstituted. When substituted, the alkynyl can be substituted at any available point of attachment, and in some embodiments, the substituents are selected from one or more of a D atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples include: methoxy, ethoxy, propoxy, butoxy, etc. The alkoxy can be substituted or unsubstituted. When substituted, the alkoxy can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). In some embodiments, the cycloalkyl has 3 to 12 ring atoms (i.e., 3- to 12-membered cycloalkyl). In some embodiments, the cycloalkyl has 3 to 8 ring atoms (i.e., 3- to 8-membered cycloalkyl). In some embodiments, the cycloalkyl has 3 to 6 ring atoms (i.e., 3- to 6-membered cycloalkyl), has 5 or 6 ring atoms (i.e., 5- or 6-membered cycloalkyl) or has 5 ring atoms (i.e., 5-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The polycyclic cycloalkyl includes: spirocycloalkyl, fused cycloalkyl and bridged cycloalkyl.

The term "spirocycloalkyl" refers to a polycyclic system in which the rings share one carbon atom (called spiro atom), wherein the ring can contain one or more double bonds, or the ring can contain one or more heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-), provided that at least one all-carbon ring is present and the point of attachment is on the all-carbon ring. The spirocycloalkyl has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). In some embodiments, the spirocycloalkyl has 6 to 14 ring atoms (i.e., 6- to 14-membered spirocycloalkyl). In some embodiments, the spirocycloalkyl has 7 to 10 ring atoms (i.e., 7- to 10-membered spirocycloalkyl). The spirocycloalkyl includes monospirocycloalkyl and polyspirocycloalkyl (such as dispirocycloalkyl); in some embodiments, the spirocycloalkyl is monospirocycloalkyl or dispirocycloalkyl; and in some embodiments, the spirocycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl. Non-limiting examples include: 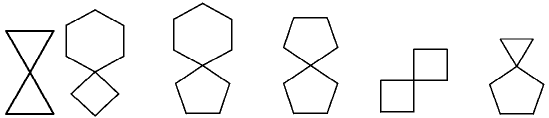 (the point of attachment can be at any position); 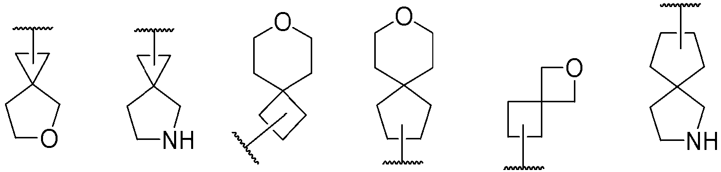 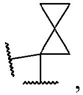 etc.

The term "fused cycloalkyl" refers to a polycyclic system in which the rings share two adjacent carbon atoms, formed by fusing monocyclic cycloalkyl with one or more monocyclic cycloalkyl, or by fusing monocyclic cycloalkyl with one or more of heterocyclyl, aryl, or heteroaryl, wherein the point of attachment is on the monocyclic cycloalkyl, and the ring can contain one or more double bonds. The fused cycloalkyl has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). In some embodiments, the fused cycloalkyl has 6 to 14 ring atoms (i.e., 6- to 14-membered fused cycloalkyl). In some embodiments, the fused cycloalkyl has 7 to 10 ring atoms (i.e., 7- to 10-membered fused cycloalkyl). The fused cycloalkyl includes bicyclic fused cycloalkyl and polycyclic fused cycloalkyl (such as tricyclic fused cycloalkyl and tetracyclic fused cycloalkyl); in some embodiments, the fused cycloalkyl is bicyclic fused cycloalkyl or tricyclic fused cycloalkyl; and in some embodiments, the fused cycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl. Non-limiting examples include: 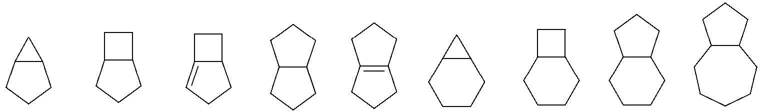 (the point of attachment can be at any position); 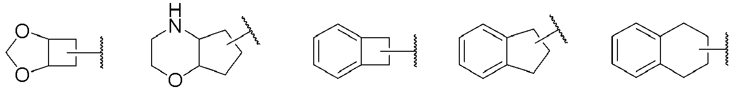 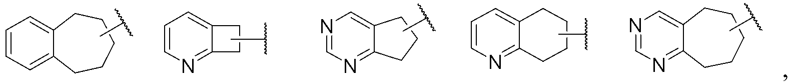 etc.

The term "bridged cycloalkyl" refers to an all-carbon polycyclic system in which the rings share two non-adjacent carbon atoms, wherein the ring can contain one or more double bonds. The bridged cycloalkyl has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). In some embodiments, the bridged cycloalkyl has 6 to 14 carbon atoms (i.e., 6- to 14-membered bridged cycloalkyl). In some embodiments, the bridged cycloalkyl has 7 to 10 carbon atoms (i.e., 7- to 10-membered bridged cycloalkyl). The bridged cycloalkyl includes bicyclic bridged cycloalkyl and polycyclic bridged cycloalkyl (such as tricyclic bridged cycloalkyl and tetracyclic bridged cycloalkyl), and in some embodiments, the bridged cycloalkyl is bicyclic bridged cycloalkyl or tricyclic bridged cycloalkyl. Non-limiting examples include: 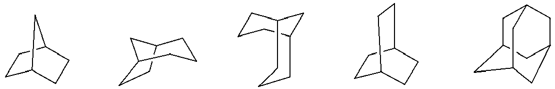 (the point of attachment can be at any position).

The cycloalkyl can be substituted or unsubstituted. When substituted, the cycloalkyl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "heterocyclyl" refers to a saturated or partially unsaturated monocyclic heterocycle (i.e., monocyclic heterocyclyl) or polycyclic heterocyclic system (i.e., polycyclic heterocyclyl), wherein the ring contains at least one (e.g., 1, 2, 3 or 4) heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-). The heterocyclyl has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). In some embodiments, the heterocyclyl has 3 to 12 ring atoms (i.e., 3- to 12-membered heterocyclyl); in some embodiments, the heterocyclyl has 3 to 8 ring atoms (i.e., 3-to 8-membered heterocyclyl); in some embodiments, the heterocyclyl has 3 to 6 ring atoms (i.e., 3- to 6-membered heterocyclyl) or has 4 to 7 ring atoms (i.e., 4- to 7-membered heterocyclyl); in some embodiments, the heterocyclyl has 5 or 6 ring atoms (i.e., 5- or 6-membered heterocyclyl) or 5 ring atoms (i.e., 5-membered heterocyclyl).

Non-limiting examples of the monocyclic heterocycly include: pyrrolidyl, dihydropyrrolyl, dihydrofuryl, tetrahydropyranyl, 1,2,3,6-tetrahydropyridyl, piperidyl, piperazinyl, morpholinyl, thiomorpholinyl, homopiperazinyl, etc.

The polycyclic heterocyclyl includes spiroheterocyclyl, fused heterocyclyl and bridged heterocyclyl. The term "spiroheterocyclyl" refers to a polycyclic heterocyclic system in which the rings share one atom (called spiro atom), wherein the ring can contain one or more double bonds, and the ring contains at least one (e.g., 1, 2, 3 or 4) heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-), provided that at least one monocyclic heterocycly is present and the point of attachment is on the monocyclic heterocycly. The spiroheterocyclyl has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). In some embodiments, the spiroheterocyclyl has 6 to 14 ring atoms (i.e., 6- to 14-membered spiroheterocyclyl). In some embodiments, the spiroheterocyclyl has 7 to 10 ring atoms (i.e., 7- to 10-membered spiroheterocyclyl). In some embodiments, the spiroheterocyclyl has 7 to 9 ring atoms (i.e., 7- to 9-membered spiroheterocyclyl). In some embodiments, the spiroheterocyclyl has 7 or 8 ring atoms (i.e., 7- or 8-membered spiroheterocyclyl). The spiroheterocyclyl includes monospiroheterocyclyl and polyspiroheterocyclyl (such as dispiroheterocyclyl); in some embodiments, the spiroheterocyclyl is monospiroheterocyclyl or dispiroheterocyclyl; and in some embodiments, the spiroheterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocyclyl. Non-limiting examples include: 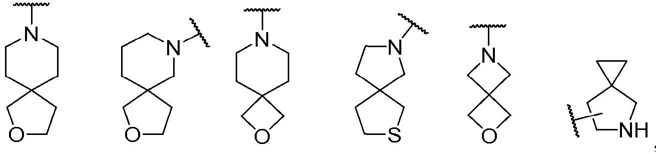 , etc.

The term "fused heterocyclyl" refers to a polycyclic heterocyclic system in which the rings share two adjacent atoms, wherein the ring can contain one or more double bonds, and the ring contains at least one (e.g., 1, 2, 3 or 4) heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-), formed by fusing monocyclic heterocyclyl with one or more monocyclic heterocyclyl, or by fusing monocyclic heterocyclyl with one or more of cycloalkyl, aryl or heteroaryl, wherein the point of attachment is on the monocyclic heterocycly. The fused heterocyclyl has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). In some embodiments, the fused heterocyclyl has 6 to 14 ring atoms (i.e., 6- to 14-membered fused heterocyclyl). In some embodiments, the fused heterocyclyl has 7 to 10 ring atoms (i.e., 7- to 10-membered fused heterocyclyl). In some embodiments, the fused heterocyclyl has 7 to 9 ring atoms (i.e., 7- to 9-membered fused heterocyclyl). In some embodiments, the fused heterocyclyl has 8 or 9 ring atoms (i.e., 8- or 9-membered fused heterocyclyl). The fused heterocyclyl includes bicyclic and polycyclic fused heterocyclyl (such as tricyclic fused heterocyclyl and tetracyclic fused heterocyclyl); in some embodiments, the fused heterocyclyl is bicyclic fused heterocyclyl or tricyclic fused heterocyclyl; in some embodiments, the fused heterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples include: 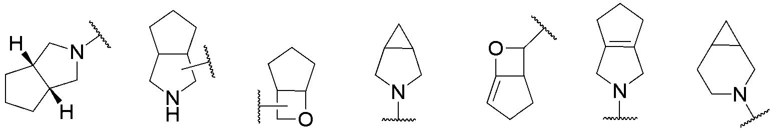 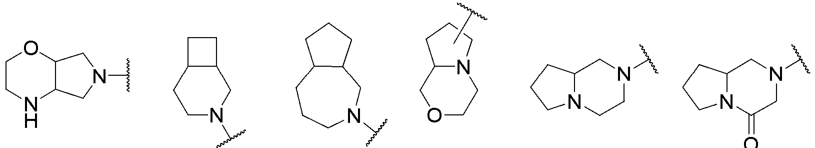 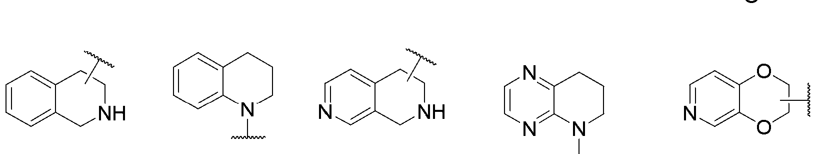 etc.

The term "bridged heterocyclyl" refers to a polycyclic heterocyclic system in which the rings share two non-adjacent atoms, wherein the ring can contain one or more double bonds, and the ring contains at least one (e.g., 1, 2, 3 or 4) heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-). The bridged heterocyclyl has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). In some embodiments, the bridged heterocyclyl has 6 to 14 ring atoms (i.e., 6- to 14-membered bridged heterocyclyl). In some embodiments, the bridged heterocyclyl has 7 to 10 ring atoms (i.e., 7- to 10-membered bridged heterocyclyl). Based on the number of constituent rings, the bridged heterocyclyl can be classified into bicyclic bridged heterocyclyl and polycyclic bridged heterocyclyl (such as tricyclic bridged heterocyclyl and tetracyclic bridged heterocyclyl); and in some embodiments, the bridged heterocyclyl is bicyclic bridged heterocyclyl or tricyclic bridged heterocyclyl. Non-limiting examples include: 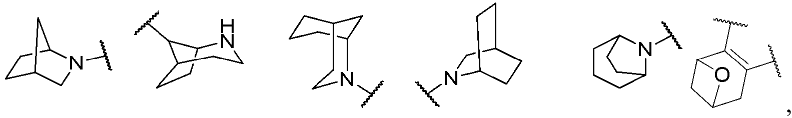 etc.

The heterocyclyl can be substituted or unsubstituted. When substituted, the heterocyclyl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "aryl" refers to a monocyclic all-carbon aromatic ring (i.e., monocyclic aryl) or polycyclic aromatic ring system (i.e., polycyclic aryl) having a conjugated π-electron system. The aryl has 6 to 22 (e.g., 6, 7, 8, 9, 10, 11, 12, 13, 14, 15, 16, 17, 18, 19, 20, 21, or 22) ring atoms (i.e., 6- to 22-membered aryl). In some embodiments, the aryl has 6 to 10 ring atoms (i.e., 6- to 10-membered aryl) or has 10 to 15 ring atoms (i.e., 10- to 15-membered aryl); in some embodiments, the aryl has 11 to 14 ring atoms (i.e., 11- to 14-membered aryl); in some embodiments, the aryl has 8 to 14 ring atoms (i.e., 8- to 14-membered aryl); in some embodiments, the aryl has 11 or 12 ring atoms (i.e., 11- or 12-membered aryl); in some embodiments, the aryl has 11 ring atoms (i.e., 11-membered aryl); the aryl is monocyclic aryl, such as phenyl. Non-limiting examples of the polycyclic aryl include: naphthyl, anthryl, phenanthryl, etc. The polycyclic aryl further includes phenyl fused with one or more of heterocyclyl or cycloalkyl, or naphthyl fused with one or more of heterocyclyl or cycloalkyl, wherein the point of attachment is on the phenyl or naphthyl. In such cases, the number of ring atoms still represents the number of ring atoms in the polycyclic aromatic ring system (in some embodiments, bicyclic aryl or tricyclic aryl), and non-limiting examples include: 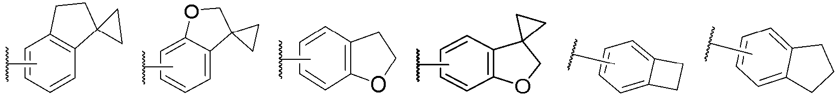 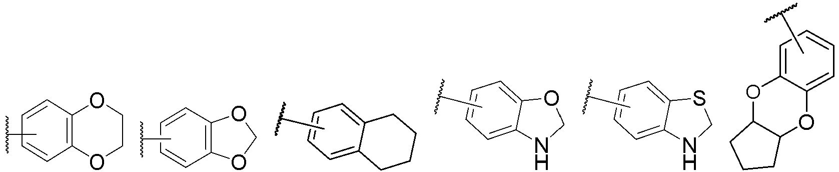 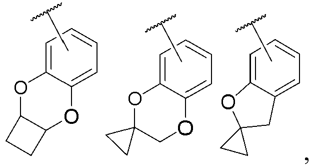 etc.

The aryl can be substituted or unsubstituted. When substituted, the aryl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "tricyclic aryl" refers to a ring system containing 3 rings, wherein the point of attachment is on the phenyl. In some embodiments, the "tricyclic aryl" has 11 to 14 ring atoms (i.e., 11- to 14-membered tricyclic aryl); in some embodiments, the tricyclic aryl has 11 or 12 ring atoms (i.e., 11- or 12-membered tricyclic aryl); in some embodiments, the tricyclic aryl has 11 ring atoms (i.e., 11-membered tricyclic aryl). Taking the ring A in general formula (I) of the present disclosure as an example, non-limiting examples of "tricyclic aryl" include: 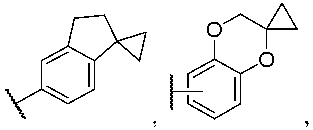 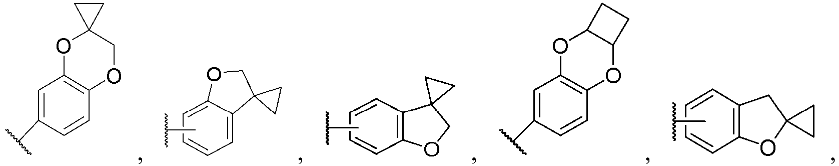 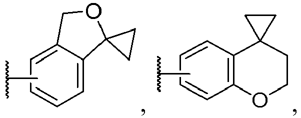 etc.

The term "tetracyclic aryl" refers to a ring system containing 4 rings, wherein the point of attachment is on the phenyl. In some embodiments, the "tetracyclic aryl" has 12 to 19 ring atoms (i.e., 12- to 19-membered tetracyclic aryl); in some embodiments, the "tetracyclic aryl" has 12 to 14 ring atoms (i.e., 12- to 14-membered tetracyclic aryl); in some embodiments, the tetracyclic aryl has 13 ring atoms (i.e., 13-membered tetracyclic aryl). Taking the ring A in general formula (I) of the present disclosure as an example, non-limiting examples of " tetracyclic aryl " include 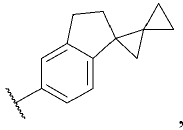 etc.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, wherein the ring contains at least one (e.g., 1, 2, 3 or 4) heteroatoms selected from nitrogen, oxygen and sulfur (the nitrogen can optionally be oxidised, i.e., to form nitrogen oxides; and the sulfur can optionally be oxidised, i.e., to form sulfoxides or sulfones, but excluding -O-O-, -O-S- or -S-S-). The heteroaryl has 5 to 22 (e.g., 5, 6, 7, 8, 9, 10, 11, 12, 13, 14, 15, 16, 17, 18, 19, 20, 21, or 22) ring atoms (i.e., 5- to 22-membered heteroaryl). In some embodiments, the heteroaryl has 5 to 14 ring atoms (i.e., 5- to 14-membered heteroaryl); in some embodiments, the heteroaryl has 5 to 10 ring atoms (i.e., 5- to 10-membered heteroaryl); in some embodiments, the heteroaryl has 9 to 14 ring atoms (i.e., 9- to 14-membered heteroaryl); in some embodiments, the heteroaryl has 8 to 14 ring atoms (i.e., 8- to 14-membered heteroaryl); in some embodiments, the heteroaryl has 11 or 12 ring atoms (i.e., 11- or 12-membered heteroaryl); in some embodiments, the heteroaryl has 10 ring atoms (i.e., 10-membered heteroaryl); in some embodiments, the heteroaryl has 5 or 6 ring atoms (i.e., 5- or 6-membered heteroaryl); in some embodiments, the heteroaryl has 5 ring atoms (i.e., 5-membered heteroaryl).

Non-limiting examples of the monocyclic heteroaryl include: furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkyl pyrrolyl, pyridyl, pyrimidyl, pyridonyl, N-alkylpyridone (such as 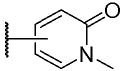 ), pyrazinyl, pyridazinyl, etc.

Non-limiting examples of the polycyclic heteroaryl (in some embodiments, 9-to 14-membered heteroaryl, and in some embodiments, 8- to 14-membered heteroaryl) include: indolyl, indazolyl, quinolyl, isoquinolyl, quinoxalinyl, phthalazinyl, benzoimidazolyl, benzothienyl, quinazolinyl, benzothiazolyl, carbazolyl, etc. The polycyclic heteroaryl further includes monocyclic heteroaryl fused with one or more aryl, wherein the point of attachment is on the aromatic ring. In such cases, the number of ring atoms still represents the number of ring atoms in the polycyclic heteroaromatic ring system. The polycyclic heteroaryl further includes monocyclic heteroaryl fused with one or more of cycloalkyl or heterocyclyl, wherein the point of attachment is on the monocyclic heteroaromatic ring. In such cases, the number of ring atoms still represents the number of ring atoms in the polycyclic heteroaromatic ring system (in some embodiments, the polycyclic heteroaryl is bicyclic heteroaryl or tricyclic heteroaryl). Non-limiting examples include: 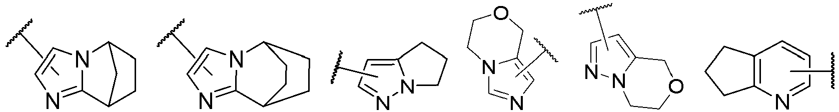 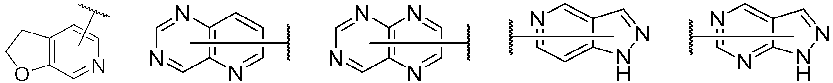 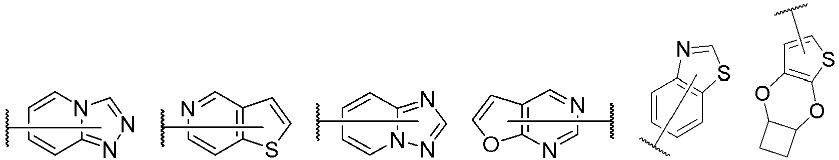 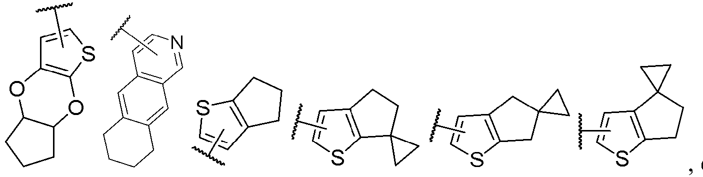 etc.

The term "tricyclic heteroaryl" refers to a ring system containing 3 rings, wherein the point of attachment is on the monocyclic heteroaromatic ring. In some embodiments, the "tricyclic heteroaryl" has 9 to 14 ring atoms (i.e., 9- to 14-membered tricyclic heteroaryl); in some embodiments, the tricyclic heteroaryl has 10 to 12 ring atoms (i.e., 10- to 12-membered tricyclic heteroaryl); in some embodiments, the tricyclic heteroaryl has 10 ring atoms (i.e., 10-membered tricyclic heteroaryl).

The heteroaryl can be substituted or unsubstituted. When substituted, the heteroaryl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "cycloalkylalkyl" refers to an alkyl group substituted with one or more cycloalkyl groups, wherein the alkyl and cycloalkyl are as defined above.

The term "heterocyclylalkyl" refers to an alkyl group substituted with one or more heterocyclyl groups, wherein the alkyl and heterocyclyl are as defined above.

The term "arylalkyl" refers to an alkyl group substituted with one or more aryl groups, wherein the alkyl and aryl are as defined above, such as -CH₂Ph.

The term "heteroarylalkyl" refers to an alkyl group substituted with one or more heteroaryl groups, wherein the alkyl and heteroaryl are as defined above.

The term "haloalkyl" refers to an alkyl group substituted with one or more halogen, wherein the alkyl is as defined above.

The term "haloalkoxy" refers to an alkoxy group substituted with one or more halogen, wherein the alkoxy is as defined above, such as-OCF₃.

The term "hydroxyalkyl" refers to an alkyl group substituted with one or more hydroxyl groups, wherein the alkyl is as defined above, such as -CH₂OH.

The term "alkoxyalkyl" refers to an alkyl group substituted with one or more alkoxy groups, wherein the alkyl and alkoxy are as defined above, such as - CH₂OCH₃.

The term "aminoalkyl" refers to an alkyl group substituted with one or more amino groups, wherein the alkyl is as defined above, such as -CH₂NH₂.

The term "methylidene" refers to=CH₂.

The term "halogen" refers to fluorine, chlorine, bromine or iodine.

The term "hydroxyl" refers to -OH.

The term "mercapto" refers to -SH.

The term "amino" refers to -NH₂.

The term "cyano" refers to -CN.

The term "nitro" refers to -NO₂.

The term "oxo" or "oxo group" refers to "=O".

The term "carbonyl" refers to C=O.

The term "carboxyl" refers to -C(O)OH.

The term "carboxylate ester group" refers to -C(O)O(alkyl), - C(O)O(cycloalkyl), (alkyl)C(O)O- or (cycloalkyl)C(O)O-, wherein the alkyl and cycloalkyl are as defined above.

In the general formula (III) of the present disclosure, when j is 0, it is equivalent to A¹ or A² being a bond.

The compounds of the present disclosure can exist in specific stereoisomeric forms. The term "stereoisomer" refers to isomers with the same structure but different spatial arrangements of atoms. It includes cis- and trans- (or *Z*- and *E*-) isomers, (-)- and (+)-isomers, (*R*)- and (*S*)-enantiomers, diastereomers, *(D*)- and (*L*)-isomers, tautomers, atropisomer, conformational isomers and mixtures thereof (such as mixtures of racemates and diastereomers). The substituents in the compounds of the present disclosure can contain additional asymmetric atoms. All these stereoisomer and mixtures thereof are encompassed within the scope of the present disclosure. Optically active (-)- and (+)-isomers, (*R*)- and (*S*)-enantiomers, and *(D*)- and (*L*)-isomers can be prepared by using chiral synthesis, chiral reagents or other conventional techniques. An isomer of a compound of the present disclosure can be prepared by using asymmetric synthesis or chiral auxiliaries. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art to obtain the pure isomers. In addition, the separation of enantiomers and diastereomers is usually performed by chromatography.

In the chemical structures of the compounds of the present disclosure, the bond " 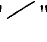 " represents an unspecified configuration; that is, if the chemical structure exists as stereoisomers, the bond " 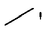 " can be " 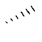 " or " 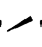 ", or both the configurations of " 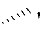 " and " 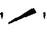 " are involved. For all carbon-carbon double bonds, both the *Z*- and *E*-forms are included, even if only one configuration is named.

The compounds of the present disclosure can also exist as different tautomeric forms, and all such forms are encompassed within the scope of the present disclosure. The term "tautomer" or "tautomeric form" refers to a structural isomer that exists in equilibrium and is easily transformed from one isomeric form to another. All possible tautomers are included, whether they exist as a single isomeric form or as a mixture in any proportion of the tautomers. Non-limiting examples include: ketoenols, imine-enamines, lactam-lactim, etc.

For example, the compound should be understood to encompass either of the following structures or a mixture of both tautomers:

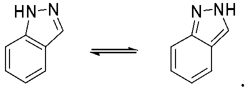

All tautomeric forms fall within the scope of the present disclosure, and the naming of the compound does not exclude any tautomer.

The compounds of the present disclosure include all suitable isotopic derivatives thereof. The term "isotopic derivative" refers to a compound in which at least one atom is replaced by an atom having the same atomic number but a different atomic mass. Examples of isotopes that can be introduced into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D),³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I. In some embodiments, the isotope is deuterium.

Deuterated drugs offer advantages over their non-deuterated counterparts, such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom can be independently replaced by a deuterium atom, where such deuterium replacement can be partial or complete, and partial deuterium replacement means that at least one hydrogen atom is replaced by at least one deuterium atom.

For the compounds of the present disclosure, when a position is specifically designated as "deuterium" or "D," it should be understood that the position has a deuterium abundance at least 1000 times greater than the natural abundance of deuterium (which is 0.015%) (i.e., at least 15% deuterium incorporation). In some embodiments, the deuterium abundance in each designated deuterium atom is at least 1000 times greater than the natural abundance of deuterium (i.e., at least 15% deuterium incorporation). In some embodiments, the deuterium abundance in each designated deuterium atom is at least 2000 times greater than the natural abundance of deuterium (i.e., at least 30% deuterium incorporation). In some embodiments, the deuterium abundance in each designated deuterium atom is at least 3000 times greater than the natural abundance of deuterium (i.e., at least 45% deuterium incorporation). In some embodiments, the deuterium abundance in each designated deuterium atom is at least 3340 times greater than the natural abundance of deuterium (i.e., at least 50.1% deuterium incorporation). In some embodiments, the deuterium abundance in each designated deuterium atom is at least 3500 times greater than the natural abundance of deuterium (i.e., at least 52.5% deuterium incorporation). In some embodiments, the deuterium abundance in each designated deuterium atom is at least 4000 times greater than the natural abundance of deuterium (i.e., at least 60% deuterium incorporation). In some embodiments, the deuterium abundance in each designated deuterium atom is at least 4500 times greater than the natural abundance of deuterium (i.e., at least 67.5% deuterium incorporation). In some embodiments, the deuterium abundance in each designated deuterium atom is at least 5000 times greater than the natural abundance of deuterium (i.e., at least 75% deuterium incorporation). In some embodiments, the deuterium abundance in each designated deuterium atom is at least 5500 times greater than the natural abundance of deuterium (i.e., at least 82.5% deuterium incorporation). In some embodiments, the deuterium abundance in each designated deuterium atom is at least 6000 times greater than the natural abundance of deuterium (i.e., at least 90% deuterium incorporation). In some embodiments, the deuterium abundance in each designated deuterium atom is at least 6333.3 times greater than the natural abundance of deuterium (i.e., at least 95% deuterium incorporation). In some embodiments, the deuterium abundance in each designated deuterium atom is at least 6466.7 times greater than the natural abundance of deuterium (i.e., at least 97% deuterium incorporation). In some embodiments, the deuterium abundance in each designated deuterium atom is at least 6600 times greater than the natural abundance of deuterium (i.e., at least 99% deuterium incorporation). In some embodiments, the deuterium abundance in each designated deuterium atom is at least 6633.3 times greater than the natural abundance of deuterium (i.e., at least 99.5% deuterium incorporation).

The term "optionally" or "optional" means that the event or circumstance subsequently described may but does not necessarily occur, encompassing both the case where it occurs and the case where it does not occur. For example, "alkyl optionally substituted with halogen or cyano" includes both the case where the alkyl is substituted with halogen or cyano and the case where the alkyl is not substituted with halogen or cyano.

The term "substitution" or "substituted" means that one or more hydrogen atoms in a group, in some embodiments 1 to 6 hydrogen atoms, and in some embodiments 1 to 3 hydrogen atoms, are each independently substituted with a corresponding number of substituents. A person skilled in the art would have been able to determine the possible or impossible substitutions (by experiment or theory) without undue effort. For example, an amino or hydroxyl group having free hydrogen may be unstable when bonded to a carbon atom bearing an unsaturated bond (e.g., in alkene).

The term "pharmaceutical composition" means a mixture containing one or more compounds described herein or the pharmaceutically acceptable salts thereof together with other chemical components, and additional components such as pharmaceutically acceptable carriers and excipients. The purpose of the pharmaceutical compositions is to facilitate administration to living organisms and facilitate the absorption of active ingredients to exert biological activity.

The term "pharmaceutically acceptable salt" refers to a salt of the compound of the present disclosure, which can be selected from an inorganic salt or an organic salt. Such salts are safe and effective when used in mammals, and possess the expected biological activity. The salts can be prepared either during the final separation and purification of the compound, or separately by reacting suitable functional groups with appropriate bases or acids. The bases commonly used to form pharmaceutically acceptable salts include inorganic bases such as sodium hydroxide and potassium hydroxide, and organic bases such as ammonia. The acids commonly used to form pharmaceutically acceptable salts include inorganic acids and organic acids.

As used herein, the term "pharmaceutically acceptable" refers to those compounds, materials, compositions and/or dosage forms that are, within the scope of sound medical judgment, suitable for use in contact with patient tissues without excessive toxicity, irritation, allergic response, or other problems or complications, commensurate with a reasonable benefit/risk ratio, and effective for the intended use.

As used herein, the singular forms "a," "an," and "the" include plural referents and vice versa, unless otherwise clearly indicated in the context.

When the term "about" is applied to parameters such as pH, concentration and temperature, it indicates that the parameter can vary by ±10%, and in some embodiments, within ±5%. As will be understood by a person skilled in the art, numerical values for non-critical parameters are generally provided for illustrative purposes only and not as limitations.

### Brief Description of the Drawings

Figure 1 shows the degree of skin swelling in mice.
Figure 2 shows percentage inhibition of skin swelling by compound 3 in mice.

### Detailed Description of Embodiments

The present disclosure will be further described below in conjunction with examples, but these examples are not intended to limit the scope of the present disclosure.

### Example

The structures of the compounds are determined by nuclear magnetic resonance (NMR) or/and mass spectrometry (MS). The NMR shift (δ) is given in the unit of 10⁻⁶ (ppm). NMR determination is performed by Bruker AVANCE NEO 500M nuclear magnetic resonance spectrometer; the solvents for determination are deuterated dimethyl sulfoxide (DMSO-*d₆*), deuterated chloroform (CDCl₃) and deuterated methanol (CD₃OD); and the internal standard is tetramethylsilane (TMS).

MS determination is carried out by Agilent 1200 /1290 DAD- 6110/6120 Quadrupole MS liquid chromatography-mass spectrometer (manufacturer: Agilent, MS model: 6110/6120 Quadrupole MS);
waters ACQuity UPLC-QD/SQD (manufacturer: waters, MS model: waters ACQuity Qda Detector/waters SQ Detector); and
THERMO Ultimate 3000-Q Exactive (manufacturer: THERMO, MS model: THERMO Q Exactive).

High-performance liquid chromatography (HPLC) analysis is carried out by Agilent HPLC 1200DAD, Agilent HPLC 1200VWD and Waters HPLC e2695-2489 high-performance liquid chromatographs.

Chiral HPLC analysis is determined by Agilent 1260 DAD high-performance liquid chromatograph.

Preparative high-performance liquid chromatography is carried out by Waters 2545-2767, Waters 2767-SQ Detecor2, Shimadzu LC-20AP and Gilson GX-281 preparative chromatographs.

Chiral preparative chromatography is carried out by Shimadzu LC-20AP preparative chromatograph.

The CombiFlash flash preparative instrument used is Combiflash Rf200 (TELEDYNE ISCO).

Yantai Huanghai HSGF254 or Qingdao GF254 silica gel plate is used as a thin layer chromatography silica plate, and the silica gel plate for the thin layer chromatography (TLC) is of the specification of 0.15 mm - 0.2 mm, and the specification when separating and purifying a product by thin layer chromatography is 0.4 mm - 0.5 mm.

For silica gel column chromatography, Yantai Huanghai silica gel 200-300 mesh silica gel is generally used as a carrier.

The determination of the average kinase inhibition rate and IC₅₀ value is performed using NovoStar microplate reader (BMG Company, Germany).

Known starting materials of the present disclosure may be synthesised using or according to methods known in the art, or may be purchased from ABCR GmbH & Co. KG, Acros Organics, Aldrich Chemical Company, Accela ChemBio Inc., Chembee Chemicals, and other companies.

Unless otherwise specified, the reactions in the examples can be carried out under an argon atmosphere or a nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Pressurised hydrogenation reactions are performed using Parr 3916EKX hydrogenator, Qinglan QL-500 hydrogen generator or HC2-SS hydrogenator.

The hydrogenation reaction usually comprises the steps of vacuumizing and charging with hydrogen, and the operation is repeated 3 times.

CEM Discover-S 908860 microwave reactor is used for the microwave reaction.

Unless otherwise specified in the examples, the solution refers to an aqueous solution.

Unless otherwise specified in the examples, the reaction temperature refers to room temperature.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, B: n-hexane/ethyl acetate system, C: petroleum ether/ethyl acetate system, wherein the volume ratio of the solvent is adjusted according to the polarity of compounds and can also be adjusted by adding a small quantity of basic reagents such as triethylamine or acidic reagents such as acetic acid.

### Example 1

### N-(3-(6-amino-5-(2-(N-methacrylamido)ethoxy)pyrimidin-4-yl)-5-fluoro-2-methylphenyl)-6'-fluoro-2',3'-dihydrospiro[cyclopropane-1,1'-indene]-5'-carboxamide 1

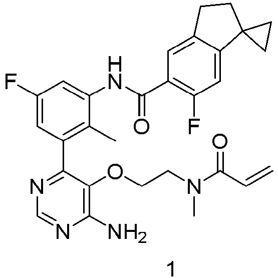

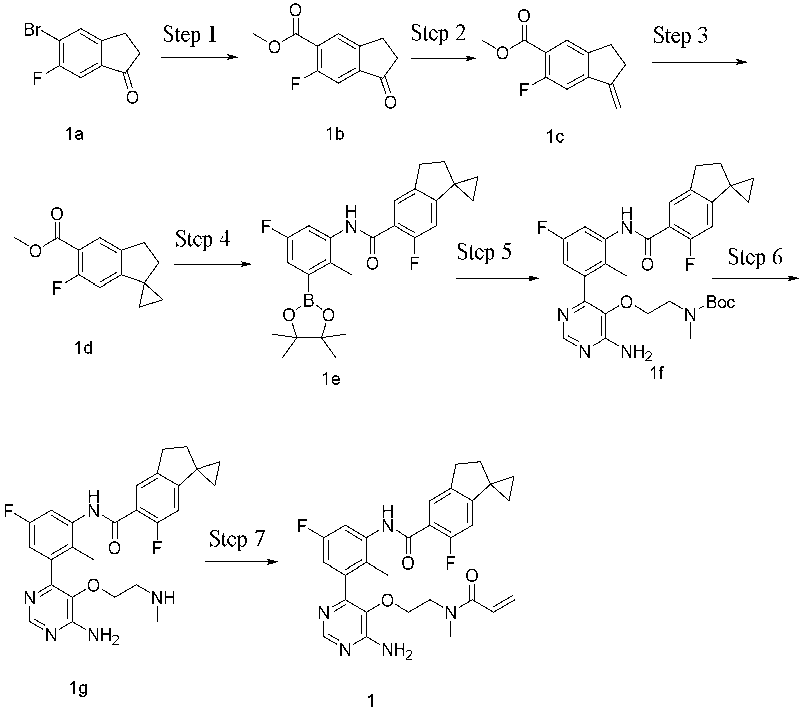

### Step 1

Methyl 6-fluoro-1-oxo-2,3-dihydro-1*H*-indene-5-carboxylate **1b** 5-Bromo-6-fluoro-2,3-dihydro-1*H*-indene-1-one **1a** (500 mg, 2.18 mmol, Bidepharm) was dissolved in *N,N*-dimethylformamide (5 mL, Sinopharm) and methanol (2.5 mL, Sinopharm). [1,1'-bis(diphenylphosphino)ferrocene]palladium dichloride (160 mg, 0.22 mmol, Adamas) and *N,N*-diisopropylethylamine (565 mg, 4.37 mmol, Adamas) were added. The mixture was purged 3 times with carbon monoxide, reacted overnight at 80°C, and concentrated under reduced pressure. The residue was purified by column chromatography with eluent system B to obtain the title compound **1b** (170 mg, yield: 37.4%).

### Step 2

### Methyl 6-fluoro-1-methylidene-2,3-dihydro-1H-indene-5-carboxylate 1c

Compound **1b** (170 mg, 0.82 mmol) was dissolved in tetrahydrofuran (5 mL, Energy Chemical), and methyltriphenylphosphonium bromide (584 mg, 1.63 mmol, Shaoyuan Technology) and potassium tert-butoxide (184 mg, 1.64 mmol, Adamas) were added. The mixture was reacted at room temperature for 2 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound **1c** (90 mg, yield: 53.4%).

MS m/z (ESI): 207.3 [M+1]

### Step 3

### Methyl 6'-fluoro-2',3'-dihydrospiro[cyclopropane-1,1'-indene]-5'-carboxylate 1d

Diethylzinc (1 M, 1.75 mL, 1.75 mmol, Adamas) was dissolved in dichloromethane (1 mL, Energy Chemical). Under a nitrogen atmosphere and at 0°C, trifluoroacetic acid (200 mg, 1.75 mmol, Adamas) was added dropwise. At 0°C, the mixture was reacted for 1 hour, and diiodomethane (468 mg, 1.75 mmol, Energy Chemical) was added dropwise. At 0°C, the mixture was reacted for 0.5 hours, and a solution of compound **1c** (90 mg, 0.44 mmol) in dichloromethane (1 mL, Energy Chemical) was added dropwise. The mixture was reacted at room temperature for 3 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound **1d** (78 mg, yield: 81.1%).

MS m/z (ESI): 221.3 [M+1]

### Step 4

### 6'-Fluoro-N-(5-fluoro-2-methyl-3-(4,4,5,5-tetramethyl-1,3,2-dioxaborolan-2-yl)phenyl)-2',3' dihydrospiro[cyclopropane-1,1'-indene]-5'-carboxamide 1e

Compound **1d** (39 mg, 0.18 mmol) was dissolved in tetrahydrofuran (1 mL, Energy Chemical), and 5-fluoro-2-methyl-3-(4,4,5,5-tetramethyl-1,3,2-dioxaborolan-2-yl)aniline (49 mg, 0.20 mmol, prepared according to the method disclosed in patent application WO 2015079417A1, description, page 31, for intermediate INT3) was added. The mixture was purged 3 times with nitrogen. Sodium bis(trimethylsilyl)amide (2 M, 0.13 mL, 0.13 mmol, Adamas) was added dropwise at 0°C, and the mixture was stirred at room temperature for 2 hours. 2 mL of saturated sodium bicarbonate solution was added, and the mixture was extracted with ethyl acetate (2 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound **1e** (30 mg, yield: 38.6%).

MS m/z (ESI): 440.5 [M+1]

### Step 5

### Tert-butyl (2-((4-amino-6-(5-fluoro-3-(6'-fluoro-2',3'-dihydrospiro[cyclopropane-1,1'-indene]-5'-carboxamido)-2-methylphenyl)pyrimidin-5-yl)oxy)ethyl)(methyl)carbamate 1f

The crude compound **1e** (30 mg, 0.07 mmol) was dissolved in ethylene glycol dimethyl ether (1 mL, Sinopharm) and water (0.3 mL, Sinopharm). Bis(triphenylphosphine)palladium chloride (4.8 mg, 0.01 mmol, Adamas), tert-butyl (2-((4-amino-6-chloropyrimidin-5-yl)oxy)ethyl))methyl)carbamate (25 mg, 0.08 mmol, prepared according to the method disclosed in patent application WO 2015079417A1, description, page 39, for intermediate INT8) and sodium carbonate (15 mg, 0.14 mmol, Sinopharm) were added. The mixture was purged 3 times with nitrogen, and reacted under microwave conditions at 110°C for 1 hour. 3 mL of water was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound **1f** (30 mg, yield: 75.8%).

MS m/z (ESI): 580.2 [M+1]

### Step 6

### N-(3-(6-amino-5-(2-(methylamino)ethoxy)pyrimidin-4-yl)-5-fluoro-2-methylphenyl)-6'-fluoro-2',3'-dihydrospiro[cyclopropane-1,1'-indene]-5'-carboxamide 1g

The crude compound **1f** (30 mg, 0.05 mmol) was dissolved in dichloromethane (1 mL, Sinopharm), and trifluoroacetic acid (462 mg, 4.05 mmol, Adamas) was added. The mixture was reacted at room temperature for 1 hour. The reaction solution was concentrated under reduced pressure. 3 mL of saturated sodium bicarbonate was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure to obtain the crude title compound **1g** (24.8 mg), which was directly used in the next reaction without purification.

MS m/z (ESI): 480.6 [M+1]

### Step 7

### N-(3-(6-amino-5-(2-(N-methacrylamido)ethoxy)pyrimidin-4-yl)-5-fluoro-2-methylphenyl)-6'-fluoro-2',3'-dihydrospiro[cyclopropane-1,1'-indene]-5'-carboxamide 1

The crude compound **1g** (24.8 mg, 0.05 mmol) was dissolved in dichloromethane (1 mL, Sinopharm), and *N,N*-diisopropylethylamine (565 mg, 4.37 mmol, Adamas) was added. Acryloyl chloride (4.7 mg, 0.05 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound 1 (12 mg, yield: 43.5%).
MS m/z (ESI): 534.5 [M+1]
¹H NMR (500 MHz, DMSO-*d₆*): δ 9.75-9.57 (m, 1H), 8.21-8.18 (m, 1H), 7.71-7.50 (m, 2H), 7.23-6.98 (m, 3H), 6.78-6.65 (m, 2H), 6.06-6.03 (m, 1H), 5.60-5.58 (m, 1H), 3.58-3.47 (m, 4H), 3.04-3.02 (m, 2H), 2.80 (s, 3H), 2.17-2.14 (m, 2H), 2.01-1.96 (m, 3H), 1.24-1.22 (m, 2H), 1.03-1.02 (m, 2H).

### Example 2

### N-(3-(6-amino-5-(2-(N-methacrylamido)ethoxy)pyrimidin-4-yl)-5-fluoro-2-methylphenyl)-2-fluoro-4-(1-methylcyclopropyl)benzamide 2

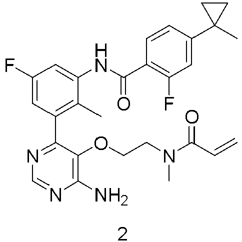

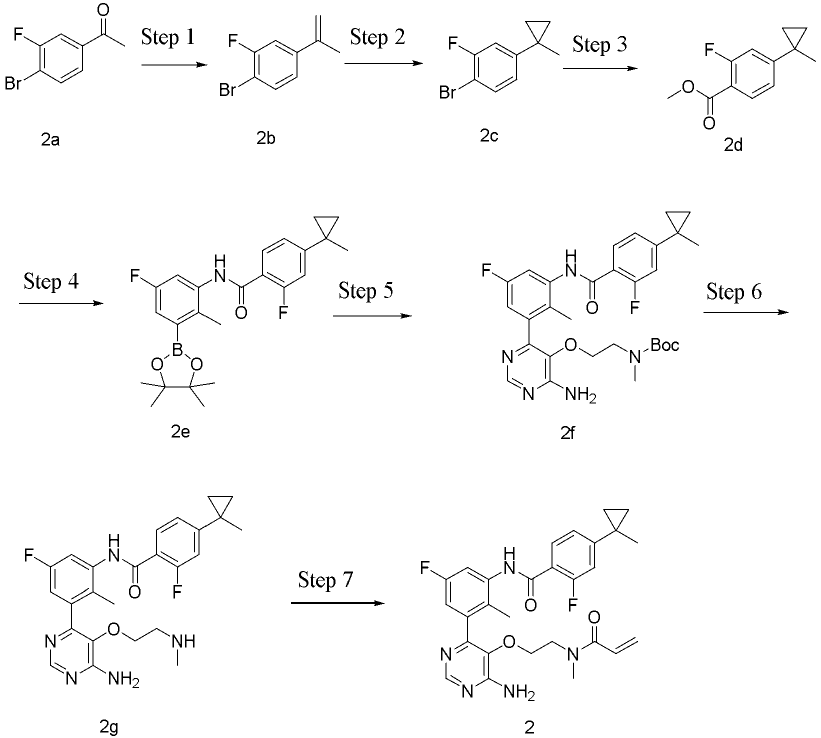

### Step 1

### 1-Bromo-2-fluoro-4-(prop-1-en-2-yl)benzene 2b

1-(4-Bromo-3-fluorophenyl)ethan-1-one **2a** (1 g, 4.61 mmol, Shaoyuan Technology) was dissolved in tetrahydrofuran (20 mL, Energy Chemical), and methyltriphenylphosphonium bromide (3.3 g, 9.24 mmol, Shaoyuan Technology) and potassium tert-butoxide (1.04 g, 9.27 mmol, Adamas) were added. The mixture was reacted at room temperature for 2 hours. 20 mL of water was added, and the mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system B to obtain the title compound **2b** (780 mg, yield: 78.7%).

### Step 2

### 1-Bromo-2-fluoro-4-(1-methylcyclopropyl)benzene 2c

Diethylzinc (1 M, 6.52 mL, 6.52 mmol, Adamas) was dissolved in dichloromethane (3 mL, Energy Chemical). Under a nitrogen atmosphere and at 0°C, trifluoroacetic acid (743 mg, 6.52 mmol, Adamas) was added dropwise. At 0°C, the mixture was reacted for 1 hour, and diiodomethane (1.75 g, 6.54 mmol, Energy Chemical) was added dropwise. At 0°C, the mixture was reacted for 0.5 hours, and a solution of compound **2b** (350 mg, 1.63 mmol) in dichloromethane (3 mL, Energy Chemical) was added dropwise. The mixture was reacted at room temperature for 3 hours. 15 mL of water was added, and the mixture was extracted with ethyl acetate (15 mL×3). The organic phases were combined, washed with 15 mL of saturated sodium chloride solution, dried over anhydrous sodium sulfate, filtered and concentrated under reduced pressure. The residue was purified by column chromatography with eluent system B to obtain the title compound **2c** (240 mg, yield: 64.4%).

### Step 3

### Methyl 2-fluoro-4-(1-methylcyclopropyl)benzoate 2d

Compound **2c** (240 mg, 1.05 mmol) was dissolved in *N,N*-dimethylacetamide (3 mL, Sinopharm) and methanol (3 mL, Sinopharm), and 1,3-bis(diphenylphosphino)propane (87 mg, 0.21 mmol, Shaoyuan Technology), *N,N-*diisopropylethylamine (565 mg, 4.37 mmol, Adamas) and palladium acetate (48 mg, 0.21 mmol, Adamas) were added. The mixture was purged 3 times with carbon monoxide, reacted overnight at 80°C, and concentrated under reduced pressure. The residue was purified by column chromatography with eluent system B to obtain the title compound **2d** (140 mg, yield: 64.2%).

### Step 4

### 2-Fluoro-N-(5-fluoro-2-methyl-3-(4,4,5,5-tetramethyl-1,3,2-dioxaborolan-2-yl)phenyl)-4-(1-methylcyclopropyl)benzamide 2e

Compound **2d** (140 mg, 0.67 mmol) was dissolved in tetrahydrofuran (3 mL, Energy Chemical), and 5-fluoro-2-methyl-3-(4,4,5,5-tetramethyl-1,3,2-dioxaborolan-2-yl)aniline (186 mg, 0.74 mmol, prepared according to the method disclosed in patent application WO 2015079417A1, description, page 31, for intermediate INT3) was added. The mixture was purged 3 times with nitrogen. Sodium bis(trimethylsilyl)amide (2 M, 0.51 mL, 1.02 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound **2e** (85 mg, yield: 29.6%).

MS m/z (ESI): 428.3 [M+1]

### Step 5

### Tert-butyl (2-((4-amino-6-(5-fluoro-3-(2-fluoro-4-(1-methylcyclopropyl)benzamido)-2-methylphenyl)pyrimidin-5-yl)oxy)ethyl)(methyl)carbamate 2f

Compound **2e** (85 mg, 0.20 mmol) was dissolved in ethylene glycol dimethyl ether (2 mL, Sinopharm) and water (0.8 mL, Sinopharm). Bis(triphenylphosphine)palladium chloride (14 mg, 0.02 mmol, Adamas), tert-butyl (2-((4-amino-6-chloropyrimidin-5-yl)oxy)ethyl)(methyl)carbamate (73 mg, 0.24 mmol, prepared according to the method disclosed in patent application WO 2015079417A1, description, page 39, for intermediate INT8) and sodium carbonate (43 mg, 0.41 mmol, Sinopharm) were added. The mixture was purged 3 times with nitrogen, and reacted under microwave conditions at 110°C for 1 hour. 10 mL of water was added, and the mixture was extracted with ethyl acetate (10 mL×3). The organic phases were combined, washed with 10 mL of saturated sodium chloride solution, dried over anhydrous sodium sulfate, filtered and concentrated under reduced pressure. The residue was purified by column chromatography with eluent system A to obtain the title compound **2f** (110 mg, yield: 97.4%).

MS m/z (ESI): 568.6 [M+1]

### Step 6

### N-(3-(6-amino-5-(2-(methylamino)ethoxy)pyrimidin-4-yl)-5-fluoro-2-methylphenyl)-2-fluoro-4-(1-methylcyclopropyl)benzamide 2g

Compound **2f** (110 mg, 0.19 mmol) was dissolved in dichloromethane (2 mL, Sinopharm), and trifluoroacetic acid (770 mg, 6.75 mmol, Adamas) was added. The mixture was reacted at room temperature for 1 hour. The reaction solution was concentrated under reduced pressure. 5 mL of saturated sodium bicarbonate was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound **2g** (90 mg, yield: 99.3%), which was directly used in the next reaction without purification.

MS m/z (ESI): 468.4 [M+1]

### Step 7

### N-(3-(6-amino-5-(2-(N-methacrylamido)ethoxy)pyrimidin-4-yl)-5-fluoro-2-methylphenyl)-2-fluoro-4-(1-methylcyclopropyl)benzamide 2

The crude compound **2g** (90 mg, 0.19 mmol) was dissolved in dichloromethane (2 mL, Sinopharm), and *N,N*-diisopropylethylamine (125 mg, 0.97 mmol, Adamas) was added. Acryloyl chloride (18 mg, 0.20 mmol, Sinopharm) was added dropwise at -20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **2** (18 mg, yield: 17.9%).
MS m/z (ESI): 522.5 [M+1]
¹H NMR (500 MHz, DMSO-*d₆*): δ 9.81-9.59 (m, 1H), 8.21-8.18 (m, 1H), 7.78-7.56 (m, 2H), 7.21-6.97 (m, 4H), 6.67-6.57 (m, 2H), 6.10-6.02 (m, 1H), 5.61-5.58 (m, 1H), 3.59-3.45 (m, 4H), 2.53 (s, 3H), 2.02-1.97 (m, 3H), 1.44-1.43 (m, 3H), 0.99-0.86 (m, 4H).

### Example 3

### N-(3-(6-amino-5-(2-(N-methacrylamido)ethoxy)pyrimidin-4-yl)-5-fluoro-2-methylphenyl)-5-fluoro-2H-spiro[benzofuran-3,1'-cyclopropane]-6-carboxamide 3

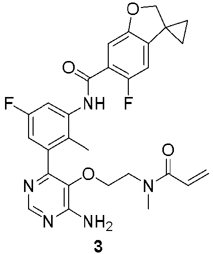

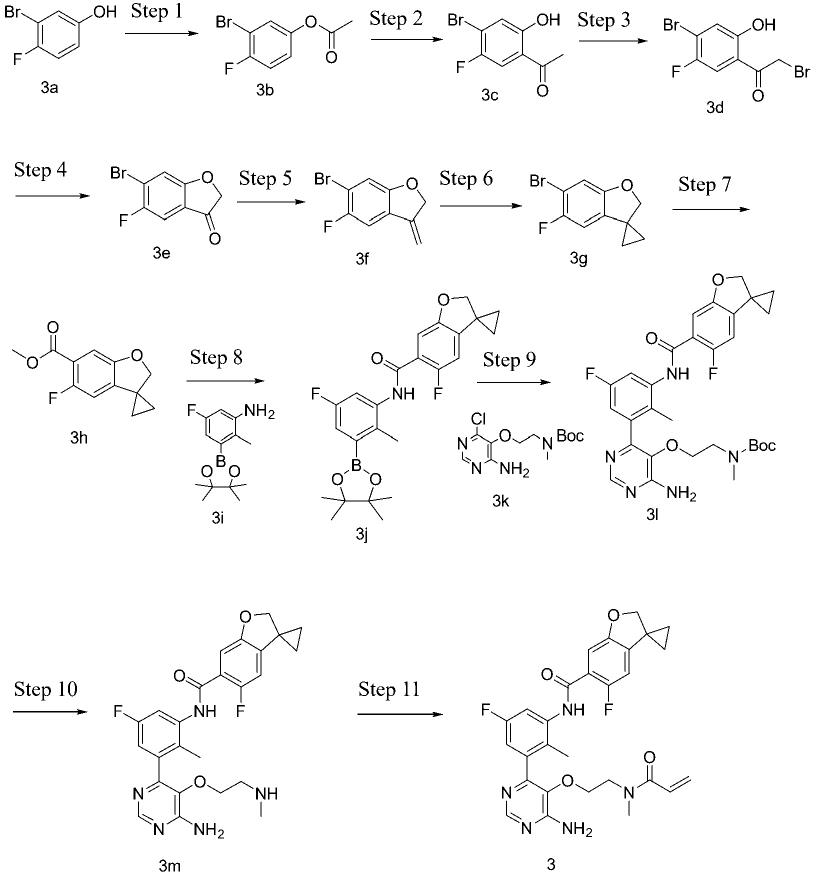

### Step 1

### 3-Bromo-4-fluorophenylacetate 3b

3-Bromo-4-fluorophenol **3a** (5 g, 26.18 mmol, Adamas) was dissolved in dichloromethane (100 mL, Sinopharm), and triethylamine (5.3 g, 52.38 mmol, Sinopharm) was added. At 0°C, acetyl chloride (2.88 g, 36.69 mmol, Sinopharm) was added dropwise. The mixture was reacted at room temperature for 1 hour, and 50 mL of water was added. The mixture was extracted with dichloromethane (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound **3b** (5.52 g, yield: 90.5%).

### Step 2

### 1-(4-Bromo-5-fluoro-2-hydroxylphenyl)ethan-1-one 3c

Compound **3b** (5.52 g, 23.69 mmol) and aluminium trichloride (6.32 g, 47.40 mmol, TCI) were added to a reaction flask. The mixture was reacted at 165°C for 3 hours, and cooled to room temperature. 50 mL of ice-cold dilute hydrochloric acid was added, and the mixture was extracted with dichloromethane (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound **3c** (5.12 g, yield: 92.8%).

MS m/z (ESI): 231.0 [M-1]

### Step 3

### 2-Bromo-1-(4-bromo-5-fluoro-2-hydroxylphenyl)ethan-1-one 3d

Compound **3c** (4 g, 17.16 mmol) was dissolved in methanol (40 mL, Sinopharm), and a solution of tetrabutylammonium tribromide (9.11 g, 18.89 mmol, Shaoyuan Technology) in dichloromethane (40 mL, Sinopharm) was added dropwise. The mixture was reacted at room temperature for 12 hours, and concentrated under reduced pressure. The residue was purified by column chromatography with eluent system B to obtain the title compound **3d** (5.3 g, yield: 99.0%).

MS m/z (ESI): 310.9 [M-1]

### Step 4

### 6-Bromo-5-fluorobenzofuran-3(2H)-one 3e

Compound **3d** (5.3 g, 16.99 mmol) was dissolved in ethanol (120 mL, Sinopharm), and sodium acetate (4.18 g, 50.95 mmol) was added. The mixture was stirred at room temperature for 1 hour. 50 mL of water was added, and the mixture was extracted with ethyl acetate (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound **3e** (1.6 g, yield: 40.8%).

### Step 5

### 6-Bromo-5-fluoro-3-methylidene-2,3-dihydrobenzofuran 3f

Compound **3e** (1.6 g, 6.93 mmol) was dissolved in tetrahydrofuran (30 mL, Adamas). Methyltriphenylphosphonium bromide (4.95 g, 13.86 mmol, Shaoyuan Technology) was added. The mixture was purged 3 times with nitrogen. At 0°C, potassium tert-butoxide (1 M 13.9 mL, 13.9 mmol) was added dropwise. The mixture was reacted at room temperature for 6 hours. 30 mL of water was added, and the mixture was extracted with ethyl acetate (30 mL×3). The organic phases were combined, washed with 30 mL of saturated sodium chloride solution, dried over anhydrous sodium sulfate, filtered and concentrated under reduced pressure. The residue was purified by column chromatography with eluent system B to obtain the title compound **3f** (260 mg, yield: 16.4%).

### Step 6

### 6-Bromo-5-fluoro-2H-spiro[benzofuran-3,1'-cyclopropane] 3g

Diethylzinc (1 M, 4.54 mL, 4.54 mmol, Adamas) was dissolved in dichloromethane (4 mL, Energy Chemical). Under a nitrogen atmosphere and at 0°C, trifluoroacetic acid (518 mg, 4.54 mmol, Adamas) was added dropwise. At 0°C, the mixture was reacted for 1 hour, and diiodomethane (1.22 g, 4.56 mmol, Energy Chemical) was added dropwise. At 0°C, the mixture was reacted for 0.5 hours, and a solution of compound **3f** (260 mg, 1.14 mmol) in dichloromethane (1 mL, Energy Chemical) was added dropwise. The mixture was reacted at room temperature for 3 hours. 15 mL of water was added, and the mixture was extracted with ethyl acetate (15 mL×3). The organic phases were combined, washed with 15 mL of saturated sodium chloride solution, dried over anhydrous sodium sulfate, filtered and concentrated under reduced pressure. The residue was purified by column chromatography with eluent system B to obtain the title compound 3g (150 mg, yield: 54.4%).

### Step 7

### Methyl 5-fluoro-2H-spiro[benzofuran-3,1'-cyclopropane]-6-carboxylate 3h

Compound **3g** (150 mg, 0.62 mmol) was dissolved in *N,N*-dimethylacetamide (2 mL, Sinopharm) and methanol (2 mL, Sinopharm). Palladium acetate (28 mg, 0.12 mmol, Adamas), N,N*-*diisopropylethylamine (565 mg, 4.37 mmol, Adamas), and 1,3-bis(diphenylphosphino)propane (51 mg, 0.12 mmol, Adamas) were added. The mixture was purged 3 times with carbon monoxide, reacted overnight at 80°C, and concentrated under reduced pressure. The residue was purified by column chromatography with eluent system B to obtain the title compound **3h** (100 mg, yield: 72.9%).

MS m/z (ESI): 223.1 [M+1]

### Step 8

### 5-Fluoro-N-(5-fluoro-2-methyl-3-(4,4,5,5-tetramethyl-1,3,2-dioxaborolan-2-yl)phenyl)-2H-spiro[benzofuran-3,1'-cyclopropane]-6-carboxamide 3j

Compound **3h** (50 mg, 0.23 mmol) was dissolved in tetrahydrofuran (2 mL, Energy Chemical), and 5-fluoro-2-methyl-3-(4,4,5,5-tetramethyl-1,3,2-dioxaborolan-2-yl)aniline **3i** (63 mg, 0.25 mmol, prepared according to the method disclosed in patent application WO 2015079417A1, description, page 31, for intermediate INT3) was added. The mixture was purged 3 times with nitrogen. Sodium bis(trimethylsilyl)amide (2 M, 0.17 mL, 0.34 mmol, Adamas) was added dropwise at 0°C, and the mixture was stirred at room temperature for 2 hours. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound 3j (60 mg, yield: 60.4%).

MS m/z (ESI): 442.3 [M+1]

### Step 9

### Tert-butyl (2-((4-amino-6-(5-fluoro-3-(5-fluoro-2H-spiro[benzofuran-3,1'-cyclopropane]-6-carboxamido)-2-methylphenyl)pyrimidin-5-yl)oxy)ethyl)(methyl)carbamate 3l

The crude compound 3j (60 mg, 0.14 mmol) was dissolved in ethylene glycol dimethyl ether (2 mL, Sinopharm) and water (0.6 mL, Sinopharm). Bis(triphenylphosphine)palladium chloride (9.6 mg, 0.02 mmol, Adamas), tert-butyl (2-((4-amino-6-chloropyrimidin-5-yl)oxy)ethyl))methyl)carbamate 3k (50 mg, 0.17 mmol, prepared according to the method disclosed in patent application WO 2015079417A1, description, page 39, for intermediate INT8) and sodium carbonate (29 mg, 0.27 mmol, Sinopharm) were added. The mixture was purged 3 times with nitrogen, and reacted under microwave conditions at 110°C for 1 hour. 5 mL of water was added, and the mixture was extracted with ethyl acetate (5 mL×3). The organic phases were combined, washed with 5 mL of saturated sodium chloride solution, dried over anhydrous sodium sulfate, filtered and concentrated under reduced pressure. The residue was purified by column chromatography with eluent system A to obtain the title compound **3l** (79 mg, yield: 99.9%).

MS m/z (ESI): 582.5 [M+1]

### Step 10

### N-(3-(6-amino-5-(2-(methylamino)ethoxy)pyrimidin-4-yl)-5-fluoro-2-methylphenyl)-5-fluoro-2H-spiro[benzofuran-3,1'-cyclopropane]-6-carboxamide 3m

Compound **3l** (79 mg, 0.14 mmol) was dissolved in dichloromethane (1 mL, Sinopharm), and trifluoroacetic acid (462 mg, 4.05 mmol, Adamas) was added. The mixture was reacted at room temperature for 1 hour. The reaction solution was concentrated under reduced pressure. 3 mL of saturated sodium bicarbonate solution was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure to obtain the crude title compound 3m (65 mg, yield: 99.4%), which was directly used in the next reaction without purification.

MS m/z (ESI): 482.1 [M+1]

### Step 11

### N-(3-(6-amino-5-(2-(N-methacrylamido)ethoxy)pyrimidin-4-yl)-5-fluoro-2-methylphenyl)-5-fluoro-2H-spiro[benzofuran-3,1'-cyclopropane]-6-carboxamide 3

The crude compound 3m (65 mg, 0.13 mmol) was dissolved in dichloromethane (2 mL, Sinopharm), and N,N-diisopropylethylamine (70 mg, 0.54 mmol, Adamas) was added. Acryloyl chloride (6.2 mg, 0.07 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound 3 (9 mg, yield: 12.4%).
MS m/z (ESI): 536.5 [M+1]
1H NMR (500 MHz, DMSO-*d*₆): δ 9.77-9.55 (m, 1H), 8.21-8.18 (m, 1H), 7.58-7.51 (m, 1H), 7.20-6.91 (m, 4H), 6.68-6.53 (m, 2H), 6.10-6.02 (m, 1H), 5.61-5.58 (m, 1H), 4.56 (s, 2H), 3.59-3.45 (m, 4H), 2.55 (s, 3H), 2.01-1.96 (m, 3H), 1.24-1.22 (m, 2H), 1.18-1.17 (m, 2H).

### Example 4

### N-(3-(6-amino-5-(2-(N-methacrylamido)ethoxy)pyrimidin-4-yl)-5-fluoro-2-methylphenyl)-7-fluoro-2,2-dimethyl-2,3-dihydrobenzofuran-6-carboxamide 4

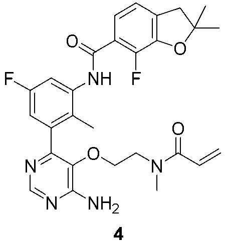

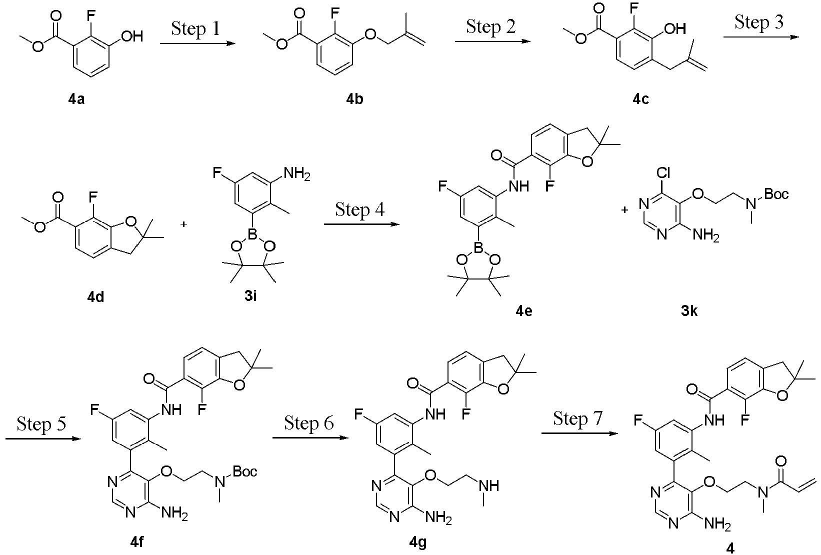

### Step 1

### Methyl 2-fluoro-3-((2-methylallyl)oxy)benzoate 4b

Methyl 2-fluoro-3-hydroxylbenzoate 4a (2.0 g, 11.75 mmol, Bidepharm) was dissolved in acetonitrile (2 mL). Potassium carbonate (1.95 g, 14.11 mmol, Sinopharm) and 3-bromo-2-methylpropene (1.91 g, 14.11 mmol, Adamas) were added, and the mixture was stirred at room temperature for 16 hours. The reaction solution was concentrated under reduced pressure, and the residue was purified by silica gel column chromatography with eluent system A to obtain the title compound 4b (1.92 g, yield: 72.8%).

MS m/z (ESI): 225.1 [M+1].

### Step 2

### Methyl 2-fluoro-3-hydroxyl-4-(2-methylallyl)benzoate 4c

Compound 4b (1.7 g, 7.58 mmol) was dissolved in biphenyl-diphenyl ether (15 mL). The reaction solution was warmed to 200°C and stirred for 2 hours. The reaction was cooled to room temperature, and directly purified by silica gel column chromatography with eluent system A to obtain the title compound 4c (570 mg, yield: 33.5%).

MS m/z (ESI): 225.1 [M+1].

### Step 3

Methyl 7-fluoro-2,2-dimethyl-2,3-dihydrobenzofuran-6-carboxylate 4d Compound 4c (445 mg, 1.98 mmol) was dissolved in formic acid (15 mL). The reaction solution was warmed to 110°C and stirred for 30 minutes. The reaction was cooled to room temperature, and saturated sodium chloride solution (20 mL) was added. The mixture was extracted with dichloromethane (20 mL×3). The organic phases were combined, dried over anhydrous sodium sulfate and filtered, and the filtrate was concentrated under reduced pressure to obtain the title compound 4d (440 mg, yield: 98.9%).

MS m/z (ESI): 225.1 [M+1].

### Step 4

### 7-Fluoro-N-(5-fluoro-2-methyl-3-(4,4,5,5-tetramethyl-1,3,2-dioxaborolan-2-yl)phenyl)-2,2-dimethyl-2,3-dihydrobenzofuran-6-carboxamide 4e

Compound 4d (200 mg, 0.89 mmol) and compound 3i (246 mg, 0.98 mmol) were dissolved in tetrahydrofuran (8 mL). The reaction solution was cooled to 0°C, and then sodium bis(trimethylsilyl)amide (245 mg, 1.34 mmol, Adamas) was added. Subsequently, the reaction solution was warmed to room temperature and stirred for 2 hours. To the reaction solution was added saturated ammonia chloride solution (20 mL), and the mixture was extracted with dichloromethane (20 mL×3). The organic phases were combined, dried over anhydrous sodium sulfate and filtered, and the filtrate was concentrated under reduced pressure. The residue was purified by silica gel column chromatography with eluent system A to obtain the title compound 4e (310 mg, yield: 78.4%).

MS m/z (ESI): 444.1 [M+1].

### Step 5

### Tert-butyl (2-((4-amino-6-(5-fluoro-3-(7-fluoro-2,2-dimethyl-2,3-dihydrobenzofuran-6-carboxamido)-2-methylphenyl)pyrimidin-5-yl)oxy)ethyl)(methyl)carbamate 4f

Compound 4e (310 mg, 0.70 mmol), compound 3k (211 mg, 0.70 mmol), tetrakis(triphenylphosphine)palladium (80 mg, 0.07 mmol, Bidepharm), and potassium carbonate (193 mg, 1.40 mmol) were dissolved in 1,4-dioxane (4 mL) and water (1 mL). The mixture was purged 3 times with nitrogen, and reacted at 110°C under microwave conditions for 30 minutes. The reaction solution was concentrated under reduced pressure, and the residue was purified by silica gel column chromatography with eluent system A to obtain the title compound 4f (380 mg, yield: 93.1%).

MS m/z (ESI): 584.3 [M+1].

### Step 6

### N-(3-(6-amino-5-(2-(methylamino)ethoxy)pyrimidin-4-yl)-5-fluoro-2-methylphenyl)-7-fluoro-2,2-dimethyl-2,3-dihydrobenzofuran-6-carboxamide 4g

Compound 4f (380 mg, 0.65 mmol) was dissolved in hydrogen chloride in dioxane (3 mL, 4 M). At room temperature, the mixture was stirred for 30 minutes. The reaction solution was concentrated under reduced pressure. 3 mL of saturated sodium bicarbonate was added, and the mixture was extracted with ethyl acetate (3 mL×3). The organic phases were combined, washed with 3 mL of saturated sodium chloride solution, dried over anhydrous sodium sulfate, filtered and concentrated under reduced pressure to obtain the title compound 4g, which was directly used in the next reaction without further purification.

MS m/z (ESI): 484.3 [M+1].

### Step 7

### N-(3-(6-amino-5-(2-(N-methacrylamido)ethoxy)pyrimidin-4-yl)-5-fluoro-2-methylphenyl)-7-fluoro-2,2-dimethyl-2,3-dihydrobenzofuran-6-carboxamide 4

Compound 4g (314 mg, 0.65 mmol) and N,N-diisopropylethylamine (420 mg, 3.25 mmol, Adamas) were dissolved in dichloromethane (5 mL). The reaction solution was cooled to -40°C. Acryloyl chloride (59 mg, 0.65 mmol, Adamas) was added, and the mixture was stirred at -40°C for 10 minutes. The reaction solution was concentrated under reduced pressure, and the residue was purified by silica gel column chromatography with eluent system B to obtain the title compound 4 (140 mg, yield: 40.1%).

MS m/z (ESI): 538.2 [M+1].

¹H NMR (500 MHz, DMSO-*d*₆) δ 9.89-9.68 (m, 1H), 8.22 (d, 1H), 7.52 (dd, 1H), 7.34 -7.05 (m, 4H), 7.00 (td, 1H), 6.61 (dt, 1H), 6.06 (ddd, 1H), 5.59 (td, 1H), 3.52 (ddt, 4H), 3.16 (s, 2H), 2.81-2.52 (m, 3H), 2.00 (d, 3H), 1.49 (s, 6H).

### Example 5

### N-(3-(6-amino-5-(2-(N-methacrylamido)ethoxy)pyrimidin-4-yl)-5-fluoro-2-methylphenyl)-7-fluoro-2H-spiro[benzofuran-3,1'-cyclopropane]-6-carboxamide 5

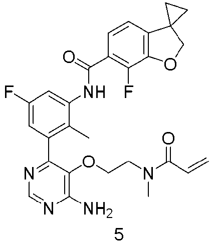

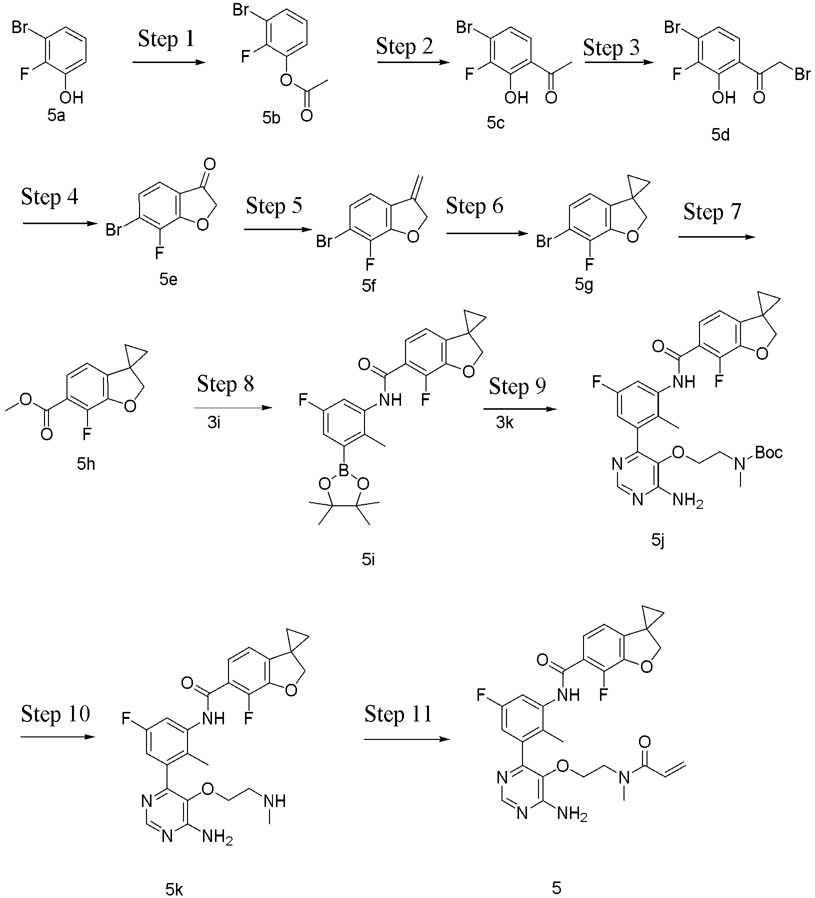

### Step 1

### 3-Bromo-2-fluorophenylacetate 5b

3-Bromo-2-fluorophenol 5a (5 g, 26.18 mmol, Bidepharm) was dissolved in dichloromethane (100 mL, Sinopharm), and triethylamine (5.3 g, 52.38 mmol, Sinopharm) was added. At 0°C, acetyl chloride (2.88 g, 36.69 mmol, Sinopharm) was added dropwise. The mixture was reacted at room temperature for 1 hour, and 50 mL of water was added. The mixture was extracted with dichloromethane (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound 5b (4.37 g, yield: 71.6%).

### Step 2

### 1-(4-Bromo-3-fluoro-2-hydroxylphenyl)ethan-1-one 5c

Compound 5b (4.37 g, 18.75 mmol) and aluminium trichloride (5 g, 37.50 mmol, Sinopharm) were added to a reaction flask. The mixture was reacted at 165°C for 3 hours, and cooled to room temperature. 50 mL of ice-cold dilute hydrochloric acid was added, and the mixture was extracted with dichloromethane (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound 5c (3.9 g, yield: 89.24%).

MS m/z (ESI): 231.0 [M-1]

### Step 3

### 2-Bromo-1-(4-bromo-3-fluoro-2-hydroxylphenyl)ethan-1-one 5d

Compound 5c (3.7 g, 15.87 mmol) was dissolved in ethyl acetate (50 mL, Sinopharm). Cupric bromide (7.1 g, 31.78 mmol, Sinopharm) was added, and the mixture was reacted overnight at 80°C. The reaction solution was filtered, and the filtrate was concentrated to obtain the title compound 5d (4.95 g, yield: 99.94%).

MS m/z (ESI): 310.9 [M-1]

### Step 4

### 6-Bromo-7-fluorobenzofuran-3(2H)-one 5e

Compound 5d (4.95 g, 15.86 mmol) was dissolved in ethanol (50 mL, Sinopharm), and sodium acetate (3.92 g, 47.78 mmol) was added. The mixture was stirred at room temperature for 1 hour. 50 mL of water was added, and the mixture was extracted with ethyl acetate (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound **5e** (1.59 g, yield: 43.37%).

### Step 5

### 6-Bromo-7-fluoro-3-methylidene-2,3-dihydrobenzofuran 5f

Compound **5e** (1.59 g, 6.88 mmol) was dissolved in tetrahydrofuran (30 mL, Adamas). Methyltriphenylphosphonium bromide (4.93 g, 13.80 mmol, Shaoyuan Technology) was added. The mixture was purged 3 times with nitrogen. At 0°C, potassium tert-butoxide (1 M 13.9 mL, 13.9 mmol) was added dropwise. The mixture was reacted at room temperature for 2 hours. 30 mL of water was added, and the mixture was extracted with ethyl acetate (30 mL×3). The organic phases were combined, washed with 30 mL of saturated sodium chloride solution, dried over anhydrous sodium sulfate, filtered and concentrated under reduced pressure. The residue was purified by column chromatography with eluent system B to obtain the title compound **5f** (190 mg, yield: 12.05%).

### Step 6

### 6-Bromo-7-fluoro-2H-spiro[benzofuran-3,1'-cyclopropane] 5g

Diethylzinc (1 M, 3.32 mL, 3.32 mmol, Adamas) was dissolved in dichloromethane (2 mL, Energy Chemical). Under a nitrogen atmosphere and at 0°C, trifluoroacetic acid (379 mg, 3.32 mmol, Adamas) was added dropwise. At 0°C, the mixture was reacted for 1 hour, and diiodomethane (889 mg, 3.32 mmol, Energy Chemical) was added dropwise. At 0°C, the mixture was reacted for 0.5 hours, and a solution of compound 5f (190 mg, 0.83 mmol) in dichloromethane (1 mL, Energy Chemical) was added dropwise. The mixture was reacted at room temperature for 3 hours. 15 mL of water was added, and the mixture was extracted with ethyl acetate (15 mL×3). The organic phases were combined, washed with 15 mL of saturated sodium chloride solution, dried over anhydrous sodium sulfate, filtered and concentrated under reduced pressure. The residue was purified by column chromatography with eluent system B to obtain the title compound 5g (140 mg, yield: 69.43%).

### Step 7

### Methyl 7-fluoro-2H-spiro[benzofuran-3,1'-cyclopropane]-6-carboxylate 5h

Compound **5g** (140 mg, 0.57 mmol) was dissolved in N,N-dimethylacetamide (2 mL, Sinopharm) and methanol (2 mL, Sinopharm). Palladium acetate (26 mg, 0.116 mmol, Adamas), N,N-diisopropylethylamine (149 mg, 1.15 mmol, Adamas), and 1,3-bis(diphenylphosphino)propane (48 mg, 0.116 mmol, Adamas) were added. The mixture was purged 3 times with carbon monoxide, reacted overnight at 80°C, and concentrated under reduced pressure. The residue was purified by column chromatography with eluent system B to obtain the title compound 5h (100 mg, yield: 78.13%).

MS m/z (ESI): 223.1 [M+1]

### Step 8

### 7-Fluoro-N-(5-fluoro-2-methyl-3-(4,4,5,5-tetramethyl-1,3,2-dioxaborolan-2-yl)phenyl)-2H-spiro[benzofuran-3,1'-cyclopropane]-6-carboxamide 5i

Compound 5h (50 mg, 0.23 mmol) was dissolved in tetrahydrofuran (2 mL, Energy Chemical), and compound 3i (63 mg, 0.25 mmol) was added. The mixture was purged 3 times with nitrogen. Sodium bis(trimethylsilyl)amide (2 M, 0.17 mL, 0.34 mmol, Adamas) was added dropwise at 0°C, and the mixture was stirred at room temperature for 2 hours. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound 5i (65 mg, yield: 65.46%).

MS m/z (ESI): 442.3 [M+1]

### Step 9

### Tert-butyl (2-((4-amino-6-(5-fluoro-3-(7-fluoro-2H-spiro[benzofuran-3,1'-cyclopropane]-6-carboxamido)-2-methylphenyl)pyrimidin-5-yl)oxy)ethyl)(methyl)carbamate 5j

The crude compound 5i (65 mg, 0.15 mmol) was dissolved in 1,4-dioxane (2 mL, Sinopharm) and water (0.6 mL, Sinopharm), and [1,1'-bis(diphenylphosphino)ferrocene]palladium dichloride (22 mg, 0.03 mmol, Bidepharm), compound 3k (54 mg, 0.18 mmol) and potassium carbonate (61 mg, 0.44 mmol, Sinopharm) were added. The mixture was purged 3 times with nitrogen, and reacted at 90°C for 2 hours. 5 mL of water was added, and the mixture was extracted with ethyl acetate (5 mL×3). The organic phases were combined, washed with 5 mL of saturated sodium chloride solution, dried over anhydrous sodium sulfate, filtered and concentrated under reduced pressure. The residue was purified by column chromatography with eluent system A to obtain the title compound **5j** (60 mg, yield: 70%).

MS m/z (ESI): 582.5 [M+1]

### Step 10

### N-(3-(6-amino-5-(2-(methylamino)ethoxy)pyrimidin-4-yl)-5-fluoro-2-methylphenyl)-7-fluoro-2H-spiro[benzofuran-3,1'-cyclopropane]-6-carboxamide 5k

The crude compound 5j (60 mg, 0.10 mmol) was dissolved in dichloromethane (1 mL, Sinopharm), and trifluoroacetic acid (462 mg, 4.05 mmol, Adamas) was added. The mixture was reacted at room temperature for 1 hour. The reaction solution was concentrated under reduced pressure. 3 mL of saturated sodium bicarbonate was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure to obtain the crude title compound 5k (49 mg, yield: 98.64%), which was directly used in the next reaction without purification.

MS m/z (ESI): 482.1 [M+1]

### Step 11

### N-(3-(6-amino-5-(2-(N-methacrylamido)ethoxy)pyrimidin-4-yl)-5-fluoro-2-methylphenyl)-7-fluoro-2H-spiro[benzofuran-3,1'-cyclopropane]-6-carboxamide 5

The crude compound 5k (49 mg, 0.1 mmol) was dissolved in dichloromethane (2 mL, Sinopharm), and N,N-diisopropylethylamine (53 mg, 0.41 mmol, Adamas) was added. Acryloyl chloride (5.53 mg, 0.06 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound 5 (9 mg, yield: 16.51%).
MS m/z (ESI): 536.5 [M+1]
¹H NMR (500 MHz, DMSO-*d₆*): δ 9.85-9.64 (m, 1H), 8.21-8.18 (m, 1H), 7.56-7.54 (m, 1H), 7.28-6.97 (m, 4H), 6.81-6.67 (m, 1H), 6.64-6.58 (m, 1H), 6.09-6.03 (m, 1H), 5.62-5.58 (m, 1H), 4.67-4.64 (m, 2H), 3.59-3.45 (m, 3H), 2.80-2.78 (m, 1H), 2.03-1.97 (m, 3H), 1.24-1.14(m, 7H).

### Example 6

### N-(3-(6-amino-5-(2-(N-methacrylamido)ethoxy)pyrimidin-4-yl)-5-fluoro-2-methylphenyl)-3-fluoro-5,5-dimethyl-5,6-dihydro-4H-cyclopenta[b]thiophene-2-carboxamide 6

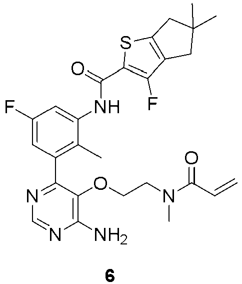

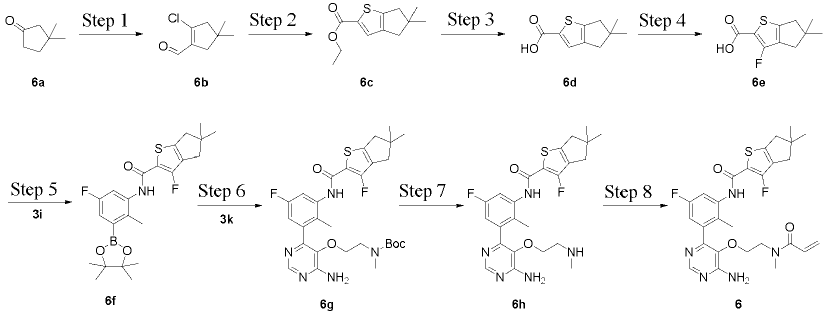

### Step 1

### 2-Chloro-4,4-dimethylcyclopent-1-ene-1-carbaldehyde 6b

3,3-Dimethylcyclopent-1-one 6a (700 mg, 6.24 mmol, Bidepharm) was dissolved in dichloromethane (40 mL). At room temperature, *N,N-*dimethylformamide (1.14 g, 15.6 mmol, Sinopharm) was added. The reaction solution was heated to 40°C, followed by the addition of phosphorus oxychloride (2.0 g, 13.1 mmol, Sinopharm), and the mixture was stirred at 40°C for 16 hours. The reaction solution was cooled to room temperature, and the reaction was quenched with aqueous potassium phosphate solution (2 mL, 4 M) and extracted with dichloromethane (20 mL×3). The organic phases were combined, dried over anhydrous sodium sulfate and filtered, and the filtrate was concentrated under reduced pressure to obtain the title compound 6b, which was directly used in the next reaction without further purification.

### Step 2

### Ethyl 5,5-dimethyl-5,6-dihydro-4H-cyclopenta[b]thiophene-2-carboxylate 6c

Compound 6b (500 mg, 3.15 mmol) was dissolved in dichloromethane (5 mL). Ethyl mercaptoacetate (416 mg, 3.46 mmol, Adamas) and triethylamine (988 mg, 9.77 mmol, Sinopharm) were added. The mixture was stirred at 40°C for 16 hours. The reaction solution was concentrated under reduced pressure, and the residue was purified by silica gel column chromatography with eluent system A to obtain the title compound 6c (340 mg, yield: 48.1%).

MS m/z (ESI): 225.1 [M+1].

### Step 3

### 5,5-Dimethyl-5,6-dihydro-4H-cyclopenta[b]thiophene-2-carboxylic acid 6d

Compound 6c (200 mg, 0.89 mmol) was dissolved in tetrahydrofuran (2 mL) and methanol (2 mL). Aqueous lithium hydroxide solution (60 mg, 1.35 mmol, 2 mL) was added, and the mixture was stirred at 60°C for 1 hour. The reaction solution was adjusted to pH = 1 with 1 N hydrochloric acid, and extracted with dichloromethane (20 mL×3). The organic phases were combined, dried over anhydrous sodium sulfate and filtered, and the filtrate was concentrated under reduced pressure to obtain the title compound 6d (170 mg, yield: 97.1%).

MS m/z (ESI): 197.2 [M+1].

### Step 4

### 3-Fluoro-5,5-dimethyl-5,6-dihydro-4H-cyclopenta[b]thiophene-2-carboxylic acid 6e

Compound 6d (83 mg, 0.42 mmol) was dissolved in tetrahydrofuran (3 mL). The reaction solution was cooled to -78°C, and n-butyllithium (68 mg, 1.06 mmol, Adamas) was added dropwise. The mixture was stirred at -78°C for 2 hours, followed by the addition of N-fluorobenzenesulfonimide (333 mg, 1.06 mmol, Shaoyuan Technology). The reaction solution was warmed to room temperature and further stirred for 1 hour. The reaction was quenched with saturated ammonium chloride solution (10 mL), and extracted with dichloromethane (20 mL×3). The organic phases were combined, dried over anhydrous sodium sulfate and filtered, and the filtrate was concentrated under reduced pressure to obtain the title compound 6e, which was directly used in the next reaction without further purification.

MS m/z (ESI): 215.1 [M+1].

### Step 5

### 3-Fluoro-N-(5-fluoro-2-methyl-3-(4,4,5,5-tetramethyl-1,3,2-dioxaborolan-2-yl)phenyl)-5,5-dimethyl-5,6-dihydro-4H-cyclopenta[b]thiophene-2-carboxamide 6f

Compound 6e (85 mg, 0.4 mmol) and compound 3i (100 mg, 0.4 mmol) were dissolved in N,N-dimethylformamide (3 mL). 2-(7-Azabenzotriazol-1-yl)-N,N,N',N'-tetramethyluronium hexafluorophosphate (228 mg, 0.6 mmol, Adamas) and N,N-diisopropylethylamine (129 mg, 1.0 mmol, Adamas) were added, and then the reaction solution was warmed to 70°C and stirred for 16 hours. The reaction solution was concentrated under reduced pressure, and the residue was purified by silica gel column chromatography with eluent system A to obtain the title compound 6f (70 mg, yield: 39.1%).

MS m/z (ESI): 448.2 [M+1].

### Step 6

### Tert-butyl (2-((4-amino-6-(5-fluoro-3-(3-fluoro-5,5-dimethyl-5,6-dihydro-4H-cyclopenta[b]thiophene-2-carboxamido)-2-methylphenyl)pyrimidin-5-yl)oxy)ethyl)(methyl)carbamate 6g

Compound 6f (70 mg, 0.15 mmol), compound 3k (50 mg, 0.16 mmol), tetrakis(triphenylphosphine)palladium (18 mg, 0.015 mmol, Bidepharm), and potassium carbonate (43 mg, 0.31 mmol, Sinopharm) were dissolved in 1,4-dioxane (2 mL) and water (0.5 mL). The mixture was purged 3 times with nitrogen, and reacted at 110°C under microwave conditions for 30 minutes. The reaction solution was concentrated under reduced pressure, and the residue was purified by silica gel column chromatography with eluent system A to obtain the title compound 6g (80 mg, yield: 87.0%).

MS m/z (ESI): 588.5 [M+1].

### Step 7

### N-(3-(6-amino-5-(2-(methylamino)ethoxy)pyrimidin-4-yl)-5-fluoro-2-methylphenyl)-3-fluoro-5,5-dimethyl-5,6-dihydro-4H-cyclopenta[b]thiophene-2-carboxamide 6h

Compound 6g (80 mg, 0.14 mmol) was dissolved in hydrogen chloride in dioxane (3 mL, 4 M). At room temperature, the mixture was stirred for 30 minutes. The reaction solution was concentrated under reduced pressure. 3 mL of saturated sodium bicarbonate solution was added, and the mixture was extracted with ethyl acetate (3 mL×3). The organic phases were combined, washed with 3 mL of saturated sodium chloride solution, dried over anhydrous sodium sulfate, filtered and concentrated under reduced pressure to obtain the title compound 6h, which was directly used in the next reaction without further purification.

MS m/z (ESI): 488.5 [M+1].

### Step 8

### N-(3-(6-amino-5-(2-(N-methacrylamido)ethoxy)pyrimidin-4-yl)-5-fluoro-2-methylphenyl)-3-fluoro-5,5-dimethyl-5,6-dihydro-4H-cyclopenta[b]thiophene-2-carboxamide 6

Compound 6h (66 mg, 0.13 mmol) and N,N-diisopropylethylamine (87 mg, 0.67 mmol, Adamas) were dissolved in dichloromethane (3 mL). The reaction solution was cooled to -40°C. Acryloyl chloride (12 mg, 0.13 mmol, Adamas) was added, and the mixture was stirred at -40°C for 10 minutes. The reaction solution was concentrated under reduced pressure, and the residue was purified by silica gel column chromatography with eluent system B to obtain the title compound 6 (12 mg, yield: 16.3%).

MS m/z (ESI): 542.3 [M+1].

¹H NMR (500 MHz, DMSO) δ 9.17-8.95 (m, 1H), 8.21-8.18 (m, 1H), 7.64-7.60 (m, 1H), 7.22-6.94 (m, 3H), 6.64 (ddd, 1H), 6.07 (ddd, 1H), 5.59 (ddd, 1H), 3.61-3.43 (m, 4H), 2.78-2.77 (m, 3H), 2.60-2.59 (m, 2H), 2.43 (s, 2H), 1.99-1.91 (m, 3H), 1.23 (s, 6H).

### Example 7

### N-(3-(6-amino-5-(2-(N-methacrylamido)ethoxy)pyrimidin-4-yl)-5-fluoro-2-methylphenyl)-7-fluoro-3,3-dimethyl-2,3-dihydrobenzofuran-6-carboxamide 7

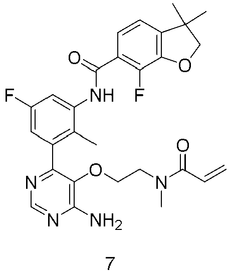

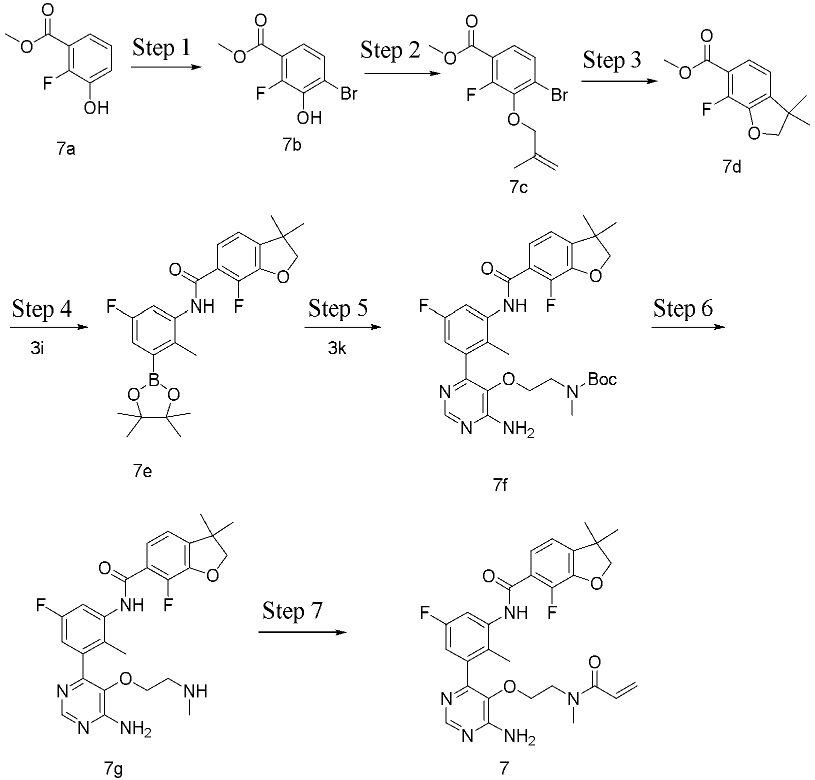

### Step 1

### Methyl 4-bromo-2-fluoro-3-hydroxylbenzoate 7b

Methyl 2-fluoro-3-hydroxylbenzoate 7a (500 mg, 2.94 mmol, Shaoyuan Technology) was dissolved in chloroform (5 mL). At 0°C, a solution of bromine (564 mg, 3.53 mmol, Sinopharm) in acetic acid (5 mL, Sinopharm) was added dropwise. The mixture was reacted at room temperature for 12 hours. 20 mL of sodium thiosulfate solution was added, and the mixture was extracted with dichloromethane (20 mL×3). The organic phases were combined, washed with 20 mL of saturated sodium chloride solution, dried over anhydrous sodium sulfate and filtered, and the filtrate was concentrated under reduced pressure. The residue was purified by column chromatography with eluent system B to obtain the title compound 7b (730 mg, yield: 99.7%).

MS m/z (ESI): 247.0 [M-1]

### Step 2

### Methyl 4-bromo-2-fluoro-3-((2-methylallyl)oxy)benzoate 7c

Compound 7b (730 mg, 2.93 mmol) was dissolved in acetonitrile (10 mL), and potassium carbonate (608 mg, 4.40 mmol, Sinopharm) was added. At 0°C, 3-bromo-2-methylprop-1-ene (475 mg, 3.52 mmol, Adamas) was added dropwise. After the addition, the mixture was reacted at room temperature for 12 hours. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound 7c (780 mg, yield: 87.8%).

### Step 3

### Methyl 7-fluoro-3,3-dimethyl-2,3-dihydrobenzofuran-6-carboxylate 7d

Compound 7c (780 mg, 2.57 mmol) was dissolved in N,Ndimethylformamide (20 mL), and tetraethylammonium chloride (470 mg, 2.84 mmol, J&K Scientific), sodium formate (193 mg, 2.84 mmol, Shaoyuan Technology), palladium acetate (58 mg, 0.26 mmol, Adamas) and anhydrous sodium acetate (634 mg, 7.73 mmol, Sinopharm) were added. The mixture was purged 3 times with nitrogen, and reacted overnight at 100°C. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound 7d (40 mg, yield: 6.9%).

### Step 4

### 7-Fluoro-N-(5-fluoro-2-methyl-3-(4,4,5,5-tetramethyl-1,3,2-dioxaborolan-2-yl)phenyl)-3,3-dimethyl-2,3-dihydrobenzofuran-6-carboxamide 7e

Compound 7d (40 mg, 0.18 mmol) was dissolved in tetrahydrofuran (2 mL), and compound 3i (50 mg, 0.20 mmol) was added. The mixture was purged 3 times with nitrogen. Sodium bis(trimethylsilyl)amide (2 M, 0.14 mL, 0.28 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 7e (53 mg, yield: 67.0%).

MS m/z (ESI): 444.3 [M+1]

### Step 5

### Tert-butyl (2-((4-amino-6-(5-fluoro-3-(7-fluoro-3,3-dimethyl-2,3-dihydrobenzofuran-6-carboxamido)-2-methylphenyl)pyrimidin-5-yl)oxy)ethyl)(methyl)carbamate 7f

Compound 7e (53 mg, 0.12 mmol) was dissolved in 1,4-dioxane (2 mL) and water (0.6 mL), and [1,1'-bis(diphenylphosphino)ferrocene]palladium dichloride (17.5 mg, 0.02 mmol, Bidepharm), compound 3k (43.5 mg, 0.14 mmol) and potassium carbonate (34 mg, 0.25 mmol, Sinopharm) were added. The mixture was purged 3 times with nitrogen, and reacted at 90°C for 2 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system A to obtain the title compound 7f (69 mg, yield: 98.9%).

MS m/z (ESI): 584.4 [M+1]

### Step 6

### N-(3-(6-amino-5-(2-(methylamino)ethoxy)pyrimidin-4-yl)-5-fluoro-2-methylphenyl)-7-fluoro-3,3-dimethyl-2,3-dihydrobenzofuran-6-carboxamide 7g

Compound 7f (69 mg, 0.12 mmol) was dissolved in dichloromethane (2 mL), and trifluoroacetic acid (770 mg, 6.75 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound 7g (57 mg, yield: 99.7%), which was directly used in the next reaction without purification.

MS m/z (ESI): 484.5 [M+1]

### Step 7

### N-(3-(6-amino-5-(2-(N-methacrylamido)ethoxy)pyrimidin-4-yl)-5-fluoro-2-methylphenyl)-7-fluoro-3,3-dimethyl-2,3-dihydrobenzofuran-6-carboxamide 7

The crude compound 7g (57 mg, 0.12 mmol) was dissolved in dichloromethane (2 mL), and N,N-diisopropylethylamine (61 mg, 0.47 mmol, Adamas) was added. Acryloyl chloride (9 mg, 0.10 mmol, Sinopharm) was added dropwise at -20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 7 (9 mg, yield: 14.2%).
MS m/z (ESI): 538.3 [M+1]
¹H NMR (500 MHz, DMSO-*d₆*): δ 9.92-9.72 (m, 1H), 8.21-8.18 (m, 1H), 7.55-7.48 (m, 1H), 7.33-6.97 (m, 5H), 6.65-6.58 (m, 1H), 6.09-6.03 (m, 1H), 5.62-5.57 (m, 1H), 4.42-4.41 (m, 2H), 3.59-3.45 (m, 4H), 2.53 (s, 3H), 2.03-1.98 (m, 3H), 1.36-1.24 (m, 6H).

### Example 8

### N-(3-(6-amino-5-(2-(N-methacrylamido)ethoxy)pyrimidin-4-yl)-5-fluoro-2-methylphenyl)-5-fluoro-3,3-dimethyl-2,3-dihydrobenzofuran-6-carboxamide 8

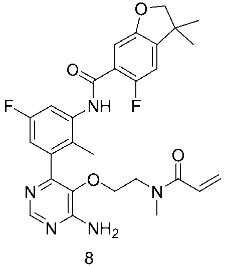

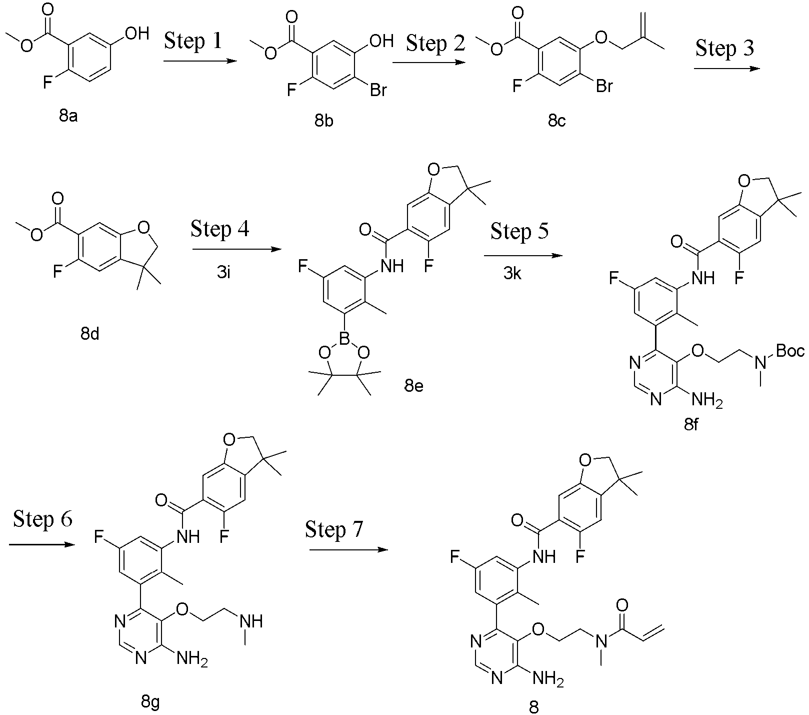

### Step 1

### Methyl 4-bromo-2-fluoro-5-hydroxylbenzoate 8b

Methyl 2-fluoro-5-hydroxylbenzoate 8a (1 g, 2.94 mmol, Bidepharm) was dissolved in chloroform (10 mL). At 0°C, a solution of bromine (1.41 g, 8.82 mmol, Sinopharm) in acetic acid (10 mL, Sinopharm) was added dropwise. The mixture was reacted at room temperature for 12 hours. 40 mL of sodium thiosulfate solution was added, and the mixture was extracted with dichloromethane (40 mL×3). The organic phases were combined, washed with 40 mL of saturated sodium chloride solution, dried over anhydrous sodium sulfate and filtered, and the filtrate was concentrated under reduced pressure. The residue was purified by column chromatography with eluent system B to obtain the title compound 8b (290 mg, yield: 19.8%).

MS m/z (ESI): 246.8 [M-1]

### Step 2

### Methyl 4-bromo-2-fluoro-5-((2-methylallyl)oxy)benzoate 8c

Compound 8b (290 mg, 1.16 mmol) was dissolved in acetonitrile (5 mL), and potassium carbonate (242 mg, 1.75 mmol, Sinopharm) was added. At 0°C, 3-bromo-2-methylprop-1-ene (475 mg, 3.52 mmol, Adamas) was added dropwise. The mixture was reacted at room temperature for 12 hours. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound **8c** (320 mg, yield: 90.7%).

### Step 3

### Methyl 5-fluoro-3,3-dimethyl-2,3-dihydrobenzofuran-6-carboxylate 8d

Compound **8c** (170 mg, 0.56 mmol) was dissolved in N,Ndimethylformamide (8 mL), and tetraethylammonium chloride (102 mg, 0.62 mmol, J&K Scientific), sodium formate (42 mg, 0.62 mmol, Shaoyuan Technology), palladium acetate (12.6 mg, 0.06 mmol, Adamas) and anhydrous sodium acetate (138 mg, 1.68 mmol, Sinopharm) were added. The mixture was purged 3 times with nitrogen, and reacted overnight at 100°C. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound 8d (65 mg, yield: 52.0%).

### Step 4

### 5-Fluoro-N-(5-fluoro-2-methyl-3-(4,4,5,5-tetramethyl-1,3,2-dioxaborolan-2-yl)phenyl)-3,3-dimethyl-2,3-dihydrobenzofuran-6-carboxamide 8e

Compound 8d (65 mg, 0.29 mmol) was dissolved in tetrahydrofuran (2 mL), and compound 3i (81 mg, 0.32 mmol) was added. The mixture was purged 3 times with nitrogen. Sodium bis(trimethylsilyl)amide (2 M, 0.22 mL, 0.44 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 8e (90 mg, yield: 70.0%).

MS m/z (ESI): 444.3 [M+1]

### Step 5

### Tert-butyl (2-((4-amino-6-(5-fluoro-3-(5-fluoro-3,3-dimethyl-2,3-dihydrobenzofuran-6-carboxamido)-2-methylphenyl)pyrimidin-5-yl)oxy)ethyl)(methyl)carbamate 8f

Compound 8e (90 mg, 0.20 mmol) was dissolved in 1,4-dioxane (2 mL) and water (0.6 mL), and [1,1'-bis(diphenylphosphino)ferrocene]palladium dichloride (30 mg, 0.04 mmol, Bidepharm), compound 3k (74 mg, 0.24 mmol) and potassium carbonate (57 mg, 0.41 mmol, Sinopharm) were added. The mixture was purged 3 times with nitrogen, and reacted at 90°C for 2 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system A to obtain the title compound 8f (110 mg, yield: 92.8%).

MS m/z (ESI): 584.6 [M+1]

### Step 6

### N-(3-(6-amino-5-(2-(methylamino)ethoxy)pyrimidin-4-yl)-5-fluoro-2-methylphenyl)-5-fluoro-3,3-dimethyl-2,3-dihydrobenzofuran-6-carboxamide 8g

Compound **8f** (110 mg, 0.19 mmol) was dissolved in dichloromethane (2 mL), and trifluoroacetic acid (770 mg, 6.75 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound 8g (91 mg, yield: 99.9%), which was directly used in the next reaction without purification.

MS m/z (ESI): 484.3 [M+1]

### Step 7

### N-(3-(6-amino-5-(2-(N-methacrylamido)ethoxy)pyrimidin-4-yl)-5-fluoro-2-methylphenyl)-5-fluoro-3,3-dimethyl-2,3-dihydrobenzofuran-6-carboxamide 8

The crude compound 8g (91 mg, 0.19 mmol) was dissolved in dichloromethane (2 mL), and N,N-diisopropylethylamine (98 mg, 0.76 mmol, Adamas) was added. Acryloyl chloride (10.3 mg, 0.11 mmol, Sinopharm) was added dropwise at -20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 8 (22 mg, yield: 21.7%).
MS m/z (ESI): 538.4 [M+1]
¹H NMR (500 MHz, DMSO-*d₆*): δ 9.85-9.64 (m, 1H), 8.21-8.18 (m, 1H), 7.63-7.50 (m, 1H), 7.33-6.96 (m, 5H), 6.67-6.58 (m, 1H), 6.09-6.03 (m, 1H), 5.62-5.57 (m, 1H), 4.30-4.29 (m, 2H), 3.59-3.45 (m, 4H), 2.53 (s, 3H), 2.03-1.98 (m, 3H), 1.34-1.24 (m, 6H).

### Example 9

### N-(3-(6-amino-5-(2-(N-methacrylamido)ethoxy)pyrimidin-4-yl)-5-fluoro-2-methylphenyl)-4-fluoro-2,2-dimethyl-2,3-dihydro-1H-indene-5-carboxamide 9

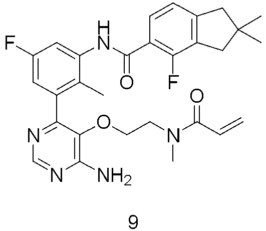

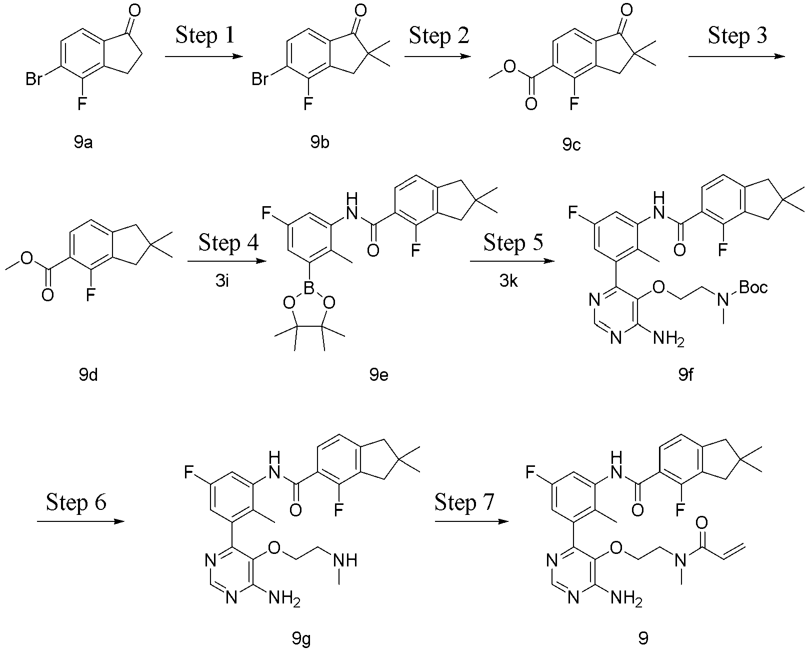

### Step 1

### 5-Bromo-4-fluoro-2,2-dimethyl-2,3-dihydro-1H-indene-1-one 9b

In a dry flask, 5-bromo-4-fluoro-2,3-dihydro-1H-indene-1-one 9a (1 g, 4.37 mmol, Bidepharm) was dissolved in N,N-dimethylformamide (20 mL). With stirring at room temperature, sodium hydride (418 mg, 10.91 mmol, purity: 60%, Sinopharm) was added. The mixture was stirred at room temperature for 10 minutes. Iodomethane (4.3 g, 30.56 mmol, Adamas) was added, and the mixture was stirred at room temperature for 12 hours. The reaction solution was slowly added dropwise to 50 mL of ice water with stirring, and extracted with diethyl ether (50 mL×2). The organic phases were combined, washed with 50 mL of water, dried over anhydrous sodium sulfate and filtered, and the filtrate was concentrated under reduced pressure. The residue was purified by column chromatography with eluent system B to obtain the title compound 9b (800 mg, yield: 71.27%).

### Step 2

### Methyl 4-fluoro-2,2-dimethyl-1-oxo-2,3-dihydro-1H-indene-5-carboxylate 9c

Compound 9b (800 mg, 3.11 mmol), triethylamine (944.6 mg, 9.33 mmol, Sinopharm), and [1,1'-bis(diphenylphosphino)ferrocene]palladium dichloride (341.5 mg, 0.47 mmol, Bidepharm) were dissolved in N,N-dimethylformamide (8 mL) and methanol (24 mL). The mixture was purged twice with nitrogen, and carbon monoxide (3 atm) was introduced. The mixture was reacted at 100°C for 12 hours. The reaction solution was cooled to room temperature, and concentrated under reduced pressure. The residue was purified by column chromatography with eluent system B to obtain the title compound **9c** (85 mg, yield: 11.56%).

### Step 3

### Methyl 4-fluoro-2,2-dimethyl-2,3-dihydro-1H-indene-5-carboxylate 9d

In a dry flask, compound **9c** (70 mg, 0.30 mmol) was dissolved in trifluoroacetic acid (2 mL, Sinopharm). With stirring at room temperature, triethylsilane (172.3 mg, 1.48 mmol, Shaoyuan Technology) was added. The mixture was stirred at room temperature for 7 hours. 10 mL of water was added to the reaction solution, and the mixture was extracted with ethyl acetate (10 mL×2). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 9d (65 mg, yield: 98.7%).

### Step 4

### 4-Fluoro-N-(5-fluoro-2-methyl-3-(4,4,5,5-tetramethyl-1,3,2-dioxaborolan-2-yl)phenyl)-2,2-dimethyl-2,3-dihydro-1H-indene-5-carboxamide 9e

Compound 9d (70 mg, 0.31 mmol) was dissolved in tetrahydrofuran (2 mL), and compound 3i (87 mg, 0.35 mmol) was added. The mixture was purged 3 times with nitrogen. Sodium bis(trimethylsilyl)amide (2 M, 0.24 mL, 0.47 mmol, Adamas) was added dropwise at 0°C, and the mixture was stirred at room temperature for 2 hours. 5 mL of saturated sodium bicarbonate solution was added, and the mixture was extracted with ethyl acetate (5 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 9e (43.7 mg, yield: 31.44%).

MS m/z (ESI): 442.3 [M+1]

### Step 5

### Tert-butyl (2-((4-amino-6-(5-fluoro-3-(4-fluoro-2,2-dimethyl-2,3-dihydro-1H-indene-5-carboxamido)-2-methylphenyl)pyrimidin-5-yl)oxy)ethyl)(methyl)carbamate 9f

Compound 9e (43.73 mg, 0.1 mmol) was dissolved in ethylene glycol dimethyl ether (1.4 mL) and water (0.5 mL). Bis(triphenylphosphine)palladium chloride (3.5 mg, 0.005 mmol, Adamas), compound 3k (30 mg, 0.1 mmol) and sodium carbonate (31.5 mg, 0.3 mmol, Sinopharm) were added. The mixture was purged with nitrogen for 10 minutes, and reacted under microwave conditions at 110°C for 40 minutes. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 9f (44 mg, yield: 76.34%).

MS m/z (ESI): 582.4 [M+1]

### Step 6

### N-(3-(6-amino-5-(2-(methylamino)ethoxy)pyrimidin-4-yl)-5-fluoro-2-methylphenyl)-4-fluoro-2,2-dimethyl-2,3-dihydro-1H-indene-5-carboxamide 9g

Compound **9f** (44 mg, 0.08 mmol) was dissolved in dichloromethane (1.5 mL), and trifluoroacetic acid (1.07 g, 9.42 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound 9g (36 mg, yield: 98.8%), which was directly used in the next reaction without purification.

MS m/z (ESI): 482.4 [M+1]

### Step 7

### N-(3-(6-amino-5-(2-(N-methacrylamido)ethoxy)pyrimidin-4-yl)-5-fluoro-2-methylphenyl)-4-fluoro-2,2-dimethyl-2,3-dihydro-1H-indene-5-carboxamide 9

The crude compound 9g (36 mg, 0.07 mmol) was dissolved in dichloromethane (1.5 mL), and N,N-diisopropylethylamine (48.3 mg, 0.37 mmol, Adamas) was added. Acryloyl chloride (6.8 mg, 0.07 mmol, Sinopharm) was added dropwise at -20°C, and the mixture was reacted at -20°C for 10 minutes. 5 mL of water was added, and the mixture was extracted with dichloromethane (5 mL×3). The organic phases were combined, washed with 5 mL of saturated sodium chloride solution, dried over anhydrous sodium sulfate and filtered, and the filtrate was concentrated under reduced pressure. The residue was purified by high-performance liquid chromatography (chromatographic column: welch Ultimate PFP, 5 µm 30*150 mm; mobile phase: aqueous phase (10 mM ammonium bicarbonate) and acetonitrile, gradient: aqueous phase 30%-95%) to obtain the title compound 9 (3.5 mg, yield: 8.74%).
MS m/z (ESI): 536.3 [M+1]
¹H NMR (500 MHz, CDCl₃): δ 8.65-8.59 (m, 1H), 8.43-8.36 (m, 1H), 8.17-8.10 (m, 1H), 8.03-8.00 (m, 1H), 7.16-7.14 (m, 1H), 7.00-6.97 (m, 1H), 6.61-6.53(m, 1H), 6.40-6.34 (m, 1H), 5.89 (s, 2H), 5.73-5.71 (m, 1H), 3.64-3.55 (m, 4H), 2.99 (s, 3H), 2.89-2.85 (m, 3H), 2.26-2.16 (m, 3H), 2.04-2.02 (m, 1H), 1.28-1.23 (m, 6H).

### Example 10

### N-(4-(6-amino-5-(2-(N-methacrylamido)ethoxy)pyrimidin-4-yl)-3-(hydroxymethyl)pyridin-2-yl)-6'-fluoro-2',3'-dihydrospiro[cyclopropane-1,1'-indene]-5'-carboxamide 10

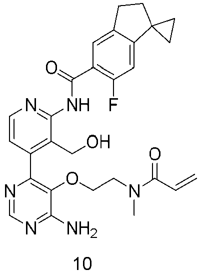

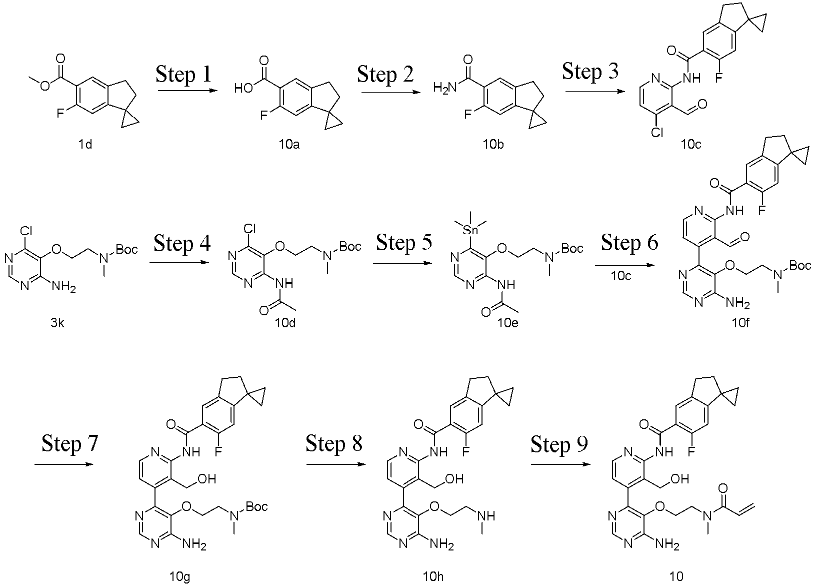

### Step 1

### 6'-Fluoro-2',3'-dihydrospiro[cyclopropane-1,1'-indene]-5'-carboxylic acid 10a

Compound **1d** (560 mg, 2.54 mmol) was dissolved in tetrahydrofuran (10 mL) and water (3 mL), and lithium hydroxide (1.07 g, 25.50 mmol, Adamas) was added. The mixture was reacted at room temperature for 2 hours. The reaction solution was adjusted to pH = 1 with 1 M hydrochloric acid, and extracted with dichloromethane (10 mL×3). The organic phases were combined, washed with 10 mL of saturated sodium chloride solution, dried over anhydrous sodium sulfate and filtered, and the filtrate was concentrated under reduced pressure to obtain the title compound 10a (524 mg, yield: 99.9%).

MS m/z (ESI): 207.1 [M+1]

### Step 2

### 6'-Fluoro-2',3'-dihydrospiro[cyclopropane-1,1'-indene]-5'-carboxamide 10b

Compound 10a (524 mg, 2.54 mmol) was dissolved in N,Ndimethylformamide (7 mL), and ammonium chloride (164 mg, 3.06 mmol, Sinopharm), *N,N-*diisopropylethylamine (1.32 g, 10.21 mmol, Adamas) and 2-(7-azabenzotriazol-1-yl)-N,N,N',N'-tetramethyluronium hexafluorophosphate (1.45 g, 3.81 mmol, Adamas) were added. The mixture was reacted at room temperature for 16 hours. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 10b (450 mg, yield: 86.3%).

MS m/z (ESI): 206.1 [M+1]

### Step 3

### N-(4-chloro-3-formylpyridin-2-yl)-6'-fluoro-2',3'-dihydrospiro[cyclopropane-1,1'-indene]-5'-carboxamide 10c

Compound 10b (200 mg, 0.97 mmol), 2-bromo-4-chloropyridine-3-carbaldehyde (257 mg, 1.16 mmol, Bidepharm), tris(dibenzylideneacetone)dipalladium (89 mg, 0.09 mmol, Bidepharm), 4,5-bis(diphenylphosphino)-9,9-dimethylxanthene (113 mg, 0.19 mmol, Shaoyuan Technology) and caesium carbonate (635 mg, 1.95 mmol, Adamas) were dissolved in 1,4-dioxane (10 mL). The mixture was purged 3 times with nitrogen, and reacted at 90°C for 5 hours. The reaction solution was concentrated under reduced pressure, and the residue was purified by silica gel column chromatography with eluent system A to obtain the title compound 10c (85 mg, yield: 25.3%).

MS m/z (ESI): 345.2 [M+1].

### Step 4

### Tert-butyl (2-((4-acetamido-6-chloropyrimidin-5-yl)oxy)ethyl)(methyl)carbamate 10d

Compound 3k (3 g, 9.91 mmol) was dissolved in dichloromethane (60 mL), and pyridine (3.13 g, 39.57 mmol, Sinopharm) and acetic anhydride (2.02 g, 19.79 mmol, Sinopharm) were added. The mixture was reacted at room temperature for 16 hours. 50 mL of water was added, and the mixture was extracted with dichloromethane (30 mL×2). The organic phases were combined, washed with 50 mL of saturated sodium chloride solution, dried over anhydrous sodium sulfate and filtered, and the filtrate was concentrated under reduced pressure. The residue was purified by column chromatography with eluent system B to obtain the title compound 10d (2.57 g, yield: 75.2%).

MS m/z (ESI): 345.3 [M+1]

### Step 5

### Tert-butyl (2-((4-acetamido-6-(trimethylstannyl)pyrimidin-5-yl)oxy)ethyl)(methyl)carbamate 10e

Compound **10d** (200 mg, 0.58 mmol) was dissolved in toluene (10 mL), and triphenylarsine (36 mg, 0.12 mmol, InnoChem), tetrakis(triphenylphosphine)palladium (67 mg, 0.06 mmol, Bidepharm) and hexamethylditin (380 mg, 1.16 mmol, Adamas) were added. The mixture was purged 3 times with nitrogen, and reacted at 120°C for 16 hours. The reaction solution was concentrated under reduced pressure to obtain the title compound 10e, which was directly used in the next reaction without purification.

MS m/z (ESI): 475.2 [M+2].

### Step 6

### Tert-butyl (2-((4-amino-6-(2-(6'-fluoro-2',3'-dihydrospiro[cyclopropane-1,1'-indene]-5'-carboxamido)-3-formylpyridin-4-yl)pyrimidin-5-yl)oxy)ethyl)(methyl)carbamate 10f

Compound 10e (236 mg, 0.50 mmol) was dissolved in 1,4-dioxane (10 mL), and compound 10c (85 mg, 0.25 mmol), cuprous iodide (95 mg, 0.50 mmol, Leyan) and bis(triphenylphosphine)palladium chloride (70 mg, 0.10 mmol, Adamas) were added. The mixture was purged 3 times with nitrogen, and reacted at 90°C for 30 minutes. The reaction solution was concentrated under reduced pressure, and the residue was purified by silica gel column chromatography with eluent system A to obtain the title compound 10f (115 mg, yield: 40.0%).

MS m/z (ESI): 577.5 [M+1].

### Step 7

### Tert-butyl (2-((4-amino-6-(2-(6'-fluoro-2',3'-dihydrospiro[cyclopropane-1,1'-indene]-5'-carboxamido)-3-(hydroxymethyl)pyridin-4-yl)pyrimidin-5-yl)oxy)ethyl)(methyl)carbamate 10g

Compound 10f (115 mg, 0.20 mmol) was dissolved in methanol (5 mL), and sodium borohydride (8 mg, 0.21 mmol, Sinopharm) was added. The mixture was reacted at room temperature for 30 minutes. The reaction was quenched with 1 N hydrochloric acid (1 mL). 10 mL of water was added, and the mixture was extracted with dichloromethane (10 mL×2). The organic phases were combined, washed with 10 mL of saturated sodium chloride solution, dried over anhydrous sodium sulfate and filtered, and the filtrate was concentrated under reduced pressure to obtain the title compound 10g (58 mg, yield: 50.4%).

MS m/z (ESI): 579.2 [M+1]

### Step 8

### N-(4-(6-amino-5-(2-(methylamino)ethoxy)pyrimidin-4-yl)-3-(hydroxymethyl)pyridin-2-yl)-6'-fluoro-2',3'-dihydrospiro[cyclopropane-1,1'-indene]-5'-carboxamide 10h

Compound **10g** (58 mg, 0.10 mmol) was dissolved in dichloromethane (2 mL), and trifluoroacetic acid (924 mg, 8.10 mmol, Adamas) was added. The mixture was reacted at room temperature for 1 hour. The reaction solution was concentrated under reduced pressure. 3 mL of saturated sodium bicarbonate was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure to obtain the crude title compound 10h (47 mg, yield: 98.1%), which was directly used in the next reaction without purification.

MS m/z (ESI): 479.2 [M+1]

### Step 9

### N-(4-(6-amino-5-(2-(N-methacrylamido)ethoxy)pyrimidin-4-yl)-3-(hydroxymethyl)pyridin-2-yl)-6'-fluoro-2',3'-dihydrospiro[cyclopropane-1,1'-indene]-5'-carboxamide 10

The crude compound 10h (47 mg, 0.10 mmol) was dissolved in dichloromethane (1 mL), and N,N-diisopropylethylamine (565 mg, 4.37 mmol, Adamas) was added. Acryloyl chloride (9.5 mg, 0.10 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound 10 (1.8 mg, yield: 3.4%).
MS m/z (ESI): 533.2 [M+1]
¹H NMR (500 MHz, DMSO-*d*₆) δ 10.64-10.37 (m, 1H), 8.58-8.41 (m, 2H), 8.09 (s, 2H), 7.57 (t, 1H), 7.31 (dd, 1H), 6.76 (d, 1H), 6.62 (ddd, 1H), 6.07 (ddd, 1H), 5.61 (ddd, 1H), 4.46 (d, 2H), 3.62-3.48 (m, 4H), 3.02 (t, 2H), 2.82-2.48 (m, 3H), 2.15 (t, 2H), 1.02 (d, 4H).

### Example 11

### N-(3-(6-amino-5-(2-(N-methacrylamido)ethoxy)pyrimidin-4-yl)-5-fluoro-2-(hydroxymethyl)phenyl)-6'-fluoro-2',3'-dihydrospiro[cyclopropane-1,1'-indene]-5'-carboxamide 11

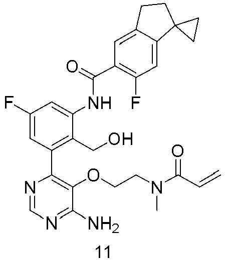

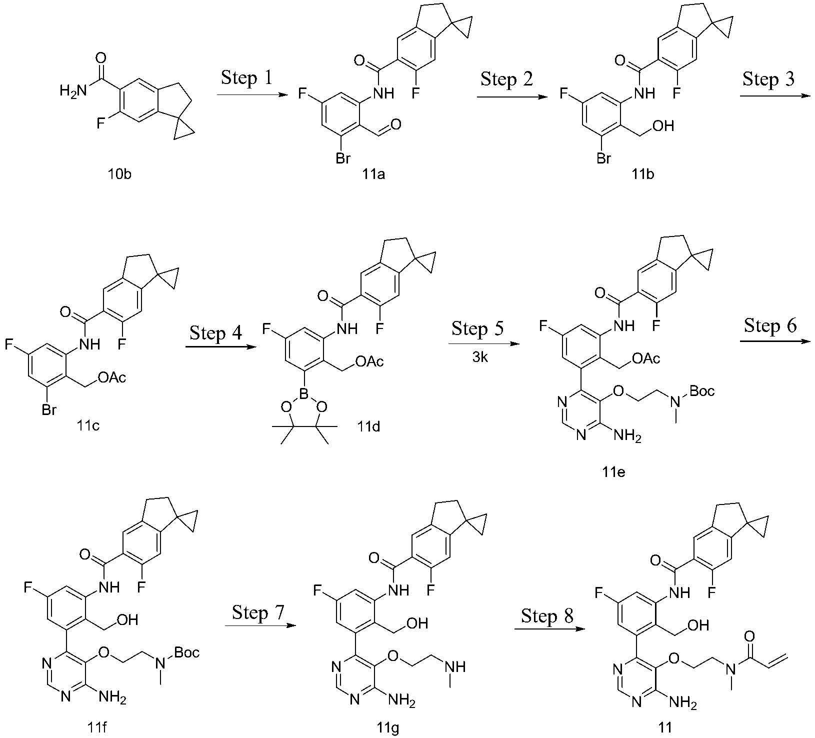

### Step 1

### N-(3-bromo-5-fluoro-2-formylphenyl)-6'-fluoro-2',3'-dihydrospiro[cyclopropane-1,1'-indene]-5'-carboxamide 11a

Compound 10b (300 mg, 1.46 mmol), 2,6-dibromo-4-fluorobenzaldehyde (825 mg, 2.92 mmol, Bidepharm), tris(dibenzylideneacetone)dipalladium (135 mg, 0.15 mmol, Bidepharm), 4,5-bis(diphenylphosphino)-9,9-dimethylxanthene (85 mg, 0.15 mmol, Shaoyuan Technology) and caesium carbonate (715 mg, 2.19 mmol, Adamas) were dissolved in 1,4-dioxane (15 mL). The mixture was purged 3 times with nitrogen, and reacted at 100°C for 16 hours. The reaction solution was concentrated under reduced pressure, and the residue was purified by silica gel column chromatography with eluent system B to obtain the title compound 11a (330 mg, yield: 55.6%).

MS m/z (ESI): 407.9 [M+2].

### Step 2

### N-(3-bromo-5-fluoro-2-(hydroxymethyl)phenyl)-6'-fluoro-2',3'-dihydrospiro[cyclopropane-1,1'-indene]-5'-carboxamide 11b

Compound **11a** (330 mg, 0.81 mmol) was dissolved in methanol (10 mL), and sodium borohydride (31 mg, 0.82 mmol, Sinopharm)was added. The mixture was reacted at room temperature for 30 minutes. The reaction was quenched with 1 N hydrochloric acid (1 mL).15 mL of water was added, and the mixture was extracted with dichloromethane (15 mL×2). The organic phases were combined, washed with 15 mL of saturated sodium chloride solution, dried over anhydrous sodium sulfate and filtered, and the filtrate was concentrated under reduced pressure to obtain the title compound 11b (330 mg, yield: 99.5%).

MS m/z (ESI): 406.2 [M-2]

### Step 3

### Benzyl 2-bromo-4-fluoro-6-(6'-fluoro-2',3'-dihydrospiro[cyclopropane-1,1'-indene]-5'-carboxamido)acetate 11c

Compound 11b (330 mg, 0.81 mmol) was dissolved in dichloromethane (10 mL), and triethylamine (82 mg, 0.81 mmol, Sinopharm) and 4-dimethylaminopyridine (10 mg, 0.08 mmol, Shaoyuan Technology) were added. At 0°C, acetic anhydride (83 mg, 0.81 mmol, Sinopharm) was added dropwise. The mixture was reacted at room temperature for 1 hour. 10 mL of water was added, and the mixture was extracted with dichloromethane (10 mL×2). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound **11c** (225 mg, yield: 61.8%).

MS m/z (ESI): 451.9 [M+2]

### Step 4

### Benzyl 4-fluoro-2-(6'-fluoro-2',3'-dihydrospiro[cyclopropane-1,1'-indene]-5'-carboxamido)-6-(4,4,5,5-tetramethyl-1,3,2-dioxaborolan-2-yl)acetate 11d

Compound **11c** (225 mg, 0.50 mmol) was dissolved in 1,4-dioxane (5 mL), and [1,1-bis(diphenylphosphino)ferrocene]palladium dichloride-dichloromethane complex (42 mg, 0.05 mmol, Bidepharm), bis(pinacolato)diboron (318 mg, 1.25 mmol, Shaoyuan Technology) and potassium acetate (147 mg, 1.5 mmol, Adamas) were added. The mixture was purged 3 times with nitrogen, and reacted at 90°C for 16 hours. The reaction solution was concentrated under reduced pressure, and the residue was purified by silica gel column chromatography with eluent system B to obtain the title compound 11d (150 mg, yield: 60.3%).

¹H NMR (500 MHz, CDCl₃) δ 9.27 (d, 1H), 8.05 (dd, 1H), 7.94 (d, 1H), 7.35 (dd, 1H), 6.45 (d, 1H), 5.48 (s, 2H), 3.08 (t, 2H), 2.21 (t, 2H), 2.08 (s, 3H), 1.28 (s, 12H), 1.09-1.06 (m, 2H), 1.00-0.96 (m, 2H).

### Step 5

### Benzyl 2-(6-amino-5-(2-((tert-butoxycarbonyl)(methyl)amino)ethoxy)pyrimidin-4-yl)-4-fluoro-6-(6'-fluoro-2',3'-dihydrospiro[cyclopropane-1,1'-indene]-5'-carboxamido)acetate 11e

Compound **11d** (150 mg, 0.30 mmol) was dissolved in 1,4-dioxane (2 mL) and water (0.5 mL), and compound 3k (92 mg, 0.30 mmol), tetrakis(triphenylphosphine)palladium (35 mg, 0.03 mmol, Bidepharm) and potassium carbonate (85 mg, 0.61 mmol, Sinopharm) were added. The mixture was purged 3 times with nitrogen, and reacted under microwave conditions at 110°C for 30 minutes. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **11e** (191 mg, yield: 99.3%).

MS m/z (ESI): 638.3 [M+1]

### Step 6

### Tert-butyl (2-((4-amino-6-(5-fluoro-3-(6'-fluoro-2',3'-dihydrospiro[cyclopropane-1,1'-indene]-5'-carboxamido)-2-(hydroxymethyl)phenyl)pyrimidin-5-yl)oxy)ethyl)(methyl)carbamate 11f

Compound **11e** (191 mg, 0.30 mmol) was dissolved in tetrahydrofuran (1.5 mL) and water (1.5 mL), and lithium hydroxide (36 mg, 1.50 mmol, Adamas) was added. The mixture was reacted at room temperature for 30 minutes. The reaction solution was adjusted to pH = 1 with 1 M hydrochloric acid, and extracted with dichloromethane (10 mL×3). The organic phases were combined, washed with 10 mL of saturated sodium chloride solution, dried over anhydrous sodium sulfate and filtered, and the filtrate was concentrated under reduced pressure to obtain the title compound **11f** (178 mg, yield: 99.7%).

MS m/z (ESI): 596.2 [M+1]

### Step 7

### N-(3-(6-amino-5-(2-(methylamino)ethoxy)pyrimidin-4-yl)-5-fluoro-2-(hydroxymethyl)phenyl)-6'-fluoro-2',3'-dihydrospiro[cyclopropane-1,1'-indene]-5'-carboxamide 11g

Compound **11f** (178 mg, 0.29 mmol) was dissolved in dichloromethane (3 mL), and trifluoroacetic acid (1.85 g, 16.2 mmol, Adamas) was added. The mixture was reacted at room temperature for 1 hour. The reaction solution was concentrated under reduced pressure. 5 mL of saturated sodium bicarbonate was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound 11g (148 mg, yield: 99.9%), which was directly used in the next reaction without purification.

MS m/z (ESI): 496.4 [M+1]

### Step 8

### N-(3-(6-amino-5-(2-(N-methacrylamido)ethoxy)pyrimidin-4-yl)-5-fluoro-2-(hydroxymethyl)phenyl)-6'-fluoro-2',3'-dihydrospiro[cyclopropane-1,1'-indene]-5'-carboxamide 11

The crude compound 11g (148 mg, 0.29 mmol) was dissolved in dichloromethane (2 mL), and N,N-diisopropylethylamine (193 mg, 1.49 mmol, Adamas) was added. Acryloyl chloride (27 mg, 0.29 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound 11 (13 mg, yield: 7.9%).
MS m/z (ESI): 550.1 [M+1]
¹H NMR (500 MHz, DMSO-*d*₆) δ 10.43-10.29 (m, 1H), 8.20 (d, 1H), 8.11 (dd, 1H), 7.74 (dd, 1H), 7.12 (s, 2H), 6.91 (ddd, 1H), 6.80 (dd, 1H), 6.67-6.51 (m, 1H), 6.02 (ddd, 1H), 5.64 (dt, 1H), 5.53 (ddd, 1H), 4.37 (dd, 2H), 3.60-3.41 (m, 4H), 3.03 (t, 2H), 2.74-2.52 (m, 3H), 2.16 (t, 2H), 1.04 (d, 4H).

### Example 12

### N-(3-(6-amino-5-(2-(N-methacrylamido)ethoxy)pyrimidin-4-yl)-5-fluoro-2-methylphenyl)-6-fluoro-1,1-dimethyl-2,3-dihydro-1H-indene-5-carboxamide 12

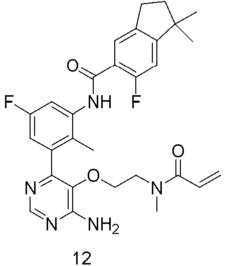

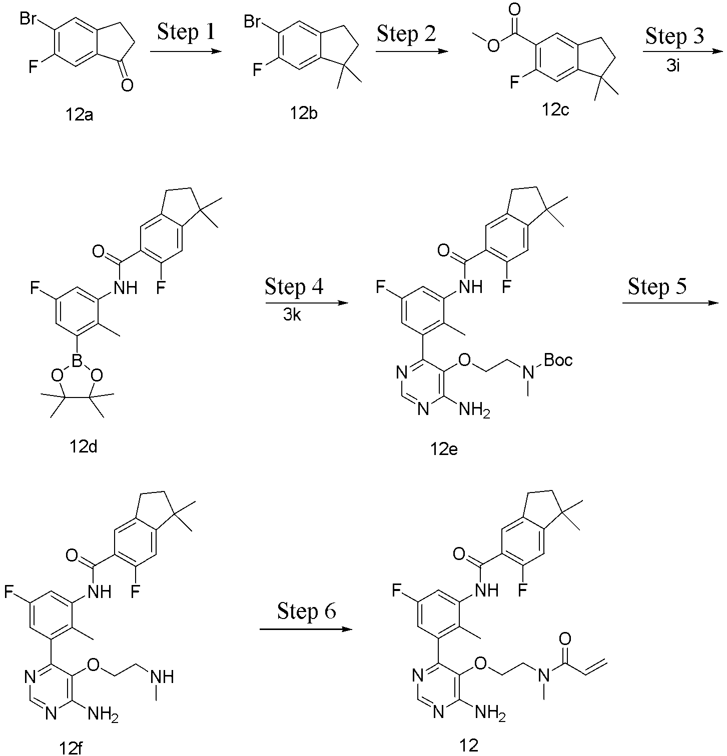

### Step 1

### 5-Bromo-6-fluoro-1,1-dimethyl-2,3-dihydro-1H-indene 12b

Titanium tetrachloride (332 mg, 1.75 mmol, Bidepharm) was dissolved in dichloromethane (3 mL). Under a nitrogen atmosphere and at -78°C, dimethylzinc (1 M, 2.62 mL, 2.62 mmol, Adamas) was added dropwise. The mixture was reacted at -78°C for 20 minutes. Then, a solution of 5-bromo-6-fluoro-2,3-dihydro-1H-indene-1-one 12a (200 mg, 0.87 mmol, Bidepharm) in dichloromethane (1.5 mL) was added dropwise. The mixture was reacted at room temperature for 12 hours. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 12b (180 mg, yield: 84.8%).

### Step 2

### Methyl 6-fluoro-1,1-dimethyl-2,3-dihydro-1H-indene-5-carboxylate 12c

Compound **12b** (180 mg, 0.74 mmol) was dissolved in *N,N-*dimethylformamide (2 mL) and methanol (2 mL), and 1,3-bis(diphenylphosphino)propane (62 mg, 0.15 mmol, Shaoyuan Technology), *N,N-*diisopropylethylamine (192 mg, 1.49 mmol, Adamas) and palladium acetate (34 mg, 0.15 mmol, Adamas) were added. The mixture was purged 3 times with carbon monoxide, and reacted at 80°C for 16 hours. The reaction solution was concentrated under reduced pressure. The residue was purified by column chromatography with eluent system B to obtain the title compound 12c (105 mg, yield: 63.8%).

### Step 3

### 6-Fluoro-N-(5-fluoro-2-methyl-3-(4,4,5,5-tetramethyl-1,3,2-dioxaborolan-2-yl)phenyl)-1,1-dimethyl-2,3-dihydro-1H-indene-5-carboxamide 12d

Compound 12c (105 mg, 0.47 mmol) was dissolved in tetrahydrofuran (2 mL), and compound 3i (131 mg, 0.52 mmol) was added. The mixture was purged 3 times with nitrogen. Sodium bis(trimethylsilyl)amide (2 M, 0.35 mL, 0.70 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound 12d (63 mg, yield: 30.2%).

MS m/z (ESI): 442.4 [M+1]

### Step 4

### Tert-butyl (2-((4-amino-6-(5-fluoro-3-(6-fluoro-1,1-dimethyl-2,3-dihydro-1H-indene-5-carboxamido)-2-methylphenyl)pyrimidin-5-yl)oxy)ethyl)(methyl)carbamate 12e

Compound 12d (63 mg, 0.14 mmol) was dissolved in ethylene glycol dimethyl ether (2 mL) and water (0.8 mL), and bis(triphenylphosphine)palladium chloride (11 mg, 0.02 mmol, Adamas), compound 3k (52 mg, 0.17 mmol) and sodium carbonate (31 mg, 0.29 mmol, Sinopharm) were added. The mixture was purged 3 times with nitrogen, and reacted at 110°C under microwave conditions for 1 hour. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 12e (63 mg, yield: 75.9%).

MS m/z (ESI): 582.6 [M+1]

### Step 5

### N-(3-(6-amino-5-(2-(methylamino)ethoxy)pyrimidin-4-yl)-5-fluoro-2-methylphenyl)-6-fluoro-1,1-dimethyl-2,3-dihydro-1H-indene-5-carboxamide 12f

Compound 12e (63 mg, 0.11 mmol) was dissolved in dichloromethane (1 mL), and trifluoroacetic acid (462 mg, 4.05 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound 12f (52 mg, yield: 99.7%), which was directly used in the next reaction without purification.

MS m/z (ESI): 482.3 [M+1]

### Step 6

### N-(3-(6-amino-5-(2-(N-methacrylamido)ethoxy)pyrimidin-4-yl)-5-fluoro-2-methylphenyl)-6-fluoro-1,1-dimethyl-2,3-dihydro-1H-indene-5-carboxamide 12

The crude compound 12f (52 mg, 0.11 mmol) was dissolved in dichloromethane (2 mL), and N,N-diisopropylethylamine (70 mg, 0.54 mmol, Adamas) was added. Acryloyl chloride (9.8 mg, 0.11 mmol, Sinopharm) was added dropwise at -20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 12 (11 mg, yield: 19.0%).
MS m/z (ESI): 536.5 [M+1]
¹H NMR (500 MHz, DMSO-*d₆*): δ 9.84-9.63 (m, 1H), 8.21-8.18 (m, 1H), 7.64-7.50 (m, 2H), 7.22-6.96 (m, 4H), 6.66-6.58 (m, 1H), 6.10-6.03 (m, 1H), 5.62-5.58 (m, 1H), 3.59-3.45 (m, 4H), 2.92-2.81 (m, 3H), 2.53 (s, 3H), 2.03-1.93 (m, 4H), 1.26-1.24 (m, 6H).

### Example 13

### N-(3-(6-amino-5-(2-(N-methacrylamido)ethoxy)pyrimidin-4-yl)-5-fluoro-2-methylphenyl)-4'-fluoro-2',3'-dihydrospiro[cyclopropane-1,1'-indene]-5'-carboxamide 13

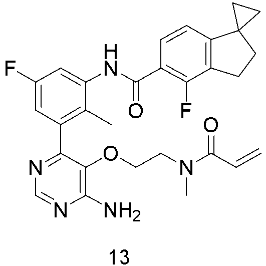

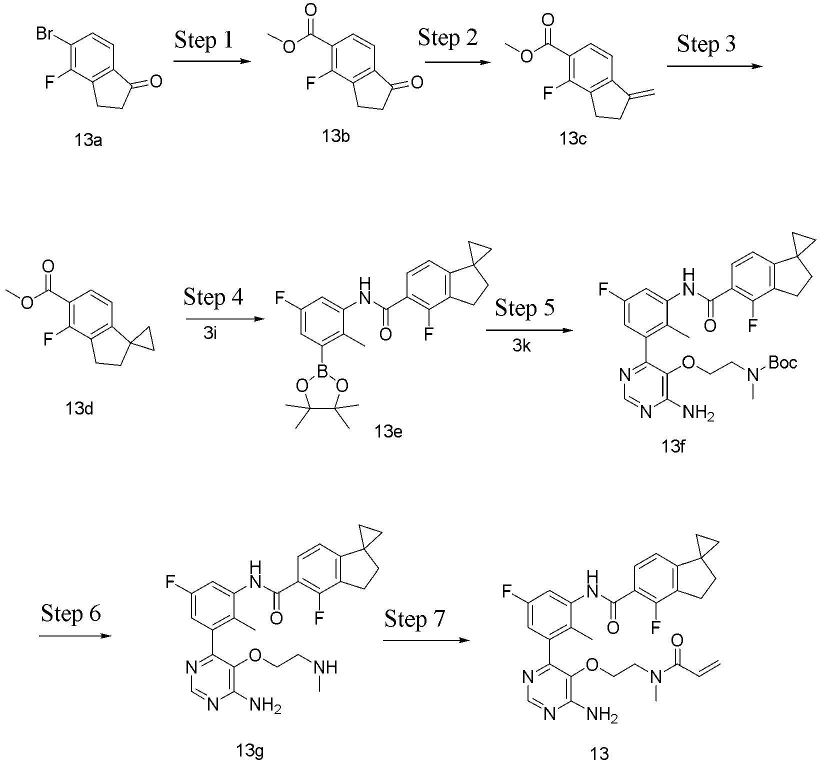

### Step 1

### Methyl 4-fluoro-1-oxo-2,3-dihydro-1H-indene-5-carboxylate 13b

5-Bromo-4-fluoro-2,3-dihydro-1*H*-indene-1-one **13a** (2 g, 8.73 mmol, Bidepharm) was dissolved in *N,N-*dimethylformamide (10 mL) and methanol (5 mL). [1,1'-bis(diphenylphosphino)ferrocene]palladium dichloride (640 mg, 0.87 mmol, Adamas) and *N,N*-diisopropylethylamine (2.26 g, 17.46 mmol, Adamas) were added. The mixture was purged 3 times with carbon monoxide, and reacted at 80°C for 16 hours. The reaction solution was concentrated under reduced pressure. The residue was purified by column chromatography with eluent system B to obtain the title compound **13b** (830 mg, yield: 45.7%).

### Step 2

### Methyl 4-fluoro-1-methylidene-2,3-dihydro-1H-indene-5-carboxylate 13c

Compound **13b** (800 mg, 3.84 mmol) was dissolved in tetrahydrofuran (15 mL), and methyltriphenylphosphonium bromide (2.61 g, 7.69 mmol, Shaoyuan Technology) and potassium tert-butoxide (1 M, 7.69 mL, 7.69 mmol, Adamas) were added. The mixture was reacted at room temperature for 2 hours. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound **13c** (490 mg, yield: 61.8%).

MS m/z (ESI): 207.3 [M+1]

### Step 3

### Methyl 4'-fluoro-2',3'-dihydrospiro[cyclopropane-1,1'-indene]-5'-carboxylate 13d

Diethylzinc (1 M, 9.50 mL, 9.50 mmol, Adamas) was dissolved in dichloromethane (4 mL). Under a nitrogen atmosphere and at 0°C, trifluoroacetic acid (1.08 g, 9.50 mmol, Adamas) was added dropwise. At 0°C, the mixture was reacted for 1 hour; then diiodomethane (2.55 mg, 9.50 mmol, Energy Chemical) was added dropwise. At 0°C, the mixture was reacted for 0.5 hours, and a solution of compound **13c** (490 mg, 0.44 mmol) in dichloromethane (4 mL) was added dropwise. The mixture was reacted at room temperature for 3 hours. 15 mL of water was added, and the mixture was extracted with ethyl acetate (15 mL×3). The organic phases were combined, washed with 15 mL of saturated sodium chloride solution, dried over anhydrous sodium sulfate and filtered, and the filtrate was concentrated under reduced pressure. The residue was purified by column chromatography with eluent system B to obtain the title compound **13d** (400 mg, yield: 76.4%).

MS m/z (ESI): 221.3 [M+1]

### Step 4

### 4'-Fluoro-N-(5-fluoro-2-methyl-3-(4,4,5,5-tetramethyl-1,3,2-dioxaborolan-2-yl)phenyl)-2',3'-dihydrospiro[cyclopropane-1,1'-indene]-5'-carboxamide 13e

Compound **13d** (150 mg, 0.68 mmol) was dissolved in tetrahydrofuran (3 mL), and compound **3i** (189 mg, 0.75 mmol) was added. The mixture was purged 3 times with nitrogen. Sodium bis(trimethylsilyl)amide (2 M, 0.51 mL, 1.02 mmol, Adamas) was added dropwise at 0°C, and the mixture was stirred at room temperature for 2 hours. 15 mL of saturated sodium bicarbonate solution was added, and the mixture was extracted with ethyl acetate (20 mL×3). The organic phases were combined, washed with 20 mL of saturated sodium chloride solution, dried over anhydrous sodium sulfate and filtered, and the filtrate was concentrated under reduced pressure. The residue was purified by column chromatography with eluent system B to obtain the title compound **13e** (230 mg, yield: 76.9%).

MS m/z (ESI): 440.5 [M+1]

### Step 5

### Tert-butyl (2-((4-amino-6-(5-fluoro-3-(4'-fluoro-2',3'-dihydrospiro[cyclopropane-1,1'-indene]-5'-carboxamido)-2-methylphenyl)pyrimidin-5-yl)oxy)ethyl)(methyl)carbamate 13f

The crude compound **13e** (230 mg, 0.52 mmol) was dissolved in ethylene glycol dimethyl ether (3 mL) and water (1 mL), and bis(triphenylphosphine)palladium chloride (37 mg, 0.05 mmol, Adamas), compound **3k** (190 mg, 0.63 mmol) and sodium carbonate (111 mg, 1.05 mmol, Sinopharm) were added. The mixture was purged 3 times with nitrogen, and reacted at 110°C under microwave conditions for 1 hour. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **13f** (200 mg, yield: 65.9%).

MS m/z (ESI): 580.6 [M+1]

### Step 6

### N-(3-(6-amino-5-(2-(methylamino)ethoxy)pyrimidin-4-yl)-5-fluoro-2-methylphenyl)-4'-fluoro-2',3'-dihydrospiro[cyclopropane-1,1'-indene]-5'-carboxamide 13g

The crude compound **13f** (100 mg, 0.17 mmol) was dissolved in dichloromethane (2 mL), and trifluoroacetic acid (770 mg, 6.75 mmol, Adamas) was added. The mixture was reacted at room temperature for 1 hour. The reaction solution was concentrated under reduced pressure. 5 mL of saturated sodium bicarbonate was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound **13g** (82 mg, yield: 99.1%), which was directly used in the next reaction without purification.

MS m/z (ESI): 480.1 [M+1]

### Step 7

### N-(3-(6-amino-5-(2-(N-methacrylamido)ethoxy)pyrimidin-4-yl)-5-fluoro-2-methylphenyl)-4'-fluoro-2',3'-dihydrospiro[cyclopropane-1,1'-indene]-5'-carboxamide 13

The crude compound **13g** (82 mg, 0.17 mmol) was dissolved in dichloromethane (2 mL), and *N,N*-diisopropylethylamine (111 mg, 0.86 mmol, Adamas) was added. Acryloyl chloride (15.5 mg, 0.86 mmol, Sinopharm) was added dropwise at -20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **13** (16 mg, yield: 17.5%).
MS m/z (ESI): 534.5 [M+1]
¹H NMR (500 MHz, DMSO-*d₆*): δ 9.78-9.57 (m, 1H), 8.21-8.18 (m, 1H), 7.63-7.54 (m, 2H), 7.10-6.96 (m, 3H), 6.75-6.58 (m, 2H), 6.10-6.03 (m, 1H), 5.61-5.58 (m, 1H), 3.60-3.45 (m, 4H), 3.10-3.06 (m, 2H), 2.80 (s, 3H), 2.22-2.18 (m, 2H), 2.03-1.97 (m, 3H), 1.25-1.23 (m, 2H), 1.02-1.00 (m, 2H).

### Example 14

### N-(3-(6-amino-5-(2-(N-methacrylamido)ethoxy)pyrimidin-4-yl)-5-fluoro-2-methylphenyl)-7-fluoro-3H-spiro[benzo[b][1,4]dioxine-2,1'-cyclopropane]-6-carboxamide 14

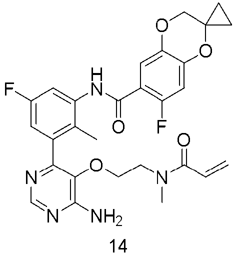

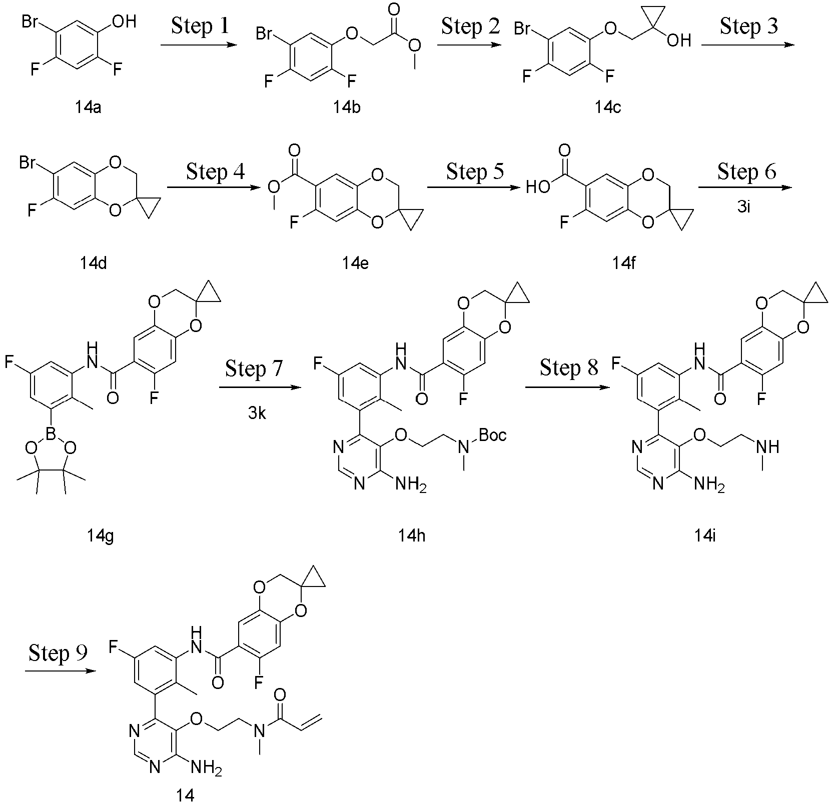

### Step 1

### Methyl 2-(5-bromo-2,4-difluorophenoxy)acetate 14b

5-Bromo-2,4-difluorophenol **14a** (1 g, 4.79 mmol, Haohong), methyl 2-bromoacetate (805 mg, 5.26 mmol, Adamas) and anhydrous potassium carbonate (992 mg, 7.17 mmol, Sinopharm) were suspended in N,N-dimethylformamide (10 mL), and the mixture was stirred at room temperature for 1.5 hours. The reaction solution was diluted in 5 mL of water, extracted with a mixed solution of petroleum ether and ethyl acetate (V/V = 1/1, 10 mL×3), dried over anhydrous sodium sulfate and filtered, and the filtrate was concentrated under reduced pressure. The residue was purified by column chromatography with eluent system B to obtain the title compound **14b** (1.25 g, yield: 92.95%).

### Step 2

### 1-((5-Bromo-2,4-difluorophenoxy)methyl)cyclopropan-1-ol 14c

In a dry, nitrogen-filled flask, compound **14b** (1 g, 3.56 mmol) was dissolved in tetrahydrofuran (15 mL) and cooled to 0°C. With stirring, tetraisopropyl titanate (1.06 g, 3.72 mmol, 1.1 mL, Adamas) was added. Then, ethylmagnesium bromide (1 M, 9.61 mL, 9.6 mmol, Adamas) was slowly added, and the mixture was warmed to room temperature and stirred for 1 hour. The reaction was cooled to 0°C, and quenched by dropwise adding 1 M dilute hydrochloric acid (20 mL). The mixture was extracted with ethyl acetate (20 mL×3), washed with 20 mL of saturated sodium chloride solution, dried over anhydrous sodium sulfate and filtered, and the filtrate was concentrated under reduced pressure. The residue was purified by column chromatography with eluent system B to obtain the title compound **14c** (750 mg, yield: 75.53%).

### Step 3

### 6-Bromo-7-fluoro-3H-spiro[benzo[b][1,4]dioxine-2,1'-cyclopropane] 14d

In a dry, nitrogen-filled flask, compound **14c** (700 mg, 2.51 mmol) was dissolved in *N,N-*dimethylformamide (20 mL), and sodium hydride (150.5 mg, 3.76 mmol, purity: 60%, Sinopharm) was added. The mixture was warmed to 80°C and stirred for 4 hours. The reaction was cooled to room temperature. The reaction solution was slowly poured into 50 mL of ice water with stirring. The mixture was extracted with ethyl acetate (50 mL×2), washed with 50 mL of saturated sodium chloride solution, dried over anhydrous sodium sulfate and filtered, and the filtrate was concentrated under reduced pressure. The residue was purified by column chromatography with eluent system B to obtain the title compound **14d** (330 mg, yield: 50.78%).

### Step 4

### Methyl 7-fluoro-3H-spiro[benzo[b][1,4]dioxine-2,1'-cyclopropane]-6-carboxylate 14e

Compound **14d** (85 mg, 0.33 mmol), triethylamine (99.6 mg, 0.98 mmol, Sinopharm), and [1,1'-bis(diphenylphosphino)ferrocene]palladium dichloride (36 mg, 0.05 mmol, Bidepharm) were dissolved in *N,N*-dimethylformamide (1.2 mL) and methanol (3.6 mL). The mixture was purged twice with nitrogen, and carbon monoxide (3 atm) was introduced. The mixture was reacted at 100°C for 12 hours. The reaction solution was cooled to room temperature, and concentrated under reduced pressure. The residue was purified by column chromatography with eluent system B to obtain the title compound **14e** (78 mg, yield: 99.8%).

### Step 5

### 7-Fluoro-3H-spiro[benzo[b][1,4]dioxine-2,1'-cyclopropane]-6-carboxylic acid 14f

Compound **14e** (78 mg, 0.33 mmol) was dissolved in tetrahydrofuran (1.5 mL) and methanol (0.5 mL). Aqueous lithium hydroxide solution (63 mg, 1.5 mmol, 1.5 mL, Sinopharm) was added, and the mixture was stirred at room temperature for 3 hours. The reaction solution was concentrated under reduced pressure, adjusted to pH = 7 with 1 *N* dilute hydrochloric acid, and extracted with ethyl acetate (15 mL×3). The organic phases were combined, dried over anhydrous sodium sulfate and filtered, and the filtrate was concentrated under reduced pressure to obtain the title compound **14f** (73 mg, yield: 99.45%).

### Step 6

### 7-Fluoro-N-(5-fluoro-2-methyl-3-(4,4,5,5-tetramethyl-1,3,2-dioxaborolan-2-yl)phenyl)-3H-spiro[benzo[b][1,4]dioxine-2,1'-cyclopropane]-6-carboxamide 14g

Compound **14f** (80 mg, 0.36 mmol) and compound **3i** (89.7 mg, 0.36 mmol) were dissolved in *N,N*-dimethylformamide (2 mL). 2-(7-Azabenzotriazol-1-yl)-*N,N,N',N*'-tetramethyluronium hexafluorophosphate (162.8 mg, 0.43 mmol, Adamas) and *N,N*-diisopropylethylamine (92.2 mg, 0.71 mmol, Adamas) were added, and then the reaction solution was warmed to 50°C and stirred for 16 hours. The reaction solution was concentrated under reduced pressure, and the residue was purified by silica gel column chromatography with eluent system A to obtain the title compound **14g** (150 mg, yield: 91.92%).

MS m/z (ESI): 458.2 [M+1].

### Step 7

### Tert-butyl (2-((4-amino-6-(5-fluoro-3-(7-fluoro-3H-spiro[benzo[b][1,4]dioxine-2,1'-cyclopropane]-6-carboxamido)-2-methylphenyl)pyrimidin-5-yl)oxy)ethyl)(methyl)carbamate 14h

Compound **14g** (152 mg, 0.33 mmol) was dissolved in ethylene glycol dimethyl ether (2.1 mL) and water (1.3 mL). Bis(triphenylphosphine)palladium chloride (12 mg, 0.02 mmol, Adamas), compound **3k** (100 mg, 0.33 mmol) and sodium carbonate (105 mg, 0.99 mmol, Sinopharm) were added. The mixture was purged with nitrogen for 10 minutes, and reacted under microwave conditions at 110°C for 40 minutes. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **14h** (132 mg, yield: 66.87%).

MS m/z (ESI): 598.6 [M+1]

### Step 8

### N-(3-(6-amino-5-(2-(methylamino)ethoxy)pyrimidin-4-yl)-5-fluoro-2-methylphenyl)-7-fluoro-3H-spiro[benzo[b][1,4]dioxine-2,1'-cyclopropane]-6-carboxamide 14i

Compound **14h** (132 mg, 0.22 mmol) was dissolved in dichloromethane (2 mL), and trifluoroacetic acid (0.2 mL, Adamas) was added. The mixture was reacted at room temperature for 12 hours. The reaction solution was concentrated under reduced pressure. 3 mL of saturated sodium bicarbonate solution was added, and the mixture was extracted with ethyl acetate (3 mL×3). The organic phases were combined, washed with 3 mL of saturated sodium chloride solution, dried over anhydrous sodium sulfate and filtered, and the filtrate was concentrated under reduced pressure to obtain the crude title compound **14i** (109 mg, yield: 99.9%), which was directly used in the next reaction without purification.

MS m/z (ESI): 498.4 [M+1]

### Step 9

### N-(3-(6-amino-5-(2-(N-methacrylamido)ethoxy)pyrimidin-4-yl)-5-fluoro-2-methylphenyl)-7-fluoro-3H-spiro[benzo[b][1,4]dioxine-2,1'-cyclopropane]-6-carboxamide 14

The crude compound **14i** (109 mg, 0.22 mmol) was dissolved in dichloromethane (3 mL), and *N,N*-diisopropylethylamine (141.6 mg, 1.1 mmol, Adamas) was added. Acryloyl chloride (20 mg, 0.22 mmol, Sinopharm) was added dropwise at -20°C, and the mixture was reacted at -20°C for 10 minutes. 5 mL of water was added, and the mixture was extracted with dichloromethane (5 mL×3). The organic phases were combined, washed with 5 mL of saturated sodium chloride solution, dried over anhydrous sodium sulfate and filtered, and the filtrate was concentrated under reduced pressure. The residue was purified by high-performance liquid chromatography (chromatographic column: welch Ultimate PFP, 5 µm 30*150 mm; mobile phase: aqueous phase (10 mM ammonium bicarbonate) and acetonitrile, gradient: aqueous phase 25%-95%) to obtain the title compound **14** (48 mg, yield: 39.72%).

MS m/z (ESI): 552.1 [M+1]
¹H NMR (500 MHz, DMSO-*d₆*): δ 9.71-9.48 (m, 1H), 8.21-8.18 (m, 1H), 7.60-7.53 (m, 1H), 7.40-7.31(m, 1H), 7.10-7.05 (m, 2H), 7.00-6.92 (m, 2H), 6.68-6.57 (m, 1H), 6.10-6.03 (m, 1H), 5.61-5.58 (m, 1H), 4.27 (s, 2H), 3.59-3.55 (m, 2H), 3.49-3.45 (m, 2H), 2.53-2.49 (m, 3H), 2.00-1.95 (m, 3H), 1.07 (d, 2H), 0.93 (t, 2H).

### Example 15

### N-(3-(6-amino-5-(2-(N-methacrylamido)ethoxy)pyrimidin-4-yl)-5-fluoro-2-methylphenyl)-5-fluoro-2,2-dimethyl-2,3-dihydrobenzo[b][1,4]dioxine-6-carboxamide 15

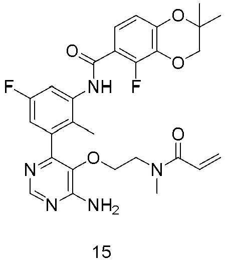

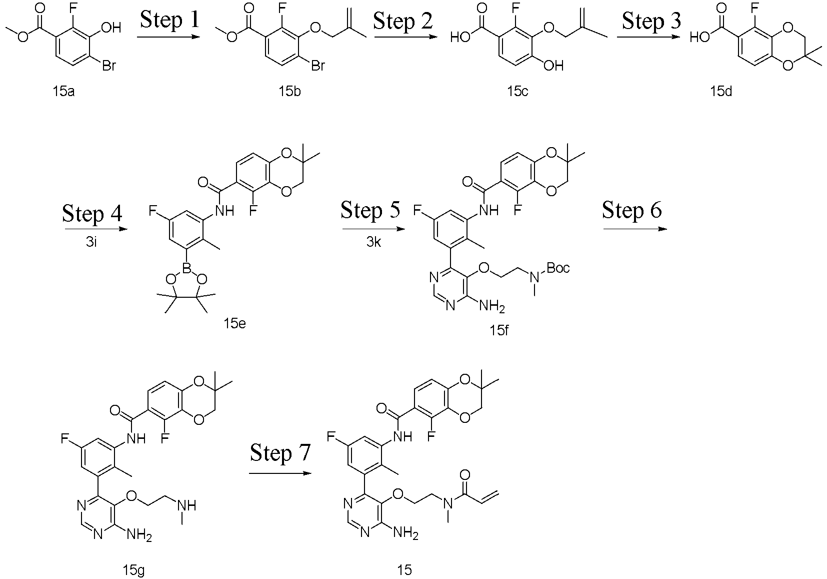

### Step 1

Methyl 4-bromo-2-fluoro-3-((2-methylallyl)oxy)benzoate **15b**

Methyl 4-bromo-2-fluoro-3-hydroxylbenzoate **15a** (4.3 g, 17.26 mmol, Bidepharm) was dissolved in *N,N*-dimethylformamide (35 mL), and potassium carbonate (2.86 g, 20.72 mmol, Sinopharm) and 3-bromo-2-methylprop-1-ene (2.8 g, 20.72 mmol, Adamas) were added. The mixture was reacted at room temperature for 16 hours. The reaction solution was filtered, and the filtrate was concentrated under reduced pressure. The residue was purified by column chromatography with eluent system B to obtain the title compound **15b** (5.08 g, yield: 97.0%)
MS m/z (ESI): 305.2 [M+2]

### Step 2

### 2-Fluoro-4-hydroxyl-3-((2-methylallyl)oxy)benzoic acid 15c

Compound **15b** (2 g, 6.6 mmol) was dissolved in 1,4-dioxane (20 mL) and water (20 mL), and tris(dibenzylideneacetone)dipalladium (604 mg, 0.66 mmol, Adamas), 2'-di-tert-butylphosphino-2,4,6-triisopropylbiphenyl (560 mg, 1.32 mmol, Bidepharm) and potassium hydroxide (740 mg, 13.19 mmol, Adamas) were added. The mixture was purged 3 times with nitrogen, and reacted at 90°C for 2 hours. 10 mL of water was added, and the mixture was extracted with dichloromethane (20 mL×2). The aqueous phases were combined, adjusted to pH = 1 with 1 M hydrochloric acid, and extracted with dichloromethane (20 mL×2). The organic phases were combined, washed with 20 mL of saturated sodium chloride solution, dried over anhydrous sodium sulfate and filtered, and the filtrate was concentrated under reduced pressure to obtain the crude title compound **15c** (500 mg, yield: 33.5%).

MS m/z (ESI): 225.0 [M-1]

### Step 3

### 5-Fluoro-2,2-dimethyl-2,3-dihydrobenzo[b][1,4]dioxine-6-carboxylic acid 15d

Compound **15c** (500 mg, 2.21 mmol) was dissolved in formic acid (15 mL, Adamas), and the mixture was reacted at 110°C for 30 minutes. The reaction solution was cooled to room temperature. 10 mL of water was added, and the mixture was extracted with dichloromethane (20 mL×2). The organic phases were combined, washed with 20 mL of saturated sodium chloride solution, dried over anhydrous sodium sulfate and filtered, and the filtrate was concentrated under reduced pressure. The residue was purified by column chromatography with eluent system A to obtain the title compound **15d** (25 mg, yield: 5.0%).

MS m/z (ESI): 227.1 [M+1]

### Step 4

### 5-Fluoro-N-(5-fluoro-2-methyl-3-(4,4,5,5-tetramethyl-1,3,2-dioxaborolan-2-yl)phenyl)-2,2-dimethyl-2,3-dihydrobenzo[b][1,4]dioxine-6-carboxamide 15e

Compound **15d** (25 mg, 0.11 mmol) was dissolved in *N,N-*dimethylformamide (3 mL). Compound **3i** (33 mg, 0.13 mmol), *N,N-*diisopropylethylamine (30 mg, 0.23 mmol, Adamas) and 2-(7-azabenzotriazol-1-yl)-*N*,*N*,*N'*,*N'*-tetramethyluronium hexafluorophosphate (50 mg, 0.13 mmol, Adamas) were added, and the mixture was reacted at 50°C for 16 hours. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound **15e** (50 mg, yield: 98.5%).

MS m/z (ESI): 460.1 [M+1]

### Step 5

### Tert-butyl (2-((4-amino-6-(5-fluoro-3-(5-fluoro-2,2-dimethyl-2,3-dihydrobenzo[b][1,4]dioxine-6-carboxamido)-2-methylphenyl)pyrimidin-5-yl)oxy)ethyl)(methyl)carbamate 15f

Compound **15e** (50 mg, 0.11 mmol) was dissolved in 1,4-dioxane (2 mL) and water (0.5 mL), and compound **3k** (40 mg, 0.13 mmol), tetrakis(triphenylphosphine)palladium (13 mg, 0.01 mmol, Bidepharm) and potassium carbonate (30 mg, 0.22 mmol, Sinopharm) were added. The mixture was purged 3 times with nitrogen, and reacted under microwave conditions at 110°C for 30 minutes. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **15f** (48 mg, yield: 73.5%).

MS m/z (ESI): 600.2 [M+1]

### Step 6

### N-(3-(6-amino-5-(2-(methylamino)ethoxy)pyrimidin-4-yl)-5-fluoro-2-methylphenyl)-5-fluoro-2,2-dimethyl-2,3-dihydrobenzo[b][1,4]dioxine-6-carboxamide 15g

Compound **15f** (48 mg, 0.08 mmol) was dissolved in dichloromethane (3 mL), and trifluoroacetic acid (100 mg, 0.88 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound **15g** (39 mg, yield: 97.5%), which was directly used in the next reaction without purification.

MS m/z (ESI): 500.2 [M+1]

### Step 7

### N-(3-(6-amino-5-(2-(N-methacrylamido)ethoxy)pyrimidin-4-yl)-5-fluoro-2-methylphenyl)-5-fluoro-2,2-dimethyl-2,3-dihydrobenzo[b][1,4]dioxine-6-carboxamide 15

The crude compound **15g** (39 mg, 0.078 mmol) was dissolved in dichloromethane (2 mL), and *N,N*-diisopropylethylamine (50 mg, 0.39 mmol, Adamas) was added. Acryloyl chloride (7 mg, 0.077 mmol, Sinopharm) was added dropwise at -20°C, and the mixture was reacted at -20°C for 1 hour. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound **15** (14 mg, yield: 32.4%).
MS m/z (ESI): 554.2 [M+1]
¹H NMR (500 MHz, DMSO-*d₆*) δ 9.86-9.63 (m, 1H), 8.50 (s, 1H), 7.66 (s, 1H), 7.37-7.08 (m, 4H), 6.84 (dd, 1H), 6.61 (ddd, 1H), 6.07 (ddd, 1H), 5.64-5.57 (m, 1H), 4.08 (d, 2H), 3.69-3.47 (m, 4H), 2.82-2.50 (m, 3H), 2.04 (d, 3H), 1.35 (s, 6H).

### Example 16-1

### N-(3-(6-amino-5-(2-(N-methacrylamido)ethoxy)pyrimidin-4-yl)-5-fluoro-2-methylphenyl)-4-fluoro-2,2-dimethyl-2,3-dihydrobenzofuran-5-carboxamide 16-1

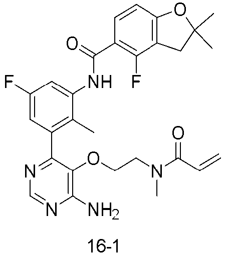

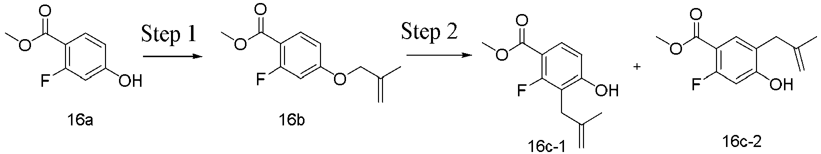

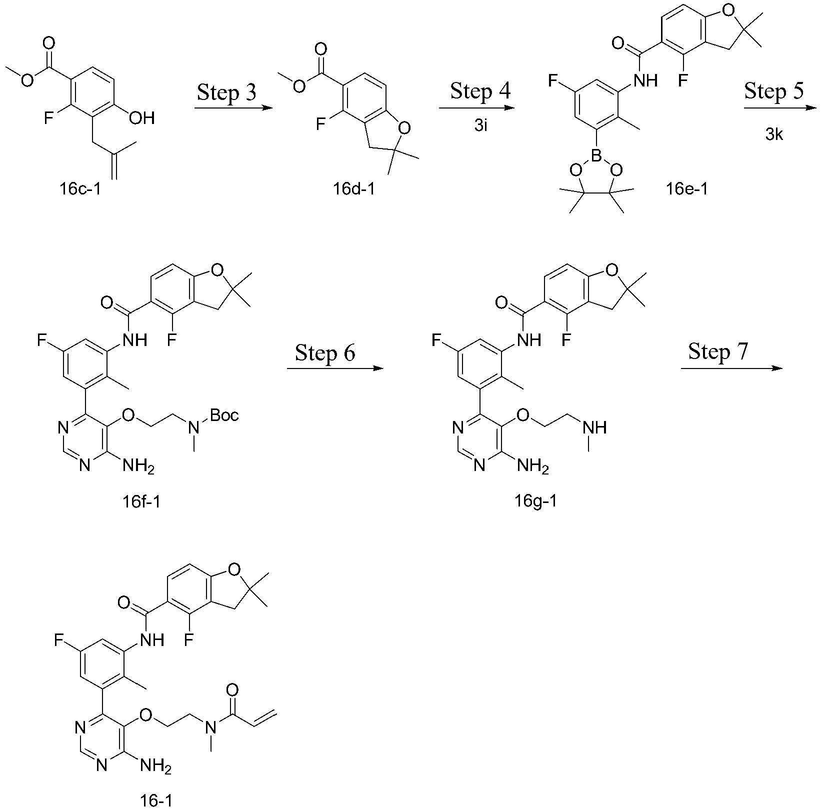

### Step 1

### Methyl 2-fluoro-4-((2-methylallyl)oxy)benzoate 16b

Methyl 2-fluoro-4-hydroxylbenzoate **16a** (2 g, 11.76 mmol, Bidepharm) was dissolved in acetonitrile (40 mL), and potassium carbonate (2.44 g, 17.65 mmol, Sinopharm) was added. The mixture was cooled to 0°C, and 3-bromo-2-methylprop-1-ene (1.91 g, 14.15 mmol, Adamas) was added dropwise. The mixture was reacted at room temperature for 12 hours. 50 mL of water was added, and the mixture was extracted with ethyl acetate (50 mL×3). The organic phases were combined, washed with 40 mL of saturated sodium chloride solution, dried over anhydrous sodium sulfate and filtered, and the filtrate was concentrated under reduced pressure. The residue was purified by column chromatography with eluent system B to obtain the title compound **16b** (2.14 g, yield: 81.2%).

### Step 2

### Methyl 2-fluoro-4-hydroxyl-3-(2-methylallyl)benzoate 16c-1

### Methyl 2-fluoro-4-hydroxyl-5-(2-methylallyl)benzoate 16c-2

Compound **16b** (2.83 g, 12.62 mmol) was dissolved in biphenyl-diphenyl ether (20 mL), and the mixture was reacted at 210°C for 2 hours. The reaction solution was cooled to room temperature and then directly purified by column chromatography with eluent system B to obtain the title compound **16c-1** (430 mg, yield: 15.2%) and compound **16c-2** (400 mg, yield: 14.1%).

MS m/z (ESI): 225.1 [M+1]

### Step 3

### Methyl 4-fluoro-2,2-dimethyl-2,3-dihydrobenzofuran-5-carboxylate 16d-1

Compound **16c-1** (420 mg, 1.87 mmol) was dissolved in formic acid (20 mL), and the mixture was reacted at 110°C for 0.5 hours. 50 mL of water was added, and the mixture was extracted with ethyl acetate (50 mL×3). The organic phases were combined, washed with 50 mL of saturated sodium bicarbonate solution, dried over anhydrous sodium sulfate and filtered, and the filtrate was concentrated under reduced pressure. The residue was purified by column chromatography with eluent system B to obtain the title compound **16d-1** (420 mg, yield: 100.0%).

MS m/z (ESI): 225.0 [M+1]

### Step 4

### 4-Fluoro-N-(5-fluoro-2-methyl-3-(4,4,5,5-tetramethyl-1,3,2-dioxaborolan-2-yl)phenyl)-2,2-dimethyl-2,3-dihydrobenzofuran-5-carboxamide 16e-1

Compound **16d-1** (150 mg, 0.67 mmol) was dissolved in tetrahydrofuran (3 mL), and compound **3i** (185 mg, 0.74 mmol) was added. The mixture was purged 3 times with nitrogen. Sodium bis(trimethylsilyl)amide (2 M, 0.50 mL, 1.00 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound **16e-1** (260 mg, yield: 87.7%).

MS m/z (ESI): 444.2 [M+1]

### Step 5

### Tert-butyl (2-((4-amino-6-(5-fluoro-3-(4-fluoro-2,2-dimethyl-2,3-dihydrobenzofuran-5-carboxamido)-2-methylphenyl)pyrimidin-5-yl)oxy)ethyl)(methyl)carbamate 16f-1

Compound **16e-1** (260 mg, 0.59 mmol) was dissolved in 1,4-dioxane (3 mL) and water (1 mL), and [1,1'-bis(diphenylphosphino)ferrocene]palladium dichloride (86 mg, 0.12 mmol, Bidepharm), compound **3k** (213 mg, 0.70 mmol) and potassium carbonate (163 mg, 1.18 mmol, Sinopharm) were added. The mixture was purged 3 times with nitrogen, and reacted at 90°C for 2 hours. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **16f-1** (290 mg, yield: 84.7%).

MS m/z (ESI): 584.5 [M+1]

### Step 6

### N-(3-(6-amino-5-(2-(methylamino)ethoxy)pyrimidin-4-yl)-5-fluoro-2-methylphenyl)-4-fluoro-2,2-dimethyl-2,3-dihydrobenzofuran-5-carboxamide 16g-1

Compound **16f-1** (290 mg, 0.50 mmol) was dissolved in dichloromethane (3 mL), and trifluoroacetic acid (1.24 g, 10.88 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound **16g-1** (240 mg, yield: 99.9%), which was directly used in the next reaction without purification.

MS m/z (ESI): 484.3 [M+1]

### Step 7

### N-(3-(6-amino-5-(2-(N-methacrylamido)ethoxy)pyrimidin-4-yl)-5-fluoro-2-methylphenyl)-4-fluoro-2,2-dimethyl-2,3-dihydrobenzofuran-5-carboxamide 16-1

The crude compound **16g-1** (240 mg, 0.50 mmol) was dissolved in dichloromethane (3 mL), and triethylamine (252 mg, 2.50 mmol, Sinopharm) was added. Acryloyl chloride (45 mg, 0.50 mmol, Sinopharm) was added dropwise at - 20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **16-1** (85 mg, yield: 31.9%).
MS m/z (ESI): 538.4 [M+1]
¹H NMR (500 MHz, DMSO-*d₆*): δ 9.62-9.41 (m, 1H), 8.21-8.18 (m, 1H), 7.71-7.55 (m, 2H), 7.20-6.95 (m, 3H), 6.75-6.57 (m, 2H), 6.10-6.02 (m, 1H), 5.60-5.58 (m, 1H), 3.59-3.45 (m, 4H), 3.14-3.11 (m, 2H), 2.53 (s, 3H), 2.03-1.96 (m, 3H), 1.49 (s, 6H).

### Example 16-2

### N-(3-(6-amino-5-(2-(N-methacrylamido)ethoxy)pyrimidin-4-yl)-5-fluoro-2-methylphenyl)-6-fluoro-2,2-dimethyl-2,3-dihydrobenzofuran-5-carboxamide 16-2

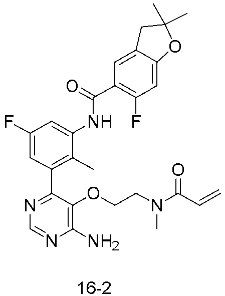

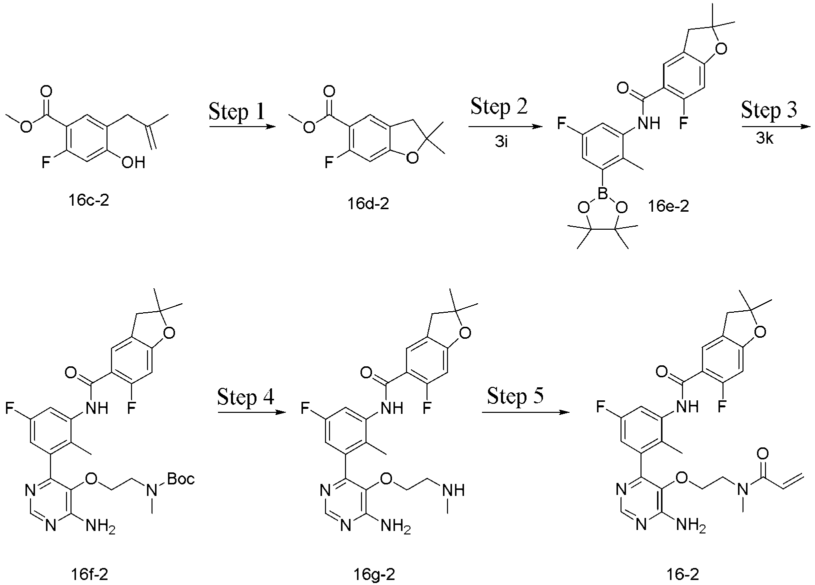

### Step 1

### Methyl 6-fluoro-2,2-dimethyl-2,3-dihydrobenzofuran-5-carboxylate 16d-2

Compound **16c-2** (400 mg, 1.78 mmol) was dissolved in formic acid (20 mL), and the mixture was reacted at 110°C for 0.5 hours. 50 mL of water was added, and the mixture was extracted with ethyl acetate (50 mL×3). The organic phases were combined, washed with 50 mL of saturated sodium bicarbonate solution, dried over anhydrous sodium sulfate and filtered, and the filtrate was concentrated under reduced pressure. The residue was purified by column chromatography with eluent system B to obtain the title compound **16d-2** (360 mg, yield: 90.0%).

MS m/z (ESI): 225.1 [M+1]

### Step 2

### 6-Fluoro-N-(5-fluoro-2-methyl-3-(4,4,5,5-tetramethyl-1,3,2-dioxaborolan-2-yl)phenyl)-2,2-dimethyl-2,3-dihydrobenzofuran-5-carboxamide 16e-2

Compound **16d-2** (120 mg, 0.54 mmol) was dissolved in tetrahydrofuran (2 mL), and compound **3i** (148 mg, 0.59 mmol) was added. The mixture was purged 3 times with nitrogen. Sodium bis(trimethylsilyl)amide (2 M, 0.40 mL, 0.80 mmol, Adamas) was added dropwise at 0°C, and the mixture was stirred at room temperature for 2 hours. 10 mL of saturated sodium bicarbonate solution was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system B to obtain the title compound **16e-2** (190 mg, yield: 80.1%).

MS m/z (ESI): 444.3 [M+1]

### Step 3

### Tert-butyl (2-((4-amino-6-(5-fluoro-3-(6-fluoro-2,2-dimethyl-2,3-dihydrobenzofuran-5-carboxamido)-2-methylphenyl)pyrimidin-5-yl)oxy)ethyl)(methyl)carbamate 16f-2

Compound **16e-2** (190 mg, 0.43 mmol) was dissolved in 1,4-dioxane (2 mL) and water (0.6 mL), and [1,1'-bis(diphenylphosphino)ferrocene]palladium dichloride (63 mg, 0.09 mmol, Bidepharm), compound **3k** (143 mg, 0.47 mmol) and potassium carbonate (119 mg, 0.86 mmol, Sinopharm) were added. The mixture was purged 3 times with nitrogen, and reacted at 90°C for 2 hours. 10 mL of water was added, and the mixture was extracted with ethyl acetate (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **16f-2** (230 mg, yield: 91.9%).

MS m/z (ESI): 584.3 [M+1]

### Step 4

### N-(3-(6-amino-5-(2-(methylamino)ethoxy)pyrimidin-4-yl)-5-fluoro-2-methylphenyl)-6-fluoro-2,2-dimethyl-2,3-dihydrobenzofuran-5-carboxamide 16g-2

Compound **16f-2** (230 mg, 0.39 mmol) was dissolved in dichloromethane (3 mL), and trifluoroacetic acid (1.24 g, 10.88 mmol, Adamas) was added. The mixture was reacted at room temperature for 1 hour. The reaction solution was concentrated under reduced pressure. 5 mL of saturated sodium bicarbonate solution was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the crude title compound **16g-2** (190 mg, yield: 99.7%), which was directly used in the next reaction without purification.

MS m/z (ESI): 484.4 [M+1]

### Step 5

*N*-(3-(6-amino-5-(2-(*N*-methacrylamido)ethoxy)pyrimidin-4-yl)-5-fluoro-2-methylphenyl)-6-fluoro-2,2-dimethyl-2,3-dihydrobenzofuran-5-carboxamide **16-2**

The crude compound **16g-2** (190 mg, 0.39 mmol) was dissolved in dichloromethane (3 mL), and triethylamine (200 mg, 1.98 mmol, Sinopharm) was added. Acryloyl chloride (35 mg, 0.39 mmol, Sinopharm) was added dropwise at - 20°C, and the mixture was reacted at -20°C for 1 hour. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **16-2** (72 mg, yield: 34.1%).
MS m/z (ESI): 538.4 [M+1]
¹H NMR (500 MHz, DMSO-*d₆*): δ 9.60-9.38 (m, 1H), 8.21-8.18 (m, 1H), 7.71-7.53 (m, 2H), 7.20-6.95 (m, 3H), 6.80-6.57 (m, 2H), 6.10-6.02 (m, 1H), 5.60-5.58 (m, 1H), 3.59-3.45 (m, 4H), 3.06-3.05 (m, 2H), 2.53 (s, 3H), 2.03-1.95 (m, 3H), 1.47 (s, 6H).

### Example 17

### N-(3-(6-amino-5-(2-(N-(methyl-d₃)acrylamido)ethoxy)pyrimidin-4-yl)-5-fluoro-2-methylphenyl)-5-fluoro-2H-spiro[benzofuran-3,1'-cyclopropane]-6-carboxamide 17

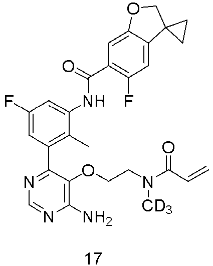

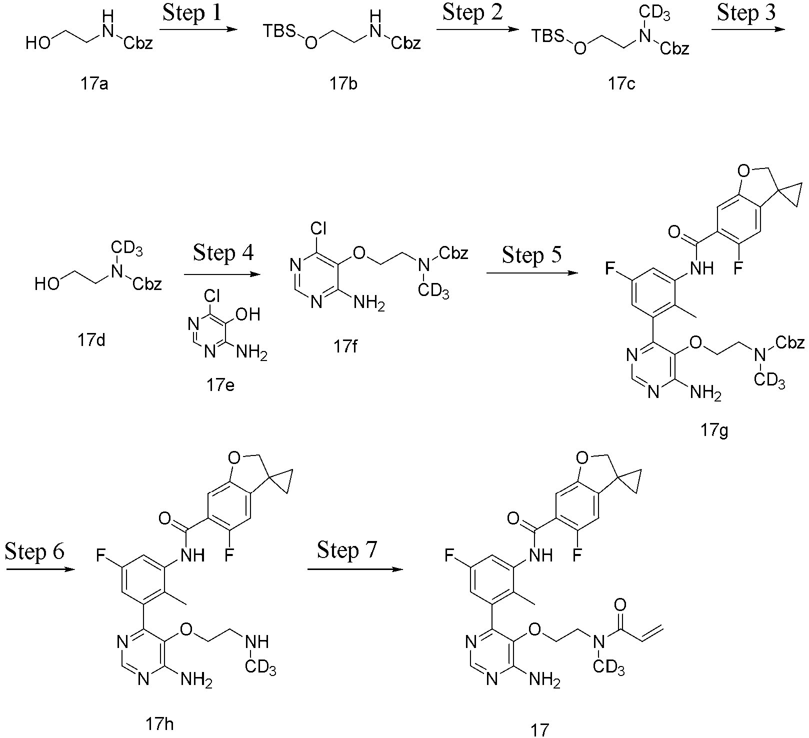

### Step 1

### Benzyl (2-((tert-butyldimethylsilyl)oxy)ethyl)carbamate 17b

Benzyl (2-hydroxyethyl)carbamate **17a** (1 g, 5.12 mmol, Bidepharm) was dissolved in dichloromethane (20 mL, Sinopharm), and imidazole (524 mg, 7.70 mmol, Sinopharm) was added. Tert-butyldimethylchlorosilane (927 mg, 6.15 mmol, Adamas) was added at 0°C. The mixture was reacted at room temperature for 12 hours. 40 mL of water was added, and the mixture was extracted with dichloromethane (40 mL×3). The organic phases were combined, washed with 40 mL of saturated sodium chloride solution, dried over anhydrous sodium sulfate and filtered, and the filtrate was concentrated under reduced pressure. The residue was purified by column chromatography with eluent system B to obtain the title compound **17b** (1.56 g, yield: 98.4%).

MS m/z (ESI): 310.4 [M+1]

### Step 2

### Benzyl (2-((tert-butyldimethylsilyl)oxy)ethyl)(methyl-d₃)carbamate 17c

Compound **17b** (1.56 g, 5.04 mmol) was dissolved in N,Ndimethylformamide (20 mL, Sinopharm). At 0°C, sodium hydride (304 mg, 7.60 mmol, 60% purity, Adamas) was added. After the addition, the mixture was reacted at 0°C for 0.5 hours, followed by the dropwise addition of deuterated iodomethane (881 mg, 6.08 mmol). After the addition, the mixture was reacted at room temperature for 1 hour. 30 mL of saturated ammonium chloride solution was added, and the mixture was extracted with ethyl acetate (30 mL×3). The organic phases were combined, washed with 30 mL of saturated sodium chloride solution, dried over anhydrous sodium sulfate, filtered and concentrated under reduced pressure. The residue was purified by column chromatography with eluent system B to obtain the title compound **17c** (1.36 g, yield: 82.6%).

MS m/z (ESI): 327.5 [M+1]

### Step 3

### Benzyl (2-hydroxyethyl)(methyl-d₃)carbamate 17d

Compound **17c** (1.36 g, 4.17 mmol) was dissolved in a solution of hydrogen chloride in 1,4-dioxane (4 M, 15 mL, Sinopharm). The mixture was reacted at room temperature for 1 hour, and concentrated under reduced pressure. The residue was purified by column chromatography with eluent system A to obtain the title compound **17d** (680 mg, yield: 76.9%).

MS m/z (ESI): 213.1 [M+1]

### Step 4

### Benzyl (2-((4-amino-6-chloropyrimidin-5-yl)oxy)ethyl)(methyl-d₃)carbamate 17f

Compound **17d** (680 mg, 3.20 mmol) was dissolved in tetrahydrofuran (5 mL, Energy Chemical), and compounds 4-amino-6-chloropyrimidin-5-ol **17e** (390 mg, 2.68 mmol, Bidepharm) and triphenylphosphine (1.76 g, 6.73 mmol, Adamas) were added. The mixture was purged 3 times with nitrogen. Diisopropyl azodicarboxylate (1.36 g, 6.73 mmol, Adamas) was added dropwise at 0°C, and the mixture was stirred at 60°C for 2 hours. 20 mL of water was added, and the mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system A to obtain the title compound **17f** (560 mg, yield: 61.5%).

MS m/z (ESI): 340.2 [M+1]

### Step 5

### Benzyl (2-((4-amino-6-(5-fluoro-3-(5-fluoro-2H-spiro[benzofuran-3,1'-cyclopropane]-6-carboxamido)-2-methylphenyl)pyrimidin-5-yl)oxy)ethyl)(methyl-d₃)carbamate 17g

Compound **17f** (200 mg, 0.59 mmol) was dissolved in 1,4-dioxane (2 mL, Sinopharm) and water (0.6 mL, Sinopharm), and [1,1'-bis(diphenylphosphino)ferrocene]palladium dichloride (34 mg, 0.05 mmol, Bidepharm), compound **3j** (100 mg, 0.23 mmol) and potassium carbonate (63 mg, 0.46 mmol, Sinopharm) were added. The mixture was purged 3 times with nitrogen, and reacted at 90°C for 2 hours. 5 mL of water was added, and the mixture was extracted with ethyl acetate (5 mL×3). The organic phases were combined, washed with 5 mL of saturated sodium chloride solution, dried over anhydrous sodium sulfate, filtered and concentrated under reduced pressure. The residue was purified by column chromatography with eluent system A to obtain the title compound **17g** (100 mg, yield: 71.3%).

MS m/z (ESI): 619.4 [M+1]

### Step 6

### N-(3-(6-amino-5-(2-((methyl-d₃)amino)ethoxy)pyrimidin-4-yl)-5-fluoro-2-methylphenyl)-5-fluoro-2H-spiro[benzofuran-3,1'-cyclopropane]-6-carboxamide 17h

Compound **17g** (100 mg, 0.19 mmol) was dissolved in methanol (3 mL, Sinopharm), and palladium on carbon (17.3 mg, 0.02 mmol, 10% purity, Bidepharm) was added. The mixture was purged 3 times with hydrogen, and reacted at room temperature for 2 hours. The reaction solution was filtered and concentrated under reduced pressure to obtain the crude title compound **17h** (78 mg, yield: 99.6%), which was directly used in the next reaction without purification.

MS m/z (ESI): 485.2 [M+1]

### Step 7

### N-(3-(6-amino-5-(2-(N-(methyl-d₃)acrylamido)ethoxy)pyrimidin-4-yl)-5-fluoro-2-methylphenyl)-5-fluoro-2H-spiro[benzofuran-3,1'-cyclopropane]-6-carboxamide 17

The crude compound **17h** (78 mg, 0.16 mmol) was dissolved in dichloromethane (2 mL, Sinopharm), and triethylamine (82 mg, 0.81 mmol, Sinopharm) was added. Acryloyl chloride (4.4 mg, 0.05 mmol, Adamas) was added dropwise at -20°C, and the mixture was reacted at -20°C for 0.5 hours. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound 17 (5 mg, yield: 5.8%).
MS m/z (ESI): 539.3 [M+1]
¹H NMR (500 MHz, DMSO-*d₆*): δ 9.77-9.55 (m, 1H), 8.21-8.18 (m, 1H), 7.57-7.51 (m, 1H), 7.20-6.91 (m, 4H), 6.67-6.57 (m, 2H), 6.09-6.03 (m, 1H), 5.60-5.58 (m, 1H), 4.56 (s, 2H), 3.58-3.44 (m, 4H), 2.03-1.98 (m, 3H), 1.24-1.17 (m, 4H).

### Example 18

### N-(3-(6-amino-5-(2-(N-(methyl-d₃)acrylamido)ethoxy)pyrimidin-4-yl)-5-fluoro-2-methylphenyl)-7-fluoro-2H-spiro[benzofuran-3,1'-cyclopropane]-6-carboxamide 18

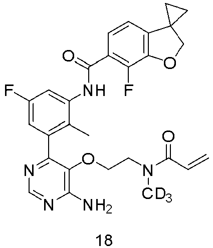

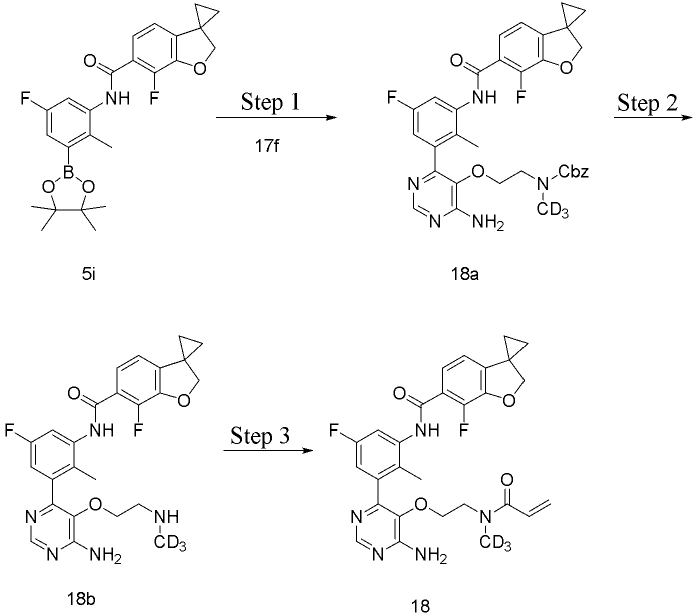

### Step 1

### Benzyl (2-((4-amino-6-(5-fluoro-3-(7-fluoro-2H-spiro[benzofuran-3,1'-cyclopropane]-6-carboxamido)-2-methylphenyl)pyrimidin-5-yl)oxy)ethyl)(methyl-d₃)carbamate 18a

The crude compound **5i** (110 mg, 0.25 mmol) was dissolved in 1,4-dioxane (2 mL) and water (0.6 mL), and [1,1'-bis(diphenylphosphino)ferrocene]palladium dichloride (36 mg, 0.05 mmol, Bidepharm), compound **17f** (350 mg, 1.03 mmol) and potassium carbonate (103 mg, 0.75 mmol, Sinopharm) were added. The mixture was purged 3 times with nitrogen, and reacted at 90°C for 2 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system A to obtain the title compound **18a** (154 mg, yield: 99%).

MS m/z (ESI): 619.3 [M+1]

### Step 2

### N-(3-(6-amino-5-(2-((methyl-d₃)amino)ethoxy)pyrimidin-4-yl)-5-fluoro-2-methylphenyl)-7-fluoro-2H-spiro[benzofuran-3,1'-cyclopropane]-6-carboxamide 18b

Compound **18a** (154 mg, 0.25 mmol) was dissolved in methanol (3 mL), and palladium on carbon (79 mg, 0.07 mmol, 10% purity, Bidepharm) was added. The mixture was purged 3 times with hydrogen, and reacted at room temperature for 2 hours. The reaction solution was filtered, and the filtrate was concentrated under reduced pressure to obtain the crude title compound **18b** (120 mg, yield: 99.6%), which was directly used in the next reaction without purification.

MS m/z (ESI): 485.3 [M+1]

### Step 3

### N-(3-(6-amino-5-(2-(N-(methyl-d₃)acrylamido)ethoxy)pyrimidin-4-yl)-5-fluoro-2-methylphenyl)-7-fluoro-2H-spiro[benzofuran-3,1'-cyclopropane]-6-carboxamide 18

The crude compound **18b** (120 mg, 0.25 mmol) was dissolved in dichloromethane (2 mL), and *N,N*-diisopropylethylamine (96 mg, 0.75 mmol, Adamas) was added. Acryloyl chloride (11.2 mg, 0.13 mmol, Sinopharm) was added dropwise at -20°C, and the mixture was reacted at -20°C for 10 minutes. 3 mL of water was added, and the mixture was extracted with dichloromethane (3 mL×3). The organic phases were combined, washed with 3 mL of saturated sodium chloride solution, dried over anhydrous sodium sulfate and filtered, and the filtrate was concentrated under reduced pressure. The residue was purified by column chromatography with eluent system A to obtain the title compound **18** (3.2 mg, yield: 2.4%).
MS m/z (ESI): 539.3 [M+1]
¹H NMR (500 MHz, DMSO-*d₆*): δ 9.85-9.64 (m, 1H), 8.21-8.18 (m, 1H), 7.57-7.47 (m, 1H), 7.28-6.96 (m, 4H), 6.82-6.77 (m, 1H), 6.66-6.58 (m, 1H), 6.09-6.01 (m, 1H), 5.62-5.54 (m, 1H), 4.68-4.64 (m, 2H), 3.62-3.42 (m, 4H), 2.04-1.91 (m, 3H), 1.23-1.13 (m, 4H).

### Example 19

### N-(3-(6-amino-5-(2-(N-(methyl-d3)acrylamido)ethoxy)pyrimidin-4-yl)-5-fluoro-2-methylphenyl)-6'-fluoro-2',3'-dihydrospiro[cyclopropane-1,1'-indene]-5'-carboxamide 19

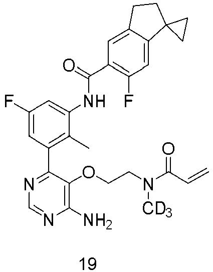

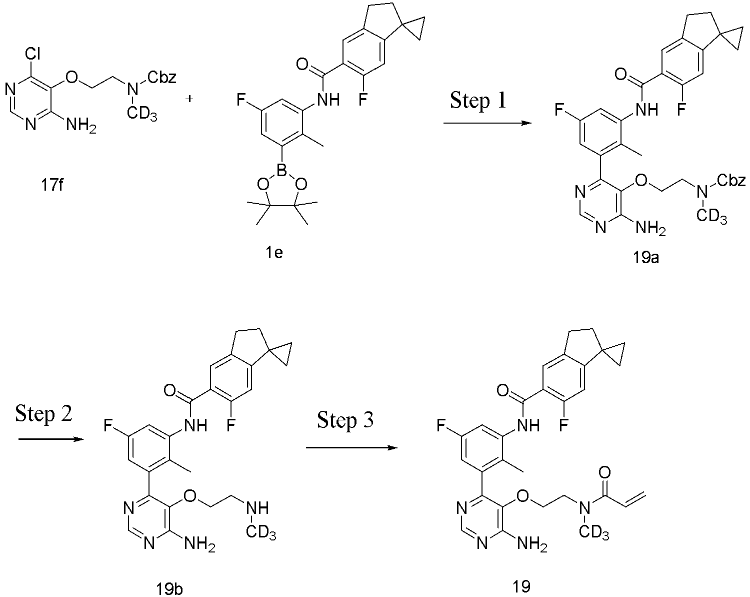

### Step 1

### Benzyl (2-((4-amino-6-(5-fluoro-3-(6'-fluoro-2',3'-dihydrospiro[cyclopropane-1,1'-indene]-5'-carboxamido)-2-methylphenyl)pyrimidin-5-yl)oxy)ethyl)(methyl-d3)carbamate 19a

The crude compound **1e** (170 mg, 0.39 mmol) was dissolved in 1,4-dioxane (5 mL) and water (1.5 mL), and [1,1'-bis(diphenylphosphino)ferrocene]palladium dichloride (57 mg, 0.08 mmol, Bidepharm), compound **17f** (158 mg, 0.46 mmol) and potassium carbonate (107 mg, 0.77 mmol, Sinopharm) were added. The mixture was purged 3 times with nitrogen, and reacted at 90°C for 2 hours. The reaction was allowed to return to room temperature. 20 mL of water was added, and the mixture was extracted with ethyl acetate (20 mL×3). The organic phases were combined, washed with 20 mL of saturated sodium chloride solution, dried over anhydrous sodium sulfate and filtered, and the filtrate was concentrated under reduced pressure. The residue was purified by column chromatography with eluent system A to obtain the title compound **19a** (238 mg, yield: 99.7%).

MS m/z (ESI): 617.5 [M+1].

### Step 2

### N-(3-(6-amino-5-(2-((methyl-d3)amino)ethoxy)pyrimidin-4-yl)-5-fluoro-2-methylphenyl)-6'-fluoro-2',3'-dihydrospiro[cyclopropane-1,1'-indene]-5'-carboxamide 19b

The crude compound **19a** (238 mg, 0.39 mmol) was dissolved in methanol (5 mL, Sinopharm), and palladium on carbon (42 mg, 0.04 mmol, 10% purity, Bidepharm) was added. The mixture was purged 3 times with hydrogen, and reacted at room temperature for 3 hours. The reaction solution was filtered and concentrated under reduced pressure to obtain the crude title compound **19b** (186 mg, yield: 99.9%), which was directly used in the next reaction without purification.

MS m/z (ESI): 483.4 [M+1].

### Step 3

### N-(3-(6-amino-5-(2-(N-(methyl-d3)acrylamido)ethoxy)pyrimidin-4-yl)-5-fluoro-2-methylphenyl)-6'-fluoro-2',3'-dihydrospiro[cyclopropane-1,1'-indene]-5'-carboxamide 19

The crude compound **19b** (186 mg, 0.39 mmol) was dissolved in dichloromethane (5 mL, Sinopharm), and triethylamine (195 mg, 1.93 mmol, Adamas) was added. Acryloyl chloride (35 mg, 0.39 mmol, Sinopharm) was added dropwise at -20°C, and the mixture was reacted at -20°C for 1 hour. The reaction was allowed to return to room temperature. 10 mL of water was added, and the mixture was extracted with dichloromethane (10 mL×3). The organic phases were combined, washed with 10 mL of saturated sodium chloride solution, dried over anhydrous sodium sulfate and filtered, and the filtrate was concentrated under reduced pressure. The residue was purified by column chromatography with eluent system A to obtain the title compound **19** (25 mg, yield: 12.1%).

MS m/z (ESI): 537.5 [M+1].

¹H NMR (500 MHz, DMSO-*d₆*): δ 9.76-9.54 (m, 1H), 8.21-8.18 (m, 1H), 7.64-7.55 (m, 2H), 7.10-6.96 (m, 3H), 6.80-6.57 (m, 2H), 6.10-6.02 (m, 1H), 5.61-5.57 (m, 1H), 3.59-3.45 (m, 3H), 3.04-3.02 (m, 2H), 2.17-2.14 (m, 2H), 2.03-1.96 (m, 3H), 1.24-1.22 (m, 2H), 1.03-1.02 (m, 3H).

### Example 20

### N-(3-(6-amino-5-(2-(N-methacrylamido)ethoxy)pyrimidin-4-yl)-5-fluoro-2-methylphenyl)-7-fluoro-3H-spiro[benzofuran-2,1'-cyclopropane]-6-carboxamide 20

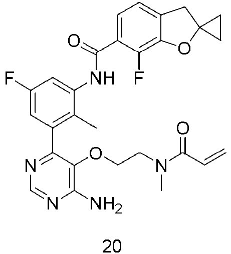

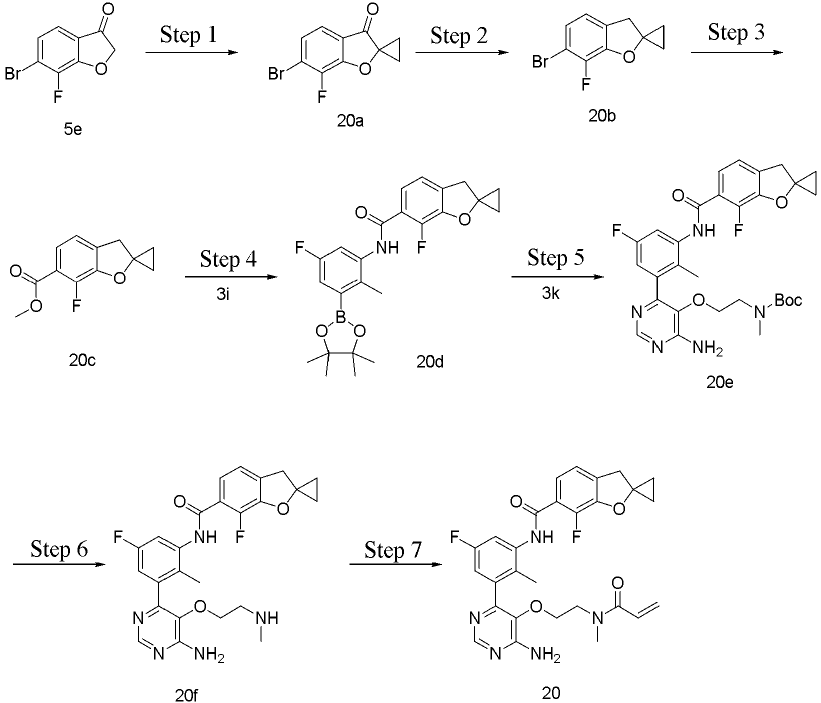

### Step 1

6-Bromo-7-fluoro-3*H*-spiro[benzofuran-2,1'-cyclopropane]-3-one **20a** Compound **5e** (2.0 g, 8.65 mmol) and 1,2-dibromoethane (2.44 g, 12.99 mmol, Adamas) were dissolved in *N,N*-dimethylformamide (40 mL, Sinopharm). At room temperature, sodium hydride (865 mg, 21.64 mmol, Sinopharm) was added. The mixture was reacted at room temperature for 1 hour. The reaction was quenched with 50 mL of ice water, and extracted with dichloromethane (50 mL×3). The organic phases were combined, washed with 50 mL of saturated sodium chloride solution, dried over anhydrous sodium sulfate and filtered, and the filtrate was concentrated under reduced pressure. The residue was purified by column chromatography with eluent system B to obtain the title compound **20a** (212 mg, yield: 9.5%).

### Step 2

### 6-Bromo-7-fluoro-3H-spiro[benzofuran-2,1'-cyclopropane] 20b

Aluminium trichloride (428 mg, 3.21 mmol, Sinopharm) and lithium aluminium hydride (1 M, 2.92 mL, 2.92 mmol, Adamas) were dissolved in diethyl ether (40 mL, Sinopharm). At 0°C, compound **20a** (750 mg, 2.92 mmol) was added. The mixture was reacted at room temperature for 2 hours. The reaction was quenched with 0.5 N aqueous sodium hydroxide solution (10 mL) and diluted in 50 mL of water. The mixture was extracted with dichloromethane (50 mL×3). The organic phases were combined, washed with 50 mL of saturated sodium chloride solution, dried over anhydrous sodium sulfate, filtered and concentrated under reduced pressure. The residue was purified by column chromatography with eluent system B to obtain the title compound **20b** (190 mg, yield: 26.8%).

### Step 3

### Methyl 7-fluoro-3H-spiro[benzofuran-2,1'-cyclopropane]-6-carboxylate 20c

Compound **20b** (190 mg, 0.78 mmol) was dissolved in*N,N*-dimethylacetamide (3 mL) and methanol (3 mL), and palladium acetate (35 mg, 0.15 mmol, Bidepharm), 1,3-bis(diphenylphosphino)propane (65 mg, 0.15 mmol, Adamas) and *N,N-*diisopropylethylamine (202 mg, 1.56 mmol, Adamas) were added. The mixture was purged 3 times with carbon monoxide, and reacted at 80°C for 16 hours. The reaction solution was cooled to room temperature, and concentrated under reduced pressure. The residue was purified by column chromatography with eluent system B to obtain the title compound **20c** (72 mg, yield: 41.4%).

### Step 4

### 7-Fluoro-N-(5-fluoro-2-methyl-3-(4,4,5,5-tetramethyl-1,3,2-dioxaborolan-2-yl)phenyl)-3H-spiro[benzofuran-2,1'-cyclopropane]-6-carboxamide 20d

Compound **20c** (95 mg, 0.43 mmol) was dissolved in tetrahydrofuran (5 mL), and compound **3i** (118 mg, 0.47 mmol) was added. The mixture was purged 3 times with nitrogen. Sodium bis(trimethylsilyl)amide (2 M, 0.32 mL, 0.64 mmol, Adamas) was added dropwise at 0°C, and the mixture was stirred at room temperature for 2 hours. 15 mL of saturated sodium bicarbonate solution was added, and the mixture was extracted with ethyl acetate (20 mL×3). The organic phases were combined, washed with 20 mL of saturated sodium chloride solution, dried over anhydrous sodium sulfate and filtered, and the filtrate was concentrated under reduced pressure. The residue was purified by column chromatography with eluent system B to obtain the title compound **20d** (70 mg, yield: 37.1%).

MS m/z (ESI): 442.2 [M+1].

### Step 5

### Tert-butyl (2-((4-amino-6-(5-fluoro-3-(7-fluoro-3H-spiro[benzofuran-2,1'-cyclopropane]-6-carboxamido)-2-methylphenyl)pyrimidin-5-yl)oxy)ethyl)(methyl)carbamate 20e

Compound **20d** (70 mg, 0.16 mmol) was dissolved in 1,4-dioxane (2 mL, Sinopharm) and water (0.5 mL, Sinopharm), and tetrakis(triphenylphosphine)palladium (19 mg, 16 µmol, Bidepharm), compound **3k** (58 mg, 0.19 mmol) and potassium carbonate (45 mg, 0.32 mmol, Sinopharm) were added. The mixture was purged 3 times with nitrogen, and reacted at 110°C under microwave conditions for 30 minutes. The reaction solution was cooled to room temperature and concentrated under reduced pressure. The residue was purified by silica gel column chromatograph with eluent system B to obtain the title compound **20e** (87 mg, yield: 94.3%).

MS m/z (ESI): 582.4 [M+1].

### Step 6

### N-(3-(6-amino-5-(2-(methylamino)ethoxy)pyrimidin-4-yl)-5-fluoro-2-methylphenyl)-7-fluoro-3H-spiro[benzofuran-2,1'-cyclopropane]-6-carboxamide 20f

Compound **20e** (87 mg, 0.15 mmol) was dissolved in hydrogen chloride in dioxane (4 M, 3 mL, Adamas). At room temperature, the mixture was stirred for 30 minutes. The reaction solution was concentrated under reduced pressure. 3 mL of saturated sodium bicarbonate solution was added, and the mixture was extracted with ethyl acetate (3 mL×3). The organic phases were combined, washed with 3 mL of saturated sodium chloride solution, dried over anhydrous sodium sulfate, filtered and concentrated under reduced pressure to obtain the title compound **20f,** which was directly used in the next reaction without further purification.

MS m/z (ESI): 482.5 [M+1].

### Step 7

### N-(3-(6-amino-5-(2-(N-methacrylamido)ethoxy)pyrimidin-4-yl)-5-fluoro-2-methylphenyl)-7-fluoro-3H-spiro[benzofuran-2,1'-cyclopropane]-6-carboxamide 20

Compound **20f** (72 mg, 0.15 mmol) and *N,N*-diisopropylethylamine (97 mg, 0.75 mmol, Adamas) were dissolved in dichloromethane (5 mL). The reaction solution was cooled to -40°C. Acryloyl chloride (13 mg, 0.14 mmol, Adamas) was added, and the mixture was stirred at -40°C for 10 minutes. The reaction solution was concentrated under reduced pressure, and the residue was purified by silica gel column chromatography with eluent system A to obtain the title compound **20** (14 mg, yield: 17.5%).

MS m/z (ESI): 536.1 [M+1].

¹H NMR (500 MHz, DMSO-*d*₆) δ 9.91-9.70 (m, 1H), 8.21-8.18 (m, 1H), 7.52 (dd, 1H), 7.35-6.95 (m, 5H), 6.61 (ddd, 1H), 6.05 (ddd, 1H), 5.59 (td, 1H), 3.66-3.42 (m, 6H), 2.80-2.51 (m, 3H), 2.01-1.97 (m, 3H), 1.21-1.16 (m, 2H), 0.88-0.85 (m, 2H).

### Example 21

### N-(3-(6-amino-5-(2-(N-methacrylamido)ethoxy)pyrimidin-4-yl)-5-fluoro-2-methylphenyl)-6'-fluoro-3'H-spiro[cyclopropane-1,1'-isobenzofuran]-5'-carboxamide 21

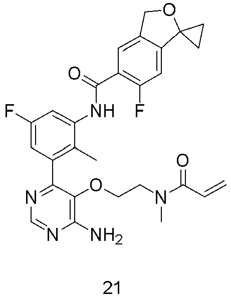

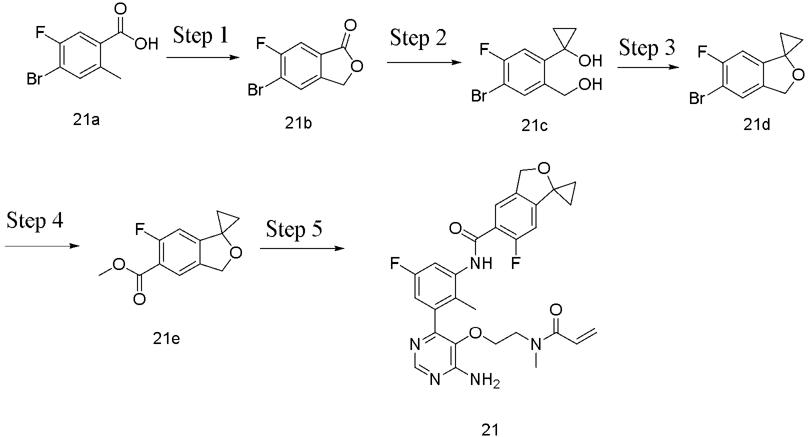

### Step 1

### 5-Bromo-6-fluoroisobenzofuran-1(3H)-one 21b

4-Bromo-5-fluoro-2-methylbenzoic acid **21a** (1 g, 4.29 mmol) was dissolved in acetonitrile (20 mL, Sinopharm), and sodium persulfate (3.07 g, 12.89 mmol) was added. Then, tetrabutylammonium bromide (2.77 g, 8.59 mmol) was added, and the mixture was stirred at 80°C for 2 hours. The reaction was allowed to return to room temperature. 20 mL of water was added, and the mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system B to obtain the title compound **21b** (260 mg, yield: 26.2%).

### Step 2

### 1-(4-Bromo-5-fluoro-2-(hydroxymethyl)phenyl)cyclopropan-1-ol 21c

Compound **21b** (260 mg, 1.13 mmol) was dissolved in tetrahydrofuran (5 mL, Adamas). The mixture was purged 3 times with nitrogen. Tetraisopropyl titanate (352 mg, 1.24 mmol) was added dropwise at 0°C, and the mixture was reacted at 0°C for 0.5 hours. Ethylmagnesium bromide (2 M, 1.35 mL, 1.24 mmol, Sinopharm) was added dropwise. At 0°C, the mixture was reacted for 1 hour. The reaction was allowed to return to room temperature. 10 mL of water was added, and the mixture was extracted with ethyl acetate (10 mL×3). The organic phases were combined, washed with 10 mL of saturated sodium chloride solution, dried over anhydrous sodium sulfate, filtered and concentrated under reduced pressure. The residue was purified by column chromatography with eluent system B to obtain the title compound **21c** (170 mg, yield: 57.9%).

MS m/z (ESI): 261.0 [M-1].

### Step 3

### 5'-Bromo-6'-fluoro-3'H spiro[cyclopropane-1,1'-isobenzofuran] 21d

Triphenylphosphine (513 mg, 1.96 mmol, Adamas) was dissolved in tetrahydrofuran (4 mL, Energy Chemical). Under a nitrogen atmosphere and at 0°C, diisopropyl azodicarboxylate (395 mg, 1.96 mmol, Adamas) was added dropwise. The mixture was reacted at 0°C for 1 hour, and a solution of compound **21c** (170 mg, 3.32 mmol) in tetrahydrofuran (4 mL, Energy Chemical) was added dropwise. The reaction was allowed to return to room temperature, reacted for 12 hours, and concentrated under reduced pressure. The residue was purified by column chromatography with eluent system B to obtain the title compound **21d** (65 mg, yield: 41.1%).

### Step 4

### Methyl 6'-fluoro-3'H-spiro[cyclopropane-1,1'-isobenzofuran]-5'-carboxylate 21e

Compound **21d** (140 mg, 0.57 mmol) was dissolved in*N,N*-dimethylacetamide (1.5 mL, Sinopharm) and methanol (1.5 mL, Sinopharm). Palladium acetate (12 mg, 0.05 mmol, Adamas), *N,N*-diisopropylethylamine (70 mg, 0.54 mmol, Adamas), and 1,3-bis(diphenylphosphino)propane (22 mg, 0.05 mmol, Adamas) were added. The mixture was purged 3 times with carbon monoxide, and reacted overnight at 80°C. The reaction was allowed to return to room temperature and concentrated under reduced pressure. The residue was purified by column chromatography with eluent system B to obtain the title compound **21e** (42 mg, yield: 70.7%),

MS m/z (ESI): 223.1 [M+1].

### Step 5

### N-(3-(6-amino-5-(2-(N-methacrylamido)ethoxy)pyrimidin-4-yl)-5-fluoro-2-methylphenyl)-6'-fluoro-3'H-spiro[cyclopropane-1,1'-isobenzofuran]-5'-carboxamide 21

Using step 4 to step 7 of the synthetic route in Example **1,** the title compound **21** (5 mg, yield: 6.9%) was obtained by replacing the starting material compound **1d** with compound **21e.**

MS m/z (ESI): 536.4 [M+1].

¹H NMR (500 MHz, DMSO-*d₆*): δ 9.91-9.70 (m, 1H), 8.21-8.18 (m, 1H), 7.75-7.50 (m, 2H), 7.08-6.98 (m, 3H), 6.66-6.58 (m, 1H), 6.09-6.03 (m, 1H), 5.62-5.58 (m, 1H), 5.13 (s, 1H), 3.59-3.45 (m, 4H), 2.81 (s, 1H), 2.03-1.96 (m, 3H), 1.29-1.24 (m, 5H), 1.03-1.02 (m, 2H), 0.96-0.85 (m, 1H).

### Example 22

### N-(3-(6-amino-5-(2-(N-methacrylamido)ethoxy)pyrimidin-4-yl)-5-fluoro-2-methylphenyl)-4-fluoro-2,2-dimethylbenzo[d][1,3]dioxole-5-carboxamide 22

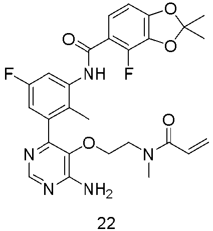

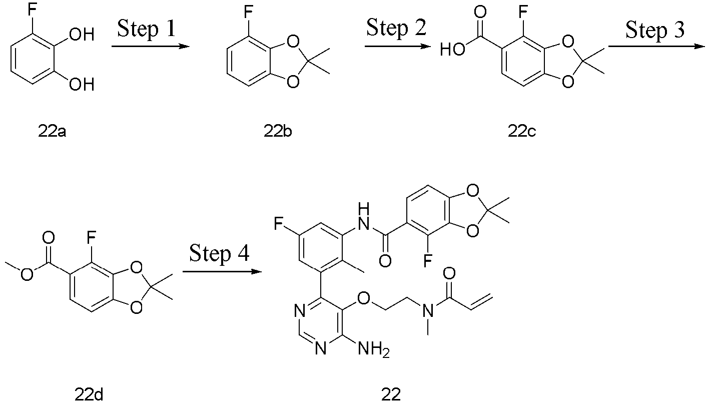

### Step 1

### 4-Fluoro-2,2-dimethylbenzo[d][1,3]dioxole 22b

3-Fluorobenzene-1,2-diol **22a** (8 g, 62.45 mmol, Leyan) and 2,2-dimethoxypropane (26 g, 249.65 mmol) were dissolved in toluene (120 mL, Sinopharm). p-Toluenesulfonic acid (1.1 g, 6.21 mmol, Sinopharm) was added, and the mixture was refluxed with water removal at 115°C overnight. The reaction solution was cooled to room temperature. 100 mL of water was added, and the mixture was extracted with ethyl acetate (50 mL×3). The organic phases were combined, dried over anhydrous sodium sulfate and filtered, and the filtrate was concentrated under reduced pressure. The residue was purified by column chromatography with eluent system B to obtain the title compound **22b** (900 mg, yield: 8.5%).

MS m/z (ESI): 169.1 [M+1].

### Step 2

### 4-Fluoro-2,2-dimethylbenzo[d][1,3]dioxole-5-carboxylic acid 22c

Compound **22b** (800 mg, 4.75 mmol) was dissolved in tetrahydrofuran (8 mL, Energy Chemical). The mixture was purged 3 times with nitrogen. At -78°C, lithium diisopropylamide (4.7 mL, 2 M in tetrahydrofuran, Adamas) was added, and the mixture was reacted for 1 hour. Dry ice pellets were added, and the mixture was further stirred for half an hour. The reaction solution was quenched with 3 mL of water. The mixture was warmed naturally to room temperature, and extracted with ethyl acetate (5 mL×2). The aqueous phase was adjusted to pH 1-2 with 2 M aqueous hydrogen chloride solution. The mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure to obtain the title compound **22c** (180 mg, yield: 17.8%).

MS m/z (ESI): 213.1 [M+1].

### Step 3

Methyl 4-fluoro-2,2-dimethylbenzo[*d*][1,3]dioxole-5-carboxylate **22d**

Compound **22c** (180 mg, 0.85 mmol) was dissolved in tetrahydrofuran (2 mL, Energy Chemical) and methanol (0.4 mL, Energy Chemical), and trimethylsilyldiazomethane (200 mg, 1.75 mmol, Adamas) was added. The reaction solution was reacted at room temperature for 2 hours. 5 mL of water was added, and the mixture was extracted with ethyl acetate (5 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system B to obtain the title compound **22d** (80 mg, yield: 41.7%).

MS m/z (ESI): 227.1 [M+1].

### Step 4

### N-(3-(6-amino-5-(2-(N-methacrylamido)ethoxy)pyrimidin-4-yl)-5-fluoro-2-methylphenyl)-4-fluoro-2,2-dimethylbenzo[d][1,3]dioxole-5-carboxamide 22

Using step 4 to step 7 of the synthetic route in Example **1,** the title compound **22** (1.5 mg, yield: 5.4%) was obtained by replacing the starting material compound **1d** with compound **22d.**

MS m/z (ESI): 540.2 [M+1].

¹H NMR (500 MHz, DMSO-*d₆*): δ 9.75-9.56 (m, 1H), 8.21-8.18 (m, 1H), 7.55-7.49 (m, 1H), 7.38-7.20 (m, 1H), 7.09-6.96 (m, 3H), 6.90-6.87 (m, 1H), 6.67-6.60 (m, 1H), 6.09-6.03 (m, 1H), 5.62-5.58 (m, 1H), 3.65-3.45 (m, 4H), 2.80 (s, 3H), 2.03-1.96 (m, 3H), 1.75 (s, 6H).

### Example 23

### N-(3-(6-amino-5-(2-(N-methacrylamido)ethoxy)pyrimidin-4-yl)-5-fluoro-2-methylphenyl)-8-fluorospiro[chromane-4,1'-cyclopropane]-7-carboxamide 23

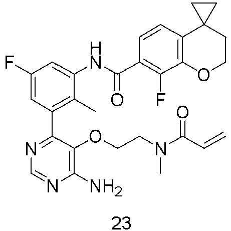

Using the synthetic route in Example **2,** the title compound **23** (1.5 mg, yield: 5.4%) was obtained by replacing the starting material compound **2a** with 7-bromo-8-fluorochromen-4-one.

MS m/z (ESI): 550.2 [M+1].

¹H NMR (500 MHz, DMSO-*d₆*): δ 9.81-9.60 (m, 1H), 8.21-8.18 (m, 1H), 7.55-7.51 (m, 1H), 7.23-7.05 (m, 3H), 7.00-6.97 (m, 1H), 6.73-6.70 (m, 1H), 6.60-6.57 (m, 1H), 6.09-6.02 (m, 1H), 5.61-5.57 (m, 1H), 4.36-4.33 (m, 2H), 3.57-3.45 (m, 4H), 2.80 (s, 3H), 2.03-1.97 (m, 3H), 1.91-1.89 (m, 2H), 1.14-1.11 (m, 2H), 1.03-0.95 (m, 2H).

### Example 24

### N-(3-(6-amino-5-(2-(N-methacrylamido)ethoxy)pyrimidin-4-yl)-5-fluoro-2-methylphenyl)-6-fluorospiro[chromane-4,1'-cyclopropane]-7-carboxamide 24

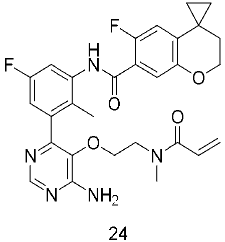

Using the synthetic route in Example **2,** the title compound **24** (110 mg, yield: 30.6%) was obtained by replacing the starting material compound **2a** with 7-bromo-6-fluorochromen-4-one.

MS m/z (ESI): 550.2 [M+1].

¹H NMR (500 MHz, DMSO-*d₆*): δ 9.75-9.53 (m, 1H), 8.21-8.18 (m, 1H), 7.55-7.51 (m, 1H), 7.19-6.96 (m, 4H), 6.81-6.77 (m, 1H), 6.67-6.60 (m, 1H), 6.10-6.00 (m, 1H), 5.60-5.57 (m, 1H), 4.26-4.22 (m, 2H), 3.57-3.45 (m, 4H), 2.80 (s, 3H), 2.00-1.94 (m, 3H), 1.87-1.84 (m, 2H), 1.16-1.11 (m, 2H), 0.99-0.85 (m, 2H).

### Example 25

### N-(3-(6-amino-5-(2-(N-methacrylamido)ethoxy)pyrimidin-4-yl)-5-fluoro-2-methylphenyl)-3-fluoro-4,5-dihydrospiro[cyclopenta[b]thiophene-6,1'-cyclopropane]-2-carboxamide 25

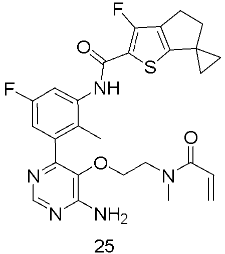

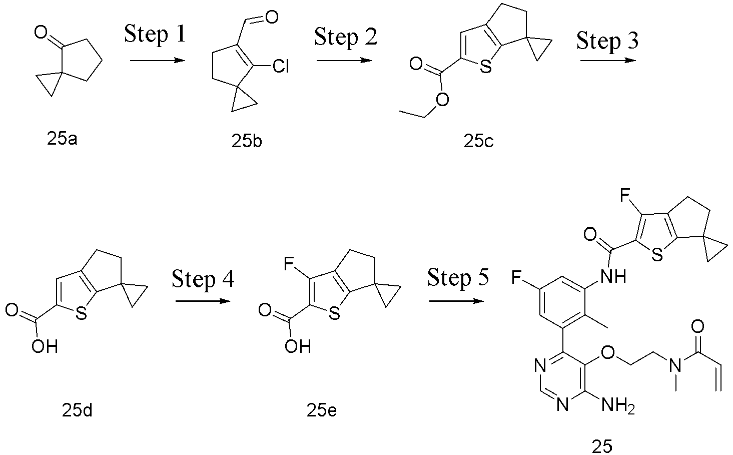

### Step 1

### 4-Chlorospiro[2.4]hept-4-ene-5-carbaldehyde 25b

Spiro[2.4]heptan-4-one **25a** (3.7 g, 33.59 mmol, Shaoyuan Technology) was dissolved in dichloromethane (80 mL, Sinopharm). *N,N*-dimethylformamide (6.2 g, 84.82 mmol, Bidepharm) and phosphorus oxychloride (10.9 g, 71.09 mmol, Bidepharm) were added, and the mixture was reacted at 40°C for 12 hours. The reaction was allowed to return to room temperature. 30 mL of saturated aqueous potassium phosphate solution was added. The mixture was extracted with dichloromethane (50 mL×3). The organic phases were combined, dried over anhydrous sodium sulfate, filtered and concentrated under reduced pressure to obtain the crude title compound **25b** (5.2 g, yield: 98.9%).

MS m/z (ESI): 156.9 [M+1].

### Step 2

### Ethyl 4,5-dihydrospiro[cyclopenta[b]thiophene-6,1'-cyclopropane]-2-carboxylate 25c

Compound **25b** (5.2 g, 33.20 mmol) was dissolved in dichloromethane (100 mL, Sinopharm). Ethyl mercaptoacetate (4.4 g, 36.62 mmol, Bidepharm) and triethylamine (10.1 g, 99.81 mmol, Bidepharm) were added. The mixture was reacted at 40°C for 0.5 hours. The reaction was allowed to return to room temperature and concentrated under reduced pressure. Ethanol (100 mL, Sinopharm) was added, and then triethylamine (5.1 g, 49.91 mmol, Bidepharm) was added. The mixture was reacted at 85°C for 2 hours. The reaction was allowed to return to room temperature and concentrated under reduced pressure. The residue was purified by column chromatography with eluent system B to obtain the title compound **25c** (2.4 g, yield: 32.9%),

MS m/z (ESI): 223.1 [M+1].

### Step 3

### 4,5-Dihydrospiro[cyclopenta[b]thiophene-6,1'-cyclopropane]-2-carboxylic acid 25d

Compound **25c** (2.4 g, 10.93 mmol) was dissolved in tetrahydrofuran (20 mL, Sinopharm), methanol (20 mL, Sinopharm) and water (20 mL, Sinopharm). Lithium hydroxide (1.4 g, 33.36 mmol, Adamas) was added, and the mixture was reacted at 60°C for 1 hour. The reaction was allowed to return to room temperature. The reaction solution was adjusted to pH 1-2 with 2 M aqueous hydrogen chloride solution, and extracted with ethyl acetate (30 mL×3). The organic phases were combined, washed with 30 mL of saturated sodium chloride solution, dried over anhydrous sodium sulfate, filtered and concentrated under reduced pressure. The residue was purified by column chromatography with eluent system A to obtain the title compound **25d** (2.1 g, yield: 98.9%).

MS m/z (ESI): 195.0 [M+1].

### Step 4

### 3-Fluoro-4,5-dihydrospiro[cyclopenta[b]thiophene-6,1'-cyclopropane]-2-carboxylic acid 25e

Compound **25d** (2.1 g, 10.81 mmol) was dissolved in tetrahydrofuran (40 mL, Sinopharm). At -78°C, n-Butyllithium (1.8 g, 28.10 mmol, Adamas) was added. The mixture was reacted at -40°C for 2 hours. At -40°C, N-fluorobenzenesulfonimide (8.6 g, 27.27 mmol, Adamas) was added. The reaction was allowed to return to room temperature and stirred for 12 hours. The reaction solution was adjusted to pH 5-6 with 2 M aqueous hydrogen chloride solution, and extracted with dichloromethane (30 mL×3). The organic phases were combined, washed with 30 mL of saturated sodium chloride solution, dried over anhydrous sodium sulfate, filtered and concentrated under reduced pressure. The residue was purified by column chromatography with eluent system A to obtain the title compound **25e** (1.2 g, yield: 52.3%).

MS m/z (ESI): 213.1 [M+1].

### Step 5

### N-(3-(6-amino-5-(2-(N-methacrylamido)ethoxy)pyrimidin-4-yl)-5-fluoro-2-methylphenyl)-3-fluoro-4,5-dihydrospiro[cyclopenta[b]thiophene-6,1'-cyclopropane]-2-carboxamide 25

Using step 5 to step 8 of the synthetic route in Example **6,** the title compound **25** (20 mg, yield: 36.7%) was obtained by replacing the starting material compound **6e** with compound **25e.**

MS m/z (ESI): 538.1 [M-1].

¹H NMR (500 MHz, CD₃OD): δ 8.23-8.20 (m, 1H), 7.76-7.73 (m, 1H), 7.01-6.98 (m, 1H), 6.77-6.59 (m, 1H), 6.28-6.17 (m, 1H), 5.71-5.65 (m, 1H), 3.73-3.61 (m, 4H), 2.99-2.95 (m, 2H), 2.89-2.60 (m, 3H), 2.54-2.51 (m, 2H), 2.10-2.04 (m, 3H), 1.11-1.04 (m, 4H).

### Example 26-1 and Example 26-2

### N-(3-(6-amino-5-(2-(N-methacrylamido)ethoxy)pyrimidin-4-yl)-5-fluoro-2-methylphenyl)-3-fluoro-4,6-dihydrospiro[cyclopenta[b]thiophene-5,1'-cyclopropane]-2-carboxamide 26-1

### N-(3-(6-amino-5-(2-(N-methacrylamido)ethoxy)pyrimidin-4-yl)-5-fluoro-2-methylphenyl)-3-fluoro-5,6-dihydrospiro[cyclopenta[b]thiophene-4,1'-cyclopropane]-2-carboxamide 26-2

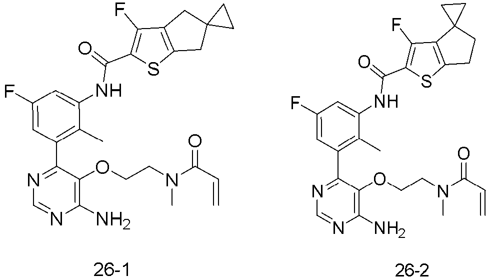

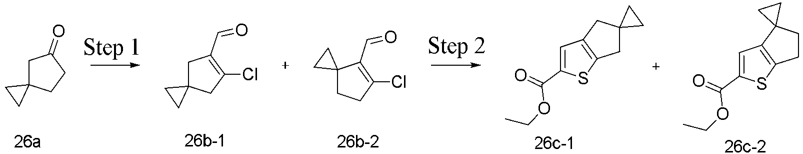

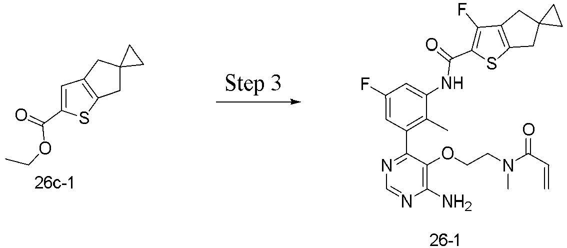

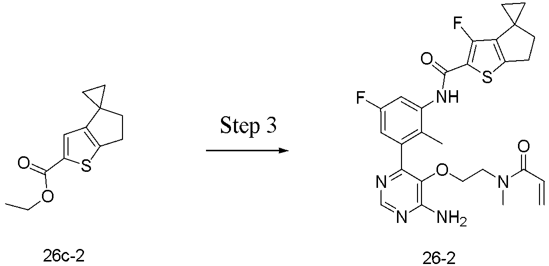

### Step 1

### 6-Chlorospiro[2.4]hept-5-ene-5-carbaldehyde 26b-1

### 5-Chlorospiro[2.4]hept-4-ene-4-carbaldehyde 26b-2

Spiro[2.4]heptan-5-one **26a** (1 g, 9.08 mmol, Shaoyuan Technology) was dissolved in dichloromethane (20 mL, Sinopharm). *N,N*-dimethylformamide (996 mg, 13.62 mmol, Bidepharm) and phosphorus oxychloride (2.93 g, 19.11 mmol, Bidepharm) were added, and the mixture was reacted at 40°C for 12 hours. The reaction was allowed to return to room temperature. 10 mL of saturated aqueous potassium phosphate solution was added. The mixture was extracted with dichloromethane (30 mL×3). The organic phases were combined, dried over anhydrous sodium sulfate, filtered and concentrated under reduced pressure to obtain a mixture of crude title compounds **26b-1** and **26b-2** (888 mg, yield: 62.5%).

### Step 2

### Ethyl 4,6-dihydrospiro[cyclopenta[b]thiophene-5,1'-cyclopropane]-2-carboxylate 26c-1

### Ethyl 5,6-dihydrospiro[cyclopenta[b]thiophene-4,1'-cyclopropane]-2-carboxylate 26c-2

The mixture of crude compounds **26b-1** and **26b-2** (888 mg, 5.62 mmol) was dissolved in dichloromethane (8 mL, Sinopharm). Ethyl mercaptoacetate (743 mg, 6.18 mmol, Bidepharm) and triethylamine (1.7 g, 16.90 mmol, Bidepharm) were added, and the mixture was reacted at 40°C for 0.5 hours. The reaction was allowed to return to room temperature and concentrated under reduced pressure. Ethanol (10 mL, Sinopharm) was added, and then triethylamine (0.8 g, 7.95 mmol, Bidepharm) was added. The mixture was reacted at 85°C for 2 hours. The reaction was allowed to return to room temperature and concentrated under reduced pressure. The residue was purified by column chromatography with eluent system B to obtain the title compounds **26c-1** (550 mg, yield: 44.1%) and **26c-2** (500 mg, yield: 40.1%).

### Compound 26c-1:

MS m/z (ESI): 223.0 [M+1].

¹H NMR (500 MHz, CD₃OD): δ 7.51 (s, 1H), 4.34-4.30 (m, 2H), 2.94 (s, 2H), 2.77 (s, 2H), 1.38-1.35 (m, 3H), 0.73-0.72 (m, 4H).

### Compound 26c-2:

MS m/z (ESI): 223.0 [M+1].

¹H NMR (500 MHz, CD₃OD): δ 7.17 (s, 1H), 4.32-4.28 (m, 2H), 3.08-3.05 (m, 2H), 2.50-2.47 (m, 2H), 1.36-1.34 (m, 3H), 0.91-0.88 (m, 4H).

### Step 3

### N-(3-(6-amino-5-(2-(N-methacrylamido)ethoxy)pyrimidin-4-yl)-5-fluoro-2-methylphenyl)-3-fluoro-4,6-dihydrospiro[cyclopenta[b]thiophene-5,1'-cyclopropane]-2-carboxamide 26-1

### N-(3-(6-amino-5-(2-(N-methacrylamido)ethoxy)pyrimidin-4-yl)-5-fluoro-2-methylphenyl)-3-fluoro-5,6-dihydrospiro[cyclopenta[b]thiophene-4,1'-cyclopropane]-2-carboxamide 26-2

Using step 3 to step 8 of the synthetic route in Example **6,** the title compound **26-1** (6 mg, yield: 2.5%) was obtained by replacing the starting material compound **6c** with compound **26c-1.**

MS m/z (ESI): 538.1 [M-1].

¹H NMR (500 MHz, CD₃OD): δ 8.23-8.20 (m, 1H), 7.77-7.74 (m, 1H), 7.02-7.00 (m, 1H), 6.79-6.59 (m, 1H), 6.27-6.17 (m, 1H), 5.72-5.66 (m, 1H), 3.74-3.62 (m, 4H), 2.98-2.95 (m, 2H), 2.89-2.83 (m, 3H), 2.61 (s, 2H), 2.11-2.05 (m, 3H), 1.12-1.05 (m, 4H).

Using step 3 to step 8 of the synthetic route in Example **6,** the title compound **26-2** (6 mg, yield: 2.5%) was obtained by replacing the starting material compound **6c** with compound **26c-2.**

MS m/z (ESI): 540.1 [M+1].

¹H NMR (500 MHz, CD₃OD): δ 8.23-8.20 (m, 1H), 7.75-7.73 (m, 1H), 7.00-6.98 (m, 1H), 6.75-6.60 (m, 1H), 6.25-6.16 (m, 1H), 5.71-5.64 (m, 1H), 3.73-3.61 (m, 4H), 3.12-3.09 (m, 2H), 2.88-2.50 (m, 3H), 2.53-2.50 (m, 2H), 2.09-2.03 (m, 3H), 1.19-1.17 (m, 2H), 0.92-0.90 (m, 2H).

### Example 27

### N-(3-(6-amino-5-(2-(N-methacrylamido)ethoxy)pyrimidin-4-yl)-5-fluoro-2-methylphenyl)-3,3-dimethyl-2,3-dihydrobenzofuran-6-carboxamide 27

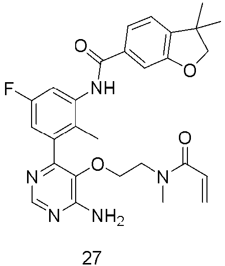

Using step 2 to step 7 of the synthetic route in Example 7, the title compound **27** (25 mg, yield: 24.3%) was obtained by replacing the starting material compound **7b** with methyl 4-bromo-3-hydroxylbenzoate.

MS m/z (ESI): 518.2 [M-1].

¹H NMR (500 MHz, DMSO-*d₆*): δ 9.88-9.64 (m, 1H), 8.21-8.18 (m, 1H), 7.61-7.53 (m, 1H), 7.43-7.35 (m, 3H), 7.11-7.00 (m, 3H), 6.75-6.69 (m, 1H), 6.14-6.11 (m, 1H), 5.64-5.59 (m, 1H), 4.31 (s, 2H), 3.59-3.46 (m, 4H), 2.82 (s, 1H), 2.53-2.50 (m, 2H), 1.98-1.93 (m, 3H), 1.34-1.24 (m, 6H).

### Example 28

### N-(3-(6-amino-5-(2-(N-methacrylamido)ethoxy-1,1-d₂)pyrimidin-4-yl)-5-fluoro-2-methylphenyl)-7-fluoro-3,3-dimethyl-2,3-dihydrobenzofuran-6-carboxamide 28

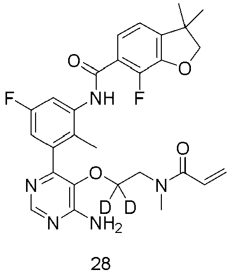

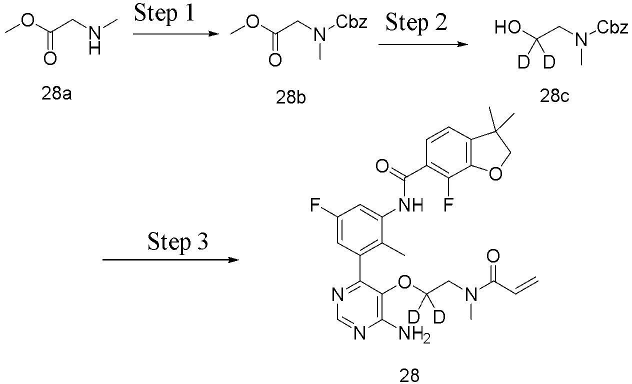

### Step 1

### Methyl N-((benzyloxy)carbonyl)-N-methylglycinate 28b

Methyl methylglycinate **28a** (1 g, 7.16 mmol, Bidepharm) was dissolved in ethyl acetate (20 mL, Sinopharm) and water (20 mL, Sinopharm), and sodium bicarbonate (1.81 g, 21.55 mmol, Sinopharm) was added. At 0°C, benzyl chloroformate (1.35 g, 7.91 mmol, Adamas) was added dropwise. The reaction was allowed to return to room temperature and stirred for 12 hours. 40 mL of water was added, and the mixture was extracted with ethyl acetate (40 mL×3). The organic phases were combined, washed with 40 mL of saturated sodium chloride solution, dried over anhydrous sodium sulfate, filtered and concentrated under reduced pressure. The residue was purified by column chromatography with eluent system B to obtain the title compound **28b** (1.28 g, yield: 75.3%).

MS m/z (ESI): 238.2 [M+1].

### Step 2

### Benzyl (2-hydroxyethyl-2,2-d₂)(methyl)carbamate 28c

Compound **28b** (1.28 g, 5.44 mmol) was dissolved in tetrahydrofuran (2.5 mL, Sinopharm) and deuterated methanol (1.25 mL, Sinopharm). Under a nitrogen atmosphere and at 0°C, sodium borodeuteride (232 mg, 5.54 mmol, Adamas) was added. The reaction was allowed to return to room temperature and stirred for 2 hours. Then, the reaction was cooled to 0°C and quenched by dropwise adding deuterium oxide. After the addition, the mixture was stirred at 0°C for 0.5 hours. The reaction was allowed to return to room temperature, and 20 mL of water was added. The mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system B to obtain the title compound **28c** (900 mg, yield: 78.4%).

MS m/z (ESI): 212.1 [M+1].

### Step 3

### N-(3-(6-amino-5-(2-(N-methacrylamido)ethoxy-1,1-d₂)pyrimidin-4-yl)-5-fluoro-2-methylphenyl)-7-fluoro-3,3-dimethyl-2,3-dihydrobenzofuran-6-carboxamide 28

Using step 4 to step 7 of the synthetic route in Example **17,** the title compound **28** (51 mg, yield: 17.8%) was obtained by replacing the starting material compound **17d** with compound **28c.**

MS m/z (ESI): 540.5 [M+1].

¹H NMR (500 MHz, DMSO-*d₆*): δ 9.92-9.72 (m, 1H), 8.21-8.18 (m, 1H), 7.55-7.48 (m, 1H), 7.33-7.18 (m, 2H), 7.05-6.97 (m, 3H), 6.66-6.58 (m, 1H), 6.09-6.03 (m, 1H), 5.62-5.58 (m, 1H), 4.42 (s, 1H), 3.52-3.45 (m, 2H), 2.81 (s, 1H), 2.52-2.50 (m, 3H), 2.03-1.98 (m, 3H), 1.48-1.24 (m, 6H).

### Example 29

### N-(3-(6-amino-5-(2-(N-ethylacrylamido)ethoxy)pyrimidin-4-yl)-5-fluoro-2-methylphenyl)-7-fluoro-3,3-dimethyl-2,3-dihydrobenzofuran-6-carboxamide 29

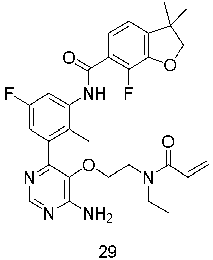

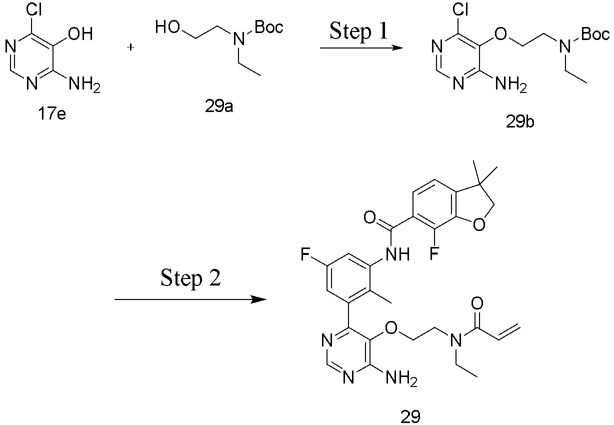

### Step 1

### Tert-butyl (2-((4-amino-6-chloropyrimidin-5-yl)oxy)ethyl)(ethyl)carbamate 29b

Tert-butyl ethyl(2-hydroxyethyl)carbamate **29a** (976 mg, 5.16 mmol, Leyan) was dissolved in tetrahydrofuran (20 mL, Energy Chemical). Compound **17e** (500 mg, 3.44 mmol, Bidepharm), triphenylphosphine (1.36 g, 5.19 mmol, Adamas) were added. The mixture was purged 3 times with nitrogen. At 0°C, diisopropyl azodicarboxylate (1.05 g, 5.19 mmol, Adamas) was added dropwise. The reaction was allowed to return to room temperature and stirred for 12 hours. 20 mL of water was added, and the mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system A to obtain the title compound **29b** (450 mg, yield: 41.4%).

MS m/z (ESI): 317.3 [M+1].

### Step 2

### N-(3-(6-amino-5-(2-(N-ethylacrylamido)ethoxy)pyrimidin-4-yl)-5-fluoro-2-methylphenyl)-7-fluoro-3,3-dimethyl-2,3-dihydrobenzofuran-6-carboxamide 29

Using step 5 to step 7 of the synthetic route in Example **7,** the title compound **29** (53 mg, yield: 15.5%) was obtained by replacing the starting material compound **3k** with compound **29b.**

MS m/z (ESI): 552.5 [M+1].

¹H NMR (500 MHz, DMSO-*d₆*): δ 9.92-9.79 (m, 1H), 8.21-8.18 (m, 1H), 7.55-7.48 (m, 1H), 7.30-7.18 (m, 2H), 7.05-6.97 (m, 2H), 6.62-6.49 (m, 2H), 6.09-6.03 (m, 1H), 5.62-5.58 (m, 1H), 4.41 (s, 1H), 3.59-3.48 (m, 4H), 3.30-3.09 (m, 2H), 2.03-1.98 (m, 3H), 1.46-1.24 (m, 7H), 0.96-0.85 (m, 3H).

### Example 30

### N-(3-(6-amino-5-(2-(N-(2-fluoroethyl)acrylamido)ethoxy)pyrimidin-4-yl)-5-fluoro-2-methylphenyl)-7-fluoro-3,3-dimethyl-2,3-dihydrobenzofuran-6-carboxamide 30

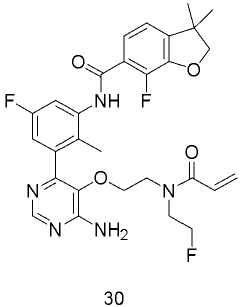

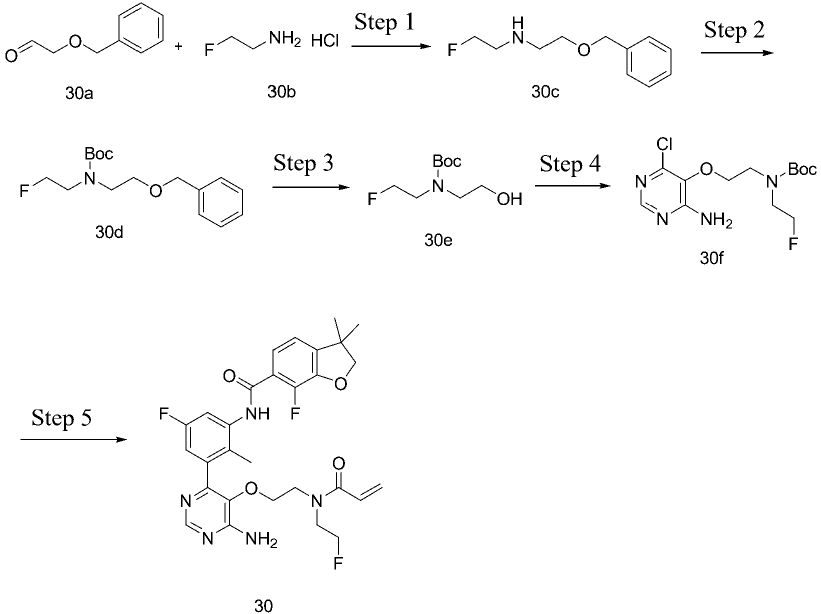

### Step 1

### 2-(Benzyloxy)-N-(2-fluoroethyl)ethan-1-amine 30e

2-(Benzyloxy)acetaldehyde **30a** (1 g, 6.66 mmol, Aikon) was dissolved in methanol (20 mL, Energy Chemical). 2-Fluoroethan-1-amine hydrochloride **30b** (730 mg, 7.33 mmol, Aikon) was added, and the mixture was stirred at room temperature for 0.5 hours. At 0°C, sodium triacetoxyborohydride (2.12 g, 10.00 mmol, Bidepharm) was added. The reaction was allowed to return to room temperature, and stirred for 4 hours. 5 mL of water was added, and the mixture was extracted with dichloromethane (20 mL×3). The organic phases were combined, washed with 5 mL of saturated sodium chloride solution, dried over anhydrous sodium sulfate and filtered, and the filtrate was concentrated under reduced pressure. The residue was purified by column chromatography with eluent system A to obtain the title compound **30c** (1.3 g, yield: 99.0%).

MS m/z (ESI): 198.1 [M+1].

### Step 2

### Tert-butyl (2-(benzyloxy)ethyl)(2-fluoroethyl)carbamate 30d

Compound **30c** (1.3 g, 6.59 mmol) was dissolved in dichloromethane (20 mL, Energy Chemical). Triethylamine (2.67 mg, 26.39 mmol, Sinopharm) and di-tert-butyl dicarbonate (1.59 g, 7.29 mmol, Adamas) were added, and the mixture was stirred at room temperature for 3 hours. The reaction solution was concentrated under reduced pressure. The residue was purified by column chromatography with eluent system B to obtain the title compound **30d** (1.24 g, yield: 63.3%).

MS m/z (ESI): 298.3 [M+1].

### Step 3

### Tert-butyl (2-fluoroethyl)(2-hydroxyethyl)carbamate 30e

Compound **30d** (1.3 g, 6.59 mmol) was dissolved in methanol (30 mL, Energy Chemical), and palladium on carbon (491 mg, 0.46 mmol, 10% purity, Bidepharm) was added. The mixture was purged 3 times with hydrogen, and stirred at room temperature for 12 hours. The reaction solution was filtered and concentrated under reduced pressure to obtain the crude title compound **30e** (950 mg, yield: 99.5%), which was directly used in the next reaction without purification.

MS m/z (ESI): 208.3 [M+1].

### Step 4

### Tert-butyl (2-((4-amino-6-chloropyrimidin-5-yl)oxy)ethyl)(2-fluoroethyl)carbamate 30f

Compound **30e** (800 mg, 3.86 mmol) was dissolved in tetrahydrofuran (15 mL, Energy Chemical). Compound **17e** (400 mg, 2.75 mmol, Bidepharm) and triphenylphosphine (1.45 g, 5.53 mmol, Adamas) were added. The mixture was purged 3 times with nitrogen. At 0°C, diisopropyl azodicarboxylate (1.12 g, 5.53 mmol, Adamas) was added dropwise. The reaction was allowed to return to room temperature, and stirred for 12 hours. 20 mL of water was added, and the mixture was extracted with ethyl acetate (20 mL×3). The organic phases were combined, washed with 20 mL of saturated sodium chloride solution, dried over anhydrous sodium sulfate, filtered and concentrated under reduced pressure. The residue was purified by column chromatography with eluent system A to obtain the title compound **30f** (300 mg, yield: 32.6%).

MS m/z (ESI): 335.3 [M+1]

### Step 5

### N-(3-(6-amino-5-(2-(N-(2-fluoroethyl)acrylamido)ethoxy)pyrimidin-4-yl)-5-fluoro-2-methylphenyl)-7-fluoro-3,3-dimethyl-2,3-dihydrobenzofuran-6-carboxamide 30

Using step 5 to step 7 of the synthetic route in Example **7,** the title compound **30** (69 mg, yield: 23.4%) was obtained by replacing the starting material compound **3k** with compound **30f.**

MS m/z (ESI): 570.5 [M+1].

¹H NMR (500 MHz, DMSO-*d₆*): δ 9.93-9.78 (m, 1H), 8.21-8.18 (m, 1H), 7.54-7.46 (m, 1H), 7.30-7.18 (m, 2H), 7.05-6.95 (m, 3H), 6.66-6.52 (m, 1H), 6.10-6.05 (m, 1H), 5.62-5.58 (m, 1H), 4.46-4.32 (m, 3H), 3.62-3.49 (m, 4H), 3.30-3.29 (m, 3H), 2.03-1.98 (m, 3H), 1.35-1.24 (m, 6H).

### Example 31

### (S)-N-(3-(5-((1-acryloylpyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide 31

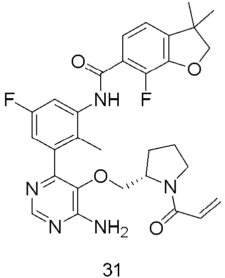

Using step 5 to step 7 of the synthetic route in Example **7,** the title compound **31** (70 mg, yield: 23.7%) was obtained by replacing the starting material compound **3k** with tert-butyl (*S*)-2-(((4-amino-6-chloropyrimidin-5-yl)oxy)methyl)pyrrolidine-1-carboxylate (prepared according to the method disclosed in patent application WO 2015079417 A1, description, page 59, for intermediate INT24).

MS m/z (ESI): 564.2 [M+1].

¹H NMR (500 MHz, DMSO-*d₆*): δ 9.94-9.89 (m, 1H), 8.21-8.18 (m, 1H), 7.55-7.51 (m, 1H), 7.26-7.18 (m, 2H), 7.10-6.90 (m, 3H), 6.50-6.45 (m, 1H), 6.11-6.06 (m, 1H), 5.63-5.56 (m, 1H), 4.41 (s, 2H), 4.17-4.03 (m, 1H), 3.65-3.36 (m, 2H), 3.27-3.10 (m, 2H), 2.03 (s, 3H), 1.76-1.57 (m, 4H), 1.35 (s, 6H).

### Example 32

### N-(3-(5-(((2S,4R)-1-acryloyl-4-fluoropyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide 32

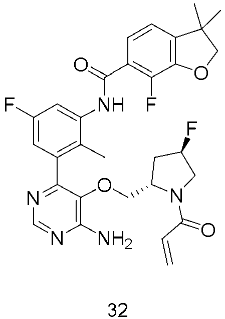

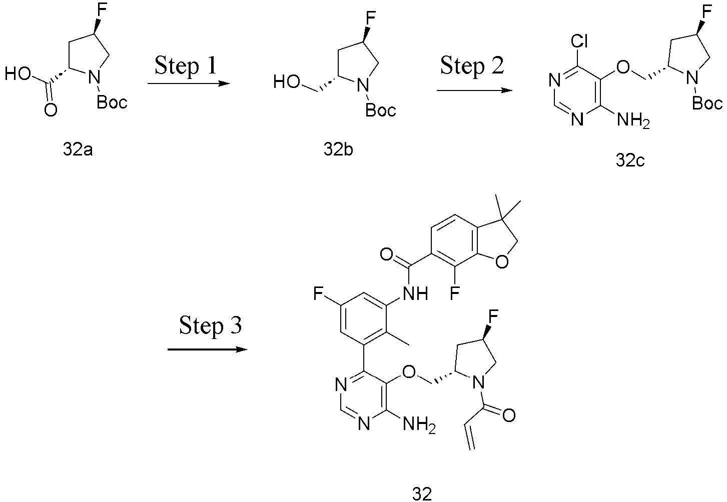

### Step 1

### Tert-butyl (2S,4R)-4-fluoro-2-(hydroxymethyl)pyrrolidine-1-carboxylate 32b

(2*S*,4*R*)-1-(tertbutoxycarbonyl)-4-fluoropyrrolidine-2-carboxylic acid **32a** (3 g, 12.86 mmol, Bidepharm) was dissolved in tetrahydrofuran (40 mL, Energy Chemical). Lithium aluminium hydride (976 mg, 25.71 mmol, Sinopharm) was added, and the mixture was stirred at room temperature for 2 hours. 20 mL of water was added, and the mixture was extracted with dichloromethane (20 mL×3). The organic phases were combined, washed with 20 mL of saturated sodium chloride solution, dried over anhydrous sodium sulfate and filtered, and the filtrate was concentrated under reduced pressure. The residue was purified by column chromatography with eluent system B to obtain the title compound **32b** (780 mg, yield: 27.7%).

### Step 2

### Tert-butyl (2S,4R)-2-(((4-amino-6-chloropyrimidin-5-yl)oxy)methyl)-4-fluoropyrrolidine-1-carboxylate 32c

Compound **32b** (780 mg, 3.56 mmol) was dissolved in toluene (20 mL, Sinopharm). Compound **17e** (517 mg, 3.55 mmol, Bidepharm) and (cyanomethylene)tri-n-butylphosphorane (1.71 g, 7.09 mmol, Adamas) were added. The mixture was purged 3 times with nitrogen, and stirred at 90°C for 2 hours. The reaction was allowed to return to room temperature and concentrated under reduced pressure. The residue was purified by column chromatography with eluent system A to obtain the title compound **32c** (300 mg, yield: 24.3%),

MS m/z (ESI): 347.2 [M+1].

### Step 3

### N-(3-(5-(((2S,4R)-1-acryloyl-4-fluoropyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide 32

Using step 5 to step 7 of the synthetic route in Example **7,** the title compound **32** (61 mg, yield: 16.1%) was obtained by replacing the starting material compound **3k** with compound **32c.**

MS m/z (ESI): 582.5 [M+1].

¹H NMR (500 MHz, DMSO-*d₆*): δ 9.98-9.90 (m, 1H), 8.22-8.19 (m, 1H), 7.52-7.45 (m, 1H), 7.28-7.18 (m, 2H), 7.02-6.90 (m, 3H), 6.51-6.45 (m, 1H), 6.13-6.08 (m, 1H), 5.67-5.60 (m, 1H), 5.33-5.07 (m, 1H), 4.41 (s, 1H), 4.35-4.33 (m, 1H), 4.16-4.14 (m, 2H), 3.94-3.36 (m, 3H), 2.06-1.89 (m, 5H), 1.35-1.24 (m, 6H).

### Example 33

### N-(3-(5-(((2S,4R)-1-acryloyl-4-methoxypyrrolidin-2-yl)methoxy)-6-aminopyrimidin-4-yl)-5-fluoro-2-methylphenyl)-7-fluoro-3,3-dimethyl-2,3-dihydrobenzofuran-6-carboxamide 33

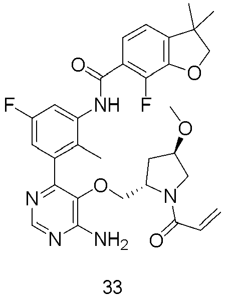

Using step 5 to step 7 of the synthetic route in Example **7,** the title compound **33** (70 mg, yield: 24.3%) was obtained by replacing the starting material compound **3k** with tert-butyl (2S,4R)-2-(((4-amino-6-chloropyrimidin-5-yl)oxy)methyl)-4-methoxypyrrolidine-1-carboxylate (prepared according to the method disclosed in patent application WO 2015079417 A1, description, page 64, for intermediate INT30).

MS m/z (ESI): 594.2 [M+1].

¹H NMR (500 MHz, DMSO-*d₆*): δ 9.96-9.90 (m, 1H), 8.21-8.18 (m, 1H), 7.55-7.48 (m, 1H), 7.25-7.18 (m, 2H), 7.10-6.90 (m, 3H), 6.51-6.46 (m, 1H), 6.12-6.06 (m, 1H), 5.61-5.57 (m, 1H), 4.41 (s, 2H), 4.17-4.04 (m, 1H), 3.83-3.40 (m, 4H), 3.15-3.10 (m, 3H), 2.01 (s, 3H), 1.88-1.74 (m, 3H), 1.37 (s, 6H).

### Biological evaluation

The present disclosure will be further described and explained below in conjunction with test examples, but these test examples are not intended to limit the scope of the present disclosure.

**Test example 1:** Inhibitory activity of compounds of the present disclosure against BTK
I. Experimental materials and instruments
   1. BTK recombinant human protein (Thermo, PV3363)
   2. HTRF KinEASE-TK kit (Cisbio, 62TK0PEC)
   3. 1 M MgCl₂ (Thermo, AM9530G)
   4. 1 M MnCl₂ (Sigma, M1787-100ML)
   5. Adenosine 5'-triphosphate (ATP) (New England Biolabs, P0756L)
   6. DTT 2M (Sangon Biotech, B645939)
   7. Ultrapure water (Invitrogen, 10977-015)
   8. OptiPlate-384, white opaque 384-well microplate (PerkinElmer, 6007290)
   9. 96-well round-bottom plate (Corning, 3788)
   10. Biochemical incubator (Shanghai Boxun Medical & Biological Instruments Co., Ltd., BSP-100)
   11. 15-mL centrifuge tube (Corning)
   12. Benchtop high-speed centrifuge (Changsha Yingtai Instrument Co., Ltd., TD4N)
   13. Multifunctional microplate reader (PerkinElmer, EnVision)
   14. Bravo liquid handling platform (Agilent Technologies, SGC120TH34702)
II. Experimental procedure

The HTRF KinEASE-TK kit is designed to detect changes in BTK enzyme activity based on two technologies, i.e., fluorescence resonance energy transfer and time-resolved fluorescence. The kit is operated in two stages: 1) enzyme reaction stage: the enzyme reaction is initiated by adding ATP, where the enzyme phosphorylates the substrate, and the compounds are then added to competitively bind to BTK against ATP, thereby inhibiting substrate phosphorylation; and 2) detection stage: after the enzyme reaction is complete, a detection reagent is added to terminate the reaction. Different groups on the phosphorylated substrate bind to the energy donor and energy acceptor, respectively, bringing the two into close proximity. During the instrumental readout, the energy donor is excited by an external light source, which enables resonance energy transfer to the proximal energy acceptor. The acceptor, in turn, becomes excited and emits light at a specific wavelength. Thus, changes in enzyme activity can be detected by measuring the signal values, and the inhibition rate of the compounds against the target enzyme can be calculated, thereby evaluating the inhibitory effect of the compounds on BTK enzyme activity.

Preparation of enzyme reaction buffer: 5× Kinase Buffer was diluted 5-fold with ultrapure water, and then the stock solutions of MgCl₂, MnCl₂ and DTT were added. The final composition of the buffer was as follows: 1× Kinase Buffer, 10 mM MgCl₂, 1 mM MnCl₂, and 1 mM DTT.

Preparation of 5 × BTK working solution: The BTK (5.17 µM) stock solution was removed from -20°C storage. Given that the BTK concentration in the enzyme reaction system was 2 nM and the concentration of the 5× working solution was 10 nM, the BTK stock solution was diluted 517-fold with the enzyme reaction buffer according to the volume required for the experiment. The diluted solution was then added to the 384-well plate at 2 µL per well. The plate was placed in a plate spinner and centrifuged at 2000 rpm for 1 minute to bring the liquid to the bottom of the wells.

Preparation of 5 × SEB working solution: The SEB (2500 nM) stock solution was removed from -20°C storage. Given that the SEB concentration in the enzyme reaction system was 50 nM and the concentration of the 5× working solution was 250 nM, the SEB stock solution was diluted 10-fold with the enzyme reaction buffer according to the volume required for the experiment. The diluted solution was then added to the 384-well plate at 2 µL per well. The plate was placed in a plate spinner and centrifuged at 2000 rpm for 1 minute to bring the liquid to the bottom of the wells.

The test compounds dissolved in 100% DMSO at 20 mM were diluted with 100% DMSO to a concentration of 200 µM, which represents the 200× concentration of the highest compound concentration point in the reaction system. The compounds at this concentration were then subjected to 10-point 3-fold serial dilution with 100% DMSO. The positive control wells and negative control wells were set with 100% DMSO. The serially diluted compounds and 100% DMSO were further diluted 40-fold in complete medium to achieve the 5× concentration, followed by thorough mixing. 2 µL of the 5× concentration solutions of the compound or DMSO were added to the 384-well microplate. The microplate was placed in a plate spinner and centrifuged at 2000 rpm for 1 minute to bring the liquid to the bottom of the wells. Subsequently, the microplate was incubated in a biochemical incubator at 25°C for 0.5 hours.

Preparation of 5× TK-Substrate-Biotin (substrate) working solution: The substrate (500 µM) stock solution was removed from -20°C storage. Given that the substrate concentration in the enzyme reaction system was 0.5 µM and the concentration of the 5× working solution was 2.5 µM, the substrate stock solution was diluted 200-fold with the enzyme reaction buffer according to the volume required for the experiment. The diluted solution was then added to the 384-well microplate at 2 µL per well. The microplate was placed in a plate spinner and centrifuged at 2000 rpm for 1 minute to bring the liquid to the bottom of the wells.

Preparation of 5× ATP working solution: The ATP (10 mM) stock solution was removed from -20°C storage. Given that the ATP concentration in the enzyme reaction system was 10 µM and the concentration of the 5× working solution was 50 µM, the ATP stock solution was diluted 200-fold with the enzyme reaction buffer according to the volume required for the experiment. The diluted solution was then added to the 384-well microplate at 2 µL per well. The microplate was placed in a plate spinner and centrifuged at 2000 rpm for 1 minute to bring the liquid to the bottom of the wells. The microplate was incubated in a biochemical incubator at 25°C for 1 hour.

Preparation of detection reagent: The Sa-XL665 (16.67 µM) stock solution and TK antibody-EU3+-cryptate (100X) stock solution were removed from -20°C storage. According to the volume required for the experiment, the Sa-XL665 stock solution was diluted 133.4-fold with the detection buffer, and the Sa-XL665 stock solution was diluted 200-fold with the detection buffer. The diluted solutions were added to the 384-well plate at 10 µL per well. The plate was then placed in a plate spinner and centrifuged at 2000 rpm for 1 minute to bring the liquid to the bottom of the wells. Subsequently, the microplate was incubated in a biochemical incubator at 25°C for 1 hour.

After incubation, the TRF signal was read using Envision. Ratio = (signal at 665 nm/signal at 620 nm)* 104. Inhibition rate = 100-100 × (ratio _{test well} - ratio _{negative well})/(ratio _{positive well} - ratio _{negative well}). Using Graphpad Prism software, an inhibition curve was plotted based on the compound concentrations and their corresponding inhibition rates. The curve was then fitted using 4PL, yielding the compound concentration at which the inhibition rate reached 50%, i.e., IC₅₀.

**Table 1. IC₅₀ values of inhibitory activity of compounds of the present disclosure against BTK**

| Example No. | IC₅₀ (nM) |
|---|---|
| 1 | 6.09 |
| 3 | 8.30 |
| 4 | 5.10 |
| 5 | 6.90 |
| 6 | 6.30 |
| 7 | 3.00 |
| 8 | 6.20 |
| 11 | 4.20 |
| 12 | 4.00 |
| 17 | 5.74 |
| 18 | 3.78 |
| 19 | 8.99 |
| 26-2 | 6.15 |
| 27 | 7.52 |
| 28 | 2.12 |
| 29 | 1.73 |
| 30 | 1.55 |
| 31 | 0.70 |
| 32 | 1.56 |
| 33 | 1.25 |

Conclusion: the compounds of the present disclosure exhibit significant inhibitory effects on BTK.

**Test example 2:** Inhibitory activity of compounds of the present disclosure against B cell activation in human whole blood
I. Experimental materials and instruments
   1. Human whole blood (internal recruitment)
   2. Goat F(ab')2 anti-human IgD-UNLB (SouthernBiotech, 2032-01)
   3. DPBS (1x) (Gibco, 14190-144)
   4. Recombinant human IL-4 (Peprotech, 200-04)
   5. Brilliant Violet 421^{™} anti-human CD19 antibody (Biolegend, 302234)
   6. APC anti-human CD69 antibody (Biolegend, 310910)
   7. APC mouse IgG1, κ isotype control antibody (Biolegend, 400120)
   8. eBioscience^{™} 10X RBC lysis buffer (multi-species) (Thermo, 004300-54)
   9. NEST 96-well deep-well plate (Wuxi NEST Biotechnology Co., Ltd., 503501)
   10. 96-well round-bottom plate (Corning, 3788)
   11. 96-well flat-bottom plate (Corning, 3599)
   12. Attune^{™} NxT acoustic focusing cytometer (ThermoFisher Scientific, 4.2.0)
   13. Cell incubator (Thermo, Forma steri-cycle i160)
II. Experimental procedure

B cells express the B cell receptor (BCR) complex on their surface. When B cells in human whole blood are induced to activate, they can trigger the downstream signalling pathways of the receptor and express the activation marker CD69. The expression of CD69 in activated B cells was detected by flow cytometry, and the IC₅₀ of the compounds were calculated to evaluate the effects of the compounds on BCR activation.

The test compounds dissolved in 100% DMSO at 20 mM were diluted with 100% DMSO to a concentration of 1 mM, which represents the 200× concentration of the highest compound concentration point in the reaction system. The compounds at this concentration were then subjected to 8-point 4-fold serial dilution with 100% DMSO. The positive control wells were set with 100% DMSO, whereas the negative control wells were set with Remiburtinib at a final concentration of 5 µM. The serially diluted compounds and 100% DMSO were further diluted 10-fold in PBS to achieve the 20× concentration, followed by thorough mixing.

After thorough mixing, the whole blood sample was transferred to a loading reservoir and then dispensed into a 96-well flat-bottom plate at 80 µl per well. The compound dilution was dispensed at 5 µl per well. The 11th column of each row was designated as the negative control well, with 5 µl of 100 µM positive control drug added to each well. After thorough mixing for 5 min, the plate was incubated in a 37°C incubator for 30 min. Human IgD antibody was diluted 1.67-fold with PBS to a concentration of 300 µg/mL, and 10 µl of the diluted solution was added to each well of the culture plate (final concentration: 30 µg/ml). Human IL-4 (stock solution at 50 µg/ml) was diluted 500-fold with PBS to a concentration of 100 ng/mL, and 5 µl of the diluted solution was added to each well of the culture plate (final concentration: 5 ng/ml). After thorough mixing for 5 min, the plate was cultured in a 37°C incubator with 5% CO₂ for 18 hours.

Flow cytometry detection: CD69-APC, CD19-BV421 and CD4-APC antibodies, and the isotype control antibodies for CD69-APC were removed from 4°C storage. Each antibody was added at 1 µl per well. After thorough mixing for 5 min, the plate was incubated for 30 min at 4°C (refrigerator) in the dark for staining. The 10X RBC lysis buffer was diluted in water to prepare 1X RBC lysis buffer. Aliquots of 1 ml of the lysis buffer were dispensed into each well of a 96-well deep-well plate in advance. The stained samples were transferred into the deep-well plate containing the lysis buffer for erythrocyte lysis. After completing the transfer of an entire plate, the plate was incubated in the dark for 15-20 min. Upon completion of lysis, the plate was centrifuged at 2000 rpm for 4 min. Each well was washed with 1 ml of PBS. Subsequently, the cells were resuspended in 200 µl of PBS, transferred to a 96-well plate, and washed twice more using the same procedure. The samples were resuspended in 200 µl of PBS per well and then subjected to flow cytometry detection.

Data analysis: based on the flow cytometry results, lymphocytes were first gated, followed by gating of CD19-positive B cells. With reference to isotype control, CD69-positive cells within the B cell gate were gated, and the percentage of CD69-positive cells was calculated. Inhibition rate = (positive control well - test well)/(positive control well - negative control well) * 100%. The IC₅₀ for each compound was determined by fitting the log [concentration] to the corresponding inhibition rate using the 4-parameter logistic model.

**Table 2. IC₅₀ values of inhibitory activity of compounds of the present disclosure against B cell activation in human whole blood**

| Example No. | IC₅₀ (nM) |
|---|---|
| 1 | 10-100 |
| 2 | 10-100 |
| 3 | 10-100 |
| 4 | 10-100 |
| 5 | 10-100 |
| 7 | 10-100 |
| 8 | 10-100 |
| 11 | 10-100 |
| 12 | 10-100 |
| 17 | 10-100 |
| 18 | 10-100 |
| 19 | 10-100 |
| 20 | 10-100 |
| 27 | 10-100 |
| 28 | 10-100 |
| 29 | 10-100 |
| 30 | 10-100 |
| 31 | 10-100 |
| 32 | 10-100 |

Conclusion: the compounds of the present disclosure exhibit inhibitory effects on B cell activation in human whole blood.

### Test example 3: hERG channel inhibition experiment

### 1. Experimental objective

To test the blocking effects of compounds of the present disclosure on hERG potassium currents using automated patch-clamp technique in cell lines stably expressing hERG potassium channels.

### 2. Experimental method

### 2.1 Experimental materials and instruments

### 2.1.1 Experimental materials:

| Reagent name | Supplier | Catalogue no. |
|---|---|---|
| External standard | Nanion | 083001 |
| External standard Ca 10 | Nanion | 083012 |
| Internal KF 110 | Nanion | 083007 |
| Foetal bovine serum (FBS) | Corning | 35-081-CV |
| TrypLE^{™} Express | Gibco | 12604021 |
| Puromycin dihydrochloride | Invitrogen | A11138-03 |
| DMEM-high glucose | Hyclone | SH30243.01 |

### 2.1.2 Experimental instruments:

| Instrument name | Supplier | Model |
|---|---|---|
| Patchliner 4 channel | Nanion | 2-03-03100-002 |
| Patchliner wash station | Nanion | 2-02-03201-005 |
| Patchliner cell bank | Nanion | 2-02-03105-000 |
| Elektrodenchloridierer Patchliner | Nanion | 3-02-03533-000 |

| | | |
|---|---|---|
| HEAK EPC10 patch-clamp amplifier | Nanion | 1-01-10012-000 |
| Vortex mixing oscillator | Corning | LSE |
| Electrode Set Patchliner 8 channel | Nanion | 6-08-1088 |
| NPC-16 Chip, Medium Resistance: 1.8-3 Mohm | Nanion | 071102 |

### 2.2 Procedures for Automated patch-clamp experiment

The HEK293-hERG stable transfected cell line was subcultured at a ratio of 1: 3 or 1 : 4 in DMEM (high glucose) containing 10% FBS and 1.5 µg/mL puromycin dihydrochloride. The automated patch-clamp experiment was performed within 48-72 hours of the subculture. On the day of the experiment, cells were digested using TrypLE^{™} Express, collected by centrifugation, and resuspended in standard external solution (external standard) to prepare a cell suspension. The cell suspension was placed on the cell bank of the Patchliner instrument. Individual cells were adsorbed onto the micropores of the NPC-16 chip using a negative pressure controller, followed by stepwise addition of internal solution (Internal KF 110) and sealing solution (External standard Ca 10) onto the chip according to the protocol. Whole-cell patch-clamp technique was used to record hERG currents. The cells were clamped at a voltage of -100 mV, then stepped to -40 mV for 1 second, followed by depolarisation to 40 mV for 1 second, and finally repolarised to -40 mV for 1 second to elicit hERG tail currents. After current stabilisation, the test compounds were perfused starting from the lowest concentration. Each concentration was perfused for 100 s, and perfusion of the next concentration was initiated only after the currents stabilised again. During the experiment, the experimental conditions of the Patchliner instrument were controlled and experimental data were collected using PatchControl HT (Nanion) and PatchMaster (Nanion) software. Subsequently, the data were analysed with Igor Pro (Nanion) software. A concentration-response curve was plotted based on the inhibition rate of each compound concentration relative to the initial current, and the IC₅₀ values of the compounds for inhibiting hERG potassium currents were calculated.

### 2.3 Experimental results

The blocking effects of compounds of the present disclosure on hERG potassium currents were expressed as IC₅₀ values, as shown in Table 3.

The positive control compound Remibrutinib (with reference to WO 2015079417 A1, Example 6) has the following structure:

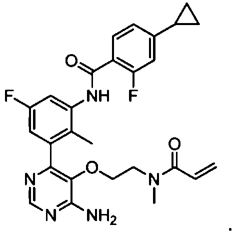

**Table 3. IC₅₀ values of blocking effects of compounds of the present disclosure on hERG potassium currents.**

| Example No. | IC₅₀ (µM) |
|---|---|
| 3 | >12.27 |
| 4 | 15.5 |
| 7 | 17.0 |
| 8 | 10.0 |
| 26-1 | 14.0 |
| 27 | 12.0 |
| 28 | 14.0 |
| 29 | 16.0 |
| 30 | 20.0 |
| 32 | 12.0 |
| Remibrutinib | 1.75 |

Conclusion: compared with the positive control compound Remibrutinib, the compounds of the present disclosure exhibit weaker inhibitory effects on hERG, which can reduce the side effects caused by hERG inhibition.

### Test example 4: Efficacy evaluation

### 1. Experimental objective

To evaluate the efficacy of compound 3 in a mouse RPA model.

### 2. Experimental drugs

### Compound 3

The administration volume of the vehicle was 10 ml/kg, and that of compound 3 was 20 ml/kg. The vehicle consisted of 0.5% MC + 0.5% Tween 80 + 99% water.

### 3. Experimental methods and experimental materials

### 3.1 Laboratory animals and housing conditions

Thirty-three female C57BL/6N mice weighing 18-20 g were purchased from Vital River Laboratories. Animal production license No.: SCXK (Zhe) 2024-0001; animal qualification certificate No.: 20240229Abzz0619000122. The mice were housed in an SPF-grade animal facility under a 12/12-hour light/dark cycle, with a constant temperature of 23°C ± 1°C and a relative humidity of 50-60%. Food and water were provided ad libitum.

### 3.2 Animal grouping

After acclimatisation, the mice were grouped and administered as follows:

**Table 4. Animal grouping and dosing regimen for in vivo efficacy experiment**

| Grouping | Dosage (mpk) | Dosing frequency and administration route | Number of animals |
|---|---|---|---|
| Naive | - | - | 5 |
| Vehicle control | - | 0.5% MC+0.5% Tween 80 + 99% water, single dose, p.o | 7 |
| Compound 3 | 30 | Single dose, p.o | 7 |
| Compound 3 | 100 | Single dose, p.o | 7 |
| Compound 3 | 150 | Single dose, p.o | 7 |

| | | | |
|---|---|---|---|
| Notes: p.o. refers to oral administration. | | | |

### 3.3 Experimental methods:

After acclimatisation, the mice were shaved on the back one day prior to the experiment for skin preparation. On the day of the experiment, the mice were administered the vehicle control or compound 3 via oral gavage at administration volumes of 10 ml/kg and 20 ml/kg, respectively. Two hours post-administration, the mice were anaesthetised with isoflurane, and the thickness of the dorsal skin was measured using a vernier calliper. Upon completion of the measurement, 30 µg of mouse anti-OVA IgG was intradermally injected into the dorsal skin of the mice, while an equal volume of PBS was injected into the control group. Simultaneously, 200 µl of 2 mg/ml OVA antigen was intravenously injected. After 3 hours, the thickness of the skin swelling at the modelling site was measured.

### 3.4 Data statistics

Data of relevant indicators for animals in each group were expressed as mean ± standard deviation (Mean ± SD) and statistically analysed and plotted using GraphPad software.

### 4. Results

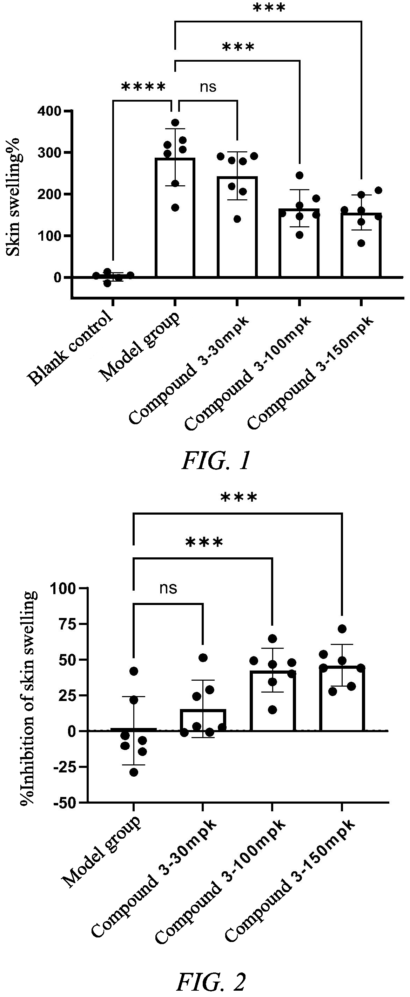

Obvious skin swelling was observed in the model group of this experiment. Based on the degree of skin swelling (Figure 1: degree of skin swelling in mice) and the percentage of drug inhibition (Figure 2: percentage inhibition of skin swelling by compound 3 in mice), compound 3 exhibited a dose-dependent inhibitory effect. Specifically, compared with the model group, compound 3 at a dose of 30 mpk showed no statistically significant difference; at doses of 100 mpk and 150 mpk, it significantly inhibited OVA-induced skin swelling in mice, with the efficacy tending to reach a plateau at the dose of 100 mpk.

### 5. Conclusion

In this experiment, an acute urticaria model in mice is induced using OVA antigens in combination with anti-OVA IgG antibodies. Five hours post-administration, compound 3 at doses of 100 mpk and 150 mpk can significantly inhibit OVA-induced skin swelling in mice, showing statistically significant differences compared with the model group.

## Claims

1. A compound as represented by general formula (I) or a pharmaceutically acceptable salt thereof: 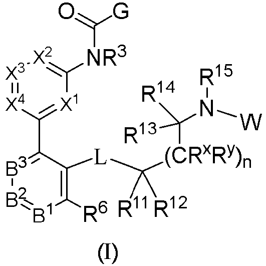 **characterised in that**:
G is selected from 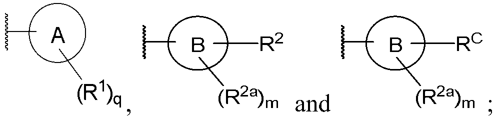
ring A is selected from polycyclic aryl, heteroaryl, cycloalkyl and heterocyclyl;
ring B is monocyclic aryl or monocyclic heteroaryl;
each R¹ and each R^{2a} are the same or different, and are each independently selected from a deuterium atom, halogen, alkyl, alkenyl, alkynyl, haloalkyl, deuterioalkyl, hydroxyalkyl, alkoxy, haloalkoxy, alkoxyalkyl, deuterioalkoxy, aminoalkyl, cyano, amino, hydroxyl, nitro, -(CR^{a}R^{b})ᵥOR¹⁷, -(CR^{a}R^{b})ᵥNR¹⁸R¹⁹, - C(O)R¹⁷, -C(O)OR¹⁷, -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl, - (CR^{a}R^{b})ᵥ heteroaryl, oxo, =S and =CR^{p}R^{q}, wherein the alkyl, alkenyl, alkynyl, alkoxy, cycloalkyl, heterocyclyl, aryl and heteroaryl are each independently and optionally substituted with one or more R⁰;
or two R¹ together with the ring atoms to which they are attached form cycloalkyl or heterocyclyl, wherein the cycloalkyl or heterocyclyl is optionally substituted with one or more R⁰;
R² is -OR^{A} or -alkylene-R^{B}, wherein the alkylene is optionally substituted with one or more R⁰;
R^{A} is selected from alkyl, cycloalkyl, heterocyclyl, aryl and heteroaryl, wherein the alkyl, cycloalkyl, heterocyclyl, aryl and heteroaryl are each independently and optionally substituted with one or more R⁰;
R^{B} is selected from cycloalkyl, heterocyclyl, aryl and heteroaryl, wherein the cycloalkyl, heterocyclyl, aryl and heteroaryl are each independently and optionally substituted with one or more R⁰;
R^{C} is selected from cycloalkyl, heterocyclyl, aryl and heteroaryl, wherein the cycloalkyl, heterocyclyl, aryl and heteroaryl are each independently substituted with one or more R^{d};
each R^{d} is the same or different, and is independently selected from a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, cyano, amino, hydroxyl, nitro, -(CR^{a}R^{b})ᵥOR¹⁷, -(CR^{a}R^{b})ᵥNR¹⁸R¹⁹, -C(O)R¹⁷, -C(O)OR¹⁷, - C(O)NR¹⁸R¹⁹, -OC(O)NR¹⁸R¹⁹, -OC(O)R¹⁷, -OC(O)OR¹⁷, -NR²⁰C(O)R¹⁷, - NR²⁰C(O)OR¹⁷, -SR¹⁷, -S(O)R¹⁷, -S(O)₂R¹⁷, -S(O)₂NR¹⁸R¹⁹, -NR²⁰S(O)₂R¹⁷, - (CR^{a}R^{b})ᵥ cycloalkyl, -(CR^{a}R^{b})ᵥ heterocyclyl, -(CR^{a}R^{b})ᵥ aryl, -(CR^{a}R^{b})ᵥ heteroaryl, oxo, =S and =CR^{p}R^{q}, wherein the alkyl, alkenyl, alkynyl, alkoxy, cycloalkyl, heterocyclyl, aryl and heteroaryl are each independently and optionally substituted with one or more R⁰;
R³ is selected from a hydrogen atom, alkyl, deuterioalkyl, alkenyl, alkynyl, haloalkyl, hydroxyalkyl, cycloalkyl, heterocyclyl, cycloalkylalkyl and heterocyclylalkyl;
X¹ is CR^{4a} or N;
X² is CR^{4b} or N;
X³ is CR^{4c} or N;
X⁴ is CR^{4d} or N;
B¹ is CR^{5a} or N;
B² is CR^{5b} or N;
B³ is CR^{5c} or N;
R^{4a}, R⁴⁶, R^{4c}, R^{4d}, R^{5a}, R^{5b} and R^{5c} are the same or different, and are each independently selected from a hydrogen atom, a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, cyano, amino, hydroxyl, nitro, - (CR^{a}R^{b})ᵥOR¹⁷, -(CR^{a}R^{b})ᵥNR¹⁸R¹⁹, -C(O)R¹⁷, -C(O)OR¹⁷, -C(O)NR¹⁸R¹⁹, - OC(O)NR¹⁸R¹⁹, -OC(O)R¹⁷, -OC(O)OR¹⁷, -NR²⁰C(O)R¹⁷, -NR²⁰C(O)OR¹⁷, -SR¹⁷, - S(O)R¹⁷, -S(O)₂R¹⁷, -S(O)₂NR¹⁸R¹⁹, -NR²⁰S(O)₂R¹⁷, -(CR^{a}R^{b})ᵥ cycloalkyl, - (CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or R^{4b} and R^{4c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{4c} and R^{4d} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5a} and R^{5b} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
or R^{5b} and R^{5c} together with the carbon atoms to which they are respectively attached form cycloalkyl, heterocyclyl, aryl or heteroaryl, wherein the cycloalkyl, heterocyclyl, aryl or heteroaryl is optionally substituted with one or more R⁰;
R⁶ is selected from a hydrogen atom, -NHR⁷ and -NHC(O)R⁷;
R⁷ is selected from a hydrogen atom, alkyl, haloalkyl, deuterioalkyl, hydroxyalkyl, alkoxyalkyl, hydroxyl, alkoxy, haloalkoxy, deuterioalkoxy, cycloalkyl, heterocyclyl, aryl, heteroaryl, cycloalkylalkyl and heterocyclylalkyl;
L is selected from CR⁸R⁹, C (O), O, NR¹⁰, S, S(O) and S(O)₂;
R⁸ and R⁹ are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, alkoxyalkyl, aminoalkyl, hydroxyl, cyano, amino, cycloalkyl and cycloalkylalkyl;
or R⁸ and R⁹ together with the carbon atoms to which they are attached form cycloalkyl or heterocyclyl, wherein the cycloalkyl or heterocyclyl is optionally substituted with one or more R⁰;
R¹⁰ is selected from a hydrogen atom, alkyl, alkenyl, alkynyl, haloalkyl, deuterioalkyl, hydroxyalkyl, alkoxyalkyl, hydroxyl, alkoxy, haloalkoxy, deuterioalkoxy, cycloalkyl, heterocyclyl, cycloalkylalkyl and heterocyclylalkyl;
R¹¹, R¹², R¹³, R¹⁴, R^{x} and R^{y} are the same or different, and are each independently selected from a hydrogen atom, a deuterium atom, halogen, alkyl, alkenyl, alkynyl, haloalkyl, deuterioalkyl, hydroxyalkyl, alkoxy, haloalkoxy, deuterioalkoxy, alkoxyalkyl, aminoalkyl, hydroxyl, cyano, amino, nitro, - (CR^{a}R^{b})ᵥOR¹⁷, -(CR^{a}R^{b})ᵥNR¹⁸R¹⁹, -(CR^{a}R^{b})ᵥ cycloalkyl, -(CR^{a}R^{b})ᵥ heterocyclyl, - (CR^{a}R^{b})ᵥ aryl and -(CR^{a}R^{b})ᵥ heteroaryl, wherein the alkyl, alkenyl, alkynyl, alkoxy, cycloalkyl, heterocyclyl, aryl and heteroaryl are each independently and optionally substituted with one or more R⁰;
or any two of R¹¹, R¹², R¹³, R¹⁴, R^{x} and R^{y}, together with the carbon atoms to which they are attached, form cycloalkyl or heterocyclyl, wherein the cycloalkyl or heterocyclyl is optionally substituted with one or more R⁰;
or R¹¹ and R¹² together form =O;
or R¹³ and R¹⁴ together form =O;
or R^{x} and R^{y} on the same carbon atom together form =O;
R¹⁵ is selected from a hydrogen atom, alkyl, alkoxy, alkenyl, alkynyl, haloalkyl, deuterioalkyl, hydroxyalkyl, cycloalkyl, heterocyclyl, aryl, heteroaryl, cycloalkylalkyl, heterocyclylalkyl, arylalkyl and heteroarylalkyl, wherein the alkyl, alkoxy, alkenyl, alkynyl, cycloalkyl, heterocyclyl, aryl, heteroaryl, cycloalkylalkyl, heterocyclylalkyl, arylalkyl and heteroarylalkyl are each independently and optionally substituted with one or more R⁰;
or any one of R¹¹, R¹², R¹³, R¹⁴, R^{x} and R^{y} together with R¹⁵ and the atoms to which they are respectively attached forms nitrogen-containing heterocyclyl, wherein the nitrogen-containing heterocyclyl is optionally substituted with one or more R⁰;
W is selected from -C(O)R¹⁶, -S(O)₂R¹⁶ and -CN;
R¹⁶ is selected from alkenyl, alkynyl and alkenyl oxide, wherein the alkenyl, alkynyl and alkenyl oxide are each independently and optionally substituted with one or more substituents selected from oxo, =S, =CR^{p}R^{q}, a deuterium atom, halogen, alkyl, haloalkyl, deuterioalkyl, hydroxyalkyl, alkoxy, haloalkoxy, deuterioalkoxy, alkoxyalkyl, aminoalkyl, hydroxyl, cyano, amino, nitro, -(CR^{a}R^{b})ᵥOR¹⁷, - (CR^{a}R^{b})ᵥNR¹⁸R¹⁹, -C(O)R¹⁷, -C(O)OR¹⁷, -C(O)NR¹⁸R¹⁹, -OC(O)R¹⁷, -OC(O)OR¹⁷, -NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and - (CR^{a}R^{b})ᵥ heteroaryl;
R⁰ at each occurrence is the same or different, and is independently selected from oxo, =S, =CR^{p}R^{q}, a deuterium atom, halogen, alkenyl, alkynyl, cyano, nitro, hydroxyl, amino, alkyl, haloalkyl, deuterioalkyl, hydroxyalkyl, alkoxy, haloalkoxy, deuterioalkoxy, alkoxyalkyl, aminoalkyl, -(CR^{a}R^{b})ᵥOR¹⁷, -(CR^{a}R^{b})ᵥNR¹⁸R¹⁹, - C(O)R¹⁷, -C(O)OR¹⁷- -C(O)NR¹⁸R¹⁹, -OC(O)NR¹⁸R¹⁹, -OC(O)R¹⁷, -OC(O)OR¹⁷, - NR²⁰C(O)R¹⁷, -NR²⁰C(O)OR¹⁷, -SR¹⁷, -S(O)R¹⁷, -S(O)₂R¹⁷, -S(O)₂NR¹⁸R¹⁹, - NR²⁰S(O)₂R¹⁷, -(CR^{a}R^{b})ᵥ cycloalkyl, -(CR^{a}R^{b})ᵥ heterocyclyl, -(CR^{a}R^{b})ᵥ aryl and - (CR^{a}R^{b})ᵥ heteroaryl;
R¹⁷, R¹⁸, R¹⁹ and R²⁰ at each occurrence are the same or different, and are each independently selected from a hydrogen atom, alkyl, alkenyl, alkynyl, -(CR^{a}R^{b})ᵥ cycloalkyl, -(CR^{a}R^{b})ᵥ heterocyclyl, -(CR^{a}R^{b})ᵥ aryl and -(CR^{a}R^{b})ᵥ heteroaryl, wherein the alkyl, alkenyl, alkynyl, cycloalkyl, heterocyclyl, aryl and heteroaryl are each independently and optionally substituted with one or more R⁰¹;
or R¹⁸ and R¹⁹ together with the nitrogen atom to which they are attached form nitrogen-containing heterocyclyl, wherein the nitrogen-containing heterocyclyl is optionally substituted with one or more R⁰¹;
R⁰¹ at each occurrence is the same or different, and is independently selected from oxo, =S, =CR^{p}R^{q}, a deuterium atom, halogen, hydroxyl, alkenyl, alkynyl, cyano, nitro, amino, -NH alkyl, -N (alkyl)₂, -C(O)O alkyl, -C(O)OH, -C(O)NH₂, - C(O)halogen, -C(O)alkyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
R^{a} and R^{b} at each occurrence are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, hydroxyl, cyano, amino, cycloalkyl and cycloalkylalkyl;
R^{p} and R^{q} at each occurrence are the same or different, and are each independently selected from a hydrogen atom, a deuterium atom, halogen, alkyl, haloalkyl, deuterioalkyl, hydroxyalkyl, alkoxy, haloalkoxy, deuterioalkoxy, hydroxyl, cyano, amino, cycloalkyl and cycloalkylalkyl;
q is an integer from 0 to 25;
m is 0, 1, 2, 3 or 4;
n is 0, 1, 2 or 3;
v is 0, 1, 2 or 3.

2. The compound as represented by general formula (I) or the pharmaceutically acceptable salt thereof according to claim 1, **characterised in that** the compound or the pharmaceutically acceptable salt thereof is a compound as represented by general formula (II) or a pharmaceutically acceptable salt thereof: 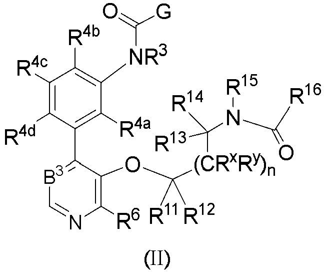 wherein:
G, R³, R^{4a}, R^{4b}, R^{4c}, R^{4d}, B³, R⁶, R¹¹, R¹², R¹³, R¹⁴, R¹⁵, R¹⁶, R^{x}, R^{y} and n are as defined in claim 1.

3. The compound as represented by general formula (I) or the pharmaceutically acceptable salt thereof according to claim 1 or 2, **characterised in that** G is selected from 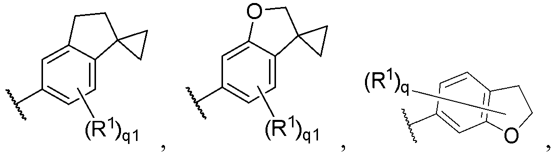 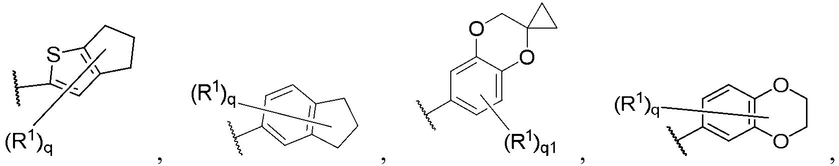 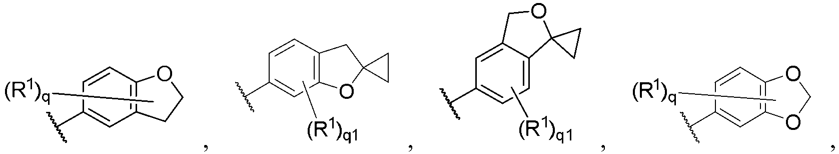 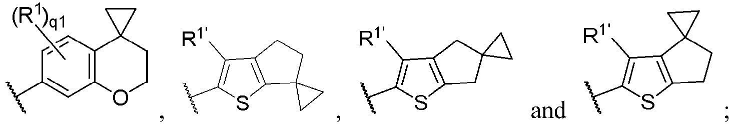 R¹' is a hydrogen atom or R¹; q1 is 0, 1, 2 or 3; q is 0, 1, 2, 3, 4, 5 or 6; R¹ is as defined in claim 1; preferably, G is selected from

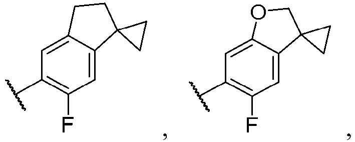

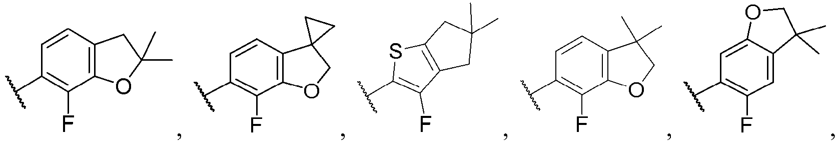

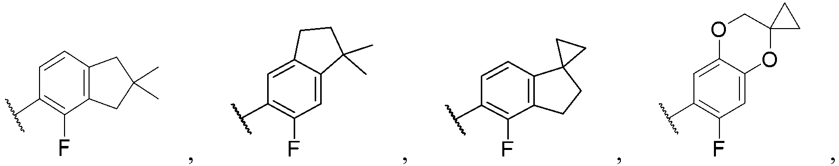

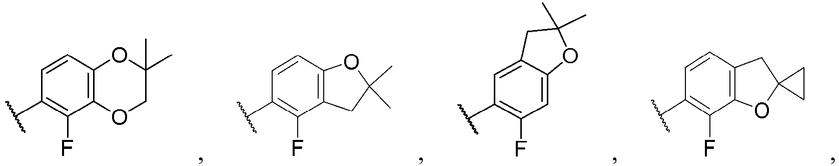

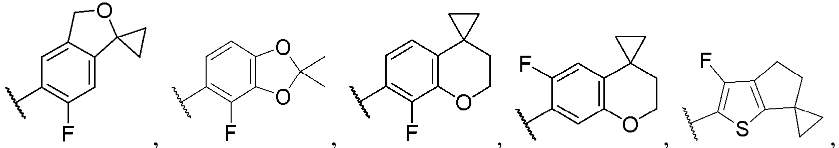

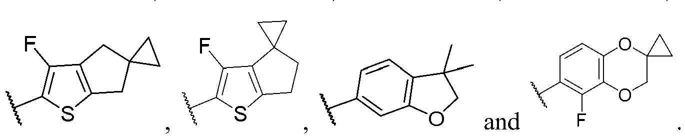

4. The compound as represented by general formula (I) or the pharmaceutically acceptable salt thereof according to claim 1 or 2, **characterised in that** the compound or the pharmaceutically acceptable salt thereof is a compound as represented by general formula (III) or a pharmaceutically acceptable salt thereof: 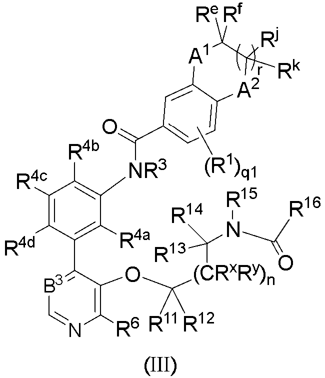 wherein:
A¹ and A² are the same or different, and are each independently selected from a bond, C (O), O, NR, S, S (O), S(O)₂ and (CR^{1b}R^{1c})ⱼ;
R¹⁶, R^{1c}, R^{e}, R^{f}, R^{j} and R^{k} are the same or different, and are each independently a hydrogen atom or R¹;
or R^{e} and R^{f} together with the carbon atoms to which they are attached form cycloalkyl or heterocyclyl, wherein the cycloalkyl or heterocyclyl is optionally substituted with 1, 2, 3 or 4 R¹;
or R^{j} and R^{k} together with the carbon atoms to which they are attached form cycloalkyl or heterocyclyl, wherein the cycloalkyl or heterocyclyl is optionally substituted with 1, 2, 3 or 4 R¹;
or R^{f} and R^{j} together with the carbon atoms to which they are attached form cycloalkyl or heterocyclyl, wherein the cycloalkyl or heterocyclyl is optionally substituted with 1, 2, 3 or 4 R¹;
or R^{1b} and R^{1b} together with the carbon atoms to which they are attached form cycloalkyl or heterocyclyl, wherein the cycloalkyl or heterocyclyl is optionally substituted with 1, 2, 3 or 4 R¹;
R is selected from a hydrogen atom, alkyl, alkenyl, alkynyl, haloalkyl, hydroxyalkyl, alkoxyalkyl, aminoalkyl, cycloalkyl, heterocyclyl, aryl and heteroaryl;
j is 0, 1, 2 or 3;
q1 is 0, 1, 2 or 3;
r is 0 or 1;
R¹, R³, R^{4a}, R^{4b}, R^{4c}, R^{4d}, B³, R⁶, R¹¹, R¹², R¹³, R¹⁴, R¹⁵, R¹⁶, R^{x}, R^{y} and n are as defined in claim 1.

5. The compound as represented by general formula (I) or the pharmaceutically acceptable salt thereof according to any one of claims 1 to 4, **characterised in that** each R¹ is the same or different, and is independently selected from halogen, C₁₋₆ alkyl, C₁₋₆ haloalkyl, C₁₋₆ alkoxy and C₁₋₆ hydroxyalkyl; preferably, each R¹ is the same or different, and is independently halogen; more preferably, R¹ is F.

6. The compound as represented by general formula (I) or the pharmaceutically acceptable salt thereof according to any one of claims 1 to 5, **characterised in that** R^{4a}, R^{4b}, R^{4c} and R^{4d} are the same or different, and are each independently selected from a hydrogen atom, halogen, C₁₋₆ alkyl, C₁₋₆ haloalkyl, C₁₋₆ alkoxy and C₁₋₆ hydroxyalkyl; preferably, R^{4a} is C₁₋₆ alkyl or C₁₋₆ hydroxyalkyl; R^{4b} is a hydrogen atom; R^{4c} is a hydrogen atom or halogen; R^{4d} is a hydrogen atom; more preferably, R^{4a} is methyl or hydroxymethyl; R^{4b} is a hydrogen atom; R^{4c} is a hydrogen atom or F; R^{4d} is a hydrogen atom.

7. The compound as represented by general formula (I) or the pharmaceutically acceptable salt thereof according to any one of claims 1 to 6, **characterised in that** B³ is CH or N; preferably, B³ is N; and/or R⁶ is amino.

8. The compound as represented by general formula (I) or the pharmaceutically acceptable salt thereof according to any one of claims 1 to 7, **characterised in that** R¹¹, R¹², R¹³ and R¹⁴ are hydrogen atoms; and/or n is 0; and/or R¹⁵ is selected from a hydrogen atom, C₁₋₆ alkyl, C₁₋₆ deuterioalkyl and 3- to 6-membered cycloalkyl; preferably, R¹⁵ is methyl or -CD₃.

9. The compound as represented by general formula (I) or the pharmaceutically acceptable salt thereof according to any one of claims 1 to 8, **characterised in that** R¹⁶ is C₂₋₆ alkenyl, wherein the C₂₋₆ alkenyl is optionally substituted with one or more substituents selected from halogen, C₁₋₆ alkyl, C₁₋₆ alkoxy and -NR¹⁸R¹⁹; R¹⁸ and R¹⁹ are the same or different, and are each independently a hydrogen atom or C₁₋₆ alkyl; preferably, R¹⁶ is -CH=CH₂.

10. The compound as represented by general formula (I) or the pharmaceutically acceptable salt thereof according to any one of claims 4 to 9, **characterised in that** A¹ and A² are the same or different, and are each independently selected from a bond, O and CH₂; preferably, A¹ is CH₂ or O, and A² is a bond.

11. The compound as represented by general formula (I) or the pharmaceutically acceptable salt thereof according to any one of claims 4 to 10, **characterised in that** R^{e}, R^{f}, R^{j} and R^{k} are the same or different, and are each independently selected from a hydrogen atom, halogen and C₁₋₆ alkyl; or R^{e} and R^{f} together with the carbon atoms to which they are attached form 3- to 6-membered cycloalkyl; or R^{j} and R^{k} together with the carbon atoms to which they are attached form 3- to 6-membered cycloalkyl; or R^{f} and R^{j} together with the carbon atoms to which they are attached form 4- to 6-membered cycloalkyl.

12. The compound as represented by general formula (I) or the pharmaceutically acceptable salt thereof according to any one of claims 1 to 11, **characterised in that** the compound is selected from any one of: 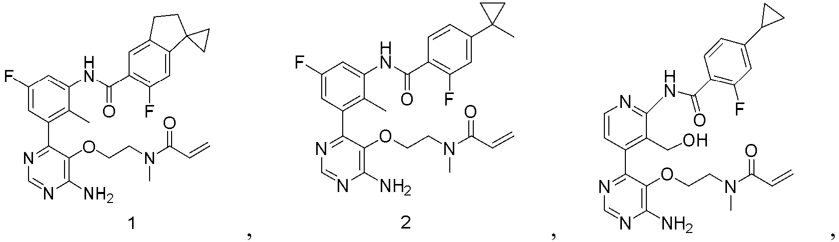 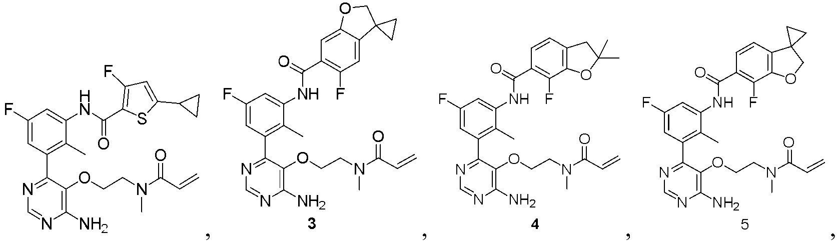 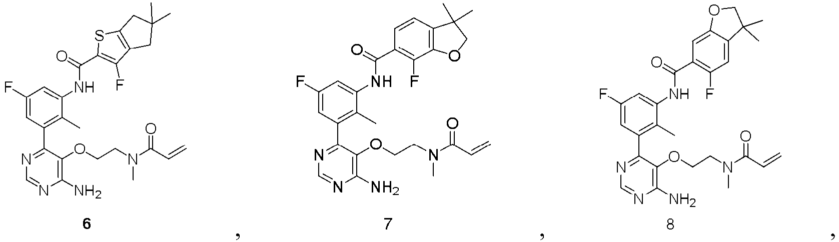 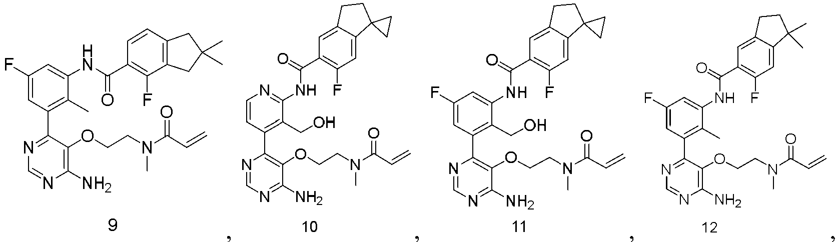 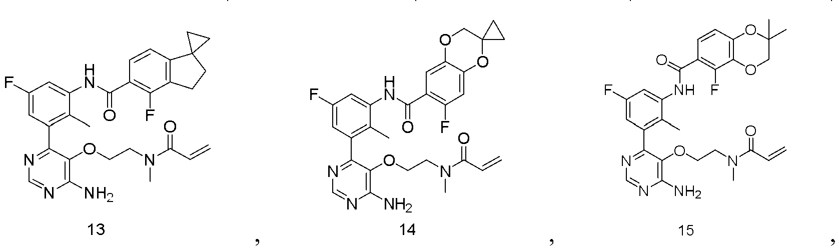 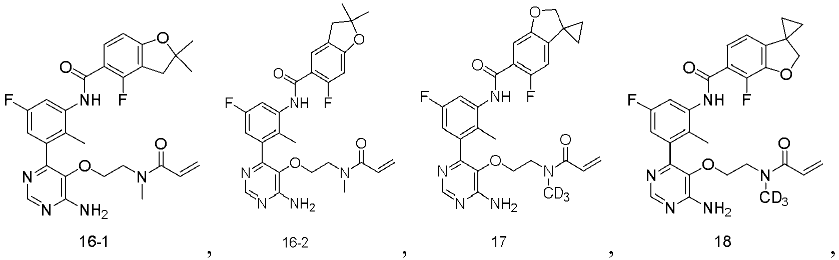 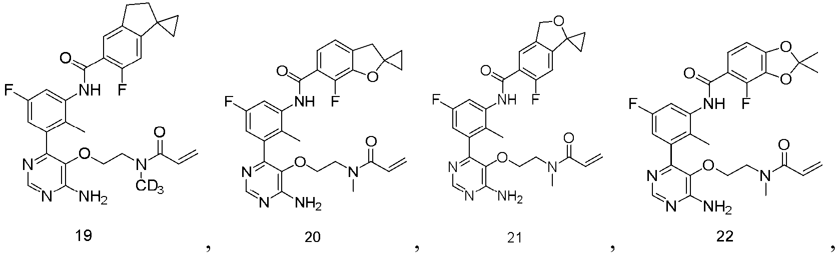 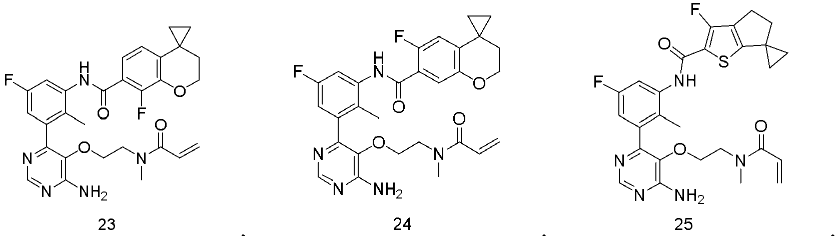 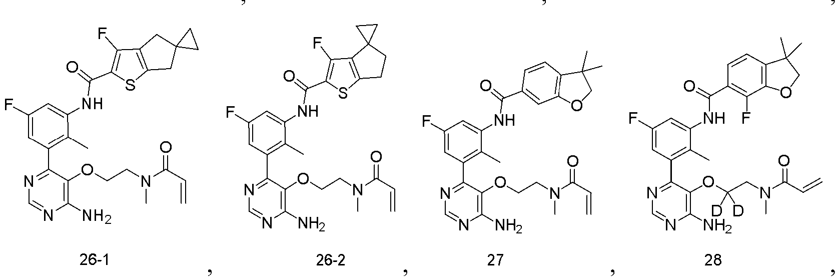 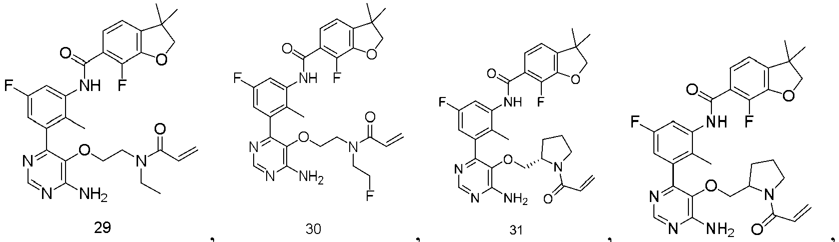 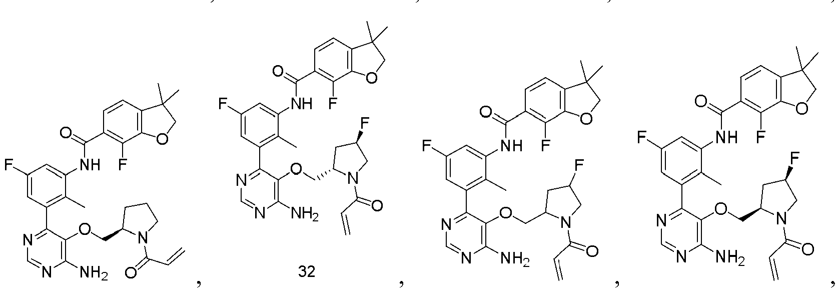 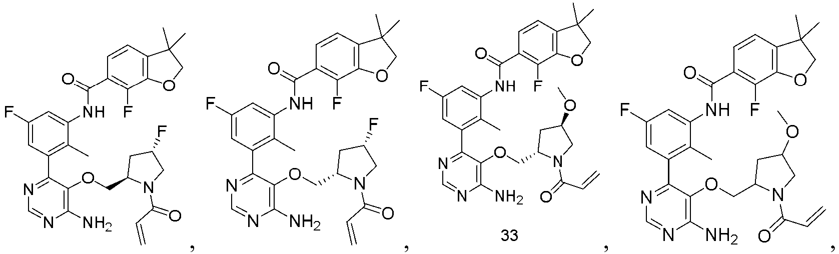 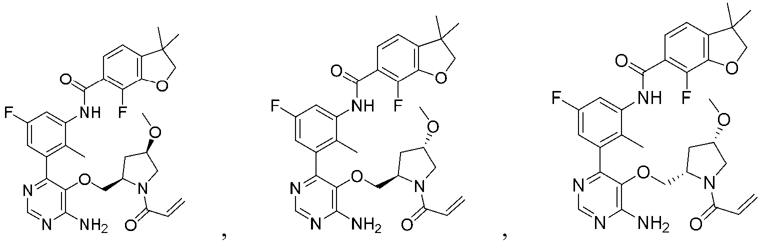 and

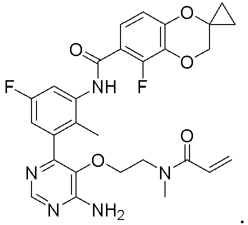

13. A compound as represented by general formula (IA) or a salt thereof: 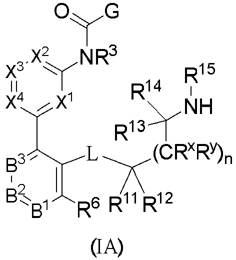 **characterised in that**:
G, R³, X¹, X², X³, X⁴, B¹, B², B³, R⁶, L, R¹¹, R¹², R¹³, R¹⁴, R¹⁵, R^{x}, R^{y} and n are as defined in claim 1.

14. A compound or a salt thereof, **characterised in that** the compound is selected from any of:

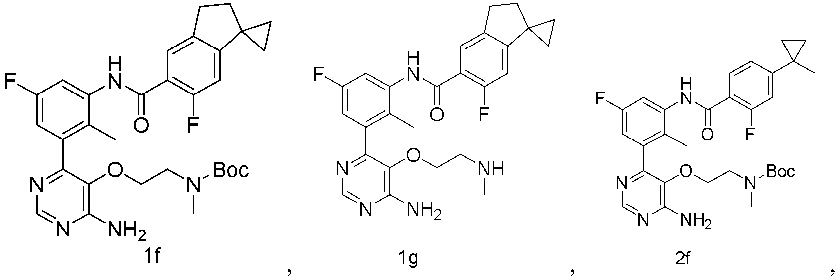

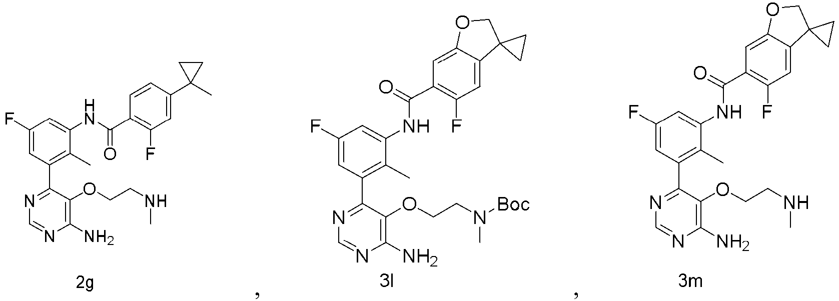

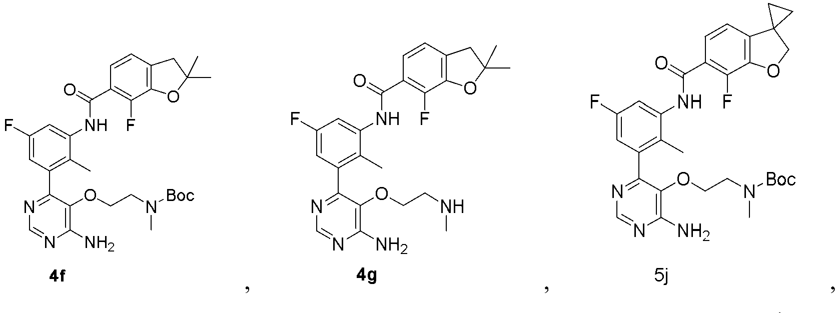

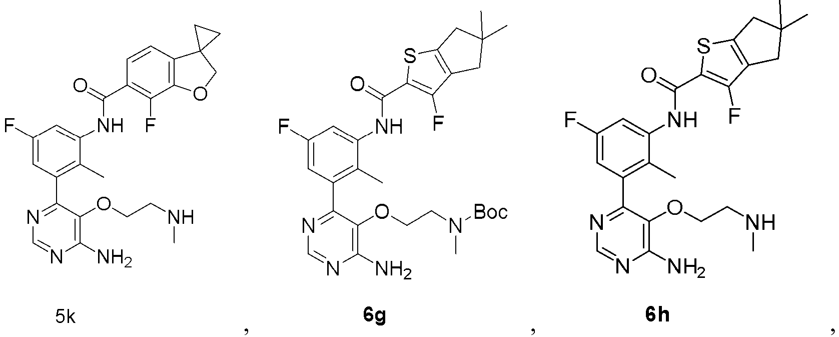

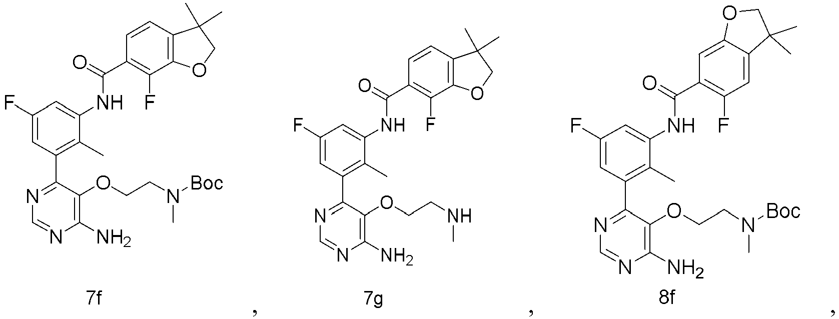

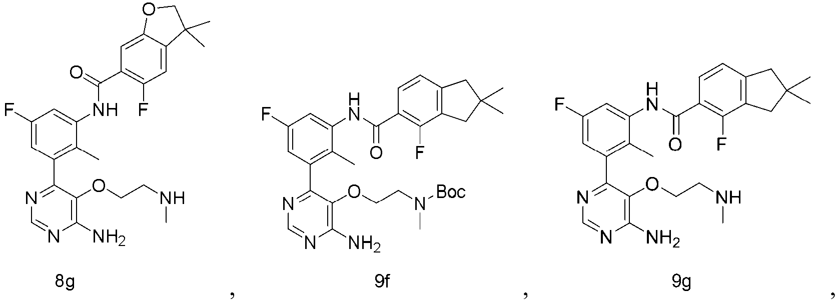

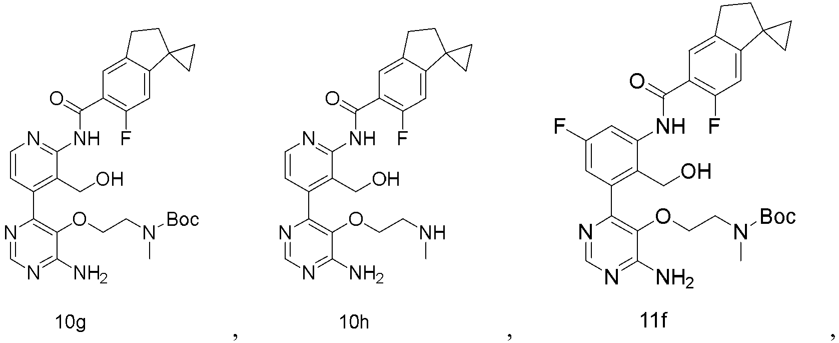

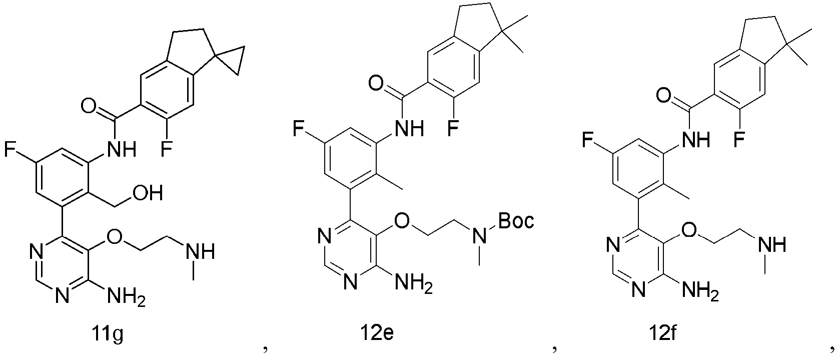

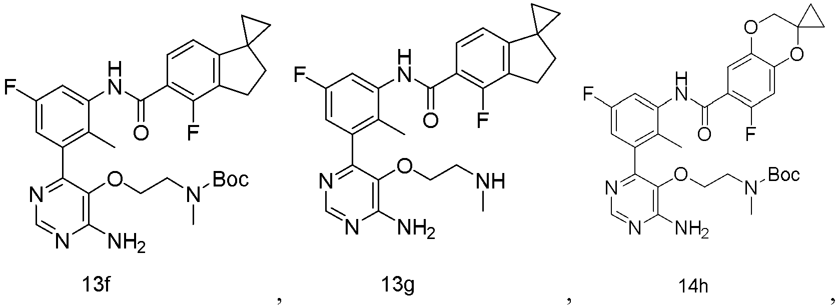

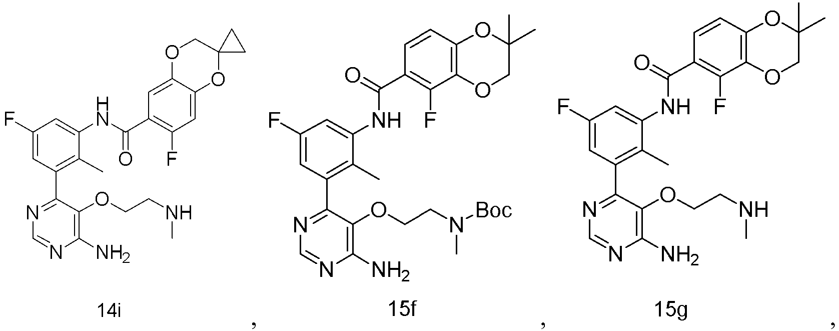

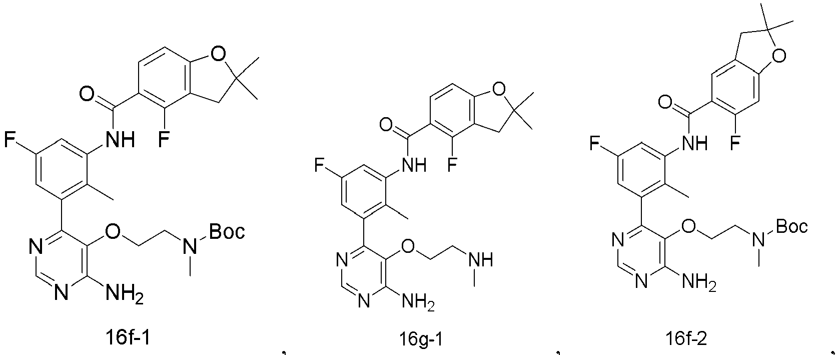

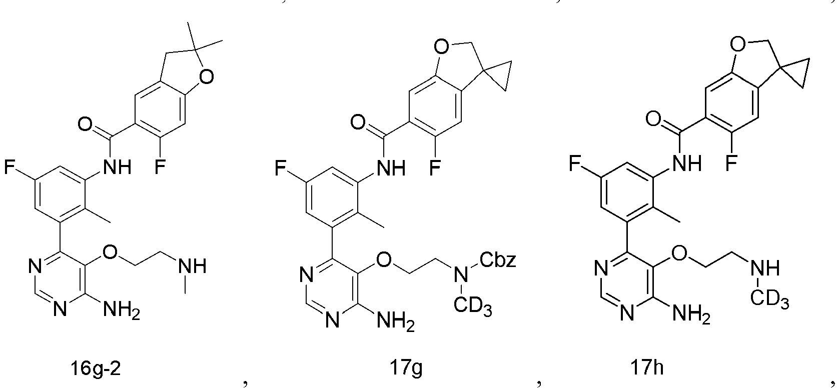

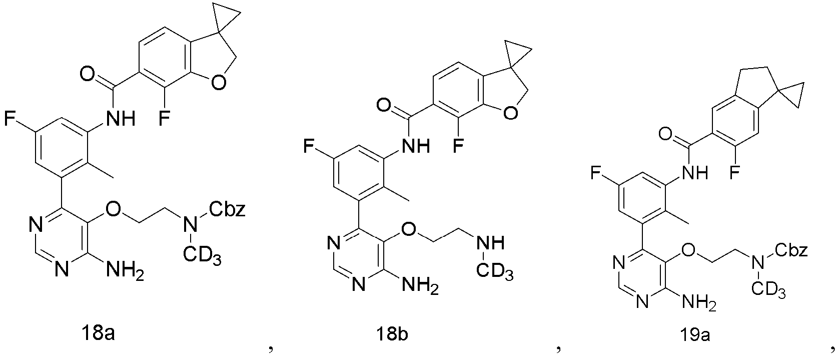

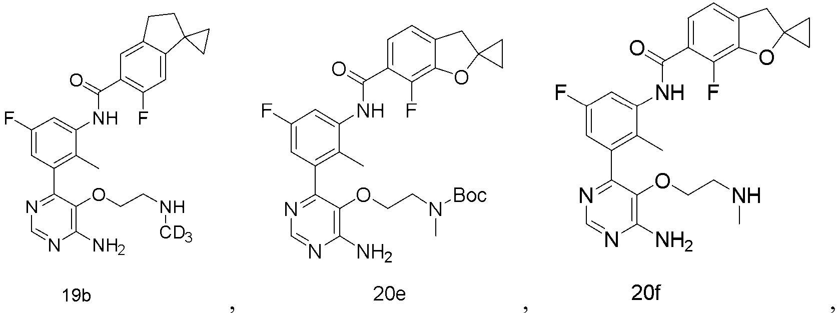

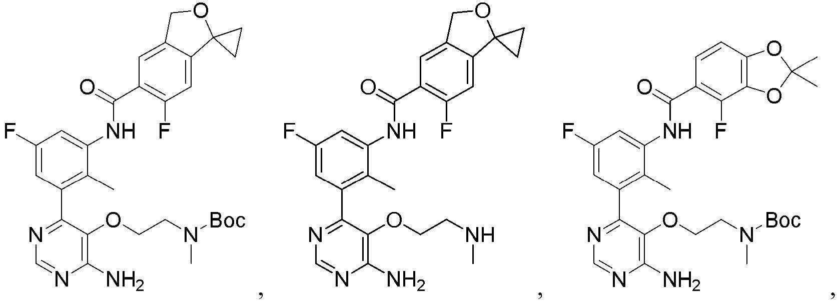

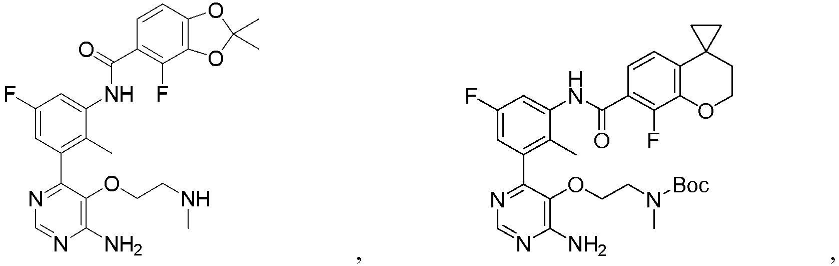

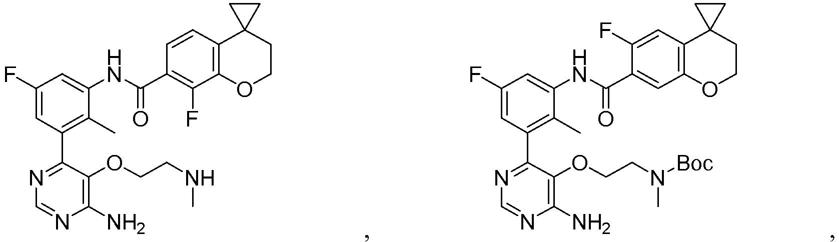

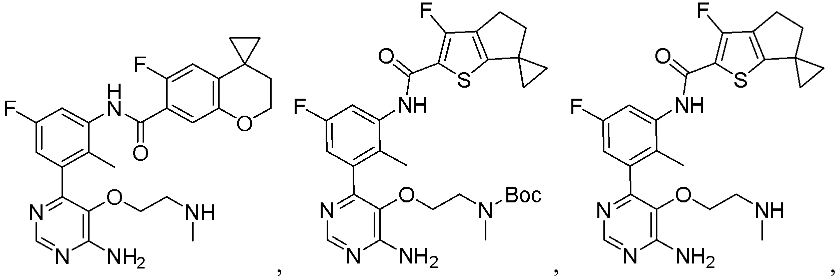

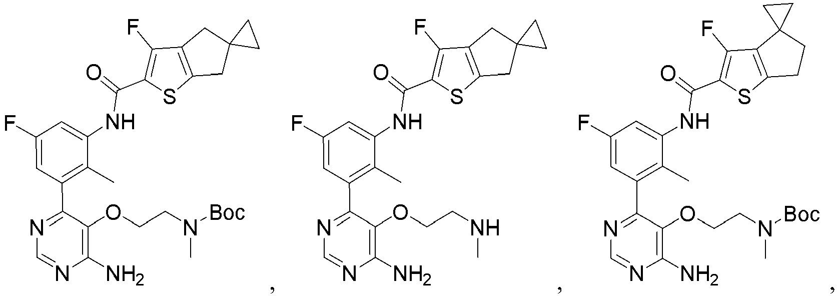

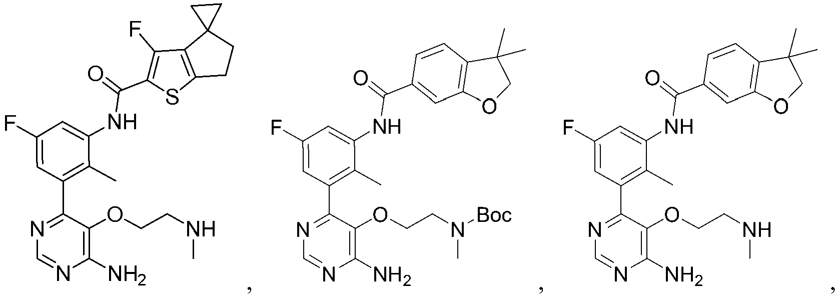

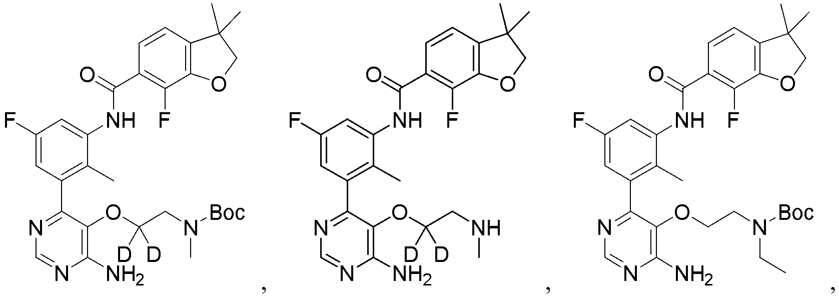

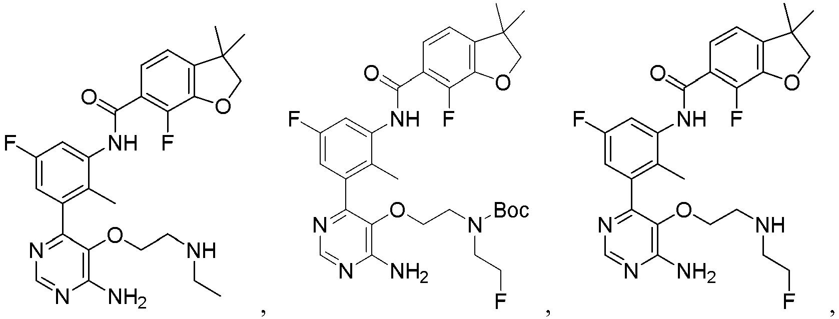

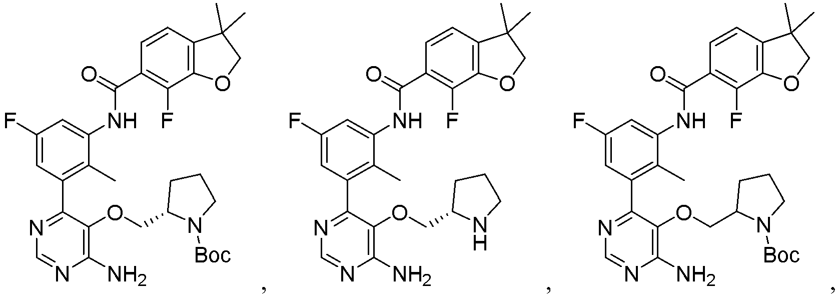

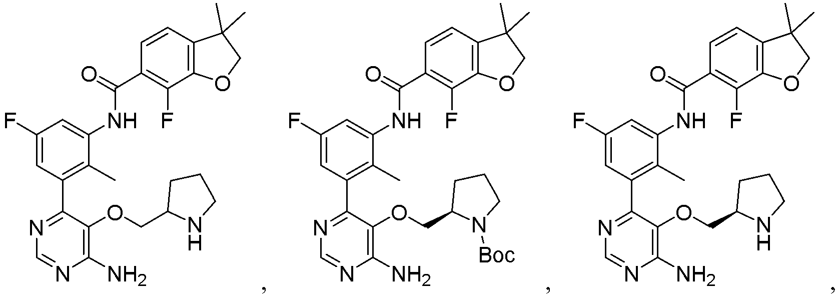

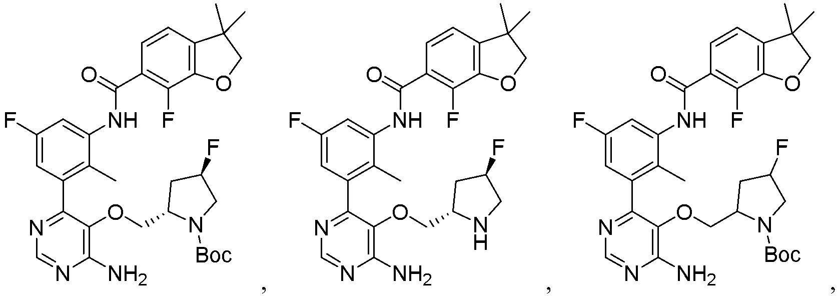

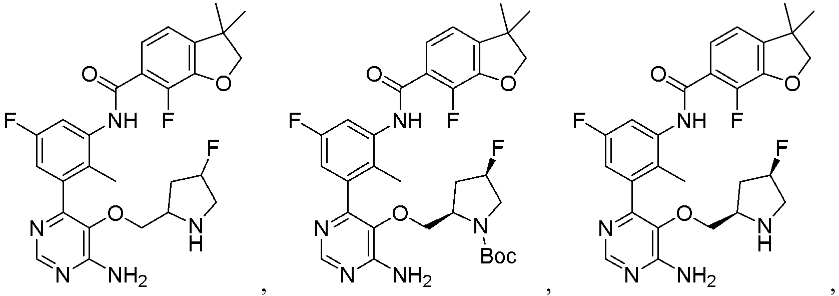

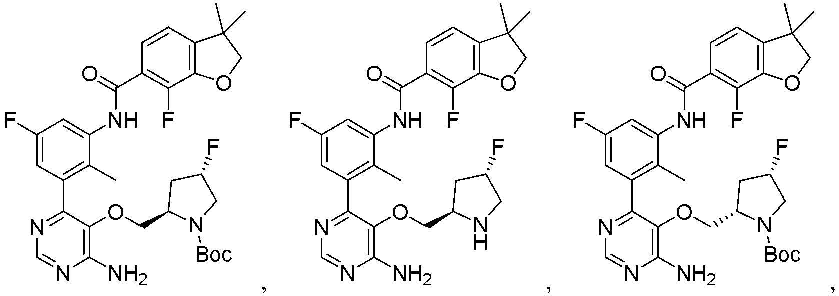

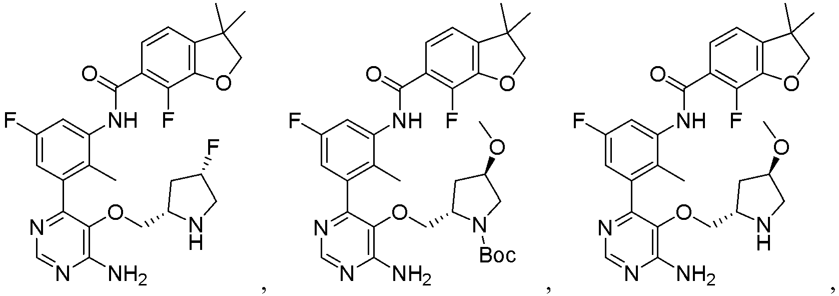

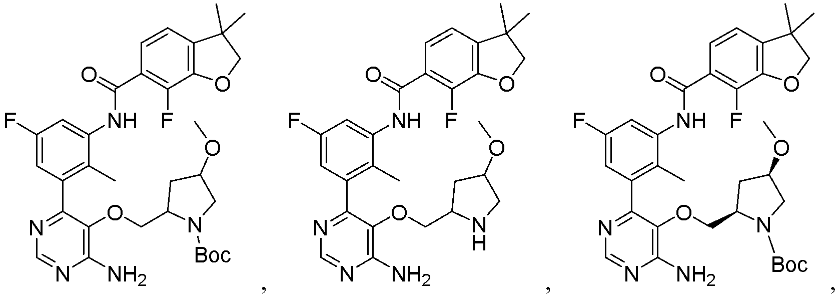

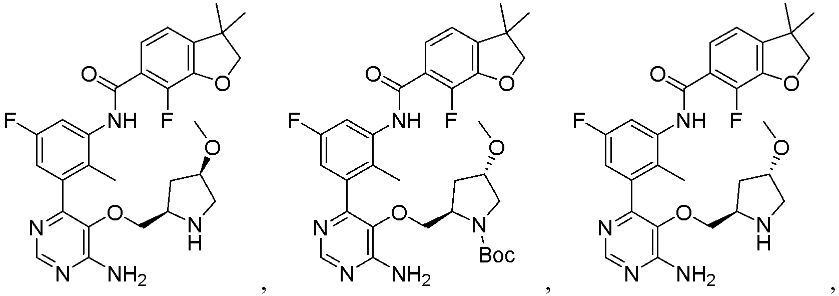

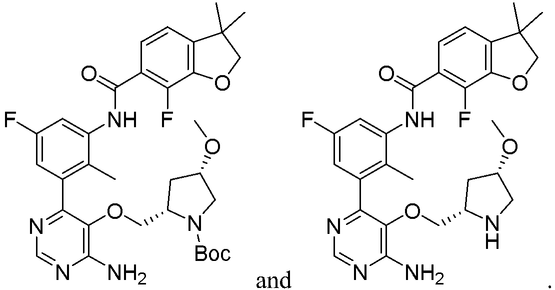

15. A method for preparing the compound as represented by general formula (I) or the pharmaceutically acceptable salt thereof according to claim 1, **characterised in that** the method comprises: 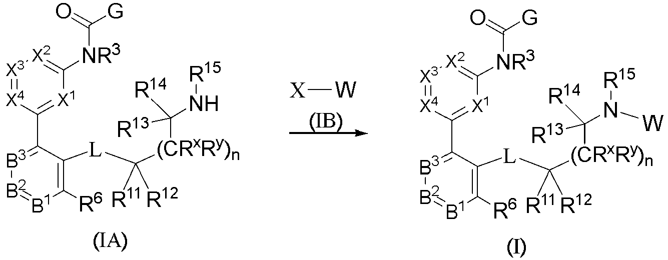
subjecting a compound as represented by general formula (IA) or a salt thereof, or a compound as represented by general formula (IB) or a salt thereof to a condensation reaction to obtain the compound as represented by general formula (I) or the pharmaceutically acceptable salt thereof,
wherein:
X is halogen, preferably Cl;
W is -C(O)R¹⁶ or -S(O)₂R¹⁶;
G, R³ , X¹, X², X³, X⁴, B¹, B², B³, R⁶, L, R¹¹, R¹², R¹³, R¹⁴, R¹⁵, R¹⁶, R^{x}, R^{y} and n are as defined in claim 1.

16. A pharmaceutical composition, **characterised in that** the pharmaceutical composition comprises the compound as represented by general formula (I) or the pharmaceutically acceptable salt thereof according to any one of claims 1 to 12, and one or more pharmaceutically acceptable carriers, diluents or excipients.

17. Use of the compound as represented by formula (I) or the pharmaceutically acceptable salt thereof according to any one of claims 1 to 12 or the pharmaceutical composition according to claim 16 in the preparation of a medicament for treating and/or preventing a BTK-mediated disease or condition.

18. Use of the compound as represented by formula (I) or the pharmaceutically acceptable salt thereof according to any one of claims 1 to 12 or the pharmaceutical composition according to claim 16 in the preparation of a medicament for treating and/or preventing a disease or condition, **characterised in that** the disease or condition is selected from cancer, autoimmune diseases, inflammatory diseases, allergic diseases, allergic reactions, respiratory diseases, cardiovascular diseases, viral infections, transplant rejection reactions, metabolic/endocrine disorders, and neurological disorders; preferably, the disease or condition is selected from autoimmune diseases, inflammatory diseases and cancer; more preferably, the disease or condition is selected from B-cell malignancies, multiple myeloma, leukaemia, malignant lymphoma, Waldenstrom macroglobulinemia, polycythaemia vera, essential thrombocythemia, myelofibrosis with myeloid metaplasia, Waldenstroem macroglobulinemia (Waldenstroem), bone cancer, bone metastasis, arthritis, multiple sclerosis, amyotrophic lateral sclerosis, osteoporosis, diabetes mellitus, arteriosclerosis, irritable bowel syndrome, inflammatory bowel disease, ulcerative colitis, Crohn's disease, Sjogren's syndrome, autoimmune thyroid diseases, Alzheimer's disease, lupus, systemic lupus erythematosus, psoriasis, urticaria, hepatic insufficiency, allergies, ankylosing spondylitis, dermatitis, pancreatitis, mastitis, meningitis, glomerulonephritis, Goodpasture syndrome, Hashimoto thyroiditis, keratitis, rhinitis, stomatitis, parotitis, pharyngitis, tonsillitis, tracheitis, bronchitis, pneumonia, myocarditis, gastritis, gastroenteritis, cholecystitis, appendicitis, Graves' disease, asthma, chronic obstructive pulmonary disease (COPD), thromboembolic diseases, myocardial infarction, angina pectoris, stroke, ischemic diseases, pulmonary embolism, gout, pemphigus vulgaris, idiopathic thrombocytopenic purpura, hidradenitis suppurativa, myasthenia gravis, autoimmune haemolytic anaemia, antineutrophil cytoplasmic antibody (ANCA)-associated vasculitis, cryoglobulinemia, thrombotic thrombocytopenic purpura, immune thrombocytopenia, complications caused by organ transplantation, xenotransplantation, antibody-mediated rejection (AMR), graft versus host disease, B cell-mediated hyperacute, and acute and chronic transplant rejection reactions.
